# EUROPEAN PATENT APPLICATION

(11) **EP 4 417 600 A1**
(43) Date of publication of application: **21.08.2024**
(21) Application number: 22880202.1
(22) Date of filing: 08.10.2022
(51) Int. Cl.: C07D 311/04, C07D 405/14, C07D 209/02, A61K 31/403, A61K 31/352, A61P 35/00

(54) **SHP2 INHIBITOR, PHARMACEUTICAL COMPOSITION COMPRISING SAME, AND USE THEREOF**

(30) Priority: 14.10.2021 CN 202111199960
(71) Applicant: Beijing Tide Pharmaceutical Co., Ltd., Beijing 100176 (CN)
(72) Inventor: LI, Wenming, Beijing 100176 (CN); LI, Xiaobo, Beijing 100176 (CN); LU, Peng, Beijing 100176 (CN); PIAO, Mingnan, Beijing 100176 (CN); WANG, Ning, Beijing 100176 (CN); YU, Guokun, Beijing 100176 (CN); LIU, Junrong, Beijing 100176 (CN); ZHANG, Yu, Beijing 100176 (CN); YU, Tiantian, Beijing 100176 (CN)
(74) Representative: Ipside
(86) International application number: PCT/CN2022/123863
(87) International publication number: WO 2023/061263

(57) **Abstract**

The present invention provides compounds, or pharmaceutically acceptable salts, esters, optical isomers, stereoisomers, polymorphs, solvates, N-oxides, isotopically labeled compounds, metabolites, chelates, complexes, clathrates, or prodrugs thereof, and pharmaceutical compositions containing the compounds of the invention. Also provides the application of the compounds in preparing medicaments for treating SHP2 phosphatase modulated diseases. The invention also provides methods for treating SHP2 phosphatase modulated diseases.

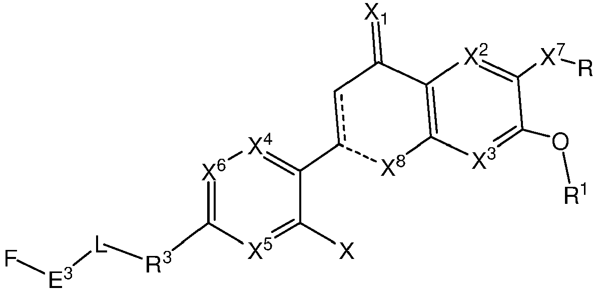

## Description

### Technical Field

The invention belongs to the field of medicinal chemistry, and particularly relates to an SHP2 inhibitor, a pharmaceutical composition containing the same and medical application thereof.

### Background of the Invention

SHP2 (The Src homology domain, The Src homology-2domain) is a non-receptor tyrosine phosphatase encoded by PTPN11 gene. It is ubiquitously expressed in various tissues and cell types and comprises a conserved tyrosine phosphatase domain, two N-terminal SH2 domains, and a C-terminal tail. The two SH2 domains determine the subcellular localization and functional regulation of SHP2. In the inactive state, the N-terminal SH2 domain binds to the PTP domain and inactivates it. When the SH2 domain binds to the receptor or to a specific tyrosine residue on the adaptor protein, the PTP domain is released. For example, stimulation by cytokines and growth factors results in exposure of catalytic sites, resulting in activation of SHP2. SHP2 plays an important role in the regulation of various signaling pathways in cellular biological processes, and is involved in the signaling pathways of various growth factors and interleukins. Within a single signaling pathway, SHP2 may play both an active (signal enhancement) and a passive (signal attenuation) role in the intracellular signaling process. SHP2 is believed to function by dephosphorylating its associated signaling molecule, thereby attenuating local signaling currents. However, the primary role of SHP2 activity in most signaling pathways (e.g., growth factors, interleukins, and extracellular matrix receptors) is to enhance signal transduction. For example, SHP2 is a positive regulator of the ERK/MAPK signaling pathway, which plays a key role in regulating cell proliferation and survival. (SHP2 phosphatase overview see, for example, K. S. Grossman et al, Adv. Cancer Res. 2010, 106, 53-89; and references cited therein).

In the basal state, SHP2 is normally self-inhibited by intramolecular interaction between its N-terminal SH2(N-SH2) domain and its catalytic (PTP) domain, blocking access to the catalytic site. Activation of the protein interacting with the SH2 domain induces a conformational change that reverses this inhibition and allows access of the substrate to the catalytic site. Mutations in the PTPN11 gene at N-SH2 or the PTP domain residues involved in the basic inhibition of SHP2 produce a more activated form of SHP2 protein that causes unregulated or increased SHP2 activity. SHP2 is widely expressed and is involved in multiple cell signaling processes, such as Ras-Erk, PI3K-Akt, Jak-Stat, Met, FGFR, EGFR, insulin receptor and NF-kB pathway, and plays an important role in cell proliferation, differentiation, cell cycle and migration.

Hyperactivation of SHP2 by germline or somatic mutations has been found in Noonan Syndrome, Leopard Syndrome, juvenile myelomonocytic leukemia, myelodysplastic Syndrome, B-cell acute lymphocytic leukemia, and acute myelogenous leukemia. In addition, activating mutations of PTPN11 are also found in solid tumors, such as lung cancer, colon cancer, melanoma, neuroblastoma, and liver cancer. Therefore, activated SHP2 or up-regulated SHP2 protein in human tumors or other diseases is a new therapeutic target, and development of SHP2 inhibitors is urgently needed.

### Summary of the Invention

The inventors of the present invention found that a compound shown in the following formula 1 has SHP2 inhibitory activity, so that an SHP2 small molecule inhibitor can be provided, and the activity of targeted degradation of SHP2 is embodied. Specifically, the invention provides a compound shown in formula I or pharmaceutically acceptable salt, ester, optical isomer, stereoisomer, polymorph, solvate, N-oxide, isotopically labeled compound, metabolite, chelate, complex, clathrate or prodrug thereof,

A compound of formula 1 or a pharmaceutically acceptable salt, ester, optical isomer, stereoisomer, polymorph, solvate, N-oxide, isotopically labeled compound, metabolite, chelate, complex, clathrate, or prodrug thereof,

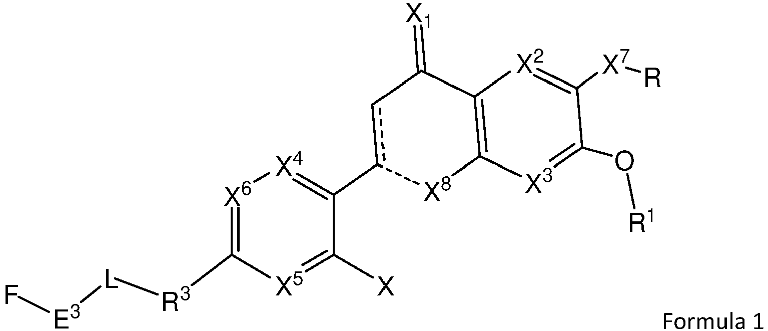

In formula 1,
"- - -" indicates the chemical bond is a double or single bond; "---" indicates that the chemical bond is a single bond or absent,
X is halogen, C1-6 alkyl, C2-6 alkenyl, C2-6 alkynyl, saturated or partially unsaturated C3-10 cyclic hydrocarbyl, preferably halogen, methyl, ethyl, isopropyl, cyclopropyl, more preferably halogen,
X¹ is NR⁴, O or S, preferably O or S, more preferably O,
X⁷ is a chemical bond or a divalent group formed by combining one or more selected from the group consisting of C1-6 alkylene, C2-6 alkenylene, C2-6 alkynylene, saturated or partially unsaturated C3-10 cyclic hydrocarbylene, -O-, -CO-, -C(= O)O-, -CONH-, -NHCO-, - NHCONH-, -NH-, -S-, -sulfinyl, and sulfonyl, preferably a chemical bond or a divalent group formed by combining one or more selected from the group consisting of C1-6 alkylene, -O-, - CO-, -CONH-, -NHCO-, -NHCONH-, and NHCONH-,
X², X³, X⁴, X⁵and X⁶ are each independently CR⁴ or N, preferably CR⁶,
When the chemical bond represented by "---" is a single bond, X⁸ is NR², O or S, preferably O or S, more preferably O,
When the chemical bond represented by "---" is absent, X⁸ is H, N(R²)₂, OR² or SR², preferably OR² or SR², more preferably OR²,
R¹ is H, C1-6 alkyl, C2-6 alkenyl, C2-6 alkynyl, saturated or partially unsaturated C3-10 cyclic hydrocarbyl, preferably C1-6 alkyl, more preferably one selected from methyl, ethyl, isopropyl and cyclopropyl,
R² is H, C1-6 alkyl, C2-6 alkenyl, C2-6 alkynyl, saturated or partially unsaturated C3-10 cyclic hydrocarbyl, preferably H, C1-6 alkyl, more preferably one selected from H, methyl and ethyl, particularly preferably H,
R³ is CR⁴R⁵, NR⁴, 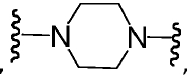 O or S, preferably NR⁴, 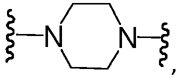 O or S, more preferably NR⁴ 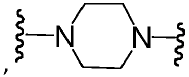 or O,
R⁴ and R⁵ are each independently H, C1-6 alkyl, C2-6 alkenyl, C2-6 alkynyl, saturated or partially unsaturated C3-10 cyclic hydrocarbyl, saturated or partially unsaturated 3-10 membered heterocyclyl, C6-10 aryl, 5-14 membered heteroaryl, or C6-12 aralkyl,
R⁶ is H, halogen, C1-6 alkyl, C2-6 alkenyl, C2-6 alkynyl, saturated or partially unsaturated C3-10 cyclic hydrocarbyl, saturated or partially unsaturated 3-10 membered heterocyclyl, C6-10 aryl, 5-14 membered heteroaryl, or C6-12 aralkyl,
R is H, halogen, NH₂, OH, SH, amino, substituted or unsubstituted C2-10 aliphatic hydrocarbon group, substituted or unsubstituted saturated or partially unsaturated 3-10 membered heterocyclyl, substituted or unsubstituted C6-10 aryl, or substituted or unsubstituted 5-14 membered heteroaryl; R is preferably halogen, OH, SH, haloalkyl, amino, saturated C3-10 cyclic hydrocarbyl, saturated or partially unsaturated 3-10 membered heterocyclyl, C1-6 alkyl substituted amino, 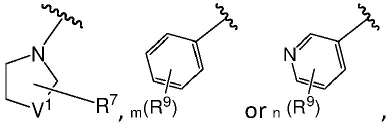
Wherein V¹ is one selected from C(R⁸)₂, C(R⁸)₂ -C(R⁸)₂, CR⁸=CR⁸, C=O, C(=O)C(R⁸)₂, C(R⁸)₂C(=O), C(=O)O, OC(=O), C(= O)NR, N=CR⁸, CR⁸=N, NR⁸-C(R⁸)₂ or C(R⁸)₂-NR⁸;
R⁷ is one selected from H, halogen, cyano, nitro, hydroxymethyl, hydroxyl, amide, C1-C6 alkyl, C1-C6 alkoxy, C1-C6 alkoxy-C1-C6 alkylene, or 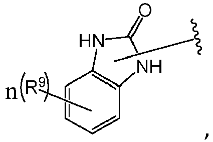
R⁹ are each independently selected from the group consisting of halogen, hydroxyl, oxo, amino, C1-6 alkyl-substituted amino, cyano, nitro, -Si(R⁸)₃, C1-6 alkyl, C1-6 alkoxy, saturated or partially unsaturated C3-6 cyclic hydrocarbyl, saturated or partially unsaturated 3-10 membered heterocyclyl, C6-10 aryl, 5-14 membered heteroaryl, C6-12 aralkyl, -C(=O)R⁸, -OC(=O)R⁸, -C(=O)OR⁸, -OR⁸, -SR⁸, -S(=O)R⁸, -S(=O)₂R⁸, -S(=O)₂N(R⁸)₂, -N(R⁸)₂, -C(=O)N(R⁸),-NR⁸-C(=O)R⁸, -NR⁸-C(=O)OR⁸, -NR⁸-S(=O)₂-R⁸, -NR⁸-C(=O)-N(R⁸)₂, -C1-6 alkylene-N(R⁸)₂, -C1-6 alkylene-OR⁸, -C1-6 alkenylene-OR⁸ and-O-C1-6 alkylene-N(R⁸)₂; m is an integer from 0 to 5, n is an integer from 0 to 4, when m is not 0, a plurality of R⁹ can be connected with each other to form a ring structure, and when n is not 0, a plurality of R⁹ can be connected with each other to form a ring structure;
R⁸ is H, C1-C6 alkyl, saturated or partially unsaturated C3-6 cyclic hydrocarbyl, saturated or partially unsaturated 3-10 membered heterocyclyl or C6-10 aryl, the expression of the ring structure with "-" represents the connecting site at any position on the ring structure that can form a bond;
L is a linking group which represents a linear or branched C3-C29 alkylene chain, wherein the linear or branched C3-C29 alkylene chain is optionally interrupted one or more times by one or more divalent groups selected from -O-, -CO-, -C(= O)O-, -CONH-, -NHCO-, - NHCONH-, -NH-, -NR⁸-, -C(R⁸)₂-, -S-, sulfinyl, sulfonyl, sulfinyloxy, sulfonyloxy, - aminosulfonylamino-, alkynylene, alkenylene, cycloalkylene, 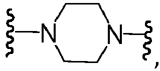 or any combination thereof,
E³ is the following group, 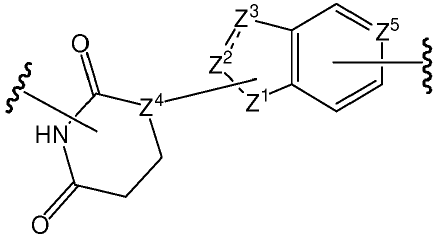
**-̅-̅-̅-̅-̅** represents a single or double bond, preferably a single bond;
Z¹ is O, S, NH, CH₂ or C=O, preferably CH₂ or C=O;
When -̅-̅-̅-̅-̅ is a double bond, Z² is N or CH, Z³ is N or CH;
When -̅-̅-̅-̅-̅ is a single bond, Z² is O, S, NH, CH₂ or C=O, preferably NH; Z³ is O, S, NH, CH₂ or C=O, preferably CH₂ or C=O;
Z⁴ is N or CH, Z⁴ is connected with any connectable position of Z¹, Z² and Z³;
Z⁵ is N or CH, preferably N;
E³ is further preferably 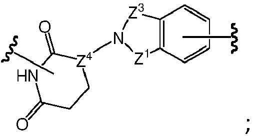
F is H or 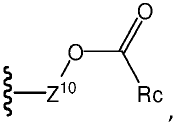
Z¹⁰ is selected from a chemical bond, C1-6 alkylene, C2-6 alkenylene, C2-6 alkynylene, saturated or partially unsaturated C3-10 cyclic hydrocarbylene, preferably a chemical bond, C1-6 alkylene;
Rc is halogen, cyano, nitro, hydroxyl, acylamino, C1-C6 alkyl, C1-C6 alkoxy, substituted C1-C6 alkyl, saturated or partially unsaturated C3-10 cyclic hydrocarbyl, saturated or partially unsaturated 3-10 membered heterocyclyl, C6-10 aryl, 5-14 membered heteroaryl or C6-12 aralkyl, preferably C1-C6 alkyl, saturated or partially unsaturated C3-10 cyclic hydrocarbyl, C6-10 aryl, C6-12 aralkyl,
In the above expression, 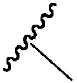 denotes the position of the linkage, and the expression of the ring straucture with "-" represents the connecting site at any position on the ring structure that can form a bond.

The compound designed and synthesized in the invention can effectively inhibit the function of SHP2, can reduce the content of SHP2 in cells, has better activity of resisting solid tumors and blood tumors in vivo and in vitro, and has lower toxicity to normal cells.

The compound of the present application is structurally inventive. This kind of compound can inhibit the phosphatase activity of SHP2, inhibit the signal channel transmission of SHP2 phosphatase dependency, inhibit the activity of proteins such as downstream proteins ERK, STAT3, STATS, c-SRC, JAK2 and the like, and inhibit malignant proliferation and distal metastasis of tumors; meanwhile, can block the downstream signal transmission independent of the important phosphatase of SHP2, inhibit the activity of protooncogenes such as KRAS and NRAS, and stabilize the tumor suppressor gene TP53, thereby achieving the purpose of inhibiting tumor growth, infiltration and metastasis; This kind of compound can also effectively improve the activity of T cells in a tumor microenvironment and prevent tumor cells from escaping immune surveillance to a certain extent. Therefore, the compound of the application can affect the function of SHP2 from two ways of SHP2 phospholipase dependence and phospholipase independence to achieve the aim of resisting tumors, so that the compound can be explained as specifically inhibiting the two functions of SHP2 and can obtain an active compound with high efficiency and low toxicity.

The respective elements of the present invention will be described in more detail below.

### Definition

Unless otherwise defined in the context, all technical and scientific terms used herein are intended to have the same meaning as commonly understood by a person skilled in the art. References to techniques employed herein are intended to refer to the techniques as commonly understood in the art, including variations on those techniques or substitutions of equivalent techniques which would be apparent to a person skilled in the art. While it is believed that the following terms will be readily understood by a person skilled in the art, the following definitions are nevertheless put forth to better illustrate the present invention.

The terms "contain", "include", "comprise", "have", or "relate to", as well as other variations used herein are inclusive or open-ended, and do not exclude additional, unrecited elements or method steps.

As used herein, the term "alkylene" refers to a saturated divalent hydrocarbyl, preferably refers to a saturated divalent hydrocarbyl having 1, 2, 3, 4, 5 or 6 carbon atoms, e.g., methylene, ethylene, propylene or butylene.

As used herein, the term "alkyl" is defined as a linear or branched saturated aliphatic hydrocarbon. In some embodiments, alkyl has 1-12, e.g., 1-6, carbon atoms. For example, as used herein, the term "C₁₋₆ alkyl" refers to a linear or branched group having 1-6 carbon atoms (such as methyl, ethyl, n-propyl, isopropyl, n-butyl, isobutyl, sec-butyl, tert-butyl, n-pentyl, isopentyl, neopentyl, or n-hexyl), which is optionally substituted with one or more (e.g., 1 to 3) suitable substituents such as halogen (in which case the group may be referred to as "haloalkyl") (e.g., CH₂F, CHF₂, CF₃, CCl₃, C₂F₅, C₂Cl₅, CH₂CF₃, CH₂Cl or - CH₂CH₂CF₃ etc.). The term "C₁₋₄ alkyl" refers to a linear or branched aliphatic hydrocarbon chain having 1-4 carbon atoms (i.e., methyl, ethyl, n-propyl, isopropyl, n-butyl, isobutyl, sec-butyl or tert-butyl).

As used herein, the term "alkenyl" refers to a linear or branched monovalent hydrocarbyl having a double bond and 2-6 carbon atoms ("C₂₋₆ alkenyl"). The alkenyl is e.g., vinyl, 1-propenyl, 2-propenyl, 2-butenyl, 3-butenyl, 2-pentenyl, 3-pentenyl, 4-pentenyl, 2-hexenyl, 3-hexenyl, 4-hexenyl, 5-hexenyl, 2-methyl-2-propenyl and 4-methyl-3-pentenyl. When the compound of the present invention contains an alkenyl group, the compound may exist as the pure E (entgegen) form, the pure Z (zusammen) form, or any mixture thereof.

As used herein, the term "alkynyl" refers to a monovalent hydrocarbyl containing one or more triple bond, and preferably having 2, 3, 4, 5 or 6 carbon atoms, e.g., ethynyl or propynyl.

As used herein, the term "cycloalkyl" refers to a saturated monocyclic or polycyclic (e.g., bicyclic) hydrocarbon ring (e.g., monocyclic, such as cyclopropyl, cyclobutyl, cyclopentyl, cyclohexyl, cycloheptyl, cyclooctyl, or cyclononyl, or bicyclic, including spiro, fused or bridged cyclic system (such as bicyclo[1.1.1]pentyl, bicyclo[2.2.1]heptyl, bicyclo[3.2.1]octyl or bicyclo[5.2.0] nonyl, or decahydronaphthalene etc.)), which is optionally substituted with one or more (e.g., 1 to 3) suitable substituents. The cycloalkyl has 3 to 15 carbon atoms. For example, the term "C₃₋₆ cycloalkyl" refers to a saturated monocyclic or polycyclic (e.g., bicyclic) hydrocarbon ring having 3 to 6 ring forming carbon atoms (e.g., cyclopropyl, cyclobutyl, cyclopentyl, or cyclohexyl), which is optionally substituted with one or more (e.g., 1 to 3) suitable substituents, e.g., methyl substituted cyclopropyl.

As used herein, the terms "cyclic hydrocarbylene", "cyclic hydrocarbyl" and "hydrocarbon ring" refer to a saturated (i.e., "cyclic hydrocarbylene" and "cycloalkyl") or unsaturated (i.e., having one or more double and/or triple bonds in the ring) monocyclic or polycyclic hydrocarbon ring having e.g., 3-10 (suitably having 3-8, and more suitably having 3-6) ring carbon atoms, including but not limited to cyclopropyl(ene) (ring), cyclobutyl(ene) (ring), cyclopentyl(ene) (ring), cyclohexyl(ene) (ring), cycloheptyl(ene) (ring), cyclooctyl(ene) (ring), cyclononyl(ene) (ring), cyclohexenyl(ene) (ring), and the like.

As used herein, the terms "heterocycly", "heterocyclylene" and "heterocycle" refer to a saturated (i.e., heterocycloalkyl) or partially unsaturated (i.e., having one or more double and/or triple bonds in the ring) cyclic group having e.g. 3-10 (suitably having 3-8, and more suitably having 3-6) ring atoms, wherein at least one ring atom is a heteroatom selected from the group consisting of N, O and S, and the remaining ring atoms are C. For example, "3- to 10-membered heterocyclyl(ene)" of "3- to 10-membered heterocycle" refers to saturated or partially unsaturated heterocyclyl(ene) or heterocycle having 2-9 (e.g., 2, 3, 4, 5, 6, 7, 8 or 9) ring carbon atoms and one or more (e.g., 1, 2, 3, or 4) heteroatoms independently selected from the group consisting of N, O and S. Examples of heterocyclylene, heterocyclyl and heterocycle include, but are not limited to oxiranyl(ene), aziridinyl(ene), azetidinyl(ene), oxetanyl(ene), tetrahydrofuranyl(ene), dioxolinyl(ene), pyrrolidinyl(ene), pyrrolidonyl(ene), imidazolidinyl(ene), pyrazolidinyl(ene), pyrrolinyl(ene), tetrahydropyranyl(ene), piperidinyl(ene), morpholinyl(ene), dithianyl(ene), thiomorpholinyl(ene), piperazinyl(ene) or trithianyl(ene). Said group also encompasses a bicyclic system, including a spiro, fused, or bridged system (e.g., 8-azaspiro[4.5]decane, 3,9-diazaspiro[5.5]undecane, 2-azabicyclo[2.2.2] octane, etc.). Heterocyclylene, heterocyclyl and heterocycle may optionally be substituted with one or more (e.g. 1, 2, 3 or 4) suitable substituents.

As used herein, the terms "aryl(ene)" and "aromatic ring" refer to an all-Carbon monocyclic or fused-Ring polycyclic aromatic group having a conjugated π electron system. For example, as used herein, the terms "C ₆₋₁₀ aryl(ene)" and "C ₆₋₁₀ aromatic ring" refer to an aromatic group containing 6 to 10 carbon atoms, such as phenyl(ene) (benzene ring) or naphthyl(ene) (naphthalene ring). Aryl(ene) or aromatic ring is optionally substituted with one or more (such as 1 to 3) suitable substituents (e.g., halogen, -OH,-CN, -NO ₂, and C₁₋₆alkyl, etc.).

As used herein, the term "aralkyl" preferably means aryl or heteroaryl substituted alkyl, wherein aryl, heteroaryl and alkyl are as defined herein. Normally, the aryl group may have 6-14 carbon atoms, the heteroaryl group may have 5-14 ring atoms, and the alkyl group may have 1-6 carbon atoms. Exemplary aralkyl group includes, but is not limited to, benzyl, phenylethyl, phenylpropyl, phenylbutyl.

As used herein, the term "halo" or "halogen" are defined to include F, Cl, Br, or I.

As used herein, the term "nitrogen containing heterocycle" refers to a saturated or unsaturated monocyclic or bicyclic group having 2, 3, 4, 5, 6, 7, 8, 9, 10, 11, 12 or 13 carbon atoms and at least one nitrogen atom in the ring, which may further optionally comprise one or more (e.g., one, two, three or four) ring members selected from the group consisting of N, O, C=O, S, S=O and S(=O) ₂. The nitrogen containing heterocycle is attached to the rest of the molecule through the nitrogen atom and any other ring atom in said nitrogen containing heterocycle. The nitrogen containing heterocycle is optionally benzO-fused, and is preferably attached to the rest of the molecule through the nitrogen atom in said nitrogen containing heterocycle and any carbon atom in the fused benzene ring.

The term "substituted" means that one or more (e.g., one, two, three, or four) hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds.

If a substituent is described as being "optionally substituted", the substituent may be either (1) not substituted, or (2) substituted. If a carbon of a substituent is described as being optionally substituted with one or more of a list of substituents, one or more of the hydrogens on the carbon (to the extent there are any) may separately and/or together be replaced with an independently selected optional substituent. If a nitrogen of a substituent is described as being optionally substituted with one or more from a list of substituents, one or more of the hydrogens on the nitrogen (to the extent there are any) may each be replaced with an independently selected optional substituent.

Preferably, "substituted" refers to the substitution of groups selected from halogen, cyano, nitro, hydroxyl, amide, C1-C6 alkyl, C1-C6 alkylyl, C1-C6 alkoxy, substituted C1-C6 alkyl, saturated or partially unsaturated C3-10 cyclic hydrocarbyl, saturated or partially unsaturated 3-10 membered heterocyclyl, C6-10 aryl, 5-14 membered heteroaryl or C6-12 aralkyl, preferably the substitution of groups selected from C1-C6 alkyl, saturated or partially unsaturated C3-10 cyclic hydrocarbyl, C6-10 aryl, C6-12 aralkyl.

If substituents are described as being "independently selected" from a group, each substituent is selected independent of the other(s). Each substituent therefore may be identical to or different from the other substituent(s).

As used herein, the term "one or more" means one or more than one (e.g., 2, 3, 4, 5 or 10) as reasonable.

As used herein, unless specified, the point of attachment of a substituent can be from any suitable position of the substituent.

When a bond to a substituent is shown to cross a bond connecting two atoms in a ring, then such substituent may be bonded to any of the ring-forming atoms in that ring that are substitutable.

The present invention also includes all pharmaceutically acceptable isotopically labeled compounds, which are identical to those of the present invention except that one or more atoms are replaced by an atom having the same atomic number, but an atomic mass or mass number different from the atomic mass or mass number which predominates in nature. Examples of isotopes suitable for inclusion in the compound of the present invention include, but are not limited to, isotopes of hydrogen, such as ²H, ³H; carbon, such as ¹¹C, ¹³C, and ¹⁴C; chlorine, such as ³⁶Cl; fluorine, such as ¹⁸F; iodine, such as ¹²³I and ¹²⁵I; nitrogen, such as ¹³N and ¹⁵N; oxygen, such as ¹⁵O, ¹⁷O, and ¹⁸O; phosphorus, such as ³²P; and sulfur, such as ³⁵S. Certain isotopically labeled compounds of the present invention, for example those incorporating a radioactive isotope, are useful in drug and/or substrate tissue distribution studies (e.g., assays). The radioactive isotopes tritium, i.e., ³H, and carbon-14, i.e., ¹⁴C, are particularly useful for this purpose in view of their ease of incorporation and ready means of detection. Substitution with positron-emitting isotopes, such as ¹¹C, ¹⁸F, ¹⁵O and ¹³N, can be useful in positron emission tomography (PET) studies for examining substrate receptor occupancy. Isotopically labeled compounds of the present invention can generally be prepared by processes analogous to those described in the accompanying Schemes and/or in the Examples and Preparations, by using an appropriate isotopically labeled reagent in place of the non-labeled reagent previously employed. Pharmaceutically acceptable solvates in accordance with the invention include those wherein the solvent of crystallization may be isotopically substituted, e.g., D₂O, acetone-d₆, or DMSO-d₆.

The term "stereoisomer" refers to isomers with at least one asymmetric center. A compound having one or more (e.g., one, two, three or four) asymmetric centers can give rise to a racemic mixture, single enantiomer, diastereomer mixture and individual diastereomer. Certain individual molecules may exist as geometric isomers (cis/trans). Similarly, the compound of the present invention may exist as a mixture of two or more structurally different forms in rapid equilibrium (generally referred to as tautomer). Typical examples of a tautomer include a ketO-enol tautomer, phenol-keto tautomer, nitrosO-oxime tautomer, imine-enamine tautomer and the like. It is to be understood that all such isomers and mixtures thereof in any proportion (such as 60%, 65%, 70%, 75%, 80%, 85%, 90%, 95%, 96%, 97%, 98%, and 99%) are encompassed within the scope of the present invention.

The chemical bonds of the compound of the present invention may be depicted herein using a solid line ( 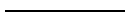 ), a solid wedge ( 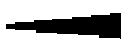 ), or a dotted wedge ( 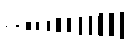 ). The use of a solid line to depict bonds to asymmetric carbon atoms is meant to indicate that all possible stereoisomers (e.g., specific enantiomers, racemic mixtures, etc.) at that carbon atom are included. The use of either a solid or dotted wedge to depict bonds to asymmetric carbon atoms is meant to indicate that the stereoisomer shown is present. When present in racemic compounds, solid and dotted wedges are used to define relative stereochemistry, rather than absolute stereochemistry. Unless stated otherwise, it is intended that the compound of the present invention can exist as stereoisomers, which include cis and trans isomers, optical isomers such as R and S enantiomers, diastereomers, geometric isomers, rotational isomers, conformational isomers, atropisomers, and mixtures thereof. The compound of the present invention may exhibit more than one type of isomerism, and consist of mixtures thereof (such as racemates and diastereomeric pairs).

The present invention includes all possible crystalline forms or polymorphs of the compound of the present invention, either as a single polymorph, or as a mixture of more than one polymorphs, in any ratio.

It also should be understood that, certain compounds of the present invention can be used for the treatment in a free form, or where appropriate, in a form of a pharmaceutically acceptable derivative. In the present invention, the pharmaceutically acceptable derivative includes, but is not limited to a pharmaceutically acceptable salt, ester, solvate, N-oxide, metabolite ,Chelate, complex, inclusion compound or prodrug, which can directly or indirectly provide the compound of the present invention or a metabolite or residue thereof after being administered to a patient in need thereof. Therefore, "the compound of the present invention" mentioned herein also means to encompass various derivative forms of the compound as mentioned above.

A pharmaceutically acceptable salt of the compound of the present invention includes an acid addition salt and a base addition salt thereof, including but not limited to salts containing hydrogen bonds or coordination bonds.

A suitable acid addition salt is formed from an acid which forms a pharmaceutically acceptable salt. Specific examples include acetate, adipate, aspartate, benzoate, besylate, bicarbonate/carbonate, bisulfate/sulfate, borate, camphorsulfonate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulfate, naphthylate, 2-Napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts.

A suitable base addition salt is formed from a base which forms a pharmaceutically acceptable salt. Specific examples include aluminum, arginine, benzathine, calcium, choline, diethylamine, diolamine, glycine, lysine, magnesium, meglumine, olamine, potassium, sodium, tromethamine and zinc salts.

For a review on suitable salts, see "Handbook of Pharmaceutical Salts: Properties, Selection, and Use" by Stahl and Wermuth (Wiley-VCH, 2002). The method for preparing a pharmaceutically acceptable salt of the compound of the present invention is known to a person skilled in the art.

As used herein, the term "ester" refers to those derived from the compounds of the various formulae in the present application, which include physiologically-hydrolyzable esters (which may be hydrolyzed under physiological conditions to release the compounds of the present invention in the form of free acids or alcohols). The compound of the present invention itself may be an ester as well.

The compound of the present invention can exist as a solvate (preferably a hydrate), wherein the compound of the present invention contains a polar solvent, in particular water, methanol or ethanol for example, as a structural element of the crystal lattice of the compound. The amount of the polar solvent, in particular water, may exist in a stoichiometric or non-stoichiometric ratio.

As can be appreciated by a person skilled in the art, not all nitrogen containing heterocycles can form N-oxides since the nitrogen requires an available lone-pair electron for oxidation to the oxide; a person skilled in the art will recognize those nitrogen containing heterocycles which can form N-oxides. A person skilled in the art will also recognize that tertiary amines can form N-oxides. Synthetic methods for the preparation of N-oxides of heterocycles and tertiary amines are well known to a person skilled in the art, and they include the oxidation of heterocycles and tertiary amines with peroxy acids such as peracetic acid and m-Chloroperbenzoic acid (MCPBA), hydrogen peroxide, alkyl hydroperoxides such as tert-butyl hydroperoxide, sodium perborate, and dioxiranes such as dimethyldioxirane. These methods for the preparation of N-oxides have been extensively described and reviewed in literatures, see e.g., T. L. Gilchrist, Comprehensive Organic Synthesis, vol. 7, pp 748-750; A. R. Katritzky and A. J. Boulton, Eds., Academic Press; and G. W. H. Cheeseman and E. S. G. Werstiuk, Advances in Heterocyclic Chemistry, vol. 22, pp 390-392, A. R. Katritzky and A. J. Boulton, Eds., Academic Press.

The metabolite of the compound of the present invention, namely a substance formed in vivo upon administration of the compound of the present invention, is also included within the scope of the present invention. Such a product may result e.g., from the oxidation, reduction, hydrolysis, amidation, de-amidation, esterification, enzymolysis, and the like, of the administered compound. Accordingly, the present invention encompasses the metabolite of the compound of the present invention, including a compound produced by a method comprising contacting the compound of the present invention with a mammal for a period of time sufficient to result in a metabolic product thereof.

Also within the scope of the present invention is a prodrug of the compound of the invention, which is certain derivative of the compound of the invention that may have little or no pharmacological activity itself, but can, when administered into or onto the body, be converted into the compound of the invention having the desired activity, for example, by hydrolytic cleavage. In general, such prodrug will be a functional derivative of the compound which is readily converted in vivo into the compound with desired therapeutic activity. Further information on the use of the prodrug may be found in "PrO-drugs as Novel Delivery Systems", Vol. 14, ACS Symposium Series (T. Higuchi and V. Stella). The prodrug in accordance with the invention can, for example, be produced by replacing appropriate functionalities present in the compound of the present invention with certain moieties known to those skilled in the art as "pro-moieties" as described, for example, in "Design of Prodrugs" by H. Bundgaard (Elsevier, 1985).

The present invention further encompasses the compound of the present invention having a protecting group. During any of the processes for preparation of the compound of the present invention, it may be necessary and/or desirable to protect sensitive or reactive groups on any of the molecules concerned, thereby resulting in the chemically protected form of the compound of the present invention. This may be achieved by means of conventional protecting groups, e.g., those described in T. W. Greene & P. G. M. Wuts, Protective Groups in Organic Synthesis, John Wiley & Sons, 1991, which is incorporated herein by reference. The protecting groups may be removed at a convenient subsequent stage using methods known from the art.

The term "about" refers to a range within ±10%, preferably within ±5%, and more preferably within ±2% of the specified value.

### Compound

The invention provides a compound shown in formula 1 or a pharmaceutically acceptable salt, ester, optical isomer, stereoisomer, polymorph, solvate, N-oxide, isotopically labeled compound, metabolite, chelate, complex, clathrate or prodrug thereof,

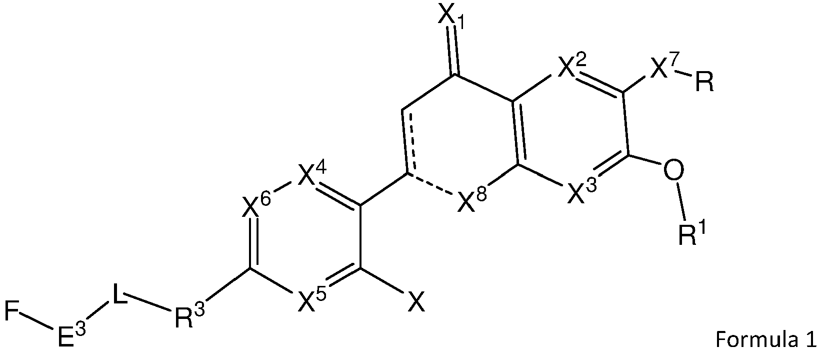

In formula 1,
"- - -" indicates the chemical bond is a double or single bond; "---" indicates that the chemical bond is a single bond or absent,
X is halogen, C1-6 alkyl, C2-6 alkenyl, C2-6 alkynyl, saturated or partially unsaturated C3-10 cyclic hydrocarbyl, preferably halogen, methyl, ethyl, isopropyl, cyclopropyl, more preferably halogen,
X¹ is NR⁴, O or S, preferably O or S, more preferably O,
X⁷ is a chemical bond or a divalent group formed by combining one or more selected from the group consisting of C1-6 alkylene, C2-6 alkenylene, C2-6 alkynylene, saturated or partially unsaturated C3-10 cyclic hydrocarbylene, -O-, -CO-, -C(= O)O-, -CONH-, -NHCO-, -NHCONH-, -NH-, -S-, -sulfinyl, and sulfonyl, preferably a chemical bond or a divalent group formed by combining one or more selected from the group consisting of C1-6 alkylene, -O-, -CO-, -CONH-, -NHCO-, -NHCONH-, and NHCONH-,
X², X³, X⁴, X⁵and X⁶ are each independently CR⁴ or N, preferably CR⁶ ,
When the chemical bond represented by "---" is a single bond, X⁸ is NR², O or S, preferably O or S, more preferably O,
When the chemical bond represented by "- - -" is absent, X⁸ is H, N (R²)₂, OR² or SR², preferably OR² or SR², more preferably OR²,
R¹ is H, C1-6 alkyl, C2-6 alkenyl, C2-6 alkynyl, saturated or partially unsaturated C3-10 cyclic hydrocarbyl, preferably C1-6 alkyl, more preferably one selected from methyl, ethyl, isopropyl and cyclopropyl,
R² is H, C1-6 alkyl, C2-6 alkenyl, C2-6 alkynyl, saturated or partially unsaturated C3-10 cyclic hydrocarbyl, preferably H, C1-6 alkyl, more preferably one selected from H, methyl and ethyl, particularly preferably H,
R³ is CR⁴ R⁵, NR⁴, 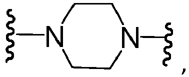 O or S, preferably NR⁴, 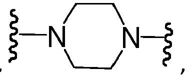 O or S, more preferably NR⁴, 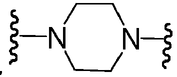 or O,
R⁴ and R⁵ are each independently H, C1-6 alkyl, C2-6 alkenyl, C2-6 alkynyl, saturated or partially unsaturated C3-10 cyclic hydrocarbyl, saturated or partially unsaturated 3-10 membered heterocyclyl, C6-10 aryl, 5-14 membered heteroaryl, or C6-12 aralkyl,
R⁶ is H, halogen, C1-6 alkyl, C2-6 alkenyl, C2-6 alkynyl, saturated or partially unsaturated C3-10 cyclic hydrocarbyl, saturated or partially unsaturated 3-10 membered heterocyclyl, C6-10 aryl, 5-14 membered heteroaryl, or C6-12 aralkyl,
R is H, halogen, NH₂, OH, SH, amino, substituted or unsubstituted C2-10 aliphatic hydrocarbon group, substituted or unsubstituted saturated or partially unsaturated 3-10 member heterocyclyl, substituted or unsubstituted C6-10 aryl group, or substituted or unsubstituted 5-14 membered heteroaryl; R is preferably halogen, OH, SH, haloalkyl, amino, saturated C3-10 cyclic hydrocarbyl, saturated or partially unsaturated 3-10 membered heterocyclyl, C1-6 alkyl substituted amino, 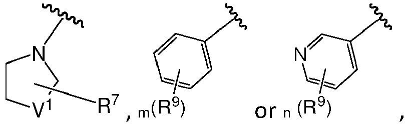
Wherein V¹ is one selected from C(R⁸)₂, C(R⁸)₂ -C(R⁸)₂, CR⁸=CR⁸, C=O, C(=O)C(R⁸)₂, C(R⁸)₂C(=O), C(=O)O, OC(=O), C(= O)NR, N=CR⁸, CR⁸=N, NR⁸-C(R⁸)₂ or C(R⁸)₂-NR⁸;
R⁷ is one selected from H, halogen, cyano, nitro, hydroxymethyl, hydroxyl, amide, C1-C6 alkyl, C1-C6 alkoxy, C1-C6 alkoxy-C1-C6 alkylene, or 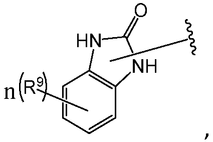
R⁹ are each independently selected from the group consisting of halogen, hydroxyl, oxo, amino, C1-6 alkyl-substituted amino, cyano, nitro, -Si(R⁸)₃, C1-6 alkyl, C1-6 alkoxy, saturated or partially unsaturated C3-6 cyclic hydrocarbyl, saturated or partially unsaturated 3-10 membered heterocyclyl, C6-10 aryl, 5-14 membered heteroaryl, C6-12 aralkyl, -C(=O)R⁸, -OC(=O)R⁸, -C(=O)OR⁸, -OR⁸, -SR⁸, -S(=O)R⁸, -S(=O)₂R⁸, -S(=O)₂N(R⁸)₂, -N(R⁸)₂, -C(=O)N(R⁸),-NR⁸-C(=O)R⁸, -NR⁸-C(=O)OR⁸, -NR⁸-S(=O)₂-R⁸, -NR⁸-C(=O)-N(R⁸)₂, -C1-6 alkylene-N(R⁸)₂, -C1-6 alkylene-OR⁸, -C1-6 alkenylene-OR⁸ and-O-C1-6 alkylene-N(R⁸)₂; m is an integer from 0 to 5, n is an integer from 0 to 4, when m is not 0, a plurality of R² can be connected with each other to form a ring structure, and when n is not 0, a plurality of R⁹⁹ can be connected with each other to form a ring structure;
R⁸ is H, C1-C6 alkyl, saturated or partially unsaturated C3-6 cyclic hydrocarbyl, saturated or partially unsaturated 3-10 member heterocyclyl or C6-10 aryl, the expression of the ring structure with "-" represents the connecting site at any position on the ring structure that can form a bond;
L is a linking group which represents a linear or branched C3-C29 alkylene chain, wherein the linear or branched C3-C29 alkylene chain is optionally interrupted one or more times by one or more divalent groups selected from -O-, -CO-, -C(=O)O-, -CONH-, - NHCO-, -NHCONH-, -NH-, -NR⁸-, -C(R⁸)₂-, -S-, sulfinyl, sulfonyl, sulfinyloxy, sulfonyloxy, - aminosulfonylamino-, alkynylene, alkenylene, cycloalkylene, 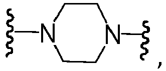 or any combination thereof,
E³ is the following group, 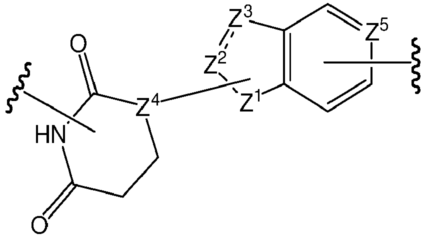
-̅-̅-̅-̅-̅Represents a single or double bond, preferably a single bond;
Z¹ is O, S, NH, CH₂ or C=O, preferably CH₂ or C=O;
When -̅-̅-̅-̅-̅ is a double bond, Z² is N or CH, and Z³ is N or CH;
When -̅-̅-̅-̅-̅ is a single bond, Z² is O, S, NH, CH₂ or C=O, preferably NH; Z³ is O, S, NH, CH₂ or C=O, preferably CH₂ or C=O;
Z⁴ is N or CH, Z⁴ is connected with any connectable position of Z¹, Z² and Z³;
Z⁵ is N or CH, preferably N;
E³ is further preferably 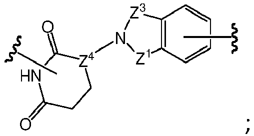
F is H or 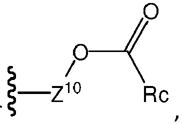
Z¹⁰ is selected from a chemical bond, C1-6 alkylene, C2-6 alkenylene, C2-6 alkynylene, saturated or partially unsaturated C3-10 cyclic hydrocarbylene, preferably a chemical bond, C1-6 alkylene;
Rc is halogen, cyano, nitro, hydroxyl, acylamino, C1-C6 alkyl, C1-C6 alkoxy, substituted C1-C6 alkyl, saturated or partially unsaturated C3-10 cyclic hydrocarbyl, saturated or partially unsaturated 3-10 membered heterocyclyl, C6-10 aryl, 5-14 membered heteroaryl or C6-12 aralkyl, preferably C1-C6 alkyl, saturated or partially unsaturated C3-10 cyclic hydrocarbyl, C6-10 aryl, C6-12 aralkyl,
In the above expression, 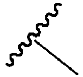 denotes the position of the linkage, and the expression of the ring straucture with "-" represents the connecting site at any position on the ring structure that can form a bond.

The substitution of the above-mentioned substituted or unsubstituted preferably refers to the substitution of groups selected from halogen, cyano, nitro, hydroxyl, amido, C1-C6 alkyl, C1-C6 alkylacyl, C1-C6 alkoxy, substituted C1-C6 alkyl, saturated or partially unsaturated C3-10 cyclic hydrocarbyl, saturated or partially unsaturated 3-10 membered heterocyclyl, C6-10 aryl, 5-14 membered heteroaryl or C6-12 aralkyl, preferably the substitution of groups selected from C1-C6 alkyl, saturated or partially unsaturated C3-10 cyclic hydrocarbyl, C6-10 aryl and C6-12 aralkyl.

In a preferred embodiment of the present invention, the compound of formula 1 has the structure of formula 2 below, 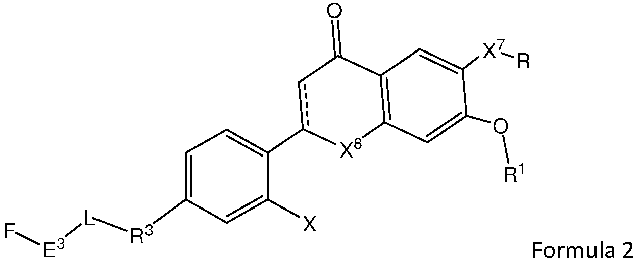
Wherein "- - -" represents a double bond or a single bond,
In formula 2, X is halogen, C1-6 alkyl, saturated or partially unsaturated C3-10 cyclic hydrocarbyl, preferably halogen,
In formula 2, R¹ is H, C1-6 alkyl, C2-6 alkenyl, C2-6 alkynyl, saturated or partially unsaturated C3-10 cyclic hydrocarbyl, preferably C1-6 alkyl, further preferably ethyl,
In formula 2, X⁸ is O or S, preferably O,
In formula 2, R³ is NR⁴, 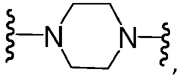 O or S, preferably NR⁴, 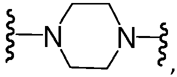 O or S, further preferably NR⁴, 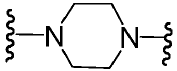 or O, in formula 2, R⁴ are each independently H, C1-6 alkyl, R⁴ is preferably H,
In formula 2, R, L, E³ and F have the same meanings as in claim 1, and in the above expression, 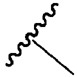 represents a position of linkage.
In a preferred embodiment of the present invention, the compound of formula 1 has a structure represented by formula 3 below, 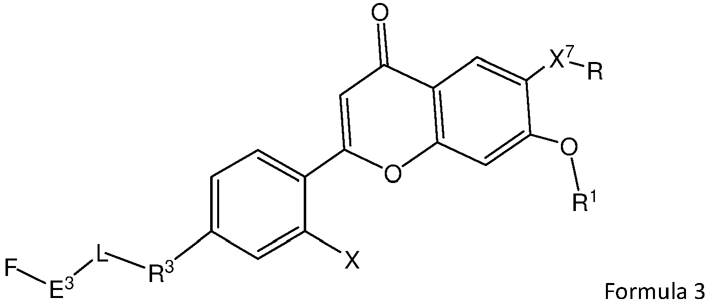
In formula 3, X is halogen, C1-6 alkyl, saturated or partially unsaturated C3-10 cyclic hydrocarbyl, preferably halogen, R¹ is H, C1-6 alkyl, C2-6 alkenyl, C2-6 alkynyl, saturated or partially unsaturated C3-10 cyclic hydrocarbyl, preferably C1-6 alkyl, further preferably ethyl,
In formula 3, R³ is CR⁴R⁵, NH, 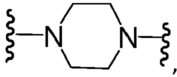 O or S, preferably NR⁴, O or S, R⁴ and R⁵ are each independently H, C1-6 alkyl, C2-6 alkenyl, C2-6 alkynyl, saturated or partially unsaturated C3-10 cyclic hydrocarbyl, saturated or partially unsaturated 3-10 membered heterocyclyl, C6-10 aryl, 5-14 membered heteroaryl or C6-12 aralkyl, R³ is further preferably NH or O,
In formula 3, R, L, E³ and F have the same meanings as in claim 1, and in the above expression, 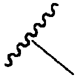 represents a position of linkage.
In a preferred embodiment of the invention, E³ is one of the following groups,

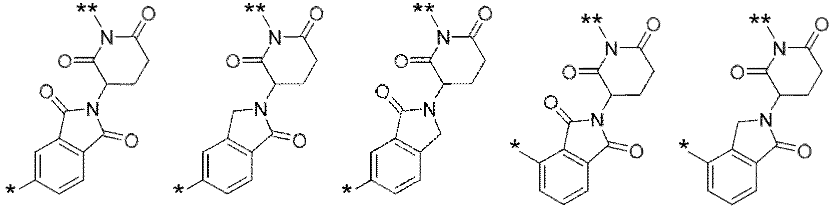

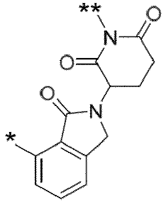

The above groups are linked to L via one of the two positions labeled with *-or**- and the other position is linked to F.

In a preferred embodiment of the invention, F is H or one of the following groups,

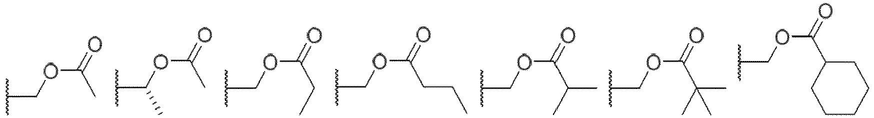

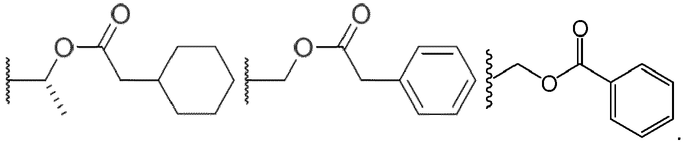

In a preferred embodiment of the invention, in the compound of the invention,
X⁷ is a chemical bond or a divalent group formed by combining one or more selected from the group consisting of methylene, ethylene, propylene, -O-, -CONH, -and NHCO-, preferably a chemical bond, methylene, ethylene, methyleneoxy, ethylene-CONH-, methylene-CONH-,
R is 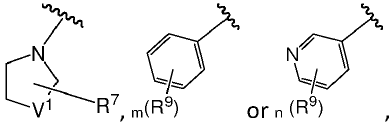 wherein V¹ is one selected from CH₂, CH₂-CH₂, CH=CH, NH-CH₂ or CH₂-NH; R⁷ is one selected from H, C1-C6 alkyl, C1-C6 alkoxy substituted C1-C6 alkylene or 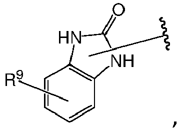 and R⁹ is as defined in claim 1; 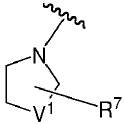
is preferably one selected from 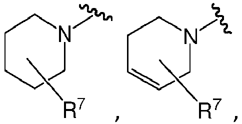 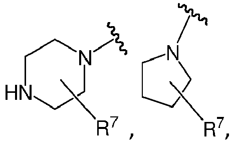 wherein R⁷ is further preferably one selected from H, methyl, ethyl, CONH₂, 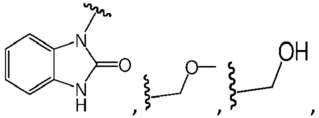
R⁹ are each independently selected from halogen, amino, C1-6 alkyl substituted amino, cyano, C1-6 alkyl, C1-6 alkoxy, saturated or partially unsaturated C3-6 cyclic hydrocarbyl, saturated or partially unsaturated 3-10 membered heterocyclyl, C6-10 aryl, C6-12 aralkyl, -C(=O)R⁸, -OC(=O)R⁸, -C(=O)OR⁸, -OR⁸, -SR⁸, -S(=O)R⁸, -S(=O)₂R⁸, - S(=O)₂N(R⁸)₂, -N(R⁸)₂, -C(=O)N(R⁸)₂, -NR⁸-C(=O)R⁸, -NR⁸-C(=O)OR⁸, -NR⁸-S(=O)₂-R⁸, -NR⁸-C(=O)-N(R⁸)₂; m is an integer from 0 to 5, n is an integer from 0 to 4, when m is not 0, a plurality of R⁹ can be connected with each other to form a ring structure, and when n is not 0, a plurality of R⁹ can be connected with each other to form a ring structure;
R⁸ is H, C1-C6 alkyl, saturated or partially unsaturated C3-6 cyclic hydrocarbyl, saturated or partially unsaturated 3-10 membered heterocyclyl, or C6-10 aryl, and the expression of the ring straucture with "-" represents the connecting site at any position on the ring structure that can form a bond, 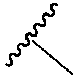
denotes the position of the linkage.
In further detail, in the compound of the present invention, R is preferably the following groups, 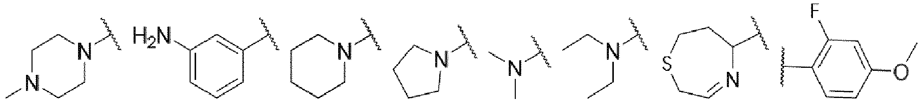 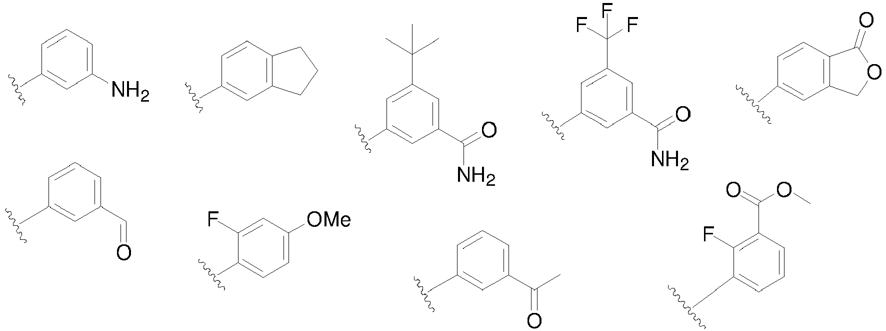 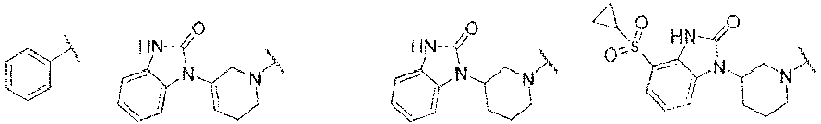 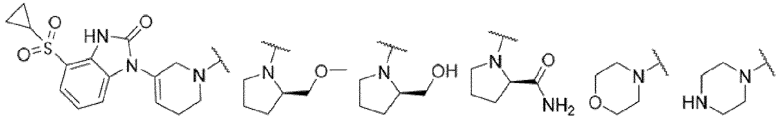 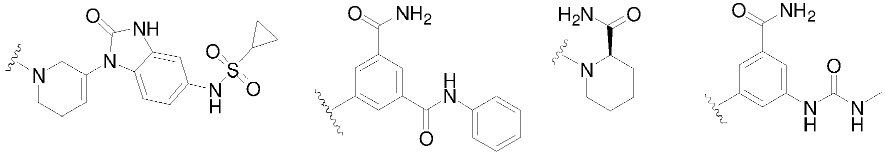 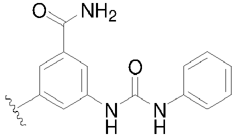 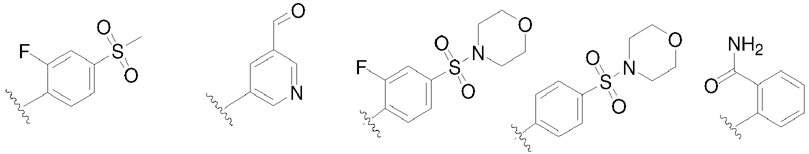 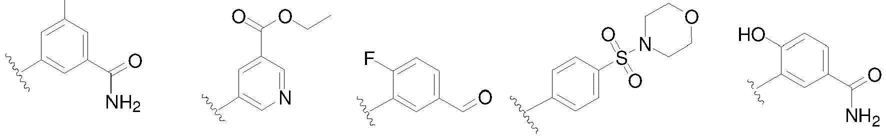 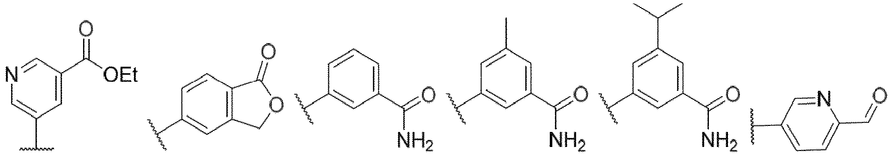 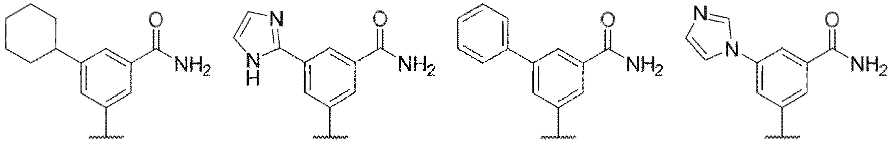 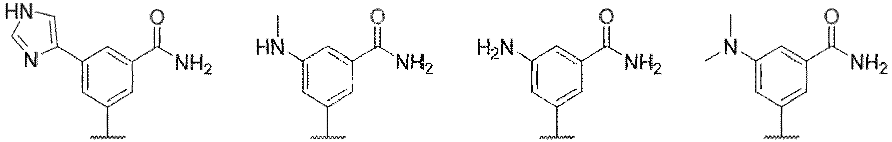 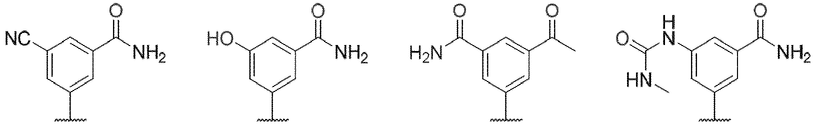 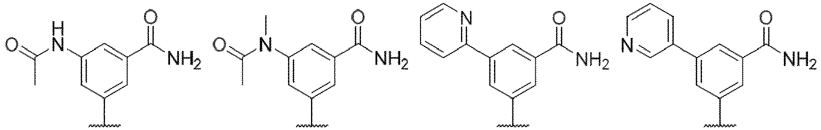 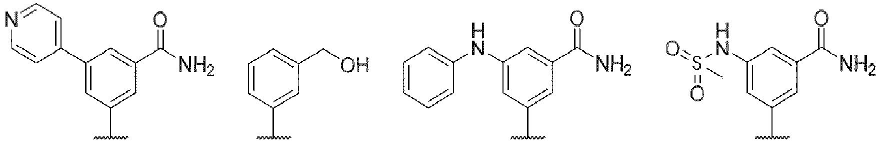 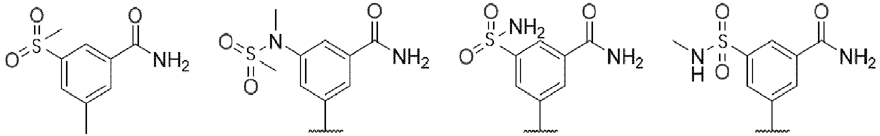 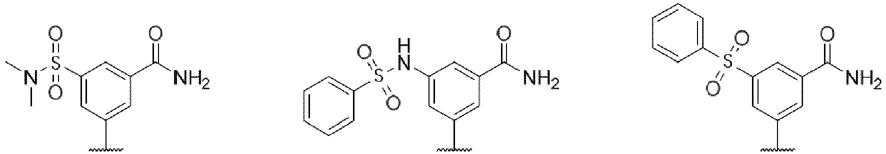 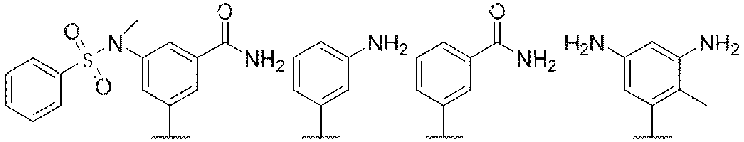 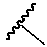
denotes the position of the linkage.
In a preferred embodiment of the present invention, L is preferably a divalent group represented by formula 5, 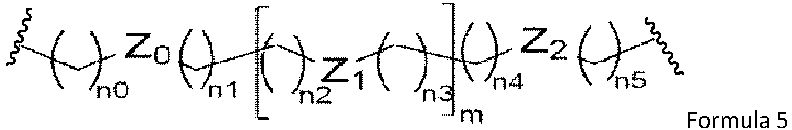
n₀ is 0 or 1,
m is an integer from 1 to 5, preferably 2 or 3; n₁ is an integer from 0 to 3, preferably 1; n₂ is an integer from 0 to 3, preferably 1; n₃ is an integer from 0 to 3, preferably 1; n₄ is an integer from 0 to 3, preferably 1; n₃ is an integer from 0 to 3, preferably 1;
Z₀ is-CH₂-, -NH-, -O-, -S-, 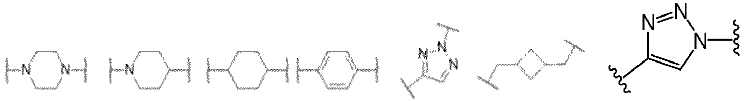 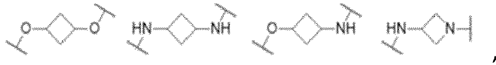 , -CO- or -C(=O)O-;
Z₁ is -CH₂-, -NH-,-O-,-S-, 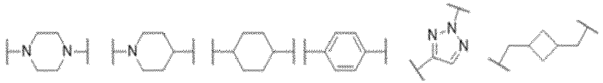 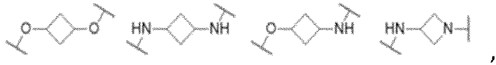 -CO- or -C(=O)O-;
Z₂ is-CH₂-, -NH-, -O-, -S-, 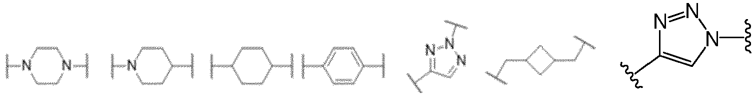 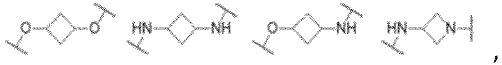 -CO- or -C(=O)O-; 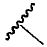
denotes the position of the linkage, and the connecting directions of the two ends are arbitrarily changed.
L is further preferably one of the following divalent groups, 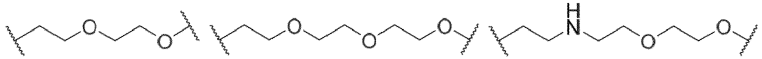 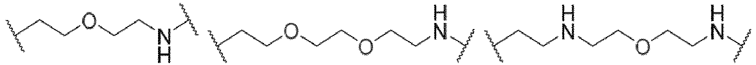 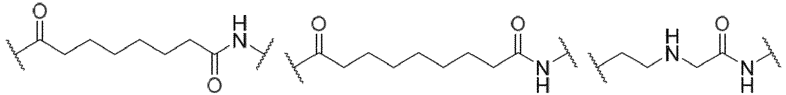 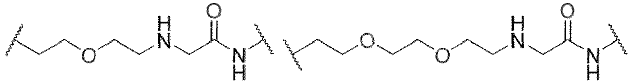 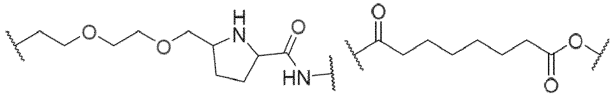 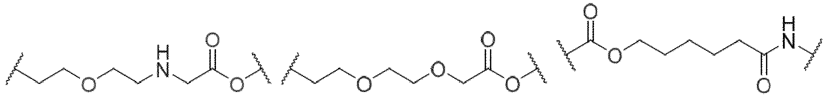 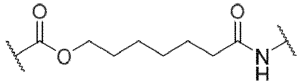 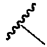
denotes the position of the linkage, and the connecting directions of the two ends are arbitrarily changed.

Although the following structures are typical specific structures for describing the compounds of the present invention in more detail, the present invention is not limited to the following structures, and the numbers and molecular weights of the corresponding molecules are shown in the table.

**Table. 1 Representative compounds of the invention and their molecular weights**

| No. | Structure | Molecular weight |
|---|---|---|
| Compound 12 | 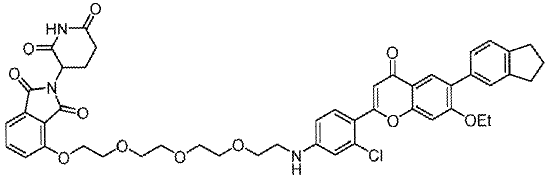 | 864.35 |
| Compound 13 | 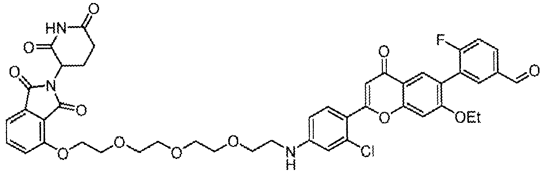 | 870.28 |
| Compound 14 | 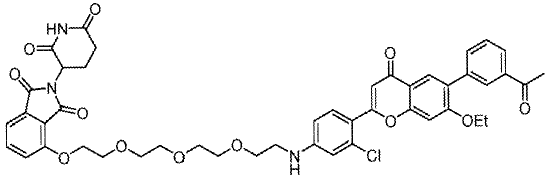 | 866.32 |
| Compound 15 | 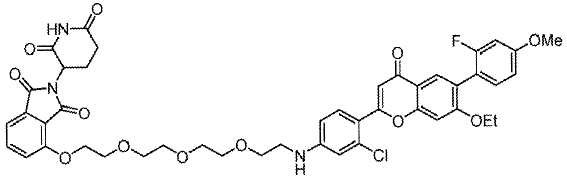 | 872.30 |
| Compound 16 | 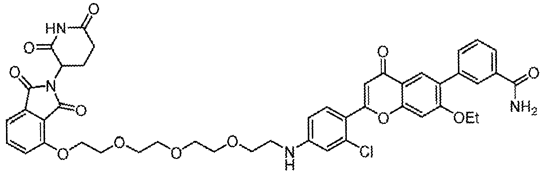 | 867.31 |
| Compound 17 | 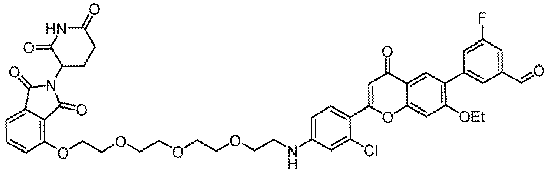 | 870.28 |
| Compound 18 | 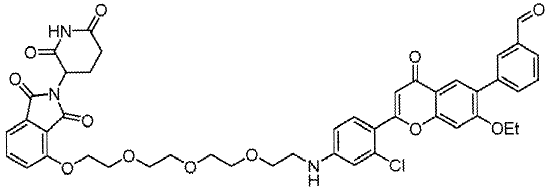 | 852.29 |
| Compound 11 | 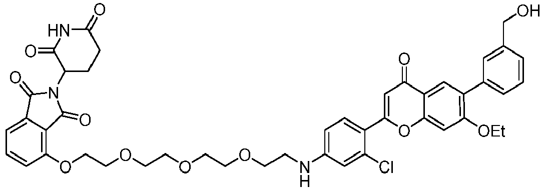 | 854.31 |
| Compound 27 | 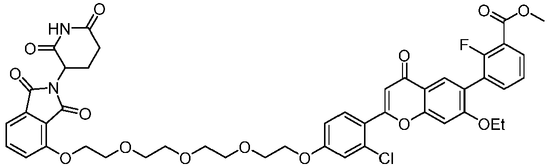 | 901.29 |
| Compound 28 | 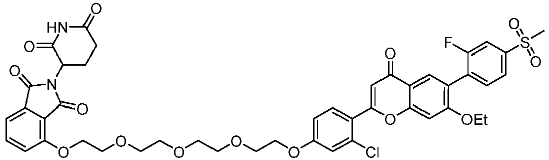 | 921.34 |
| Compound 29 | 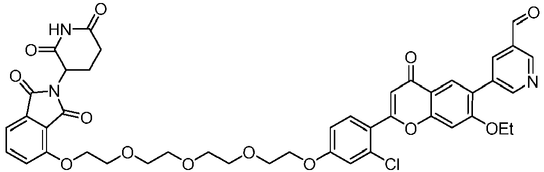 | 854.26 |
| Compound 30 | 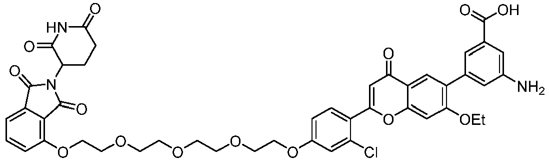 | 884.29 |
| Compound 31 | 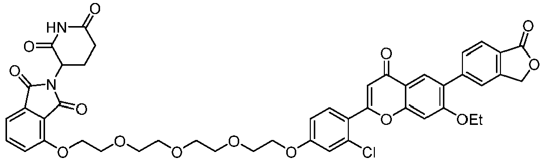 | 881.28 |
| Compound 32 | 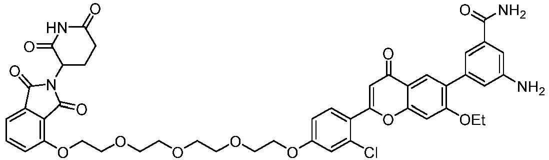 | 883.30 |
| Compound 26 | 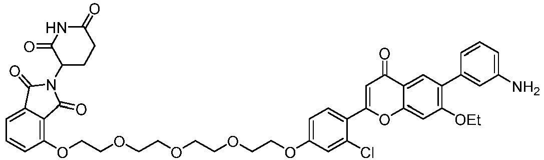 | 840.28 |
| Compound 33 | 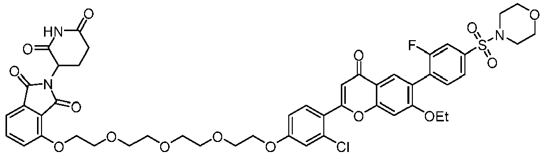 | 992.42 |
| Compound 40 | 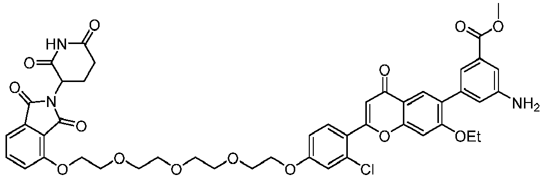 | 898.32 |
| Compound 34 | 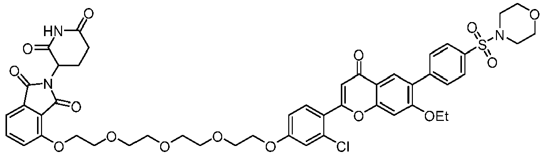 | 974.43 |
| Compound 35 | 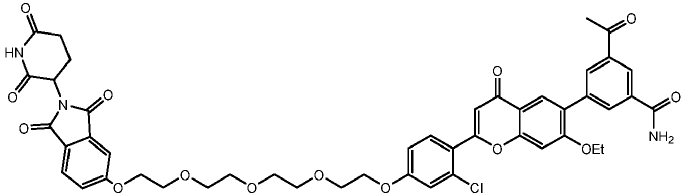 | 910.33 |
| Compound 36 | 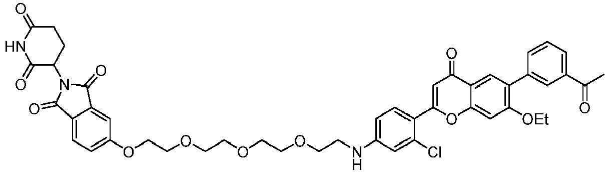 | 866.26 |
| Compound 41 | 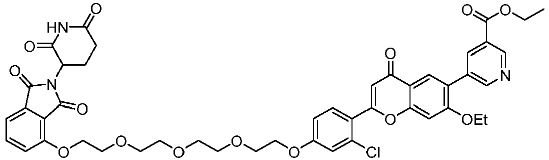 | 898.32 |
| Compound 42 | 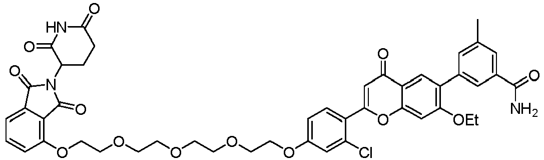 | 882.32 |
| Compound 43 | 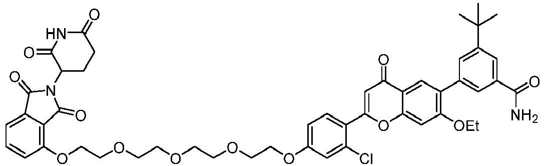 | 924.40 |
| Compound 44 | 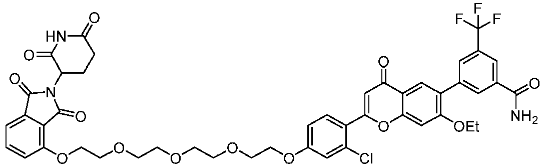 | 936.29 |
| Compound 45 | 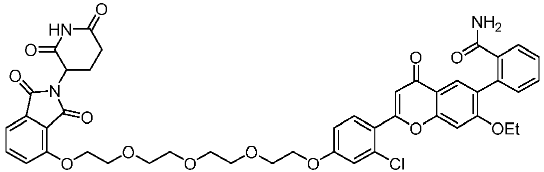 | 868.29 |
| Compound 46 | 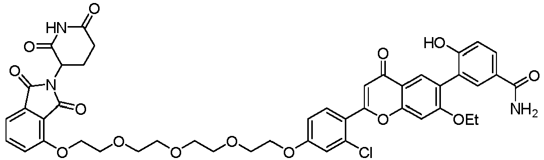 | 884.29 |

### Continued after Table 1

| No. | Structure | Molecular weight |
|---|---|---|
| Compound 50 | 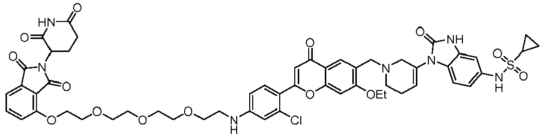 | 1094.59 |
| Compound 51 | 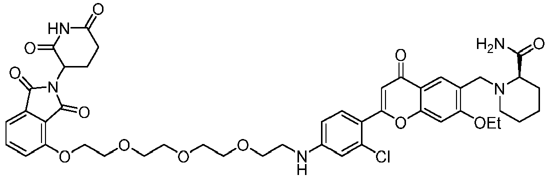 | 888.37 |
| Compound 52 | 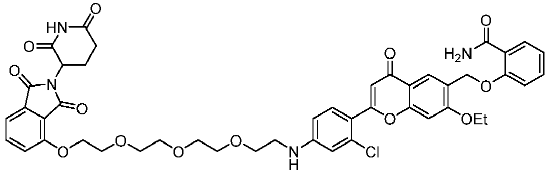 | 897.33 |
| Compound 53 | 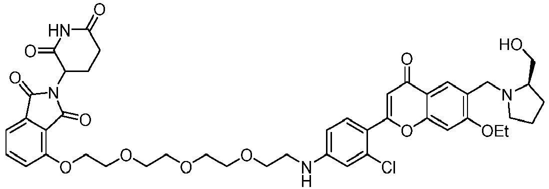 | 861.34 |
| Compound 54 | 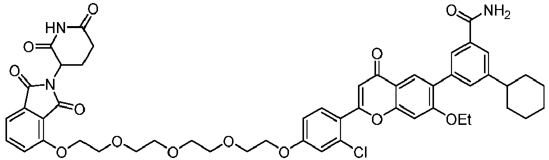 | 950.44 |
| Compound 55 | 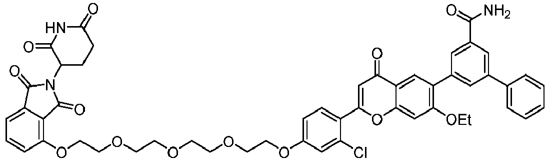 | 944.39 |
| Compound 56 | 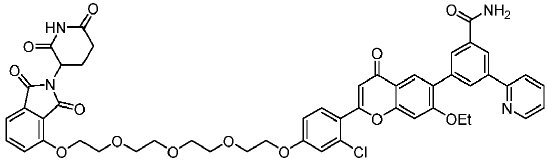 | 945.38 |
| Compound 57 | 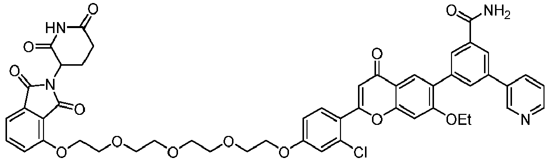 | 945.38 |
| Compound 58 | 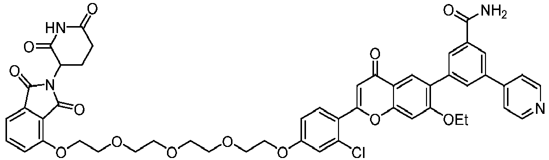 | 945.38 |
| Compound 59 | 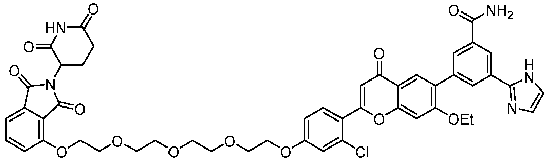 | 934.35 |
| Compound 60 | 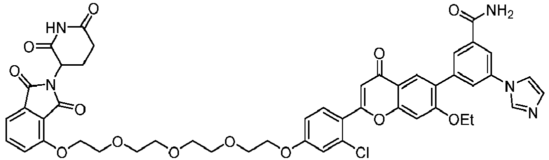 | 934.35 |
| Compound 61 | 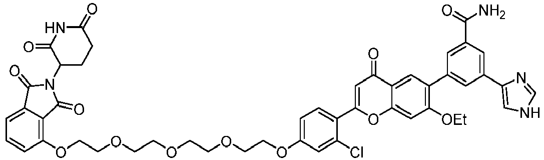 | 934.35 |
| Compound 62 | 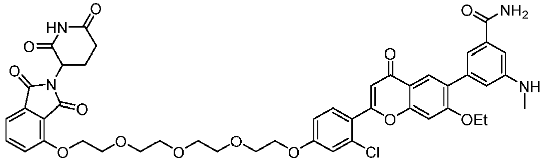 | 897.33 |
| Compound 63 | 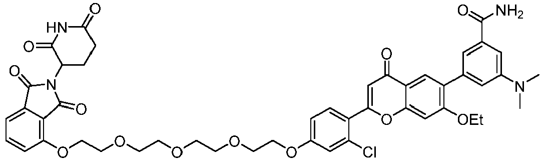 | 911.36 |
| Compound 64 | 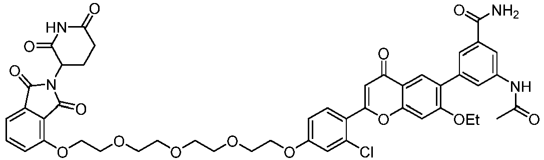 | 925.34 |
| Compound 65 | 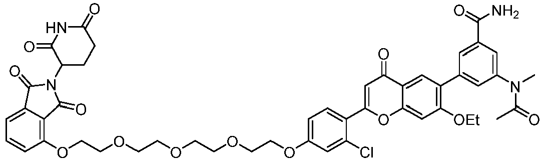 | 939.37 |
| Compound 66 | 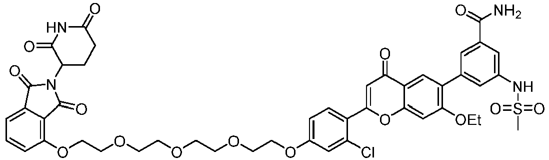 | 961.39 |
| Compound 67 | 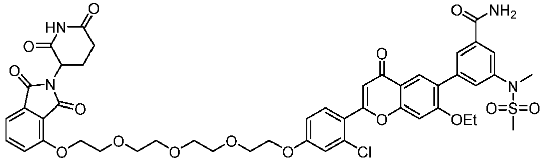 | 975.42 |
| Compound 68 | 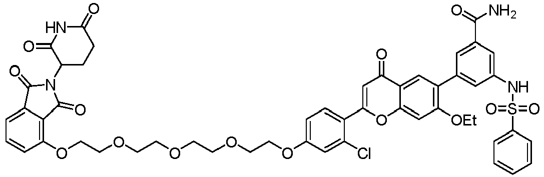 | 1023.46 |
| Compound 69 | 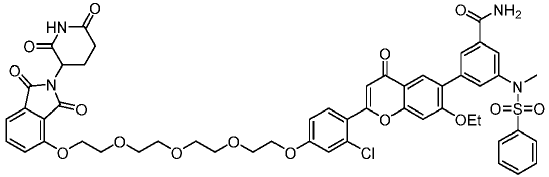 | 1037.49 |
| Compound 70 | 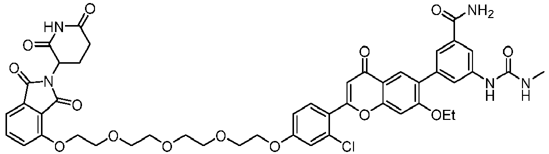 | 940.36 |
| Compound 71 | 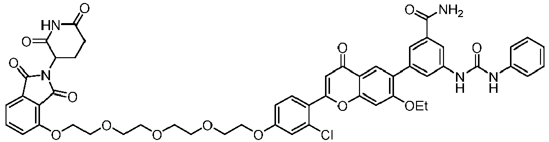 | 1002.43 |
| Compound 72 | 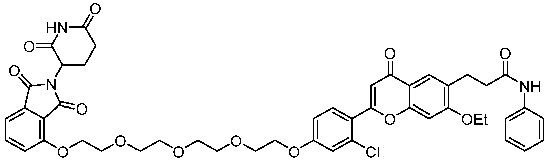 | 896.34 |
| Compound 73 | 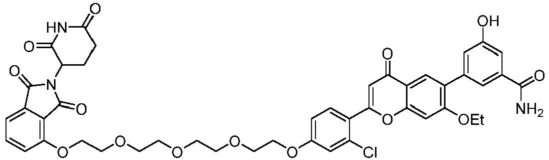 | 884.29 |
| Compound 74 | 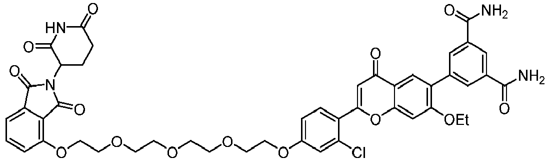 | 911.31 |
| Compound 75 | 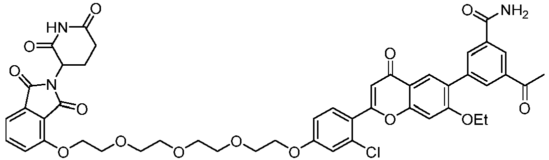 | 910.33 |
| Compound 76 | 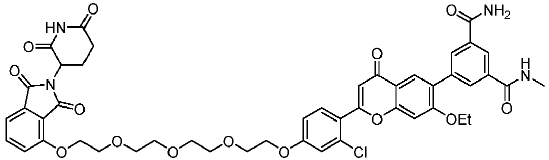 | 925.34 |
| Compound 77 | 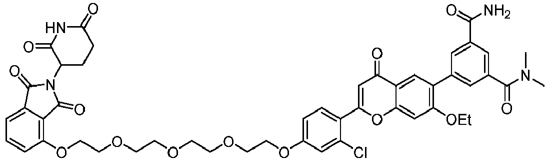 | 939.37 |
| Compound 78 | 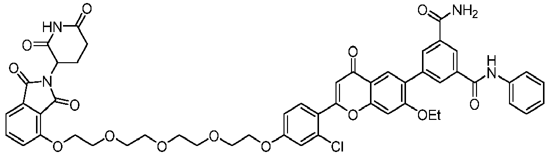 | 987.41 |
| Compound 79 | 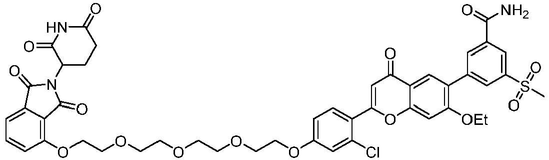 | 946.37 |
| Compound 80 | 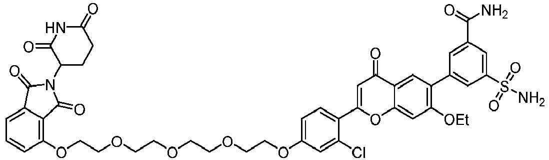 | 947.36 |
| Compound 81 | 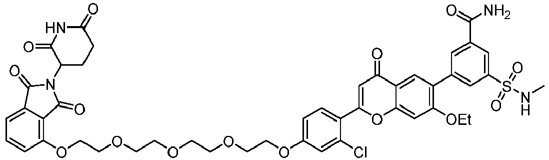 | 961.39 |
| Compound 82 | 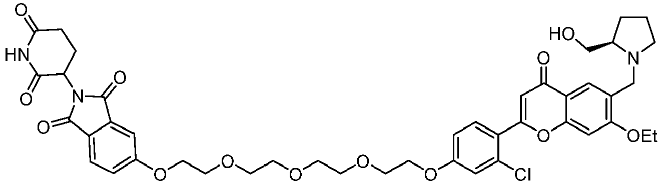 | 862.32 |
| Compound 83 | 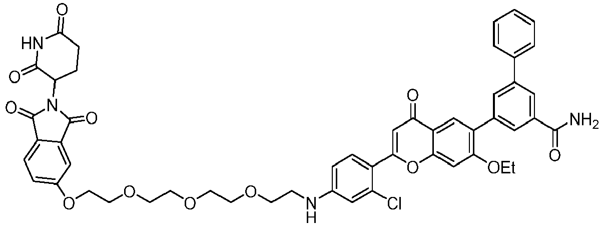 | 943.39 |
| Compound 84 | 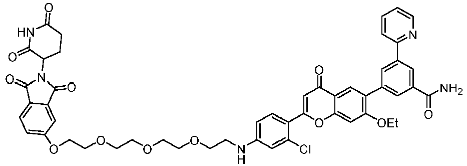 | 943.28 |
| Compound 85 | 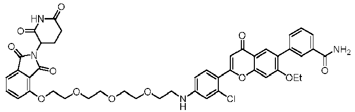 | 867.30 |
| Compound 86 | 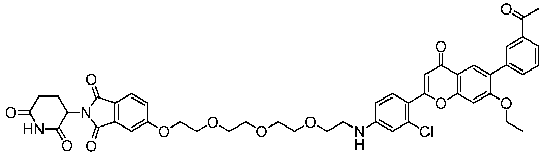 | 866.31 |
| Compound 87 | 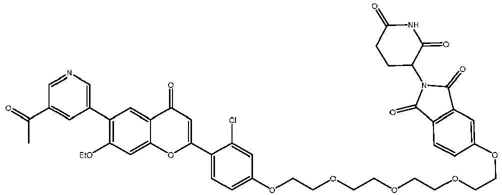 | 868.29 |
| Compound 88 | 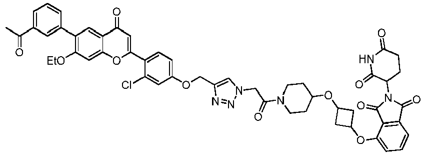 | 983.42 |
| Compound 89 | 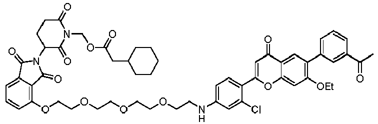 | 1020.51 |
| Compound 90 | 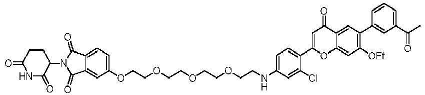 | 866.31 |
| Compound 91 | 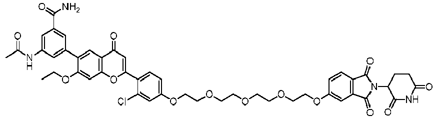 | 925.33 |

### General procedure for obtaining compounds of the invention

The compound represented by formula 1 of the present invention can be obtained by a known method, for example, by a known organic synthesis method. Exemplary synthetic routes are given below, but may be obtained by other methods known to those skilled in the art.

Representative technical routes can be divided into the following route one and route two, the main difference being the group at the R³ position. The synthesis method of the first route can refer to synthesis examples 1 to 13, and the synthesis method of the second route can refer to synthesis examples 14 to 27. In the present invention, the synthesis method of the compound is described by specific examples described later, and it should be noted that the synthesis route of the representative compound of formula 1 in which X¹ is O, and X² X³ X⁴ and X⁵ are all CH is exemplified, and the synthesis of the compounds in which X¹ to X⁵ are other parallel schemes can be realized by replacing the corresponding raw materials.

### Pharmaceutical compositions and methods of treatment

The present invention provides a pharmaceutical composition comprising a prophylactically or therapeutically effective amount of a compound of the present invention, or a pharmaceutically acceptable salt, ester, optical isomer, stereoisomer, polymorph, solvate, N-oxide, isotopically labeled compound, metabolite, chelate, complex, clathrate, or prodrug thereof, and a pharmaceutically acceptable carrier, said pharmaceutical compositionis preferably in a solid, semi-solid, liquid, or gaseous formulation.

The present invention provides a use of the compound of the invention, or a pharmaceutically acceptable salt, ester, optical isomer, stereoisomer, polymorph, solvate, N-oxide, isotopically-labeled compound, metabolite, chelate, complex, clathrate, or prodrug thereof, or a pharmaceutical composition, in the manufacture of a medicament for the treatment of a SHP2 phosphatase modulated disease.

The SHP2 phosphatase modulated disease is a tumor, such as a solid tumor, a hematological tumor, a malignant tumor, a refractory tumor, a primary or metastatic recurrent tumor, and the like.

The SHP2 phosphatasemodulated disease is generally selected from noonan syndrome, leopard syndrome, juvenile myelomonocytic leukemia, neuroblastoma, melanoma, acute myelogenous leukemia, breast cancer, esophageal cancer, lung cancer, colon cancer, brain cancer, neuroblastoma, squamous carcinoma of the head and neck, gastric cancer, anaplastic largecell lymphoma and glioblastoma, but is not limited to these.

The present invention provides a method of treatment of a SHP2 phosphatase modulated disease, comprising administering to a human in need of such treatment an effective amount of a compound of the invention, or a pharmaceutically acceptable salt, ester, optical isomer, stereoisomer, polymorph, solvate, N-oxide, isotopically labeled compound, metabolite, chelate, complex, clathrate, or prodrug thereof, or a pharmaceutical composition of the invention.

The target disease of the method of treatment of the present invention is selected from advanced solid tumors, including primary or metastatic recurrent NSCLC, squamous cell lung carcinoma, adenocarcinoma of lung, squamous carcinoma of the head and neck, gastric cancer, colorectal cancer, pancreatic cancer, and the like; also included are breast cancer, esophageal cancer, lung cancer, colon cancer, brain cancer, neuroblastoma, neuroblastoma, melanoma, squamous carcinoma of the head and neck, anaplastic large cell lymphoma and glioblastoma; malignant hematological tumor diseases include juvenile myelomonocytic leukemia, acute myelogenous leukemia; other diseases related to SHP2 abnormal expression, such as Noonan syndrome, leopard syndrome, type II diabetes, obesity, and the like.

By "pharmaceutically acceptable carrier" herein is meant a diluent, adjuvant, excipient, or vehicle that is administered with the therapeutic agent and which is, within the scope of sound medical judgment, suitable for contact with the tissues of humans and/or other animals without excessive toxicity, irritation, allergic response, or other problem or complication commensurate with a reasonable benefit/risk ratio.

Pharmaceutically acceptable carrier that can be used in the pharmaceutical compositions of the invention include, but are not limited to, sterile liquids, such as water and oils, including those of petroleum, animal, vegetable or synthetic origin, such as peanut oil, soybean oil, mineral oil, sesame oil and the like. Water is an exemplary carrier when the pharmaceutical composition is administered intravenously. Physiological brine and aqueous dextrose and glycerol solutions can also be employed as liquid carriers, particularly for injectable solutions. Suitable pharmaceutical excipients include starch, glucose, lactose, sucrose, gelatin, maltose, chalk, silica gel, sodium stearate, glycerol monostearate, talc, sodium chloride, dried skim milk, glycerol, propylene glycol, water, ethanol and the like. The composition may also optionally contain minor amounts of wetting agents, emulsifying agents or pH buffering agents. Oral formulations may contain standard carriers such as pharmaceutical grades of mannitol, lactose, starch, magnesium stearate, sodium saccharine, cellulose, magnesium carbonate, and the like. Examples of suitable pharmaceutically acceptable carriers are described in Remington's Pharmaceutical Sciences (1990).

The pharmaceutical compositions of the present invention may act systemically and/or locally. For this purpose, they may be administered by a suitable route, for example by injection (e.g. intravenous, intra-arterial, subcutaneous, intraperitoneal, intramuscular injection, including drip) or transdermally; or by oral, buccal, nasal, transmucosal, topical, in the form of ophthalmic preparations or by inhalation.

For these routes of administration, the pharmaceutical compositions of the present invention may be administered in suitable dosage forms.

Such dosage forms include, but are not limited to, tablets, capsules, lozenges, hard candies, powders, sprays, creams, ointments, suppositories, gels, pastes, lotions, ointments, aqueous suspensions, injectable solutions, elixirs, syrups.

The term "effective amount" as used herein refers to an amount of a compound that, when administered, will alleviate one or more symptoms of the condition being treated to some extent.

The dosing regimen may be adjusted to provide the best desired response. For example, a single bolus may be administered, several divided doses may be administered over time, or the dose may be proportionally reduced or increased as indicated by the exigencies of the therapeutic situation. It is noted that dosage values may vary with the type and severity of the condition being alleviated, and may include single or multiple doses. It is further understood that for any particular individual, the specific dosage regimen will be adjusted over time according to the individual need and the professional judgment of the person administering the composition or supervising the administration of the composition.

The amount of a compound of the invention administered will depend on the subject being treated, the severity of the disorder or condition, the rate of administration, the disposition of the compound, and the judgment of the prescribing physician. Generally, an effective dose is from about 0.0001 to about 50 mg per kg body weight per day, e.g., from about 0.01 to about 10 mg/kg/day (single or divided administration). For a 70kg person, this may amount to about 0.007 mg/day to about 3500 mg/day, e.g., about 0.7 mg/day to about 700 mg/day. In some cases, dosage levels no higher than the lower limit of the aforesaid range may be sufficient, while in other cases still larger doses may be employed without causing any harmful side effects, provided that the larger dose is first divided into several smaller doses for administration throughout the day.

The amount or dosage of the compound of the invention in a pharmaceutical composition may be from about 0.01 mg to about 1000 mg, suitably 0.1 to 500 mg, preferably 0.5 to 300 mg, more preferably 1 to 150 mg, especially 1 to 50 mg, for example 1.5 mg, 2 mg, 4 mg, 10 mg, 25 mg etc.

As used herein, unless otherwise specified, the term "treating" means reversing, alleviating, inhibiting the progression of, or preventing such a disorder or condition, or one or more symptoms of such a disorder or condition, to which such term applies.

As used herein, "individual" includes a human or non-human animal. Exemplary human individuals include human individuals (referred to as patients) with a disease (e.g., a disease described herein) or normal individuals. "non-human animals" in the context of the present invention include all vertebrates, such as non-mammals (e.g., birds, amphibians, reptiles) and mammals, such as non-human primates, livestock and/or domesticated animals (e.g., sheep, dogs, cats, cows, pigs, etc.).

In some embodiments, the pharmaceutical compositions of the present invention may further comprise one or more additional therapeutic or prophylactic agents.

### Detailed Description of Embodiments

The present invention is described in detail below by way of examples, but is not meant to be limited in any way. Having described the invention in detail and having disclosed specific embodiments thereof, it will be apparent to those skilled in the art that various changes and modifications can be made therein without departing from the spirit and scope thereof.

### Examples

These examples are not provided to limit the scope of the present invention.

The structure of the compound was confirmed by nuclear magnetic resonance spectroscopy (¹H NMR) and/or Mass Spectrometry (MS).

Chemical shifts (δ) are given in parts per million (ppm). ¹H NMR were measured on a Bruker 400 or Varian 300 nuclear magnetic instrument using deuterated methanol (CD₃OD), deuterated chloroform (CDCl₃) or hexadeuterated dimethyl sulfoxide (DMSO-d₆) as the test solvent and Tetramethylsilane (TMS) as the internal standard.

The LC-MS measurements were performed on Agilent LC-MS-1110 LC-MS, Agilent LC-MS-6110 LC-MS, Agilent LC-MS-6120 LC-MS (manufacturer: Agilent) or Shimadzu LC-MS 2020.

Preparative high performance liquid chromatography was performed using an MS triggered automatic purification system (Waters), Gilson GX-281(Gilson) or semi-preparative liquid chromatography (Chuangxintongheng LC3000(Ddlsogel, C18, 30mm × 250mm 10 µm)).

The Thin Layer Chromatography (TLC) is carried out by using HUANGHAI HSGF 254(5×20cm) silica gel plate, and the preparative thin layer chromatography is carried out by using a Yantai made GF 254 (0.4-0.5 nm) silica gel plate.

The reaction is detected by Thin Layer Chromatography (TLC) or LC-MS, and the developing agent system used comprises dichloromethane and methanol system, n-hexane and ethyl acetate system, petroleum ether and ethyl acetate system, and is adjusted according to the polarity of the compound to be separated (by adjusting the volume ratio of the solvent or adding triethylamine and the like).

The microwave reaction was carried out using a CEM Discovery Sp (400W, RT^{∼}300°C) microwave reactor.

Yucheng Chemical 200-300 mesh silica gel is generally used as the stationary phase of the column chromatography. The system of the eluent comprises a dichloromethane and methanol system and a normal hexane and ethyl acetate system, and the eluent system is adjusted according to the polarity of the compound to be separated (by adjusting the volume ratio of the solvent or adding triethylamine and the like).

The reaction temperature is room temperature (20°C-30°C), unless otherwise specified in the examples.

The reagents used in the examples were purchased from Aldrich Chemical Company, Shanghai Bidepharm technology Co., Ltd., Beijing Greenchem Co., Ltd., Accela ChemBio (Shanghai) Co., Ltd., or IBO technology Co., Ltd., etc.

### Synthesis example 1

### Synthesis of Compound 3

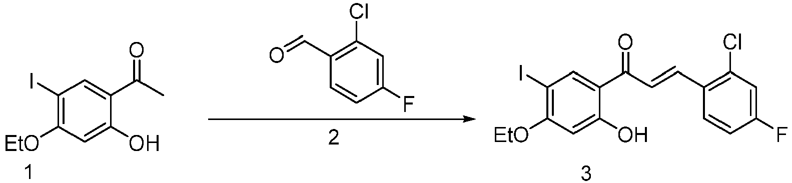

7 g of compound 1 and 3.6 g of compound 2 were added into 150 mL THF and stirred for 10 min, 5.12 g potassium tert-butoxide was added at room temperature, stirred for 30 min and slowly raised to 60°C for 1-10 h, the product was detected by TLC rf =0.4 (DCM:MeOH=15:1), and the reaction was stopped by adding water. Extracted, dried and spin-dried to obtain 16 g of the product and was directly used for the next step. MS: [M+H]=447; ¹H NMR (400 MHz, CDCl3) δ 13.27 (s, 1H), 8.25-8.19 (m, 2H), 7.80 (dd, J = 8.8, 5.9 Hz, 1H), 7.43 (d, *J* = 15.4 Hz, 1H), 7.22 (dd, *J* = 8.4, 2.6 Hz, 1H), 7.09 (td, *J* = 8.2, 2.6 Hz, 1H), 6.43 (s, 1H), 4.15 (q, *J* = 7.0 Hz, 2H), 1.52 (t, J = 7.0 Hz, 3H).

### Synthesis example 2

### Synthesis of Compound 4

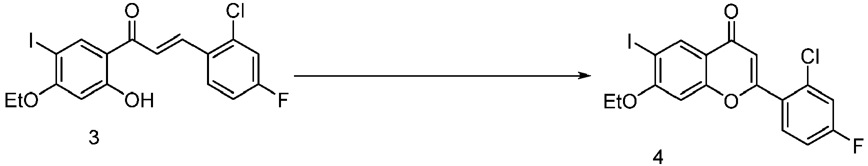

16 g of compound 3 was added into 500 ml DMSO, stirred to dissolve, then added with 2.8 g of iodine, heated to 150°C and reacted for 1-10 h, the completion of the reaction was monitored by LCMS and cooled to room temperature, 500 ml of saturated brine was added. Extracted with ethyl acetate, dried over NaSO₄ and spin-dried. The product was detected by TLC rf=0.5 (DCM:MeOH=10:1), column chromatography gave 9g of compound 4 ( total yield of the two steps 89%, purity 94%).

MS: [M+H]=445; ¹H NMR (400 MHz, CDCl3) δ 8.65 (s, 1H), 7.62 (dd, *J* = 8.7, 5.9 Hz, 1H), 7.29 (dd, *J* = 8.4, 2.5 Hz, 1H), 7.18-7.09 (m, 1H), 6.80 (s, 1H), 6.57 (s, 1H), 4.18 (q, *J* = 7.0 Hz, 2H), 1.56 (t, *J* = 7.0 Hz, 3H).

### Synthesis example 3

### Synthesis of Compound 6

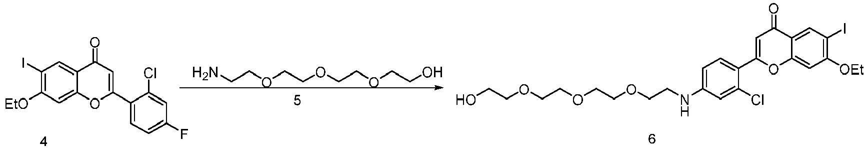

5 g of compound 4 was added into 108 g of compound 5 and stirred, heated to 40-100°C and continued to react for 3-24 h, the completion of the reaction was monitored by LCMS and cooled to room temperature, added 150 ml of ethyl acetate to dissolve, extracted with 150 ml of saturated brine, dryied with NaSO₄, and spin-dried. Column chromatography gave 3.5 g of Compound 6 (yield 50%, purity 80%). MS: [M+H]=618

### Synthesis example 4

### Synthesis of Compound 7

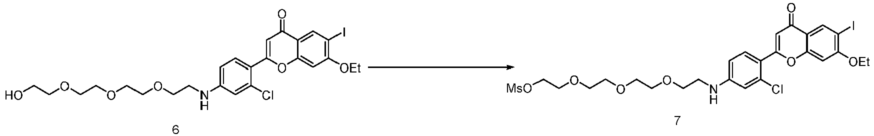

2 g of compound 6 and 350 mg of triethylamine were added into 30 ml of DCM and stirred, the temperature was reduced to 0°C, 443 mg of MsCI was added dropwise and continued to react for 1-12 h, the completion of the reaction was monitored by LCMS, and directly spin-dried. Column chromatography gave 1.2 g of Compound 7 (yield 70%, purity 90%). MS: [M+H]=696; ¹H NMR (400 MHz, CDCl3) δ 8.64 (s, 1H), 7.46 (d, *J* = 8.6 Hz, 1H), 6.81 (s, 1H), 6.75 (d, *J* = 2.3 Hz, 1H), 6.63 (d, *J* = 7.7 Hz, 2H), 5.30 (br, 1H), 4.41-4.35 (m, 2H), 4.19 (t, *J* = 7.0 Hz, 2H), 3.80- 3.76 (m, 2H), 3.73 (t, *J* = 5.1 Hz, 2H), 3.68 (d, *J* = 7.5 Hz, 8H), 3.36 (t, *J* = 5.1 Hz, 2H), 3.06 (s, 3H), 1.55 (t, *J* = 7.0 Hz, 3H).

### Synthesis example 5

### Synthesis of Compound 9

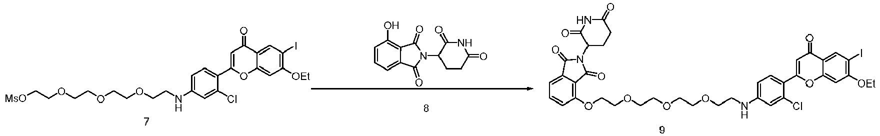

1.2 g of compound 7 and 475 mg of compound 8 were added into 10 ml of DMF and stirred, 174 mg of NaHCOs and 320 mg of KI were added at room temperature and the temperature was raised to 40-100°C and continued to react for 2-6 h, the completion of the reaction was monitored by LCMS and cooled to room temperature. Direct column chromatography gave 750 mg of Compound 9 (yield 57%, purity 93%). MS: [M+H]=873.

### Synthesis example 6

### Synthesis of Compound 11

### (1) Synthesis of Compound 11-11A

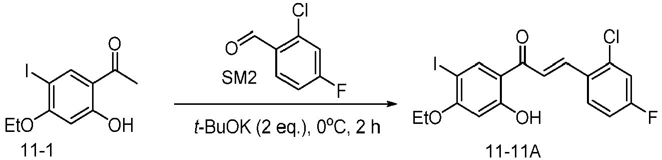

11-1(7 g, 1 eq.) and SM2(7.2 g, 2.0 eq.) were added into 150 mL THF and stirred for 10 min, then added t-BuOK (5.12 g, 2 eq.) at 0 deg.C and stirred for 30 min, slowly raised to room temperature and continued the reaction for 2 h, 11-1 disappeared on spot plate, the product was detected by TLC rf=0.4 (DCM:MeOH=25:1), and added water to stop the reaction. Extracted with ethyl acetate, dryied with NaSO₄, and spin-dried to obtain 16 g of crude product, which was directly used in the next step. MS: [M+H]=447

¹H NMR (400 MHz, CDCl3) δ 13.27 (s, 1H), 8.25-8.19 (m, 2H), 7.80 (dd, *J* = 8.8, 5.9 Hz, 1H), 7.43 (d, *J* = 15.4 Hz, 1H), 7.22 (dd, *J* = 8.4, 2.6 Hz, 1H), 7.09 (td, *J* = 8.2, 2.6 Hz, 1H), 6.43 (s, 1H), 4.15 (q, *J* = 7.0 Hz, 2H), 1.52 (t, *J* = 7.0 Hz, 3H).

### (2) Synthesis of Compound 11-12

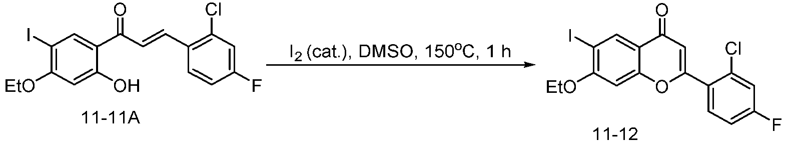

11-11A (16 g, 1 eq, crude product) was added into 100 ml DMSO and stirred to dissolve, then added I₂ (280 mg, 0.05 eq), heated to 150°C and continued to react for 1.5 h, the completion of the reaction was monitored by LCMS and cooled to room temperature, added 700 ml of saturated brine. Extracted with ethyl acetate, dried over NaSO₄ and spin-dried. The product was detected by TLC rf=0.5 (DCM:MeOH=30:1) and passed through normal phase Flash (column 330 g, DCM:MeOH=20:1) to obtain 9g (total yield of the two steps 89%, purity 94%).
MS: [M+H]=445,
¹H NMR (400 MHz, CDCl3) δ 8.65 (s, 1H), 7.62 (dd, *J* = 8.7, 5.9 Hz, 1H), 7.29 (dd, *J* = 8.4, 2.5 Hz, 1H), 7.18-7.09 (m, 1H), 6.80 (s, 1H), 6.57 (s, 1H), 4.18 (q, *J* = 7.0 Hz, 2H), 1.56 (t, *J* = 7.0 Hz, 3H).

### (3) Synthesis of Compound 11-14

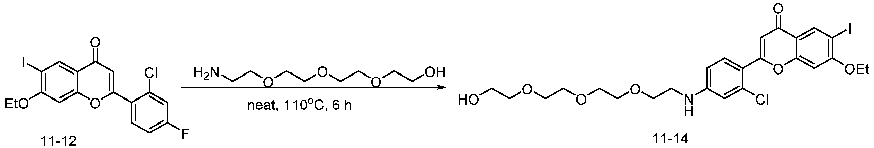

11-12 (5 g, 1 eq.) was added into 2- (2- (2- (2-aminoethoxy)ethoxy)ethoxy) ethane-1-ol (10.8 g, 5.0 eq.) and stirred, raised the temperature to 110°C and continued the reaction for 6 h, the completion of the reaction was monitored by LCMS and cooled to room temperature, added ethyl acetate (150 ml) to dissolve, added 150 ml saturated brine and 5 ml concentrated hydrochloric acid. Extracted, dryied with NaSO₄, and spin-dried. Reversed-phase Flash (column 330g, ACN/0.05% FA in water) gave 3.5 g (yield 50%, purity 80%). MS: [M+H]=618

### (4) Synthesis of Compound 11-15

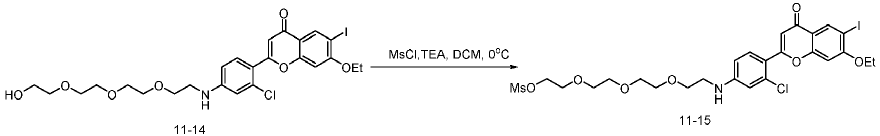

11-14 (2 g, 1 eq.) and triethylamine (650 mg, 2.0 eq.) were added into DCM (30 ml) and stirred, cooled to 0°C and added MsCI (443 mg,1.2 eq.) dropwise to continue the reaction for 2 h, the completion of the reaction was monitored by LCMS and was directly spin-dried. Normal phase Flash (column 330g, DCM:EA=3:1) gave 1.2 g (yield 70%, purity 90%). MS: [M+H]=696.

¹H NMR (400 MHz, CDCl3) δ 8.64 (s, 1H), 7.46 (d, *J* = 8.6 Hz, 1H), 6.81 (s, 1H), 6.75 (d, *J* = 2.3 Hz, 1H), 6.63 (d, *J* = 7.7 Hz, 2H), 5.30 (br, 1H), 4.41-4.35 (m, 2H), 4.19 (t, *J* = 7.0 Hz, 2H), 3.80- 3.76 (m, 2H), 3.73 (t, *J* = 5.1 Hz, 2H), 3.68 (d, *J* = 7.5 Hz, 8H), 3.36 (t, *J* = 5.1 Hz, 2H), 3.06 (s, 3H), 1.55 (t, *J* = 7.0 Hz, 3H).

### (5) Synthesis of Compound 11-16

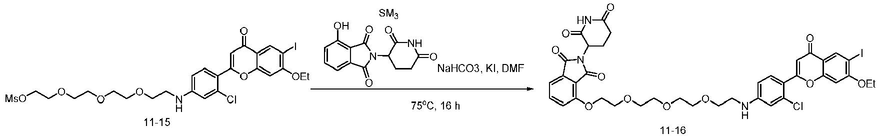

11-15 (1.2 g,1 eq.), SM3(570 mg,1.2 eq.) were added into DMF (10 ml) and stirred, NaHCO₃(290 mg, 2eq.) and KI (320 mg,1.2 eq.) were added at room temperature, raised the temperature to 75°C and continued the reaction for 16 h, the completion of the reaction was monitored by LCMS and cooled to room temperature. Reversed-phase Flash (column C18, 330 g, ACN/0.05% FA in water) gave 750 mg (yield 57%, purity 93%). MS: [M+H]=873.

### (6) Synthesis of Compound 11

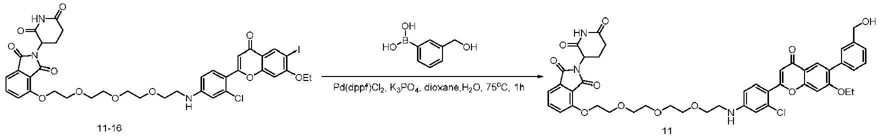

11-16(100 mg, 1 eq.), 3-hydroxymethylphenylboronic acid (53 mg,3 eq.) and K₃PO₄ (72.8 mg, 3.0 eq.) were added into 1,4-dioxane (3 ml) and water (1 ml) with stirring, Pd (dppf)Cl₂ (8.3 mg, 0.1 eq.) was added under N₂ atmosphere, raised the temperature to 75°C and continued the reaction for 1 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 35 mg off-white solid (yield 36%, purity 94%). MS: [M+H]=854.31

¹H NMR (400 MHz, DMSO-d6) δ 11.10 (s, 1H), 7.87 (s, 1H), 7.79 (dd, J = 8.5, 7.3 Hz, 1H), 7.56 (d, J = 8.7 Hz, 1H), 7.52 (s, 1H), 7.50 (d, J = 2.3 Hz, 1H), 7.45-7.42 (m, 2H), 7.39 (t, J = 7.5 Hz, 1H), 7.32 (d, J = 7.4 Hz, 1H), 7.27 (s, 1H), 6.79 (d, J = 2.3 Hz, 1H), 6.70 (dd, J = 8.7, 2.3 Hz, 1H), 6.65 (t, J = 5.6 Hz, 1H), 6.50 (s, 1H), 5.23 (t, J = 5.8 Hz, 1H), 5.08 (dd, J = 12.9, 5.4 Hz, 1H), 4.56 (d, J = 5.8 Hz, 2H), 4.33 (s, 2H), 4.21 (q, J = 6.9 Hz, 2H), 3.80 (t, J = 4.6 Hz, 2H), 3.65 (dd, J = 5.9, 3.6 Hz, 2H), 3.60-3.53 (m, 8H), 3.28 (q, J = 5.4 Hz, 2H), 2.88 (ddd, J = 17.7, 14.0, 5.3 Hz, 1H), 2.71- 2.52(m, 2H), 2.08-1.95 (m, 1H), 1.33 (t, J = 6.9 Hz, 3H).

### Synthesis example 7

### Synthesis of Compound 12

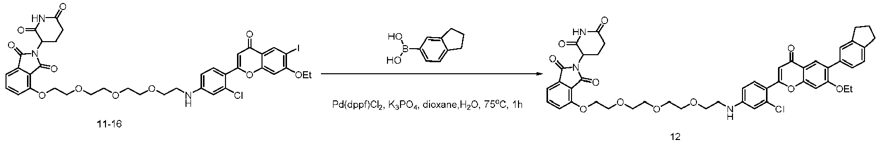

11-16(100 mg, 1 eq.), 2, 3-dihydro-1H-indene-5-boronic acid (55.6 mg,3 eq.) and K₃PO₄ (72.8 mg, 3.0 eq.) were added into 1,4-dioxane (3 ml) and water (1 ml) with stirring, Pd (dppf)Cl₂ (8.3 mg, 0.1 eq.) was added under N₂ atmosphere, raised the temperature to 75°C and continued the reaction for 1 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 46.28 mg off-white solid (yield 46.8%, purity 93%). MS: [M+H]=864.35.

¹H NMR (400 MHz, DMSO-*d*₆) δ 11.10 (s, 1H), 7.82 (s, 1H), 7.79 (dd, *J* = 8.5, 7.3 Hz, 1H), 7.55 (d, *J* = 8.7 Hz, 1H), 7.51 (d, *J* = 8.5 Hz, 1H), 7.44 (d, *J* = 7.2 Hz, 1H), 7.38 (s, 1H), 7.29 (d, *J* = 1.8 Hz, 2H), 7.24 (s, 1H), 6.79 (d, *J* = 2.2 Hz, 1H), 6.70 (dd, *J* = 8.7, 2.3 Hz, 1H), 6.65 (t, *J* = 5.6 Hz, 1H), 6.49 (s, 1H), 5.08 (dd, *J* = 12.8, 5.4 Hz, 1H), 4.33 (t, *J* = 4.6 Hz, 2H), 4.20 (q, *J* = 6.9 Hz, 2H), 3.80 (t, *J* = 4.5 Hz, 2H), 3.65 (dd, J = 5.9, 3.6 Hz, 2H), 3.60- 3.54 (m, 8H), 3.30-3.26 (m, 2H), 2.91 (td, *J* = 7.3, 4.8 Hz, 5H), 2.58 (d, J = 17.7 Hz, 2H), 2.06 (p, *J* = 7.5 Hz, 3H), 1.32 (t, *J* = 7.0 Hz, 3H).

### Synthesis example 8

### Synthesis of Compound 13

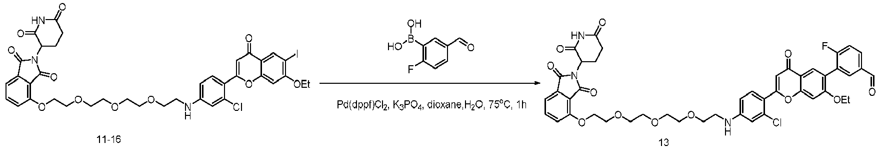

11-16(100 mg, 1 eq.), 2-fluoro-5-aldehyde phenylboronic acid (57.6 mg,3 eq.) and K₃PO₄ (72.8 mg, 3.0 eq.) were added into 1,4-dioxane (3 ml) and water (1 ml) with stirring, Pd (dppf)Cl₂ (8.3 mg, 0.1 eq.) was added under N₂ atmosphere, raised the temperature to 75°C and continued the reaction for 1 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 75.92 mg off-white solid (yield 76%, purity 93%). MS: [M+H]=870.28.

¹H NMR (400 MHz, DMSO-*d*₆) δ 11.10 (s, 1H), 10.04 (s, 1H), 8.04 (d, *J* = 6.3 Hz, 1H), 7.92 (s, 1H), 7.83-7.75 (m, 1H), 7.59-7.54 (m, 2H), 7.51 (d, *J* = 8.6 Hz, 2H), 7.44 (d, *J* = 7.2 Hz, 1H), 7.34 (s, 1H), 6.80 (d, *J* = 2.3 Hz, 1H), 6.75- 6.62 (m, 2H), 6.53 (s, 1H), 5.08 (dd, *J =* 12.8, 5.4 Hz, 1H), 4.33 (t, *J* = 4.6 Hz, 2H), 4.22 (q, *J* = 7.0 Hz, 2H), 3.80 (t, *J* = 4.5 Hz, 2H), 3.65 (dd, *J* = 5.9, 3.5 Hz, 2H), 3.58- 3.54 (m, 8H), 3.28 (d, *J* = 5.7 Hz, 2H), 2.93-2.86 (m, 1H), 2.69-2.52 (m, 2H), 2.02-1.95 (m, 1H),1.26 (s, 3H).

### Synthesis example 9

### Synthesis of Compound 14

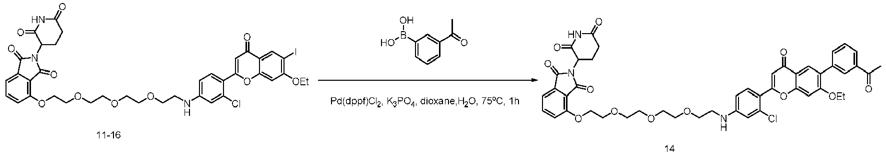

11-16(100 mg, 1 eq.), 3-acetylphenylboronic acid (56.3 mg, 3 eq.) and K₃PO₄ (72.8 mg, 3.0 eq.) were added into 1,4-dioxane (3 ml) and water (1 ml) with stirring, Pd(dppf)Cl₂ (8.3 mg, 0.1 eq.) was added under N₂ atmosphere, raised the temperature to 75°C and continued the reaction for 1 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 31.76 mg off-white solid (yield 32%, purity 92%). MS: [M+H]=866.32.

¹H NMR (400 MHz, DMSO-*d*₆) δ 11.10 (s, 1H), 8.15 (d, *J* = 1.8 Hz, 1H), 7.99-7.95 (m, 1H), 7.93 (s, 1H), 7.82 (dd, *J* = 7.6, 5.8 Hz, 1H), 7.80-7.76 (m, 1H), 7.61 (t, *J* = 7.7 Hz, 1H), 7.57 (d, *J* = 8.6 Hz, 1H), 7.51 (dd, *J* = 8.0, 4.1 Hz, 1H), 7.44 (d, *J* = 7.2 Hz, 1H), 7.31 (s, 1H), 6.80 (d, *J* = 2.2 Hz, 1H), 6.71 (dd, *J* = 8.8, 2.3 Hz, 1H), 6.66 (t, *J* = 5.7 Hz, 1H), 6.52 (s, 1H), 5.08 (dd, *J* = 12.8, 5.3 Hz, 1H), 4.37- 4.30 (m, 2H), 4.23 (q, *J* = 6.9 Hz, 2H), 3.80 (t, *J* = 4.6 Hz, 2H), 3.65 (dd, *J* = 5.9, 3.6 Hz, 2H), 3.59-3.54 (m, 8H), 3.28 (d, *J* = 5.6 Hz, 2H), 2.88 (ddd, *J* = 17.4, 13.9, 5.3 Hz, 1H), 2.64 (s, 3H), 2.61 -2.54 (m, 2H), 2.01 (s, 1H), 1.33 (t, *J* = 6.9 Hz, 3H).

### Synthesis example 10

### Synthesis of Compound 15

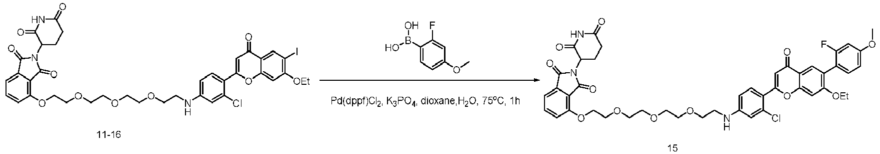

11-16(100 mg, 1 eq.), 2-fluoro-4-methoxyphenylboronic acid (58.3 mg,3 eq.) and K₃PO₄ (72.8 mg, 3.0 eq.) were added into 1,4-dioxane (3 ml) and water (1 ml) with stirring, Pd(dppf)Cl₂ (8.3 mg, 0.1 eq.) was added under N₂ atmosphere, raised the temperature to 75°C and continued the reaction for 1 h, the completion of the reaction was monitored by LCMS and cooled to room temperature. The reaction flask was charged with anhydrous sodium sulfate directly to remove water, filtered, spin-dried, and passed through pre-TLC (DCM:MeOH=20:1) to obtain 39.04 mg off-white solid (yield 39%, purity 93%). MS: [M+H]=872.30

¹H NMR (400 MHz, DMSO-*d*₆) δ 11.10 (s, 1H), 7.81-7.76 (m, 2H), 7.56 (d, *J* = 8.6 Hz, 1H), 7.51 (d, *J* = 8.5 Hz, 1H), 7.44 (d, *J* = 7.2 Hz, 1H), 7.35 (t, *J* = 8.6 Hz, 1H), 7.26 (s, 1H), 6.91 (dd, *J* = 12.1, 2.5 Hz, 1H), 6.87 (dd, *J* = 8.5, 2.6 Hz, 1H), 6.79 (d, *J* = 2.2 Hz, 1H), 6.70 (dd, *J* = 8.7, 2.3 Hz, 1H), 6.65 (t, *J* = 5.6 Hz, 1H), 6.50 (s, 1H), 5.08 (dd, *J* = 12.9, 5.4 Hz, 1H), 4.33 (t, *J* = 4.6 Hz, 2H), 4.19 (q, *J* = 6.9 Hz, 2H), 3.83 (s, 3H), 3.80 (dd, *J* = 5.6, 3.5 Hz, 2H), 3.65 (dd, *J* = 5.9, 3.6 Hz, 2H), 3.58-3.53(m,8H), 3.30-3.26 (m, 2H), 2.88 (ddd, *J* = 17.2, 14.0, 5.4 Hz, 1H), 2.68-2.51 (m, 2H), 2.06- 1.95 (m, 1H), 1.27 (t, *J* = 6.9 Hz, 3H).

### Synthesis example 11

### Synthesis of Compound 16

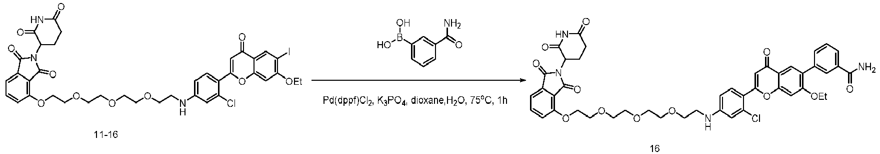

11-16(100 mg, 1 eq.), 3-carbamyl phenylboronic acid (56.6 mg,3 eq.) and K₃PO₄ (72.8 mg, 3.0 eq.) were added into 1,4-dioxane (3 ml) and water (1 ml) with stirring, Pd(dppf)Cl₂ (8.3 mg, 0.1 eq.) was added under N₂ atmosphere, raised the temperature to 75°C and continued the reaction for 1 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 58.13 mg off-white solid (yield 58%, purity 93%). MS: [M+H]=867.31.

¹H NMR (400 MHz, DMSO-*d*₆) δ 11.10 (s, 1H), 8.11-8.04 (m, 2H), 7.94 (s, 1H), 7.88 (dt, *J* = 7.8, 1.5 Hz, 1H), 7.79 (dd, *J* = 8.5, 7.2 Hz, 1H), 7.74-7.71 (m, 1H), 7.57 (d, *J* = 8.7 Hz, 1H), 7.52 (t, *J* = 7.8 Hz, 2H), 7.44 (d, *J* = 7.2 Hz, 1H), 7.39 (s, 1H), 7.30 (s, 1H), 6.80 (d, J *=* 2.3 Hz, 1H), 6.71 (dd, *J* = 8.8, 2.3 Hz, 1H), 6.66 (t, *J* = 5.6 Hz, 1H), 6.52 (s, 1H), 5.08 (dd, *J* = 12.8, 5.4 Hz, 1H), 4.33 (dd, *J* = 5.7, 3.4 Hz, 2H), 4.22 (q, *J* = 6.9 Hz, 2H), 3.80 (dd, *J* = 5.4, 3.5 Hz, 2H), 3.65 (dd, *J* = 5.9, 3.6 Hz, 2H), 3.58-3.53(m,8H), 3.28 (d, *J* = 5.6 Hz, 2H), 2.88 (ddd, *J* = 17.3, 14.0, 5.3 Hz, 1H), 2.57 (dd, *J* = 16.1, 11.8 Hz, 2H), 2.07- 1.96 (m, 1H), 1.32 (t, *J* = 6.9 Hz, 3H).

### Synthesis example 12

### Synthesis of Compound 17

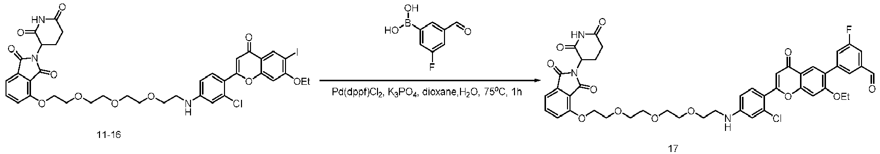

11-16(100 mg, 1 eq.), 3-fluoro-5-formylphenylboronic acid (57.6 mg,3 eq.) and K₃PO₄ (72.8 mg, 3.0 eq.) were added into 1,4-dioxane (3 ml) and water (1 ml) with stirring, Pd(dppf)Cl₂ (8.3 mg, 0.1 eq.) was added under N₂ atmosphere, raised the temperature to 75°C and continued the reaction for 1 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 70.95 mg off-white solid (yield 71%, purity 93%). MS: [M+H]=870.28.

¹H NMR (400 MHz, DMSO-*d*₆) δ 11.15 (s, 1H), 10.08 (s, 1H), 8.00 (d, *J* = 3.8 Hz, 2H), 7.84-7.76 (m, 2H), 7.70 (d, *J* = 8.4 Hz, 1H), 7.57 (d, *J* = 8.4 Hz, 1H), 7.51 (d, *J* = 8.7 Hz, 1H), 7.44 (d, *J* = 7.1 Hz, 1H), 7.33 (s, 1H), 6.80 (s, 1H), 6.73- 6.64 (m, 2H), 6.53 (s, 1H), 5.08 (s, 1H), 4.33 (s, 2H), 4.25 (d, *J* = 7.1 Hz, 2H), 3.81 (s, 2H), 3.65 (s, 2H), 3.58-3.53(m,8H), 3.28 (s, 2H), 2.88 (ddd, *J* = 17.3, 14.0, 5.3 Hz, 1H), 2.58 (d, *J* = 17.3 Hz, 2H), 2.02 (s, 1H), 1.34 (t, *J* = 7.0 Hz, 3H).

### Synthesis example 13

### Synthesis of Compound 18

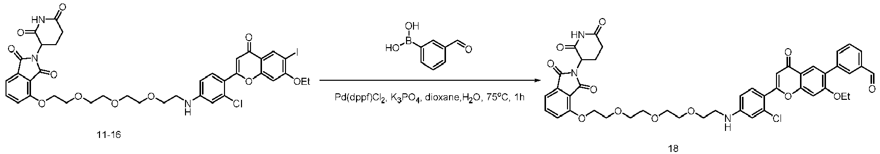

11-16(100 mg, 1 eq.), 3-formylphenylboronic acid (51.4 mg,3 eq.) and K₃PO₄ (72.8 mg, 3.0 eq.) were added into 1,4-dioxane (3 ml) and water (1 ml) with stirring, Pd(dppf)Cl₂ (8.3 mg, 0.1 eq.) was added under N₂ atmosphere, raised the temperature to 75°C and continued the reaction for 16 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 64.73 mg off-white solid (yield 66%, purity 93%). MS: [M+H]=852.29.

¹H NMR (400 MHz, DMSO-*d*₆) δ 11.10 (s, 1H), 10.09 (s, 1H), 8.11 (t, *J* = 1.8 Hz, 1H), 7.95 (s, 1H), 7.93 (ddd, *J* = 7.8, 3.0, 1.5 Hz, 2H), 7.79 (dd, *J* = 8.5, 7.2 Hz, 1H), 7.69 (t, J = 7.7 Hz, 1H), 7.58 (s, 1H), 7.51 (d, *J* = 8.5 Hz, 1H), 7.44 (d, *J* = 7.2 Hz, 1H), 7.32 (s, 1H), 6.80 (d, J = 2.3 Hz, 1H), 6.71 (dd, *J* = 8.7, 2.3 Hz, 1H), 6.67 (t, *J* = 5.6 Hz, 1H), 6.53 (s, 1H), 5.08 (dd, *J* = 12.9, 5.4 Hz, 1H), 4.33 (t, *J* = 4.6 Hz, 2H), 4.24 (q, *J* = 6.8 Hz, 2H), 3.80 (dd, *J* = 5.3, 3.6 Hz, 2H), 3.65 (dd, *J* = 6.0, 3.6 Hz, 2H), 3.60-3.53 (m, 8H), 3.28 (q, *J* = 5.4 Hz, 2H), 2.88 (ddd, *J* = 17.1, 14.0, 5.3 Hz, 1H), 2.57 (dd, *J* = 15.9, 12.0 Hz, 2H), 2.02 (dq, *J* = 11.3, 6.2, 4.5 Hz, 1H), 1.33 (t, *J* = 6.9 Hz, 3H).

### Synthesis example 14

### Synthesis of Compound 20

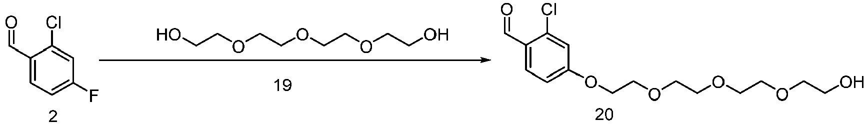

3g of compound 2 was added into 184g of compound 19, stirred for 2-50 minutes, 1.6g of NaHCOs solid was added at room temperature, stirred for 30 minutes and slowly raised to 40-100°C to continue the reaction for 2-24 h, the disappearance of the raw materials was monitored by LCMS, the product was detected with TLC rf=0.5 (DCM:MeOH=15:1), and water was added into stop the reaction. Extracted with ethyl acetate, dried over NaSO₄, spin-dried, and column chromatography gave 4.5 g light yellow oil compound 20. MS: [M+H]=333

### Synthesis example 15

### Synthesis of Compound 21

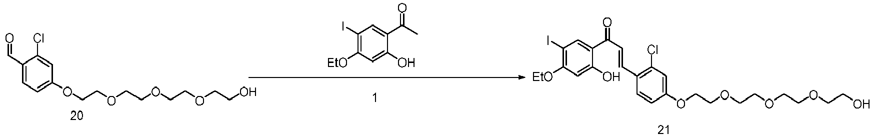

2.71g compound 20, 2.5 g compound 1 were added into 100 ml THF and 50 ml MeOH respectively and stirred to dissolve at the temperature of 0-30°C, then 1.35 g potassium tert-butoxide was added, stirred for 5 min and slowly raised to 50-90°C and continued the reaction for 1-12 h, the completion of the reaction was monitored by LCMS, added 700 ml saturated brine. Extracted with ethyl acetate, dried over NaSO₄, and spin-dried to obtain 6g of crude compound 21 and was directly used for the next step. MS: [M+H]=621.

### Synthesis example 16

### Synthesis of Compound 22

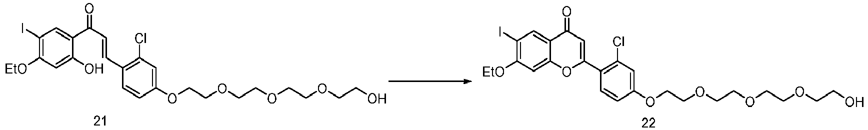

6 g of crude compound 21 was dissolved in 50 ml of DMSO and stirred, then added 1.0 g of elemental iodine, heated to 40-150°C to continue the reaction for 2-12 h, the completion of the reaction was monitored by LCMS and cooled to room temperature, added ethyl acetate (150 ml) to dissolve, and 150 ml of saturated brine. Extracted, dried over NaSO₄, and spin-dried. Column chromatography gave 4g of compound 22 (total yield of the two steps 80%, purity 93%). MS: [M+H]=619.

### Synthesis example 17

### Synthesis of Compound 23

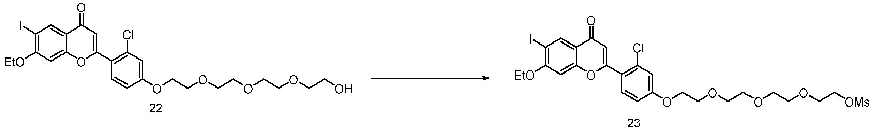

Added 2.8 g compound 22 and 549 mg triethylamine into 30 ml DCM and stirred, cooled to -20°C and added 1034 mg MsCI dropwise to continue the reaction for 2-12 h, the completion of the reaction was monitored by LCMS, and directly spin-dried. Column chromatography gave 2.5 g of compound 23 (yield 80%, purity 94%). MS: [M+H]=697.

### Synthesis example 18

### Synthesis of Compound 25

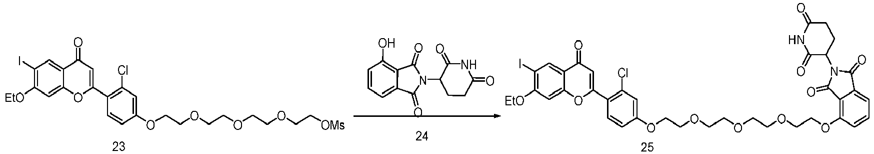

2.4 g of compound 23, 1.92g of compound 24 were stirred in 30 ml of DMF, 402 mg of NaHCOs and 1.28g of potassium iodide were added at 40°C, raised the temperature to 60-120°C to continue the reaction for 2-10 h, the completion of the reaction was monitored by LCMS and cooled to room temperature. Direct column chromatography gave 2.1g of compound 25 (yield 70%, purity 93%). MS: [M+H]=875.

### Synthetic example 19

### Synthesis of Compound 26

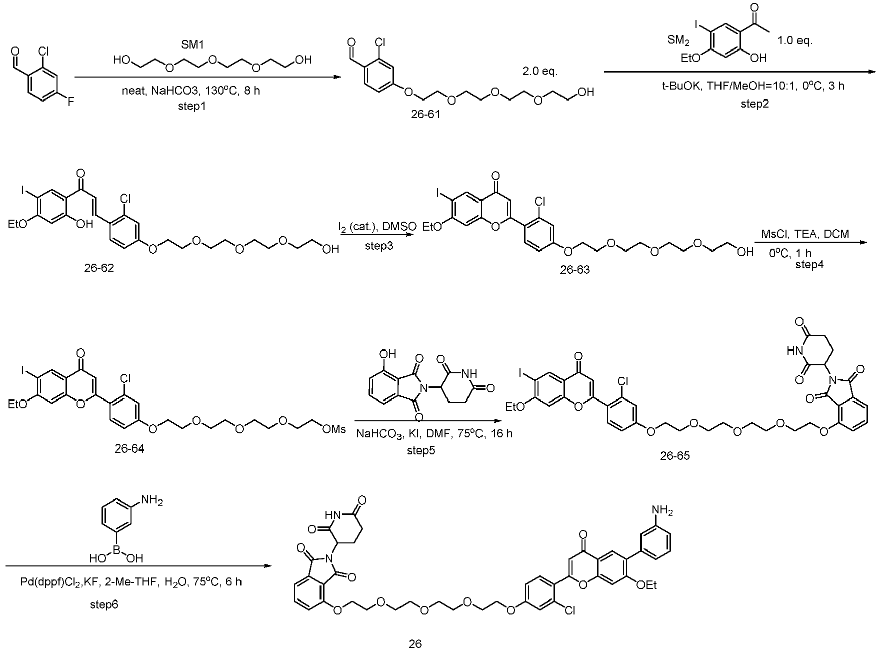

### Synthesis of 26-61

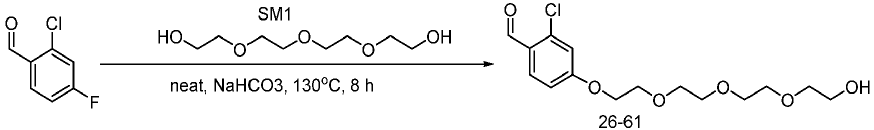

### (1) Synthesis of Compound 26-61

2-chloro-4-fluorobenzaldehyde (3 g, 1 eq.) was added into SM1(18.4 g, 5.0 eq.) and stirred for 2 minutes, added NaHCOs (3.2 g, 2 eq.) at room temperature, stirred for 30 minutes and slowly raised to 130°C to continue the reaction for 16 h, the disappearance of the raw materials was monitored by LCMS, the product was detected with TLC rf=0.5 (DCM:MeOH=25:1), and water was added into stop the reaction. Extracted with ethyl acetate, dried over NaSO₄, spin-dried, passed through normal phase Flash (column 330g, DCM:MeOH=20:1) gave 4.5 g light yellow oil. MS: [M+H]=333

### (2) Synthesis of Compound 26-62

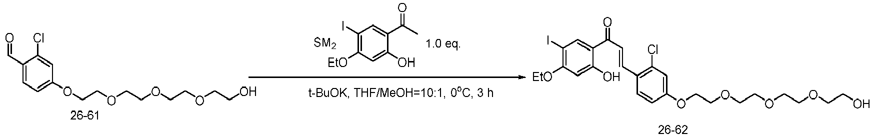

26-61 (5.42 g, 2 eq.) and SM2(2.5 g, 1 eq.) were added into THF (100 ml) and MeOH(10 ml) at 0°C under N₂ atmosphere, stirred to dissolve and added potassium tert-butoxide (3.6 g, 4 eq.), stirred for 30 minutes and slowly warmed to room temperature to continue the reaction for 1.5 h, the completion of the reaction was monitored by LCMS, added 700 ml of saturated brine. Extracted with ethyl acetate, dried over NaSO₄, spin-dried to obtain 6g of crude product and was directly used for the next step. MS: [M+H]=621.

### (3) Synthesis of Compound 26-63

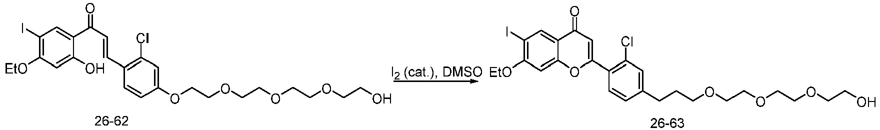

26-62 (6 g, crude product.) was dissolved in DMSO (50 ml) and stirred, and added elemental iodine (100 mg, 0.05 eq.), heated to 130°C to continue the reaction for 2 h, the completion of the reaction was monitored by LCMS and cooled to room temperature, added ethyl acetate (150 ml) to cdissolve, and added 150 ml of saturated brine. Extracted, dried over NaSO₄, and spin-dried. Reversed-phase Flash (column 330g, ACN/0.05% FA in water) gave 4g (total yield of the two steps 80%, purity 93%). MS: [M+H]=619.

### (4) Synthesis of Compounds 26-64

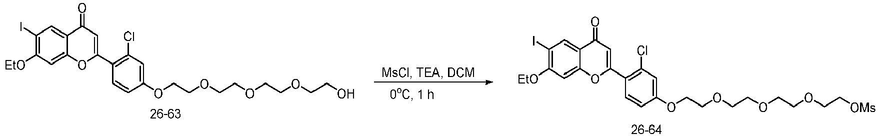

26-63 (2.8 g,1 eq.) and triethylamine (915 mg, 2.0 eq.) were added into DCM (30 ml) and stirred, cooled to 0°C and added MsCI (620 mg,1.2 eq.) dropwise to continue the reaction for 2 h, the completion of the reaction was monitored by LCMS and was directly spin-dried. Normal phase Flash (column 330g, DCM:EA =3:1) gave 2.5 g (yield 80%, purity 94%). MS: [M+H]=697.18.

### (5) Synthesis of Compounds 26-65

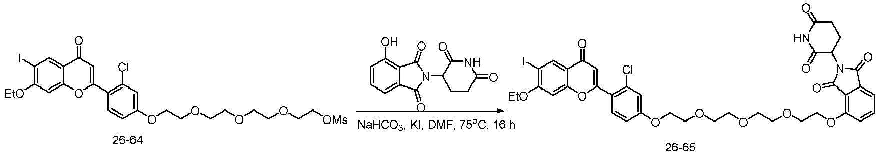

26-64 (2.4 g,1 eq.), 2- (2, 6-dioxo-piperidin-3-yl) -4-hydroxy-isoindole-1, 3-dione (1.15g,1.2 eq.) was stirred in DMF (30 ml), NaHCO₃(670 mg, 2eq.) and KI (770 mg,1.2 eq.) were added at room temperature, raised the temperature to 75°C and continued the reaction for 16 h, the completion of the reaction was monitored by LCMS and cooled to room temperature. Directly passed through reversed-phase Flash (column C18, 330g, ACN/0.05% FA in water) gave 2.1g (yield 70%, purity 93%). MS: [M+H]=875.

### Synthesis of 26

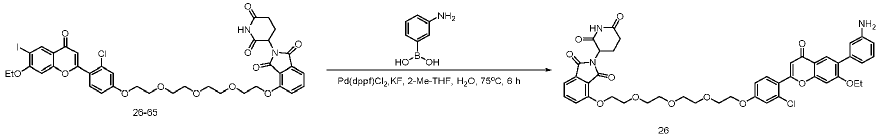

26-65(100 mg, 1 eq.), 3-aminophenylboronic acid (47 mg, 3 eq.) and KF (40 mg, 6.0 eq.) were added into 2-methyltetrahydrofuran (3 ml) and water (1 ml) with stirring, Pd(dppf)Cl₂ (8.3 mg, 0.1 eq.) was added under N₂ atmosphere, raised the temperature to 75°C to continue the reaction for 8 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 68.33 mg off-white solid (yield 70%, purity 92%). MS: [M+H]=840.28.

¹H NMR (400 MHz, DMSO-*d*₆) δ 11.11 (s, 1H), 7.84 (s, 1H), 7.79 (dd, *J* = 8.5, 7.3 Hz, 1H), 7.75-7.72 (m, 1H), 7.53 (p, *J* = 8.8, 7.8 Hz, 3H), 7.44 (d, *J* = 7.2 Hz, 1H), 7.28- 7.25 (m, 2H), 7.12 (dd, *J* = 8.7, 2.5 Hz, 1H), 7.07 (t, *J* = 7.8 Hz, 1H), 6.76 (t, *J* = 2.0 Hz, 1H), 6.72-6.68 (m, 1H), 6.57 (ddd, *J* = 8.0, 2.3, 1.0 Hz, 1H), 6.52 (s, 1H), 5.08 (dd, *J* = 12.9, 5.4 Hz, 1H), 4.34 (t, *J* = 4.6 Hz, 2H), 4.21 (q, *J* = 2.9, 2.0 Hz, 4H), 3.81 (dd, *J* = 5.6, 3.5 Hz, 2H), 3.76 (dd, *J* = 5.3, 3.4 Hz, 2H), 3.65 (dd, *J* = 5.9, 3.6 Hz, 2H), 3.61-3.58 (m, 2H), 3.57-3.55 (m, 4H), 2.64-2.51 (m, 1H), 2.44 (t, *J* = 7.3 Hz, 2H), 2.01 (td, *J* = 7.5, 7.0, 4.1 Hz, 1H), 1.33 (d, *J* = 7.0 Hz, 3H).

### Synthesis example 20

### Synthesis of Compound 27

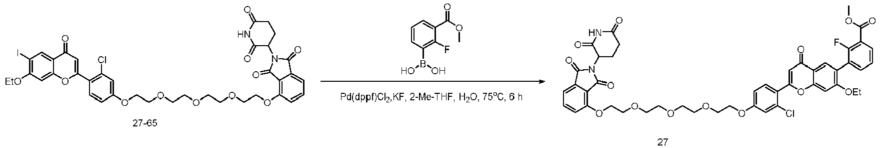

2765(100 mg, 1 eq.), 2-fluoro-3-methoxycarbonylphenylboronic acid (67.9 mg, 3 eq.) and KF (40 mg, 6.0 eq.) were added into 2-methyltetrahydrofuran (3 ml) and water (1 ml) with stirring, Pd(dppf)Cl₂ (8.3 mg, 0.1 eq.) was added under N₂ atmosphere, raised the temperature to 75°C to continue the reaction for 8 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 63.97 mg off-white solid (yield 64%, purity 92%). MS: [M+H]=901.29.

¹H NMR (400 MHz, DMSO-*d*₆) δ 11.11 (s, 1H), 7.89 (s, 1H), 7.89-7.84 (m, 1H), 7.82-7.74 (m, 4H), 7.71 (ddd, *J* = 7.6, 6.6, 1.9 Hz, 1H), 7.36 (s, 1H), 7.29-7.26 (m, 2H), 7.12 (dd, *J* = 8.8, 2.5 Hz, 1H), 6.56 (s, 1H), 5.09 (dd, *J* = 12.8, 5.4 Hz, -1H), 4.35-4.33 (m, 2H), 4.24-4.20 (m, 4H), 3.88 (s, 3H), 3.83-3.81 (m, 2H), 3.78-3.76 (m, 2H), 3.66 (dd, *J* = 5.9, 3.5 Hz, 2H), 3.61-3.58 (m, 2H), 3.57-3.55 (m, 4H), 2.89 (ddd, *J* = 17.5, 14.0, 5.4 Hz, 1H), 2.69-2.52 (m, 2H), 2.03 (ddd, *J* = 11.8, 6.1, 3.6 Hz, 1H), 1.26 (d, *J* = 7.0 Hz, 3H).

### Synthesis example 21

### Synthesis of Compound 28

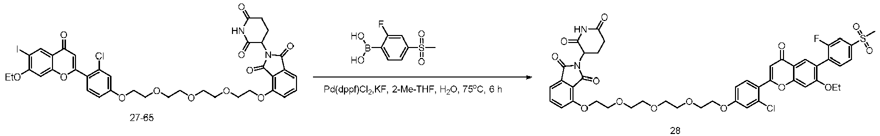

27-65(100 mg, 1 eq.), 2-fluoro-4-(methylsulfonyl) phenylboronic acid (74.8 mg, 3 eq.) and KF (40 mg, 6.0 eq.) were added into 2-methyltetrahydrofuran (3 ml) and water (1 ml) with stirring, Pd(dppf)Cl₂ (8.3 mg, 0.1 eq.) was added under N₂ atmosphere, raised the temperature to 75°C to continue the reaction for 8 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 35.41 mg off-white solid (yield 33%, purity 92%). MS: [M+H]=921.34.

¹H NMR (400 MHz, DMSO-*d*₆) δ 11.10 (s, 1H), 7.92 (s, 1H), 7.87 (ddd, *J* = 11.7, 8.5, 1.8 Hz, 2H), 7.82-7.73 (m, 3H), 7.52 (d, *J* = 8.6 Hz, 1H), 7.44 (d, *J* = 7.2 Hz, 1H), 7.40 (s, 1H), 7.27 (d, *J* = 2.5 Hz, 1H), 7.13 (dd, *J* = 8.8, 2.5 Hz, 1H), 6.57 (s, 1H), 5.08 (dd, *J* = 12.9, 5.4 Hz, 1H), 4.34 (t, *J* = 4.6 Hz, 2H), 4.27-4.20 (m, 4H), 3.83- 3.79 (m, 2H), 3.77 (t, *J* = 4.6 Hz, 2H), 3.65 (dd, *J* = 5.9, 3.6 Hz, 2H), 3.59 (dd, *J* = 6.1, 3.3 Hz, 2H), 3.57-3.54 (m, 4H), 3.36 (s, 3H), 2.88 (ddd, *J* = 17.8, 14.0, 5.3 Hz, 1H), 2.57 (td, *J* = 13.9, 3.9 Hz, 2H), 2.06-1.98 (m, 1H), 1.28 (t, *J* = 6.9 Hz, 3H).

### Synthesis example 22

### Synthesis of Compound 29

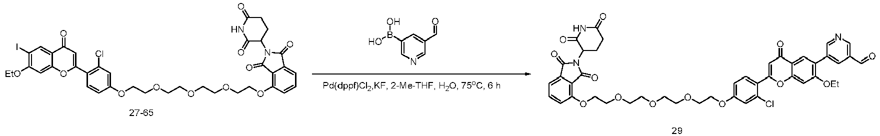

27-65 (100 mg, 1 eq.), 5-aldehyde pyridine-3-boric acid (51.8 mg, 3 eq.) and KF (40 mg, 6.0 eq.) were added into 2-methyltetrahydrofuran (3 ml) and water (1 ml) with stirring, Pd(dppf)Cl₂ (8.3 mg, 0.1 eq.) was added under N₂ atmosphere, raised the temperature to 75°C to continue the reaction for 8 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 27.89mg off-white solid (yield 28.4%, purity 92%). MS: [M+H]=854.26.

¹H NMR (400 MHz, DMSO-*d*₆) δ 11.09 (s, 1H), 10.19 (s, 1H), 9.07 (dd, *J* = 5.9, 2.1 Hz, 2H), 8.44 (t, *J* = 2.1 Hz, 1H), 8.05 (s, 1H), 7.82-7.73 (m, 2H), 7.52 (d, *J* = 8.4 Hz, 1H), 7.44 (d, *J* = 7.3 Hz, 1H), 7.40 (s, 1H), 7.27 (d, *J* = 2.5 Hz, 1H), 7.13 (dd, *J* = 8.7, 2.5 Hz, 1H), 6.58 (s, 1H), 5.08 (dd, *J* = 12.9, 5.4 Hz, 1H), 4.34 (t, *J* = 4.6 Hz, 2H), 4.29-4.21 (m, 4H), 3.81 (t, *J* = 4.6 Hz, 2H), 3.77 (dd, J = 5.4, 3.7 Hz, 2H), 3.65 (dd, *J* = 6.0, 3.6 Hz, 2H), 3.59 (dd, *J* = 6.3, 3.5 Hz, 2H), 3.56 (dd, *J* = 5.9, 3.4 Hz, 4H), 2.88 (ddd, *J* = 18.0, 14.1, 5.4 Hz, 1H), 2.71-2.52 (m, 2H), 2.01 (dd, *J* = 11.5, 5.9 Hz, 1H), 1.33 (t, *J* = 6.9 Hz, 3H).

### Synthesis example 23

### Synthesis of Compound 30

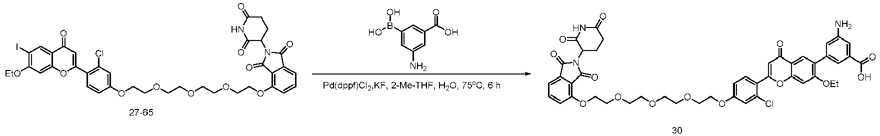

27-65 (100 mg, 1 eq.), (2-fluoro-4-(morpholinosulfophenyl) phenyl) boronic acid (62 mg, 3 eq.) and KF (40 mg, 6.0 eq.) were added into 2-methyltetrahydrofuran (3 ml) and water (1 ml) with stirring, Pd(dppf)Cl₂ (8.3 mg, 0.1 eq.) was added under N₂ atmosphere, raised the temperature to 75°C to continue the reaction for 8 h, the completion of the reaction was monitored by LCMS and cooled to room temperature. The reaction flask was charged directly with anhydrous sodium sulfate to remove water, filtered, spin-dried, and passed through pre-TLC (DCM:MeOH=20:1) to obtain 16.14 mg off-white solid (yield 16%, purity 92%). MS: [M+H]=884.29.

¹H NMR (400 MHz, DMSO-*d*₆) δ 11.10 (s, 1H), 7.87 (d, J = 2.1 Hz, 1H), 7.82-7.77 (m, 1H), 7.74 (d, *J* = 8.6 Hz, 1H), 7.52 (d, J = 8.6 Hz, 1H), 7.44 (d, *J* = 7.3 Hz, 1H), 7.30 (d, J = 3.3 Hz, 2H), 7.26 (d, *J* = 2.4 Hz, 1H), 7.19 (s, 1H), 7.12 (dd, *J* = 8.6, 2.5 Hz, 1H), 6.98 (s, 1H), 6.54 (d, *J* = 4.5 Hz, 1H), 5.39 (s, 2H), 5.08 (dd, *J* = 12.9, 5.4 Hz, 1H), 4.34 (t, *J* = 4.5 Hz, 2H), 4.22 (d, *J* = 6.6 Hz, 4H), 3.81 (t, *J* = 4.7 Hz, 2H), 3.79-3.74 (m, 2H), 3.65 (dd, *J* = 6.0, 3.6 Hz, 2H), 3.59 (d, *J* = 6.0 Hz, 2H), 3.56 (dd, *J* = 6.0, 3.6 Hz, 4H), 2.95-2.78 (m, 1H), 2.70-2.52 (m, 2H), 2.01 (s, 1H), 1.34 (t, *J* = 6.9 Hz, 3H).

### Synthesis example 24

### Synthesis of Compound 31

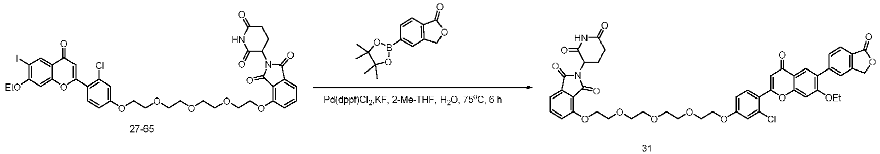

27-65(100 mg, 1 eq.) isobenzofuran-1(3H)-one-5-boronic acid pinacol ester (89mg, 3 eq.) and KF (40 mg, 6.0 eq.) were added into 2-methyltetrahydrofuran (3 ml) and water (1 ml) with stirring, Pd(dppf)Cl₂ (8.3 mg, 0.1 eq.) was added under N₂ atmosphere, raised the temperature to 75°C to continue the reaction for 8 h, the completion of the reaction was monitored by LCMS and cooled to room temperature. The reaction flask was charged directly with anhydrous sodium sulfate to remove water, filtered, spin-dried, and passed through pre-TLC (DCM:MeOH=20:1) to obtain 46.04 mg off-white solid (yield 45%, purity 92%). MS: [M+H]=881.28.

¹H NMR (400 MHz, DMSO-*d*₆) δ 11.11 (s, 1H), 7.97 (s, 1H), 7.91 (d, *J* = 8.0 Hz, 1H), 7.85 (d, *J* = 4.0 Hz, 1H), 7.82-7.77 (m, 2H), 7.75 (d, *J* = 8.7 Hz, 1H), 7.52 (d, *J* = 8.5 Hz, 1H), 7.44 (d, *J* = 7.2 Hz, 1H), 7.38 (s, 1H), 7.27 (d, *J* = 2.5 Hz, 1H), 7.13 (dd, *J* = 8.7, 2.5 Hz, 1H), 6.57 (s, 1H), 5.48 (s, 2H), 5.08 (dd, *J* = 12.9, 5.4 Hz, 1H), 4.34 (t, *J* = 4.6 Hz, 2H), 4.27-4.21 (m, 4H), 3.83-3.79 (m, 2H), 3.77 (dd, *J* = 5.5, 3.6 Hz, 2H), 3.65 (dd, *J* = 6.0, 3.5 Hz, 2H), 3.61-3.58 (m, 2H), 3.56 (dd, *J* = 6.1, 3.4 Hz, 4H), 2.95-2.81 (m, 1H), 2.68-2.52 (m, 2H), 2.06-1.98 (m, 1H), 1.33 (s, 3H).

### Synthesis example 25

### Synthesis of Compound 32

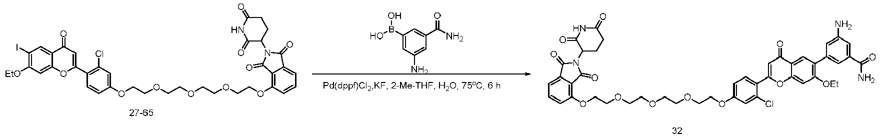

27-65(100 mg, 1 eq.), (3-amino-5-carbarylphenyl)boronic acid (61.7 mg, 3 eq.) and KF (40 mg, 6.0 eq.) were added into 2-methyltetrahydrofuran (3 ml) and water (1 ml) with stirring, Pd(dppf)Cl₂ (8.3 mg, 0.1 eq.) was added under N₂ atmosphere, raised the temperature to 75°C to continue the reaction for 8 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 8.67 mg off-white solid (yield 8.6%, purity 92%). MS: [M+H]=883.30.

¹H NMR (400 MHz, DMSO-*d*₆) δ 11.10 (s, 1H), 7.91 (s, 1H), 7.84-7.73 (m, 3H), 7.52 (d, *J* = 8.6 Hz, 1H), 7.44 (d, *J* = 7.2 Hz, 1H), 7.29 (s, 1H), 7.26 (d, *J* = 2.4 Hz, 1H), 7.18 (s, 1H), 7.17-7.10 (m, 2H), 7.06 (s, 1H), 6.91 (s, 1H), 6.54 (s, 1H), 5.29 (br, 2H), 5.08 (dd, *J* = 12.8, 5.3 Hz, 1H), 4.34 (d, *J* = 4.9 Hz, 2H), 4.21 (d, *J* = 6.7 Hz, 4H), 3.81-3.79 (m, 2H), 3.76-3.74 (m, 2H), 3.67-3.64 (m, 2H), 3.61-3.58 (m, 2H), 3.57- 3.54 (m, 4H), 2.95-2.81 (m, 1H), 2.58 (d, *J* = 17.4 Hz, 2H), 2.00 (d, *J* = 7.4 Hz, 1H), 1.34 (s, 3H).

### Synthesis example 26

### Synthesis of Compound 33

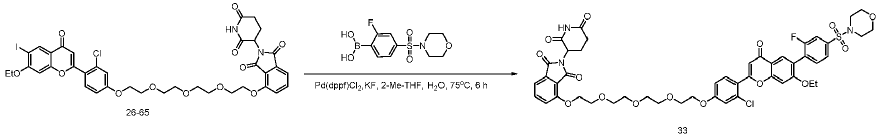

26-65(100 mg, 1 eq.), 2-fluoro-4-(morpholinosulfonyl) phenylboronic acid (99 mg, 3 eq.) and KF (40 mg, 6.0 eq.) were added into 2-methyltetrahydrofuran (3 ml) and water (1 ml) with stirring, Pd(dppf)Cl₂ (8.3 mg, 0.1 eq.) was added under N₂ atmosphere, raised the temperature to 75°C to continue the reaction for 8 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 51.18 mg off-white solid (yield 45%, purity 92%). MS: [M+H]=992.42.

¹H NMR (400 MHz, DMSO-*d*₆) δ 11.11 (s, 1H), 8.02 (s, 1H), 7.82 (d, *J* = 8.2 Hz, 1H), 7.80-7.76 (m, 2H), 7.76-7.73 (m, 1H), 7.67 (dd, *J* = 8.2, 1.6 Hz, 1H), 7.52 (d, *J* = 8.5 Hz, 1H), 7.44 (d, *J* = 7.2 Hz, 1H), 7.39 (s, 1H), 7.27 (d, *J* = 2.5 Hz, 1H), 7.12 (dd, *J* = 8.8, 2.5 Hz, 1H), 6.57 (s, 1H), 5.09 (dd, *J* = 12.9, 5.4 Hz, 1H), 4.37-4.32 (m, 2H), 4.28-4.21 (m, 4H), 3.81 (t, *J* = 4.5 Hz, 2H), 3.79- 3.75 (m, 2H), 3.67 (dt, *J* = 6.5, 3.2 Hz, 6H), 3.60-3.58 (m, 2H), 3.57-3.55 (m, 4H), 3.09 (t, *J* = 4.7 Hz, 4H), 2.95- 2.82 (m, 1H), 2.57 (dd, *J* = 16.1, 12.1 Hz, 2H), 2.09-1.95 (m, 1H), 1.35 (t, *J* = 6.9 Hz, 3H).

### Synthesis example 27

### Synthesis of Compound 34

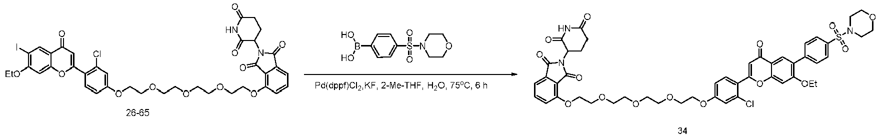

26-65(100 mg, 1 eq.), 4-(morpholinosulfonyl) phenylboronic acid (93 mg, 3 eq.) and KF (40 mg, 6.0 eq.) were added into 2-methyltetrahydrofuran (3 ml) and water (1 ml) with stirring, Pd(dppf)Cl₂ (8.3 mg, 0.1 eq.) was added under N₂ atmosphere, raised the temperature to 75°C to continue the reaction for 8 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 53.1 mg off-white solid (yield 45%, purity 92%). MS: [M+H]=974.43.

¹H NMR (400 MHz, DMSO-*d*₆) δ 11.10 (s, 1H), 7.99 (s, 1H), 7.88 (d, *J* = 8.2 Hz, 2H), 7.81 (d, *J* = 8.4 Hz, 2H), 7.77 (d, *J* = 4.2 Hz, 1H), 7.54 (dd, *J* = 15.0, 8.6 Hz, 2H), 7.38 (s, 1H), 7.27 (d, *J* = 2.5 Hz, 1H), 7.12 (dd, *J* = 8.7, 2.5 Hz, 1H), 6.97 (d, *J* = 8.8 Hz, 1H), 6.57 (s, 1H), 5.08 (dd, *J* = 12.9, 5.4 Hz, 1H), 4.35 (q, *J* = 4.8 Hz, 2H), 4.28-4.21 (m, 4H), 3.81 (t, *J* = 4.6 Hz, 2H), 3.77 (t, *J* = 4.5 Hz, 2H), 3.66 (p, *J* = 2.9 Hz, 6H), 3.61-3.59 (m, 2H), 3.56 (dd, *J* = 6.0, 3.4 Hz, 4H), 2.94 (t, *J* = 4.6 Hz, 4H), 2.83 (d, *J* = 12.2 Hz, 1H), 2.63-2.52 (m, 2H), 2.02 (d, *J* = 12.3 Hz, 1H), 1.34 (t, *J* = 6.9 Hz, 3H).

### Synthesis example 28

### Synthesis of Compound 35

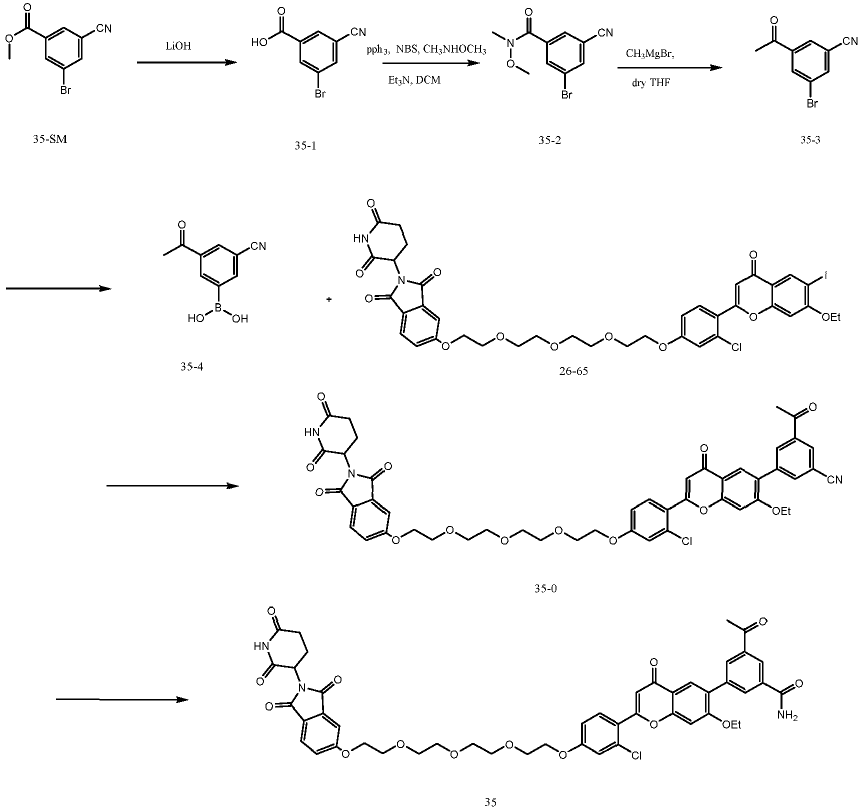

### (1) Synthesis of Compound 35-1

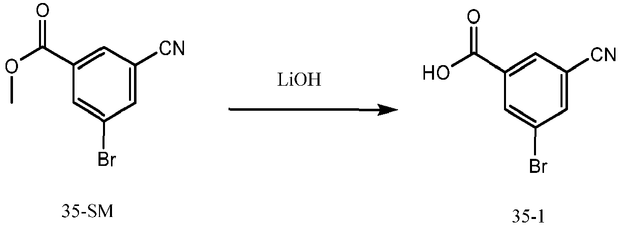

### Experimental procedures

2.00 g 35-SM was dissolved in 20 ml THF, 4 ml MeOH, 4 ml H₂O were added, and 1.75 g LiOH.H₂O was added with stirring.

After 3h, the complete reaction of the raw materials was monitored by TLC (PE:EA=3:1).

The reaction solution was concentrated, 80 ml of water was added into the concentrate, 1N HCl was added with stirring until pH was 3, a white solid precipitated, extracted with EA 50 ml for 3 times, the organic phases were combined, washed once with 80 ml of saturated brine, dried over anhydrous sodium sulfate, filtered, and concentrated to obtain 1.9g white solid, which was used directly in the next reaction.

### (2) Synthesis of Compound 35-2

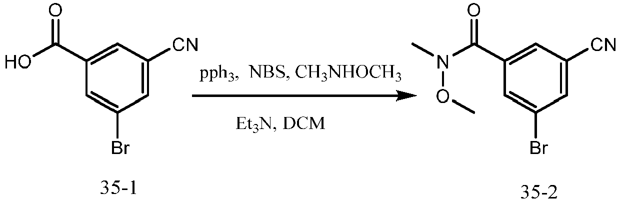

1.00 g of 35-1 was dissolved in DCM, at 0°C, adding 1.74g of triphenylphosphine, a white suspension, 1.18 g of NBS, an orange clear solution, the mixture is warmed to room temperature after 15min, 0.65 g of dimethylhydroxylamine hydrochloride and 0.49g of triethylamine were sequentially added, and the color gradually becomes dark.

After 1h, a sample was taken and sent to LC-MS showing mainly as target product 268.

The reaction mixture was poured into 100 ml of saturated sodium bicarbonate solution, with white smoke, extracted 3 times with 75 ml of DCM, the organic phases were combined, washed once with 100 ml of saturated brine, dried over anhydrous sodium sulfate and concentrated.

Purification: dissolved in DCM, stirred with 3g of silica gel, eluted with 0-50% EA\PE, collected and concentrated to obtain 0.99g white solid.

### (3) Synthesis of Compound 35-3

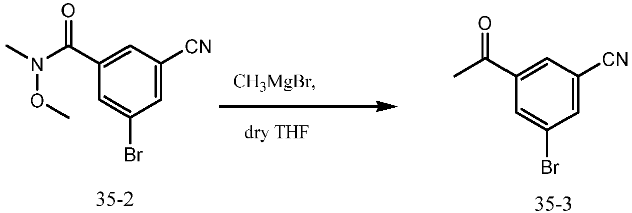

77 mg of 35-2 was put into a single-mouth bottle, protected by nitrogen, added with 2ml of ultra-dry tetrahydrofuran for dissolution, and added 0.3 ml of 1M tetrahydrofuran solution of methyl magnesium bromide at 0°C to obtain a light yellow clear liquid.

### Stirred for 1 h at 0°C.

5 ml of 5% HCl\EtOH solution was added, after stirring for 5min, 20 ml of saturated brine was added, after which extraction with 20 ml of DCM was carried out 3 times, the organic phases were combined, washed once with saturated brine, dried over anhydrous sodium sulfate, filtered and concentrated to obtain 56 mg white solid.

### (4) Synthesis of Compound 35-4

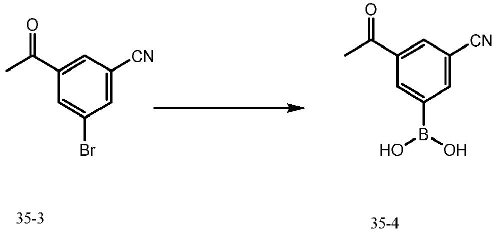

100 mg 35-3 was dissolved in 5 ml 1, 4-dioxane, 170 mg bisphenopinacol borate, 131 mg potassium acetate, 98 mg Pd(dppf)Cl₂ was added, protected by N₂, replaced for 3 times, and stirred at 80°C overnight. Sample was taken and sent to LC-MS, mainly ionizing to 190, and concentrated the reaction solution. Purification: 80 g C18 silica gel column was pre-filled and the concentrate was loaded with 2ml MeOH. Eluted with 0-60% ACN/H₂O at 40°C and concentrated to obtain 66 mg off-white solid, borate was hydrolyzed to boric acid during purification.

### (5) Synthesis of Compound 35-0

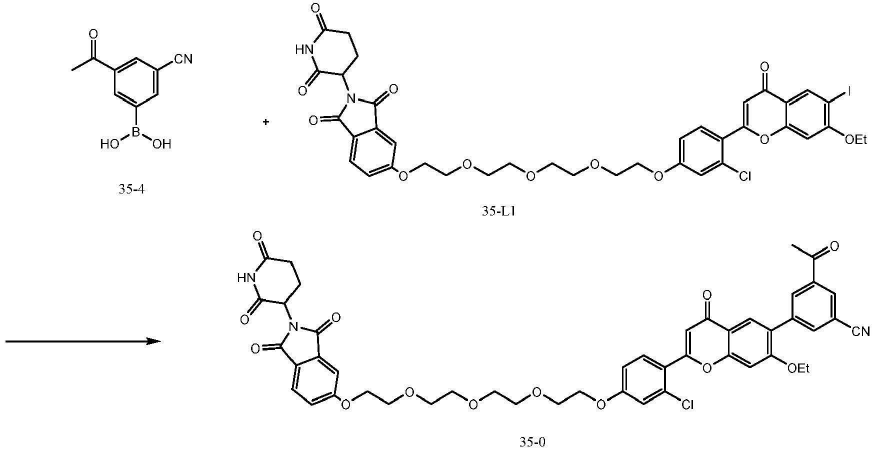

107 mg 35-4 and 100 mg 35-L1 were dissolved in 2-MTHF, KF and 25 mg Pd(dppf)Cl₂ were added, followed by 1 ml water, protected with N₂, 70°C, stirred overnight, anhydrous sodium sulfate was added into the reaction solution, filtered, washed 2 times with DCM, and concentrated for purification: 30 g of silica gel column was pre-filled and dissolved and loaded the sample with DCM, eluted with 0-5% DCM\MeOH, collected chromatographic solution, and concentrated to obtain 78 mg white solid.

### (6) Synthesis of Compound 35

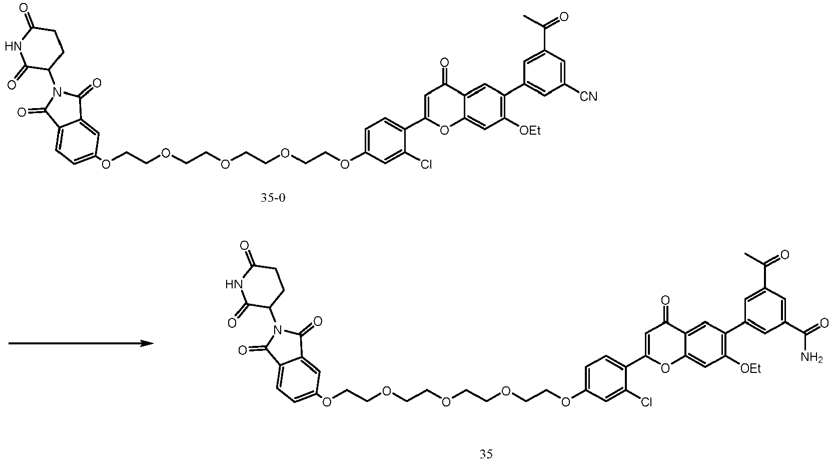

78 mg 35-0 was dissolved in ultra-dry acetonitrile, a catalytic amount of hydrogenated (dimethylphosphinic acid-kP)[hydrobis(dimethylphosphinic acid-kP)] platinum (II) was added, stirred overnight, filtered, concentrated for purification: 30 g of silica gel column was pre-filled, dissolved and loaded the sample by DCM, eluted with 0-5% DCM\MeOH, collected chromatographic solution, and concentrated to obtain 56 mg white solid. MS:[M+H]: 910.33, purity: 91.66%.

¹H NMR (400 MHz, DMSO-*d*₆) δ 11.10 (s, 1H), 8.44 (s, 1H), 8.34 - 8.27 (m, 3H), 8.06 (s, 1H), 7.79 (dd, *J* = 17.5, 8.6 Hz, 2H), 7.44 (d, *J* = 2.3 Hz, 1H), 7.40 - 7.32 (m, 2H), 7.27 (d, *J* = 2.5 Hz, 1H), 7.13 (dd, *J* = 8.6, 2.5 Hz, 1H), 6.57 (s, 1H), 5.11 (dd, *J* = 13.1, 5.2 Hz, 1H), 4.31 (s, 2H), 4.28 - 4.21 (m, 4H), 3.65 - 3.54 (m, 8H), 3.29 (s, 1H), 2.68 (s, 3H), 2.05 - 1.95 (m, 3H), 1.37 - 1.21 (m, 17H).

### Synthetic example 29

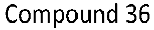

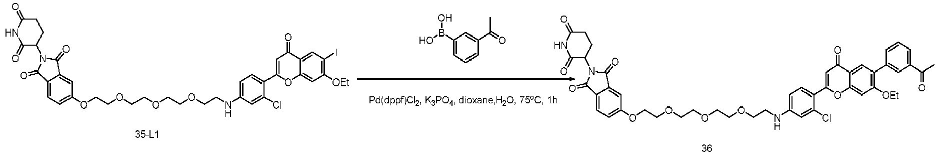

35-L1 (100 mg, 1 eq.), 3-acetylphenylboronic acid (56.3 mg, 3 eq.) and K₃PO₄ (72.8 mg, 3.0 eq.) were added into 1,4-dioxane (3 ml) and water (1 ml) with stirring, Pd(dppf)Cl₂ (8.3 mg, 0.1 eq.) was added under the condition of N₂ gas, raised the temperature to 75°C to continue the reaction for 8 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 30.55 mg off-white solid (yield 31%, purity 96.2%). MS: [M+H]=866.26.

¹H NMR (400 MHz, DMSO-*d*₆) δ 8.11 - 8.01 (m, 2H), 7.91 (m, H), 7.68 - 7.52 (m, 4H), 7.51 - 7.40 (m, 1H), 7.10 (s, 1H), 7.05 (m, H), 6.84 (d, *J* = 2.2 Hz, 1H), 6.74 (s, 1H), 6.45 (m, 1H), 5.87 (m, , 1H), 5.23 (m, 1H), 4.17 - 4.05 (m, 4H), 3.78 (m, 2H), 3.74 - 3.57 (m, 11H), 3.42 (m, H), 2.93 - 2.80 (m, 1H), 2.68 - 2.48 (m, 5H), 2.15 - 2.02 (m, 1H), 1.32 (m, 3H).

### Synthesis example 30

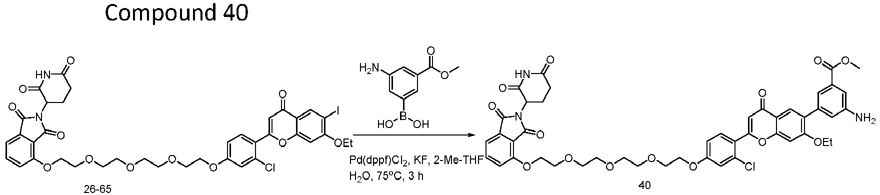

26-65(150 mg, 1 eq.), (3-amino-5-(methoxycarbonyl) phenyl) boronic acid (100 mg, 3 eq.) and KF (62 mg, 6.0 eq.) were added into 2-methyltetrahydrofuran (3 ml) and water (1 ml) with stirring, Pd(dppf)Cl₂ (12.5 mg, 0.1 eq.) was added under N₂ atmosphere, raised the temperature to 75°C to continue the reaction for 8 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 40 mg off-white solid. MS: [M+H]=898.32.

¹H NMR (400 MHz, DMSO-*d*₆) δ 11.10 (s, 1H), 7.88 (s, 1H), 7.83 - 7.71 (m, 2H), 7.57 - 7.43 (m, 4H), 7.32 (d, *J* = 8.7 Hz, 2H), 7.26 (s, 1H), 7.21 (s, 1H), 7.12 (d, *J* = 8.8 Hz, 1H), 7.03 (s, 1H), 6.54 (s, 1H), 5.17 - 5.01 (m, 1H), 4.34 (s, 2H), 4.22 (d, *J* = 6.3 Hz, 4H), 3.83 (s, 5H), 3.76 (s, 2H), 3.65 (d, *J* = 5.3 Hz, 2H), 3.57 (s, 6H), 2.95 - 2.81 (m, 1H), 2.58 (d, *J* = 17.3 Hz, 2H), 2.02 (s, 1H), 1.34 (t, *J* = 7.0 Hz, 3H).

### Synthesis example 31

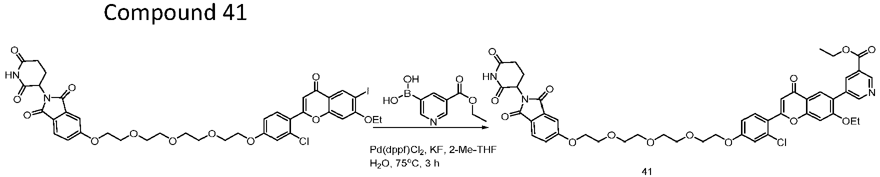

5-(2-(2-(2-(2-(3-chloro-4-(7-ethoxy-6-iodo-4-oxo-4H-chromen-2-yl)phenoxy) ethoxy) ethoxy) ethoxy)-2-(2,6-dioxoperidin-3-yl) isoindoline-1,3-dione (100 mg, 1 eq.), (5-(ethoxycarbonyl) pyridine-3-yl) boronic acid (92 mg, 3 eq.) and KF (80 mg, 6.0 eq.) were added into 2-methyltetrahydrofuran (3 ml) and water (1 ml) with stirring, Pd(dppf)Cl₂ (8 mg, 0.1 eq.) was added under N₂ atmosphere, raised the temperature to 75°C to continue the reaction for 8 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 55 mg off-white solid. MS: [M+H]=898.32.

¹H NMR (400 MHz, DMSO-*d*₆) δ 11.11 (s, 1H), 9.08 (s, 1H), 9.00 (d, *J* = 1.6 Hz, 1H), 8.46 (s, 1H), 8.01 (s, 1H), 7.81 (d, *J* = 8.3 Hz, 1H), 7.75 (d, *J* = 8.7 Hz, 1H), 7.44 (s, 1H), 7.36 (d, *J* = 10.2 Hz, 2H), 7.26 (d, *J* = 2.0 Hz, 1H), 7.12 (dd, *J* = 8.7, 2.0 Hz, 1H), 6.57 (s, 1H), 5.11 (dd, *J* = 12.9, 5.3 Hz, 1H), 4.39 (q, *J* = 7.1 Hz, 2H), 4.34 - 4.21 (m, 6H), 3.79 (s, 4H), 3.64 - 3.54 (m, 8H), 2.96 - 2.83 (m, 1H), 2.64 - 2.52 (m, 2H), 2.07 - 1.99 (m, 1H), 1.35 (dt, *J* = 12.0, 7.0 Hz, 6H).

### Synthesis example 32

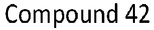

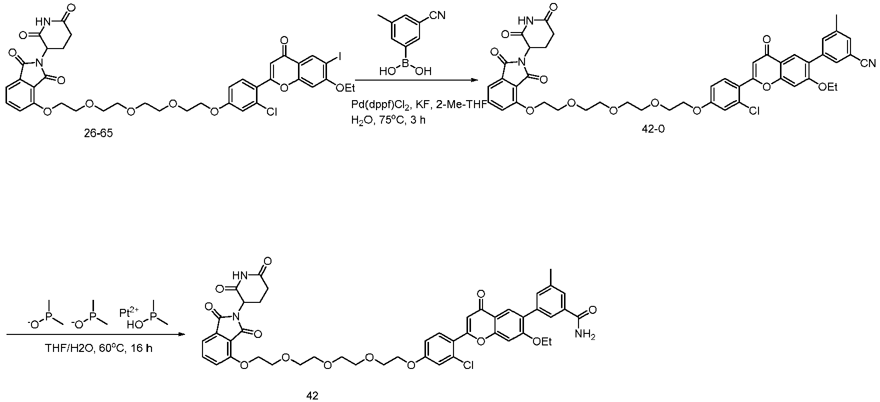

### (1) Synthesis of Compound 42-0

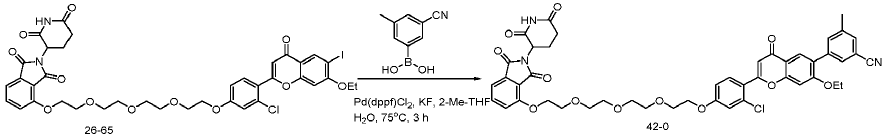

26-65(150 mg, 1 eq.), (3-cyano-5-methylphenyl) boronic acid (83 mg, 3 eq.) and KF (83 mg, 6.0 eq.) were added into 2-methyltetrahydrofuran (3 ml) and water (1 ml) with stirring, Pd(dppf)Cl₂ (15 mg, 0.1 eq.) was added under N₂ atmosphere, raised the temperature to 75°C to continue the reaction for 8 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 100 mg off-white solid. MS: [M+H]=864.21.

¹H NMR (400 MHz, DMSO-*d*₆) δ 11.10 (s, 1H), 8.02 (s, 1H), 7.95 (s, 1H), 7.87 (s, 1H), 7.79 (t, *J* = 7.8 Hz, 1H), 7.75 (d, *J* = 8.7 Hz, 1H), 7.71 (s, 1H), 7.56 - 7.49 (m, 2H), 7.44 (d, *J* = 7.2 Hz, 1H), 7.33 (d, *J* = 6.5 Hz, 2H), 7.26 (d, *J* = 2.4 Hz, 1H), 7.12 (dd, *J* = 8.7, 2.5 Hz, 1H), 6.56 (s, 1H), 5.08 (dd, *J* = 12.8, 5.4 Hz, 1H), 4.34 (t, *J* = 4.6 Hz, 2H), 4.22 (q, *J* = 7.1 Hz, 4H), 3.81 (t, *J* = 4.6 Hz, 2H), 3.77 (t, *J* = 4.5 Hz, 2H), 3.65 (dd, *J* = 5.9, 3.6 Hz, 2H), 3.59 (dd, *J* = 6.2, 3.3 Hz, 2H), 3.56 (dd, *J* = 5.9, 3.4 Hz, 4H), 2.96 - 2.81 (m, 1H), 2.57 (dd, *J* = 15.9, 11.7 Hz, 2H), 2.42 (s, 3H), 2.01 (s, 1H), 1.32 (t, *J* = 6.9 Hz, 3H).

### (2) Synthesis of Compound 42

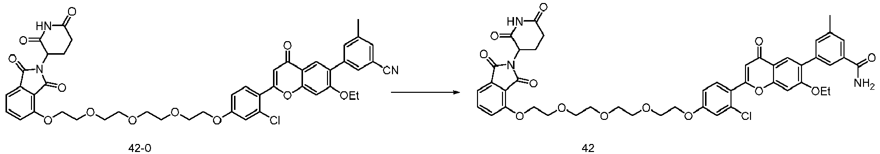

42-0 (100 mg, 1 eq.) and Pt (cas 173416-05-2) (0.1 eq.) were added into tetrahydrofuran (3 ml) and water (3 ml) with stirring, raised the temperature to 60°C to continue the reaction for 16 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 50 mg off-white solid. MS: [M+H]=882.32.

¹H NMR (400 MHz, DMSO-*d*₆) δ 11.10 (s, 1H), 8.02 (s, 1H), 7.95 (s, 1H), 7.87 (s, 1H), 7.79 (t, *J* = 7.8 Hz, 1H), 7.75 (d, *J* = 8.7 Hz, 1H), 7.71 (s, 1H), 7.56 - 7.49 (m, 2H), 7.44 (d, *J* = 7.2 Hz, 1H), 7.33 (d, *J* = 6.5 Hz, 2H), 7.26 (d, *J* = 2.4 Hz, 1H), 7.12 (dd, *J* = 8.7, 2.5 Hz, 1H), 6.56 (s, 1H), 5.08 (dd, *J* = 12.8, 5.4 Hz, 1H), 4.34 (t, *J* = 4.6 Hz, 2H), 4.22 (q, *J* = 7.1 Hz, 4H), 3.81 (t, *J* = 4.6 Hz, 2H), 3.77 (t, *J* = 4.5 Hz, 2H), 3.65 (dd, *J* = 5.9, 3.6 Hz, 2H), 3.59 (dd, *J* = 6.2, 3.3 Hz, 2H), 3.56 (dd, *J* = 5.9, 3.4 Hz, 4H), 2.96 - 2.81 (m, 1H), 2.57 (dd, *J* = 15.9, 11.7 Hz, 2H), 2.42 (s, 3H), 2.01 (s, 1H), 1.32 (t, *J* = 6.9 Hz, 3H).

### Synthesis example 33

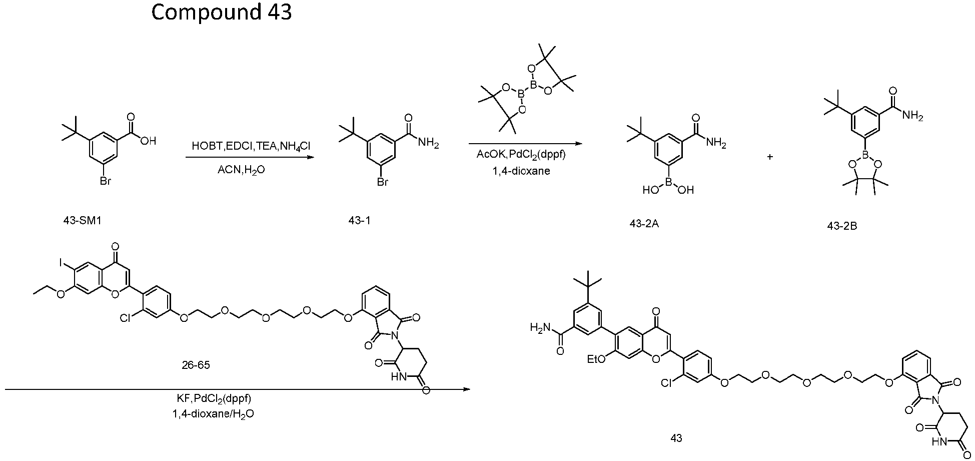

### (1) Synthesis of Compound 43-1

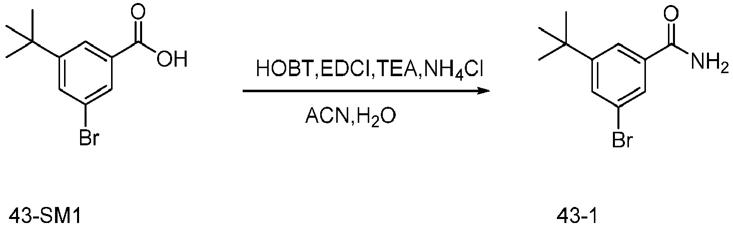

572 mg 43-SM1 was added into 10 ml ACN followed by 4493 mg TEA, 1503 mg HOBT, 2124 mg EDCI, 595 mg NH₄Cl, 1 ml H₂O, stirred after the addition, monitored by LC-MS and supplemented with TEA, HOBT, EDCI, NH₄Cl until TDP102150-SM1 remained unchanged. The system was brought to pH=3 with concentrated HCl, 100 ml H₂O, 3×100 ml EA were added for extraction, and the EA phases were combined, concentrated to dryness at 40°C under reduced pressure, the residue was separated by reversed-phase Flash, and eluted with 75% ACN to obtain obtain 230 mg target product (yield: 40.81%, purity: 98.20%), MS: [M+H] 256, 258.

### (2) Synthesis of Compound 43-2

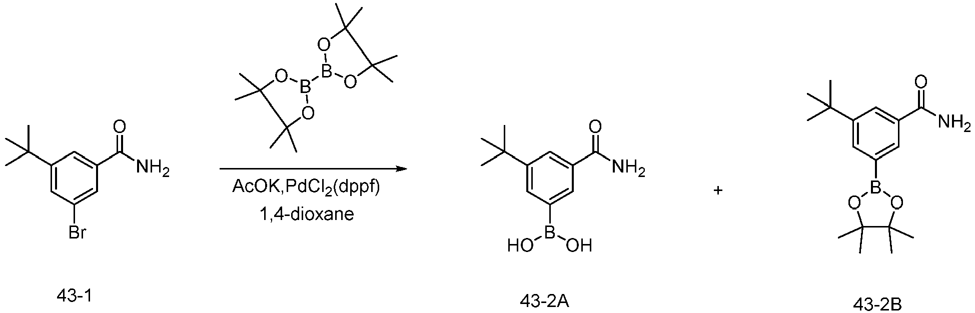

430 mg of 43-1 was added into 10 ml of 1,4-dioxane, then 642 mg of BPDB, 498 mg of AcOK, 372 mg of PdCl₂(dppf) were added, and after the addition replaced with N₂, raised the temperature to 85°C under the protection of N₂ and the mixture was stirred. Monitored by LC-MS until no TDP102150-1 remained. Concentrated to dryness at 40°C under reduced pressure, separated the residue by reversed-phase column chromatography, eluted with 45% ACN to obtain 132 mg target product, MS: [M+H]222.

### (3) Synthesis of Compound 43

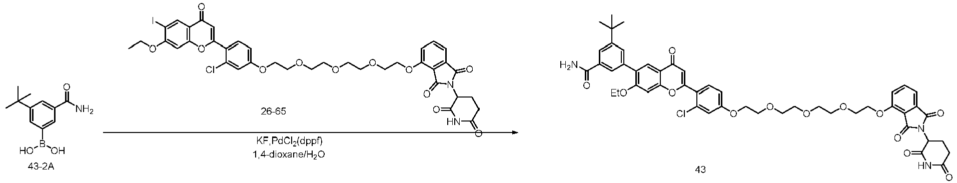

40 mg of 26-65 was added into 3 ml of 2-MTHF, then 44 mg of 43-2A, 16 mg of KF, 10 mg of PdCl₂(dppf) and 1 ml of H₂O were added, after the addition replaced with N₂, the temperature was raised to 70°C under the protection of N₂, and the mixture was stirred. Monitored by LC-MS until no TDP102117-66 remained. Added a proper amount of DCM, dried over Na₂SO₄, performed suction filtration, the filtrate was concentrated to dryness at 40°C under reduced pressure, performed Pre-TLC separation on the residue (DCM:MeOH=20:1), performed reversed-phase column chromatography, and eluted with 85% ACN to obtain 25 mg target product (yield: 58.88%, purity: 97.14%), MS: [M+H] 924.40.

¹H NMR (400 MHz, DMSO-*d*₆) δ 11.11 (s, 1H), 8.12 (s, 1H), 8.00 (s, 1H), 7.91 (s, 1H), 7.86 (s, 1H), 7.81 (d, J = 8.5 Hz, 2H), 7.76 (d, *J* = 8.7 Hz, 1H), 7.44 (d, *J* = 2.0 Hz, 1H), 7.40 - 7.31 (m, 3H), 7.27 (d, *J* = 2.3 Hz, 1H), 7.13 (dd, *J* = 8.7, 2.2 Hz, 1H), 6.56 (s, 1H), 5.11 (dd, *J* = 12.9, 5.3 Hz, 1H), 4.31 (s, 2H), 4.22 (q, *J* = 6.5 Hz, 4H), 3.79 (s, 4H), 3.58 (dd, *J* = 13.7, 3.8 Hz, 8H), 3.02 - 2.77 (m, 1H), 2.56 (s, 2H), 2.06 - 1.98 (m, 1H), 1.36 (s, 9H), 1.32 (d, *J* = 7.0 Hz, 3H).

### Synthesis example 34

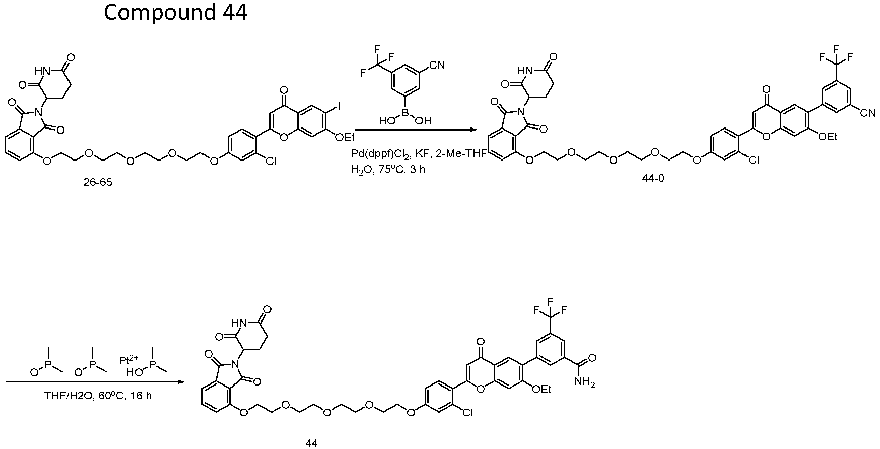

### (1) Synthesis of Compound 44-0

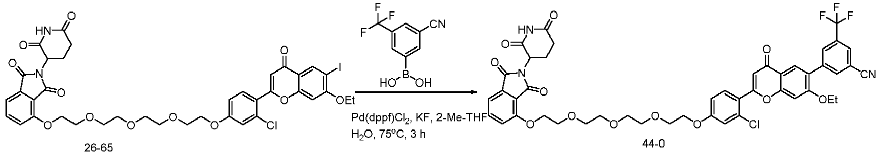

26-65 (150 mg, 1 eq.), (3-cyano-5-(trifluoromethyl) phenyl) boronic acid (111 mg, 3 eq.) and KF (70 mg, 6.0 eq.) were added into 2-methyltetrahydrofuran (3 ml) and water (1 ml) with stirring, Pd(dppf)Cl₂ (15 mg, 0.1 eq.) was added under N₂ atmosphere, raised the temperature to 75°C to continue the reaction for 8 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 80 mg off-white solid. MS: [M+H]=918.21.

¹H NMR (400 MHz, DMSO-*d*₆) δ 11.09 (s, 1H), 8.40 (s, 1H), 8.35 (s, 1H), 8.31 (s, 1H), 8.08 (s, 1H), 7.83 - 7.73 (m, 2H), 7.52 (d, *J* = 8.5 Hz, 1H), 7.44 (d, *J* = 7.2 Hz, 1H), 7.38 (s, 1H), 7.27 (d, *J* = 2.5 Hz, 1H), 7.12 (dd, *J* = 8.7, 2.5 Hz, 1H), 6.58 (s, 1H), 5.08 (dd, *J* = 12.9, 5.4 Hz, 1H), 4.36 - 4.32 (m, 2H), 4.28 - 4.20 (m, 4H), 3.81 (t, *J* = 4.6 Hz, 2H), 3.77 (t, *J* = 4.5 Hz, 2H), 3.65 (dd, *J* = 5.9, 3.6 Hz, 2H), 3.62 - 3.58 (m, 2H), 3.56 (dd, *J* = 5.9, 3.5 Hz, 4H), 2.94 - 2.81 (m, 1H), 2.58 (d, *J* = 17.3 Hz, 2H), 2.02 (d, *J* = 11.8 Hz, 1H), 1.32 (t, *J* = 6.9 Hz, 3H).

### (2) Synthesis of Compound 44

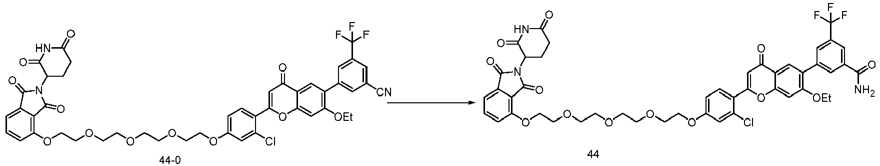

44-0 (70 mg, 1 eq.) and Pt (cas 173416-05-2) (0.1 eq.) were added into tetrahydrofuran (3 ml) and water (3 ml) with stirring, raised the temperature to 60°C to continue the reaction for 16 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 35 mg off-white solid. MS: [M+H]=936.29.

¹H NMR (400 MHz, DMSO-*d*₆) δ 11.09 (s, 1H), 8.36 (s, 2H), 8.21 (s, 1H), 8.11 (d, *J* = 15.7 Hz, 2H), 7.84 - 7.73 (m, 2H), 7.66 (s, 1H), 7.52 (d, *J* = 8.6 Hz, 1H), 7.44 (d, *J* = 7.2 Hz, 1H), 7.38 (s, 1H), 7.27 (d, *J* = 2.5 Hz, 1H), 7.13 (dd, *J* = 8.7, 2.5 Hz, 1H), 6.58 (s, 1H), 5.08 (dd, *J* = 12.9, 5.4 Hz, 1H), 4.34 (t, *J* = 4.6 Hz, 2H), 4.28 - 4.20 (m, 4H), 3.79 (dt, *J* = 17.7, 4.6 Hz, 4H), 3.65 (dd, *J* = 5.9, 3.7 Hz, 2H), 3.58 (ddt, *J* = 9.4, 6.5, 4.0 Hz, 6H), 2.88 (ddd, *J* = 17.9, 14.1, 5.4 Hz, 1H), 2.63 - 2.52 (m, 2H), 2.06 - 1.98 (m, 1H), 1.32 (t, *J* = 6.9 Hz, 3H).

### Synthesis example 35

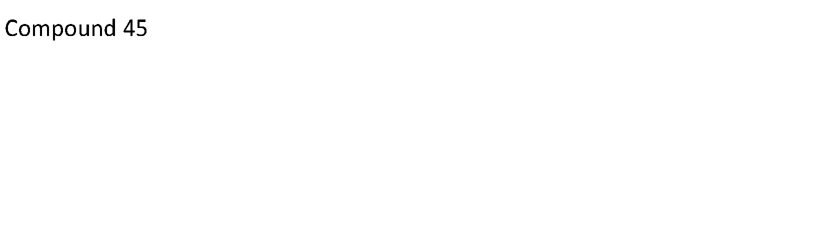

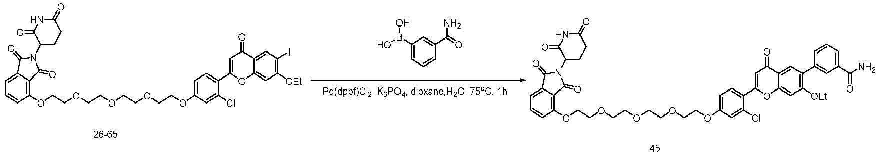

26-656 (100 mg, 1 eq.), 3-carbamyl phenylboronic acid (56.5 mg,3 eq.) and K₃PO₄ (72.7 mg, 3.0 eq.) were added into 1,4-dioxane (3 ml) and water (1 ml) with stirring, Pd(dppf)Cl₂ (8.3 mg, 0.1 eq.) was added under N₂ atmosphere, raised the temperature to 75°C to continue the reaction for 1 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 58.13 mg off-white solid (yield 58%, purity 93%). MS: [M+H]=868.29.

¹H NMR (400 MHz, DMSO-*d*₆) δ 11.10 (s, 1H), 8.11-8.04 (m, 2H), 7.94 (s, 1H), 7.88 (dt, *J* = 7.8, 1.5 Hz, 1H), 7.79 (dd, *J* = 8.5, 7.2 Hz, 1H), 7.74-7.71 (m, 1H), 7.57 (d, *J* = 8.7 Hz, 1H), 7.52 (t, *J* = 7.8 Hz, 2H), 7.44 (d, *J* = 7.2 Hz, 1H), 7.39 (s, 1H), 7.30 (s, 1H), 6.80 (d, *J =* 2.3 Hz, 1H), 6.71 (dd, *J* = 8.8, 2.3 Hz, 1H), 6.66 (t, *J* = 5.6 Hz, 1H), 6.52 (s, 1H), 5.08 (dd, *J =* 12.8, 5.4 Hz, 1H), 4.33 (dd, *J* = 5.7, 3.4 Hz, 2H), 4.22 (q, *J* = 6.9 Hz, 2H), 3.80 (dd, *J* = 5.4, 3.5 Hz, 2H), 3.65 (dd, *J* = 5.9, 3.6 Hz, 2H), 3.58-3.53(m,8H), 3.28 (d, *J* = 5.6 Hz, 2H), 2.88 (ddd, *J =* 17.3, 14.0, 5.3 Hz, 1H), 2.57 (dd, *J* = 16.1, 11.8 Hz, 2H), 2.07- 1.96 (m, 1H), 1.32 (t, *J* = 6.9 Hz, 3H).

### Synthesis example 36

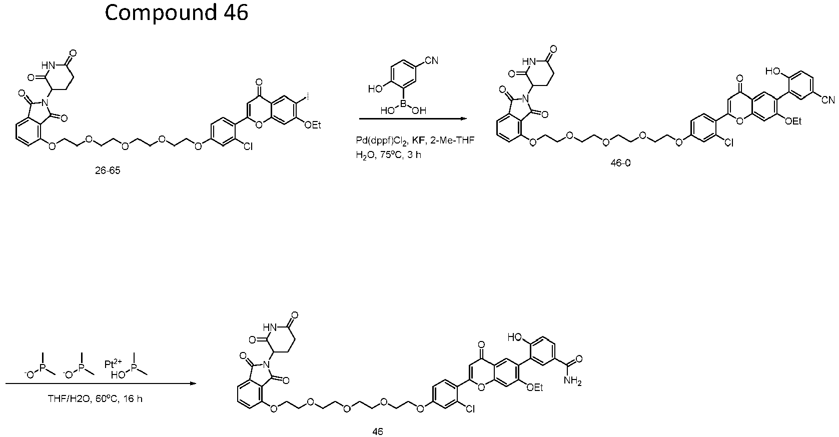

### (1) Synthesis of Compound 46-0

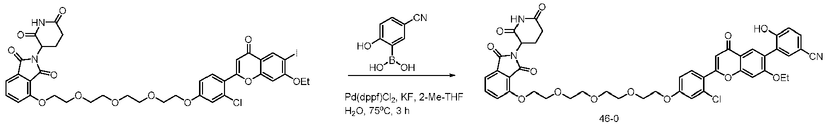

26-65 (90 mg, 1 eq.), (5-cyano-2-hydroxyphenyl) boronic acid (85 mg, 3 eq.) and KF (70 mg, 6.0 eq.) were added into 2-methyltetrahydrofuran (3 ml) and water (1 ml) with stirring, Pd(dppf)Cl₂ (11 mg, 0.1 eq.) was added under N₂ atmosphere, raised the temperature to 75°C to continue the reaction for 8 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 50 mg off-white solid. MS: [M+H]=866.2.

¹H NMR (400 MHz, DMSO-*d*₆) δ 11.10 (s, 1H), 10.65 (s, 1H), 7.82 (d, *J* = 8.9 Hz, 2H), 7.74 (d, *J* = 8.6 Hz, 1H), 7.66 (d, *J* = 7.5 Hz, 2H), 7.44 (s, 1H), 7.27 (d, *J* = 6.5 Hz, 2H), 7.12 (d, *J* = 8.2 Hz, 1H), 7.06 (d, *J* = 8.5 Hz, 1H), 6.52 (s, 1H), 5.11 (dd, *J* = 12.6, 5.2 Hz, 1H), 4.31 (s, 2H), 4.26 - 4.14 (m, 4H), 3.78 (s, 4H), 3.59 (d, *J* = 10.4 Hz, 8H), 2.56 (s, 2H), 1.25 (d, *J* = 6.8 Hz, 3H).

### (2) Synthesis of Compound 46

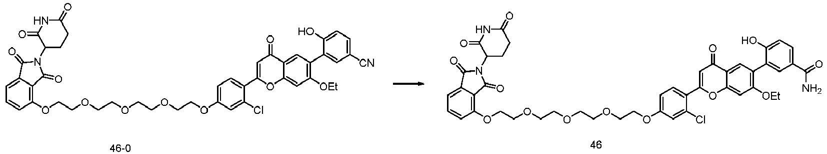

46-0 (35 mg, 1 eq.) and Pt (cas 173416-05-2) (0.1 eq.) were added into tetrahydrofuran (3 ml) and water (3 ml) with stirring, raised the temperature to 60°C and continued the reaction for 16 h under N₂ atmosphere, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 21 mg off-white solid. MS: [M+H]=884.29.

¹H NMR (400 MHz, DMSO-*d*₆) δ 11.11 (s, 1H), 9.94 (s, 1H), 7.85 - 7.80 (m, 2H), 7.75 (dd, *J* = 5.3, 3.1 Hz, 4H), 7.45 (d, *J* = 2.0 Hz, 1H), 7.36 (dd, *J* = 8.3, 2.1 Hz, 1H), 7.27 (s, 2H), 7.16 - 7.03 (m, 2H), 6.93 (d, *J* = 9.1 Hz, 1H), 6.52 (s, 1H), 5.11 (dd, *J* = 12.9, 5.3 Hz, 1H), 4.31 (d, *J* = 4.6 Hz, 2H), 4.22 (d, *J* = 4.4 Hz, 2H), 4.17 (q, *J* = 6.9 Hz, 2H), 3.78 (s, 4H), 3.60 (dd, *J* = 5.3, 2.7 Hz, 4H), 3.57 (d, *J* = 4.9 Hz, 4H), 2.67 - 2.52 (m, 2H), 1.25 (d, *J* = 6.9 Hz, 3H).

### Synthesis example 37

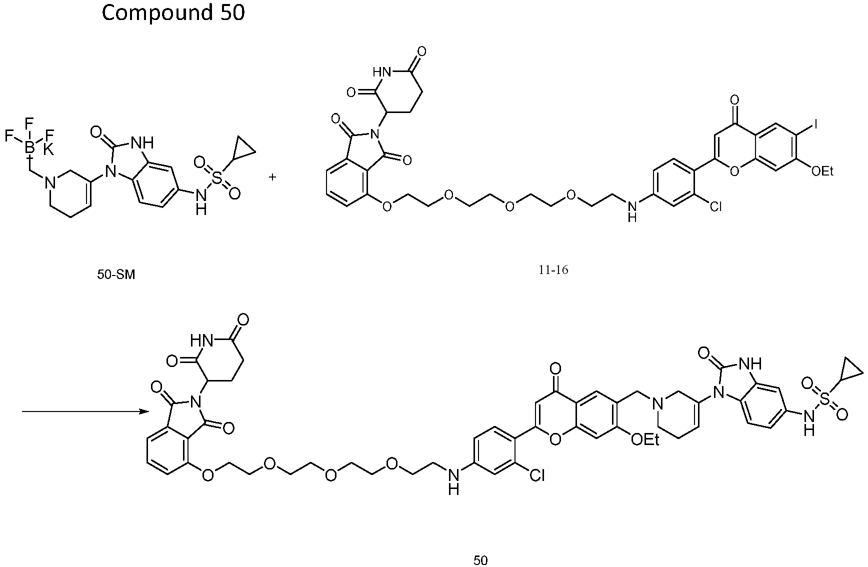

237 mg of 50-SM was dissolved in 3 ml THF, 100 mg 11-16, 56 mg caesium carbonate, 17 mg of palladium diacetate, 11 mg XPhos, 1 ml water were added, protected by N₂ and stirred overnight at 70°C.

Sample was taken and sent to LC-MS, the reaction solution was dried over anhydrous sodium sulfate, dried, filtered and concentrated.

Purification: 40 g C18 silica gel column pre-filled and the concentrate was loaded with 4 ml MeOH.

Eluted with 0-60% ACN\H₂O, concentrated, lyophilized to obtain 46 mg off-white solid.
MS: [M+H]=1094.59, purity: 94.9%
¹H NMR (500 MHz, Chloroform-*d*) δ 9.12 (s, 1H), 8.06 (s, 1H), 7.95 (s, 1H), 7.83 (d, 1H), 7.68 - 7.58 (m, 2H), 7.41 - 7.34 (m, 2H), 7.02 (s, 1H), 6.83 (s, 1H), 6.67 - 6.57 (m, 2H), 6.22 (dd, 1H), 5.12 (t, 1H), 5.00 (s, 1H), 4.91 (ddd, 1H), 4.12 - 3.99 (m, 3H), 3.77 - 3.67 (m, 3H), 3.61 (tdd, 2H), 3.52 - 3.38 (m, 4H), 3.35 - 3.14 (m, 9H), 2.96 - 2.84 (m, 1H), 2.70 (t, 2H), 2.65 - 2.53 (m, 2H), 2.13 - 2.02 (m, 3H), 1.72 (p, 1H), 1.43 (t, 3H), 0.98 (qd, 1H), 0.89 (qd, 1H), 0.79 (qd, 1H), 0.67 (qd, 1H).

### Synthesis example 38

### Synthesis of Compound 51

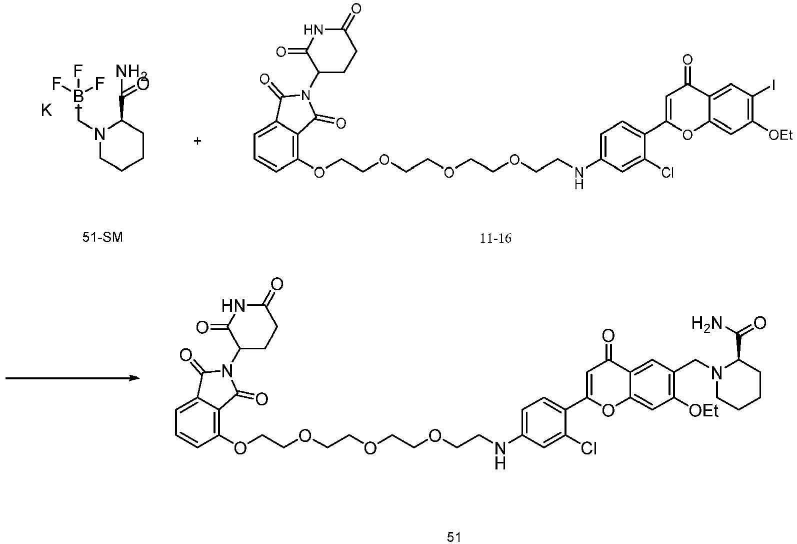

119 mg of 51-SM was dissolved in 3 ml THF, 100 mg T11-16, 56 mg caesium carbonate, 17 mg palladium diacetate, 11 mg XPhos, 1 ml water were added, protected with N₂ and stirred overnight at 70°C.

Sample was taken and sent to LC-MS, the reaction solution was dried over anhydrous sodium sulfate, filtered and concentrated.

Purification: 40 g C18 silica gel column pre-filled and the concentrate was loaded with 4 ml MeOH.

Eluted with 0-60% ACN\H₂O.

Concentrated, lyophilized to obtain 37 mg off-white solid.

MS: [M+H]=888.37, purity: 95.5%.

¹H NMR (500 MHz, Chloroform-d) δ 9.12 (s, 1H), 7.62 (t, 1H), 7.49 (dd, 1H), 7.36 (d, 1H), 7.22 (dd, 1H), 7.02 (s, 1H), 6.83 (s, 1H), 6.63 (dd, 1H), 5.24 (s, 2H), 5.11 (t, 1H), 4.60 (dq, 1H), 4.32 (s, 1H), 4.14 (dqd, 2H), 4.01 (dq, 1H), 3.75 (dd, 1H), 3.71 - 3.61 (m, 1H), 3.61 - 3.37 (m, 6H), 3.40 - 3.27 (m, 4H), 3.30 - 3.21 (m, 4H), 3.25 - 3.11 (m, 2H), 2.97 - 2.85 (m, 2H), 2.65 - 2.53 (m, 2H), 2.26 (dt, 1H), 2.13 - 2.02 (m, 1H), 1.87 - 1.63 (m, 3H), 1.56 (ddt, 2H), 1.43 (t, 3H), 1.39 - 1.27 (m, 1H).

### Synthesis example 39

### Synthesis of Compound 52

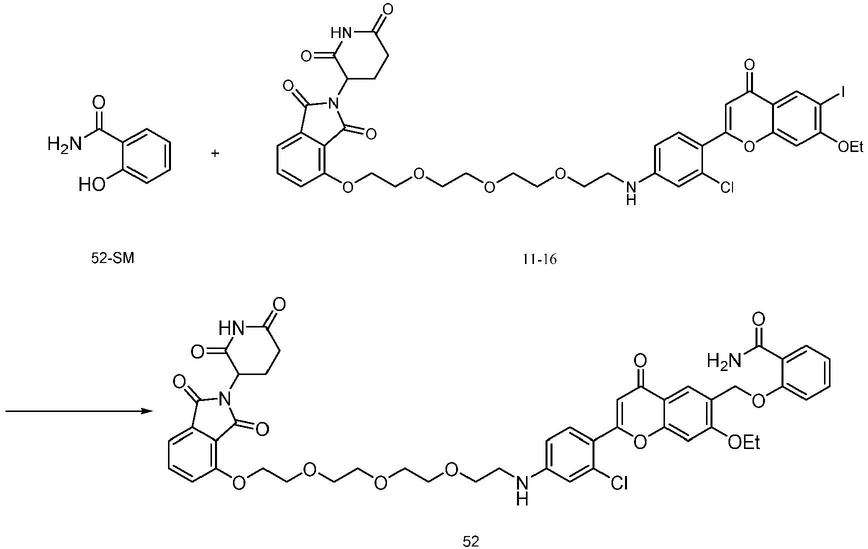

78 mg of 52-SM was dissolved in 5 ml of DMSO, 100 mg of 11-16 and 38 mg of potassium tert-butoxide were added, and the reaction was carried out at 150°C for 1 h by microwave reaction.

Sample was taken and sent to LC-MS, added 50 ml water into reaction solution, extracted by EA for 3 times, combined, washed by brine once, dried over anhydrous sodium sulfate, filtered and concentrated.

Purification: 40 g C18 silica gel column pre-filled and the concentrate was loaded with 4 ml MeOH.

Eluted with 0-60% ACN\H₂O.

Concentrated, lyophilize to obtain 32 mg off-white solid.

MS: [M+H]=897.33, purity: 97.5%.

¹H NMR (500 MHz, Chloroform-*d*) δ 9.12 (s, 1H), 7.78 (dd, 1H), 7.71 (s, 2H), 7.70 - 7.61 (m, 2H), 7.50 (td, 1H), 7.44 - 7.35 (m, 2H), 7.25 (dd, 1H), 7.11 - 7.01 (m, 3H), 6.88 (s, 1H), 6.63 (dd, 1H), 5.24 - 5.08 (m, 3H), 4.29 (s, 1H), 4.36 - 4.10 (m, 3H), 3.94 (ddd, 1H), 3.84 - 3.61 (m, 3H), 3.50 (ddd, 1H), 3.39 - 3.26 (m, 3H), 3.28 (s, 2H), 3.29 - 3.18 (m, 5H), 2.96 - 2.84 (m, 1H), 2.65 - 2.53 (m, 2H), 2.13 - 2.02 (m, 1H), 1.43 (t, 3H).

### Synthesis example 40

### Synthesis of Compound 53

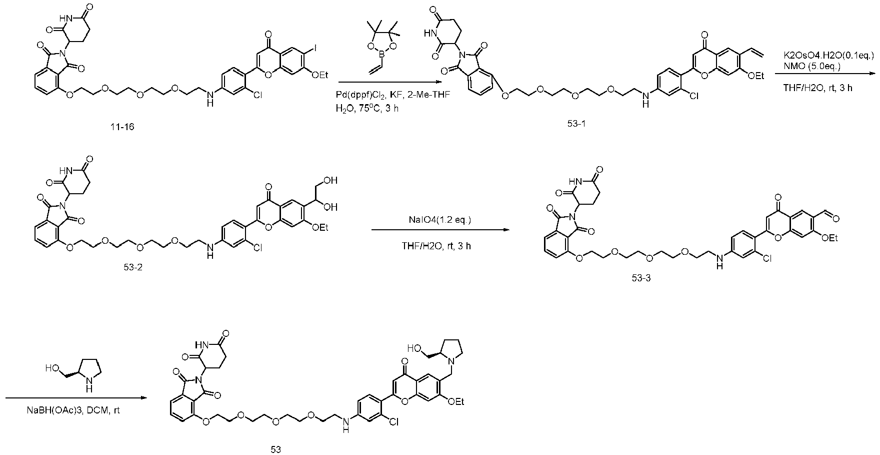

### (1) Synthesis of Compound 53-1

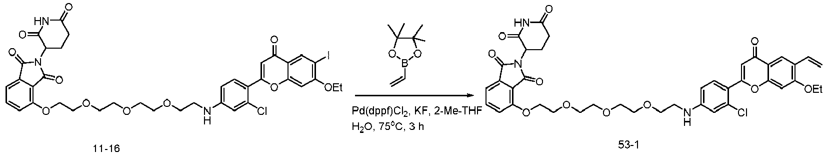

11-16 (200 mg, 1 eq.), 4,4,5,5-tetramethyl-2-vinyl-1,3,2-dioxaborolane (106 mg, 3 eq.) and KF (83 mg, 6.0 eq.) were added into 2-methyltetrahydrofuran (3 ml) and water (1 ml) with stirring, Pd(dppf)Cl₂ (17 mg, 0.1 eq.) was added under N₂ atmosphere, raised the temperature to 75°C to continue the reaction for 8 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 124 mg white solid. MS: [M+H]=775.2.

### (2) Synthesis of Compound 53-2

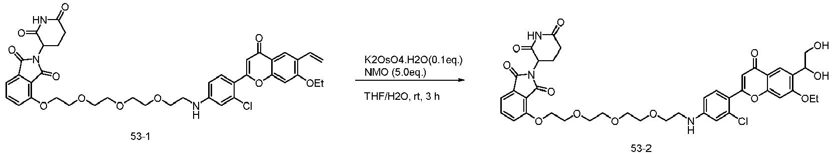

53-1 (124 mg, 1 eq.) K₂OsO₄.H₂O (7 mg, 0.1 eq.) and NMO (130 mg, 5.0 eq.) were added into 2-methyltetrahydrofuran (3 ml) and water (3 ml) with stirring for 3 h, followed by LCMS to monitor completion of the reaction. Saturated brine and EA were added directly to the reaction flask, extracted, filtered, spin-dried, and passed through pre-TLC (DCM:MeOH=20:1) to obtain 100 mg white solid. MS: [M+H]=809.2.

### (3) Synthesis of Compound 53-3

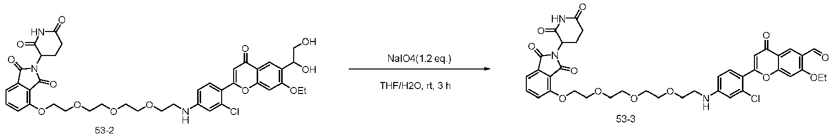

53-2 (100 mg, 1 eq.) was added into 2-methyltetrahydrofuran (3 ml) and water (3 ml) followed by sodium periodate (1.5 eq.) and stirred for 3 h, and the reaction was monitored by LCMS for completion. Saturated brine and EA were added directly to the reaction flask, extracted, filtered, spin-dried, and passed through pre-TLC (DCM:MeOH=20:1) to obtain 90 mg white solid. MS: [M+H]=777.2.

### (4) Synthesis of Compound 53

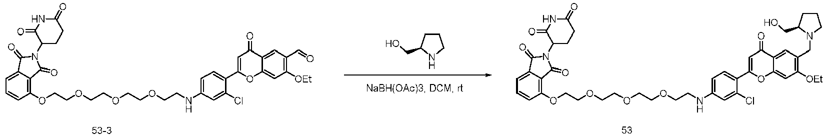

53-3 (35 mg, 1 eq.) and (R)-pyrrolidin-2-ylmethanol (3 eq.13.6 mg) were added into DCM (4 ml) followed by NaBH(OAc)₃ (1.5 eq.) and stirred for 2 h, and the reaction was monitored by LCMS for completion. The reaction was spin-dried directly and passed through pre-TLC (DCM:MeOH=20:1) to obtain 14 mg white solid. MS: [M+H]=861.34.

¹H NMR (400 MHz, DMSO-d6) δ 11.10 (s, 1H), 7.99 (s, 1H), 7.81 (d, J = 8.3 Hz, 1H), 7.72 (d, J = 8.7 Hz, 1H), 7.44 (d, J = 2.0 Hz, 1H), 7.35 (dd, J = 8.3, 2.2 Hz, 1H), 7.25 (d, J = 2.4 Hz, 1H), 7.17 (s, 1H), 7.11 (dd, J = 8.7, 2.4 Hz, 1H), 6.50 (s, 1H), 5.11 (dd, J = 12.9, 5.3 Hz, 1H), 4.33 - 4.26 (m, 2H), 4.24 - 4.15 (m, 4H), 3.78 (dt, J = 8.9, 4.9 Hz, 4H), 3.60 (dd, J = 5.4, 2.8 Hz, 4H), 3.54 (dd, J = 18.5, 4.9 Hz, 6H), 3.38 (s, 1H), 2.96 - 2.82 (m, 2H), 2.67 - 2.52 (m, 2H), 2.33 (s, 1H), 2.03 (dd, J = 10.1, 5.0 Hz, 1H), 1.91 (s, 2H), 1.65 (dd, J = 15.7, 6.9 Hz, 4H), 1.40 (t, J = 6.9 Hz, 3H).

### Synthesis example 41

### Synthesis of Compound 82

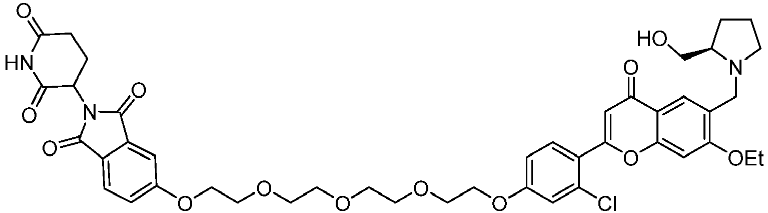

The preparation method is the same as the preparation method of compound 53, wherein the intermediate 11-16 was converted to intermediate 26-65.

MS: [M+H]=862.32.

¹H NMR (400 MHz, DMSO-d6) δ 11.10 (s, 1H), 7.99 (s, 1H), 7.81 (d, J = 8.3 Hz, 1H), 7.72 (d, J = 8.7 Hz, 1H), 7.44 (d, J = 2.0 Hz, 1H), 7.35 (dd, J = 8.3, 2.2 Hz, 1H), 7.25 (d, J = 2.4 Hz, 1H), 7.17 (s, 1H), 7.11 (dd, J = 8.7, 2.4 Hz, 1H), 6.50 (s, 1H), 5.11 (dd, J = 12.9, 5.3 Hz, 1H), 4.33 - 4.26 (m, 2H), 4.24 - 4.15 (m, 4H), 3.78 (dt, J = 8.9, 4.9 Hz, 4H), 3.60 (dd, J = 5.4, 2.8 Hz, 4H), 3.54 (dd, J = 18.5, 4.9 Hz, 6H), 3.38 (s, 1H), 2.96 - 2.82 (m, 2H), 2.67 - 2.52 (m, 2H), 2.33 (s, 1H), 2.03 (dd, J = 10.1, 5.0 Hz, 1H), 1.91 (s, 2H), 1.65 (dd, J = 15.7, 6.9 Hz, 4H), 1.40 (t, J = 6.9 Hz, 3H).

### Synthesis example 42

### Synthesis of Compound 54

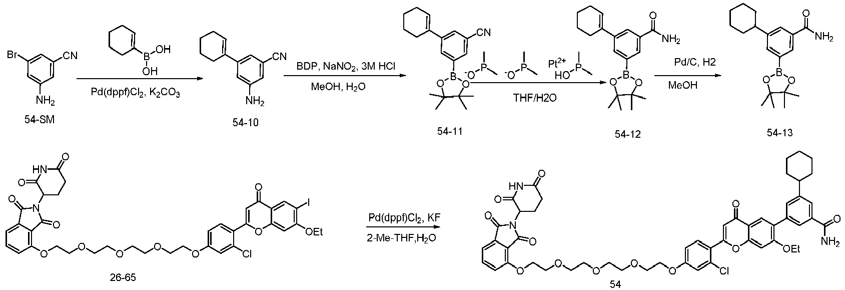

### Synthesis of 54-10

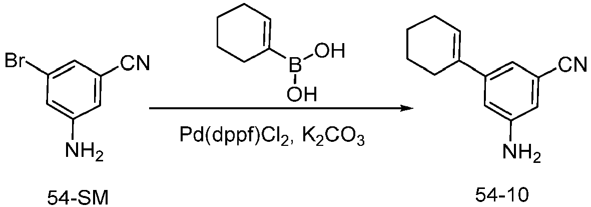

### (1) Synthesis of Compound 54-10

5 g T54-SM was added into 200 mL dioxane and 20 mL H₂O, followed by 4.1 g cyclohexeneboronic acid, 1.85 g Pd(dppf)Cl₂ and 6.98 g K₂CO₃, stirred after the addition, then stirred at 90°C for 16h under N₂ protection. The disappearance of the raw materials and the generation of product were confirmed by liquid quality monitor, extracted with ethyl acetate, the organic phase was dried, the system was concentrated, and silica gel column purification was carried out, eluted with 20% EtOAc in petroleum ether, concentrated to obtain 3.5 g of the target product (yield: 70%, purity: 97.90%). MS: [M+H] 199.11

¹H NMR (400 MHz, Chloroform-d) δ 7.03 (s, 1H), 6.89 - 6.83 (m, 1H), 6.76 (s, 1H), 6.11 (dt, *J* = 3.9, 2.3 Hz, 1H), 3.84 (s, 2H), 2.31 (q, *J* = 6.0 Hz, 2H), 2.20 (tt, *J* = 6.1, 3.1 Hz, 2H), 1.80-1.72 (m, 2H), 1.67-1.61 (m, 2H).

### (2) Synthesis of Compound 54-11

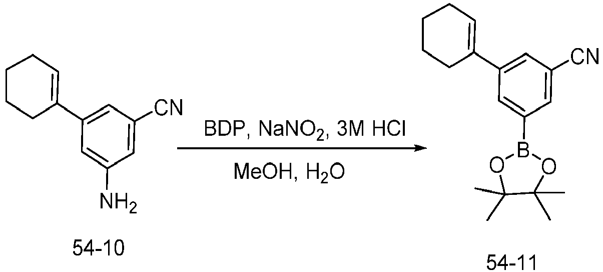

3 g of 54-10 was added into 50 mL of MeOH and 30 mL of H₂O, followed by 3M HCl (4.5 mL), an aqueous solution of 1g of sodium nitrite was added at 0°C, and further 11.5 g of pinacol diborate was added, stirred after the addition, then stirred for 16h under N₂ protection. The disappearance of the raw materials and the generation of product were confirmed by liquid quality monitor, extracted with ethyl acetate, the organic phase was dried, the system was concentrated, and silica gel column purification was carried out, eluted with 20% EtOAc in petroleum ether, concentrated to obtain 1.2 g of crude product (yield: 22%, purity: 97.90%). MS: [M+H] 310.24.

¹H NMR (400 MHz, Chloroform-*d*) δ 8.02 - 7.98 (m, 1H), 7.92 (t, *J* = 1.4 Hz, 1H), 7.69 (t, *J* = 1.7 Hz, 1H), 6.19 (hept, *J* = 1.8 Hz, 1H), 2.43 - 2.35 (m, 2H), 2.22 (tt, *J* = 6.1, 3.1 Hz, 2H), 1.84 - 1.75 (m, 2H), 1.69 - 1.64 (m, 2H), 1.35 (s, 12H).

### (3) Synthesis of Compound 54-12

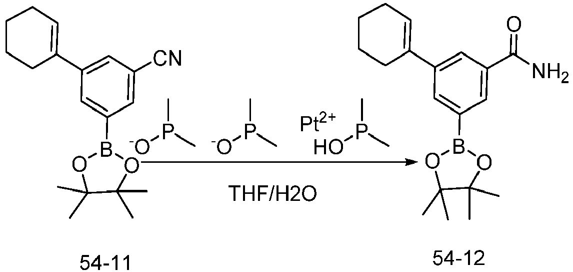

1.2 g of 54-11 was added into 100 mL of THF and 20 mL of H₂O, followed by 100 mg of hydrogenated (dimethylphosphinic acid-kP) [hydrobis (dimethylphosphinic acid-kP)] platinum (II), stirred after the addition, then stirred at 90°C for 16h under N₂ protection. The disappearance of the raw materials and the generation of product were confirmed by liquid quality monitor, extracted with ethyl acetate, the organic phase was dried, the system was concentrated, and silica gel column purification was carried out, eluted with 20% EtOAc in petroleum ether, concentrated to obtain 700 mg of the crude target product (yield: 55%, purity: 95.16%), MS: [M+H] 328.09.

### (4) Synthesis of Compound 54-13

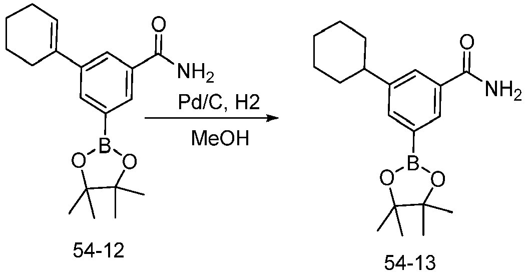

700 mg of 54-12 was added into 100 ml of MeOH, followed by 100 mg of Pd/C, stirred after the addition, then stirred at 90°C for 16h under N₂ protection. The disappearance of the raw materials and the generation of product were confirmed by liquid quality monitor, the reaction liquid was filtered by diatomite, and the organic phase system was concentrated and evaporated to directly obtain 500 mg of the target product (yield: 71%, purity: 98.80%). MS: [M+H] 330.27.

¹H NMR (400 MHz, DMSO-*d*₆) δ 8.14 (s, 1H), 8.03 (d, *J* = 5.5 Hz, 1H), 7.87 (s, 1H), 7.77 (d, *J* = 19.3 Hz, 1H), 7.65 (s, 1H), 2.58 (s, 1H), 1.82 (d, *J* = 10.6 Hz, 6H), 1.43 (dt, *J =* 24.5, 11.1 Hz, 6H), 1.33 (s, 9H).

### (5) Synthesis of Compound 54

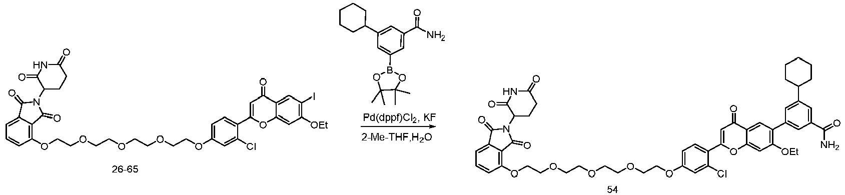

30 mg of 54-13 and 53 mg of 26-65 were added into 10 ml of 2-Me-THF and 2 mL of H₂O, followed by 8 mg of Pd(dppf)Cl₂ and 40 mg of KF, stirred after the addition, then stirred at 90°C for 16h under N₂ protection. The disappearance of the raw materials and the generation of product were confirmed by liquid quality monitor, the system was concentrated and evaporated, a proper amount of DMF was used for dissolving the residue, a C18 reversed-phase column was used for loading, reversed-phase separation was carried out, Eluted with 10% ACN to obtain 17.8 mg of the target product (yield: 31%, purity: 95.16%), MS: [M+H] 950.44.

¹H NMR (400 MHz, DMSO-*d*₆) δ 11.11 (s, 1H), 8.05 (s, 1H), 7.97 (s, 1H), 7.88 (s, 1H), 7.84 - 7.74 (m, 3H), 7.60 (s, 1H), 7.59 - 7.46 (m, 2H), 7.44 (d, *J* = 1.9 Hz, 1H), 7.39 - 7.30 (m, 3H), 7.27 (d, *J* = 2.3 Hz, 1H), 7.13 (dd, *J* = 8.7, 2.3 Hz, 1H), 6.56 (s, 1H), 5.11 (dd, *J* = 13.0, 5.4 Hz, 1H), 4.30 (d, *J* = 4.4 Hz, 2H), 4.21 (q, *J* = 6.8 Hz, 4H), 3.79 (s, 4H), 3.58 (dd, *J* = 13.7, 3.8 Hz, 8H), 2.56 (s, 3H), 2.06 - 1.99 (m, 1H), 1.84 (t, *J* = 14.0 Hz, 4H), 1.45 (dt, *J* = 25.1, 12.1 Hz, 4H), 1.33 (t, *J* = 6.9 Hz, 4H).

### Synthesis example 43

### Synthesis of Compound 55

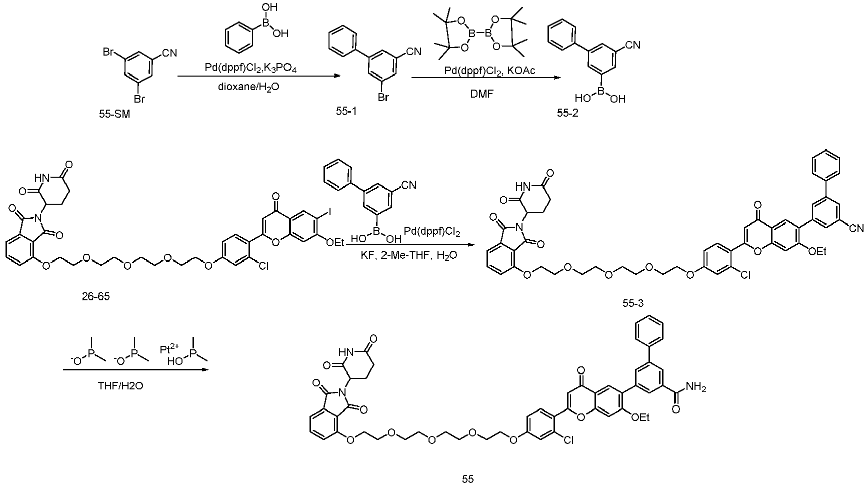

### (1) Synthesis of Compound 55-1

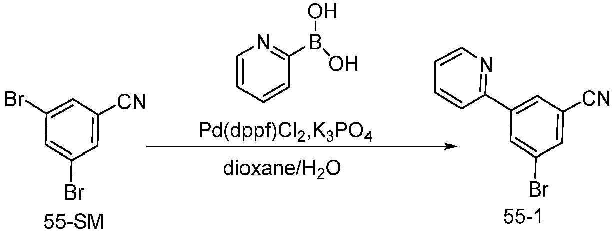

500 mg of 55-SM was added into 50 mL of dioxane and 10 mL of H₂O, followed 518 mg of phenylboronic acid, 282 mg of Pd(dppf)Cl₂ and 1.62 g of K₃PO₄ , stirred after the addition, then stirred at 90°C for 16h under N₂ protection. The disappearance of the raw materials and the generation of product were confirmed by liquid quality monitor, the system was concentrated and evaporated, a proper amount of DMF was used for dissolving the residue, a C18 reversed-phase column was used for loading, reversed-phase separation was carried out, Eluted with 60% ACN to obtain 200 mg of the target product (yield: 40%, purity: 97.90%). MS: [M+H] 258.12

### (2) Synthesis of Compound 55-2

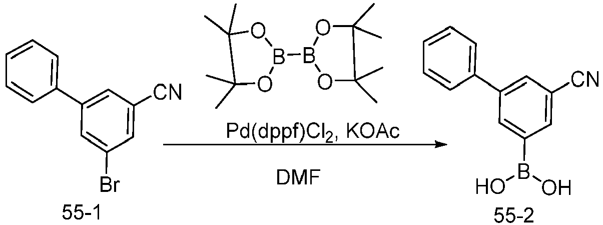

200 mg of 55-1 was added into 50 ml of DMF, followed by 253 mg of bisphenopinacol borate, 56.14 mg of Pd(dppf)Cl₂ and 150 mg of KOAc were added, stirred after the addition, then stirred at 90°C for 16h under N₂ protection. The disappearance of the raw materials and the generation of product were confirmed by liquid quality monitor, the system was concentrated and evaporated, a proper amount of DMF was used for dissolving the residue, a C18 reversed-phase column was used for loading, reversed-phase separation was carried out, Eluted with 10% ACN to obtain 150 mg of the target product (yield: 86%, purity: 97.90%). MS: [M+H] 224.22.

### (3) Synthesis of Compound 55-3

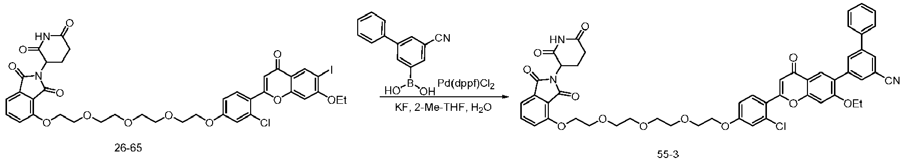

100 mg of 55-2 and 100 mg of 26-65 were added into 10 mL of 2-Me-THF and 2 mL of H₂O, followed by 17 mg of Pd(dppf)Cl₂ and 40 mg of KF, stirred after the addition, then stirred at 90°C for 16h under N₂ protection. The disappearance of the raw materials and the generation of product were confirmed by liquid quality monitor, the system was concentrated and evaporated, a proper amount of DMF was used for dissolving the residue, a C18 reversed-phase column was used for loading, reversed-phase separation was carried out, Eluted with 10% ACN to obtain 40 mg of the target product (yield: 38%, purity: 95.16%), MS: [M+H] 925.19.

¹H NMR (400 MHz, DMSO-d6) δ 11.11 (s, 1H), 8.20 - 8.14 (m, 2H), 8.04 (d, J = 10.6 Hz, 2H), 7.81 (d, J = 8.1 Hz, 3H), 7.76 (d, J = 8.7 Hz, 1H), 7.53 (t, J = 7.5 Hz, 2H), 7.48 - 7.42 (m, 2H), 7.40 - 7.32 (m, 2H), 7.27 (d, J = 2.4 Hz, 1H), 7.13 (dd, J = 8.8, 2.4 Hz, 1H), 6.57 (s, 1H), 5.11 (dd, J = 12.9, 5.4 Hz, 1H), 4.27 (ddt, J = 19.4, 8.8, 4.6 Hz, 6H), 3.78 (q, J = 6.2 Hz, 4H), 3.64 - 3.54 (m, 8H), 2.95 - 2.81 (m, 1H), 2.63 - 2.51 (m, 2H), 2.06 - 1.99 (m, 1H), 1.35 (t, J = 6.9 Hz, 3H).

### (4) Synthesis of Compound 55

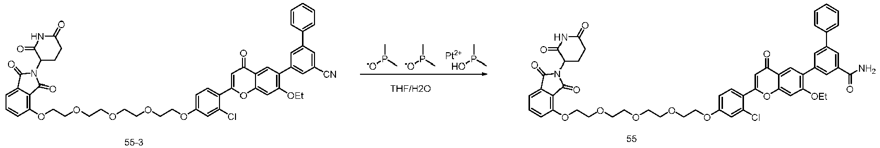

40 mg of 55-3 was added into 10 mL of THF and 2 mL of H₂O, followed by 20 mg of hydrogenated (dimethylphosphinic acid-kP) [hydrobis (dimethylphosphinic acid-kP)] platinum (II), stirred after the addition, then stirred at 90°C for 16h under N₂ protection. The disappearance of the raw materials and the generation of product were confirmed by liquid quality monitor, anhydrous sodium sulfate was added, and the organic phase system was concentrated and evaporated, a proper amount of DCM was used for dissolving the residue and loaded on Prep-TLC, developed with DCM/MeOH(15:1), and freeze-dried to obtain 17.8 mg of the target product (yield: 44.5%, purity: 98.80%). MS: [M+H] 944.39.

¹H NMR (400 MHz, CDCL3) δ 9.51 (s, 1H), 8.25 (s, 1H), 7.99 (t, *J* = 2.1 Hz, 1H), 7.96 - 7.87 (m, 2H), 7.66 (d, *J* = 8.5 Hz, 1H), 7.62 - 7.32 (m, 9H), 7.06 - 6.99 (m, 2H), 6.92 (d, *J* = 2.1 Hz, 1H), 6.84 - 6.73 (m, 2H), 5.24 (t, *J* = 3.5 Hz, 1H), 4.17 - 4.07 (m, 6H), 3.78 (t, *J* = 5.0 Hz, 4H), 3.74 - 3.62 (m, 7H), 2.92 - 2.81 (m, 1H), 2.64 - 2.49 (m, 2H), 2.15 - 2.03 (m, 1H), 1.37 (d, *J* = 12.5 Hz, 1H).

### Synthesis example 44

### Synthesis of Compound 83

Intermediate 26-65 was replaced with 11-16 in the same manner as compound 55.

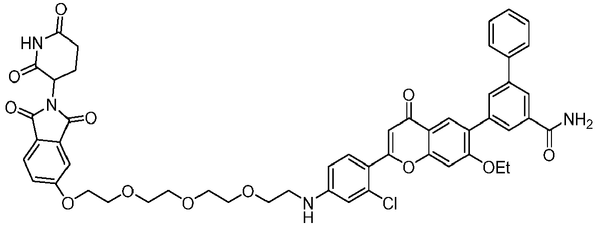

MS: [M+H]943.39, ¹H NMR (400 MHz, DMSO-d6) δ 11.12 (s, 1H), 8.23 (s, 1H), 8.17 (s, 1H), 8.04 (d, J = 14.8 Hz, 3H), 7.85-7.74 (m, 4H), 7.53 (t, J = 7.7 Hz, 3H), 7.43 (d, J = 8.4 Hz, 2H), 7.39-7.32 (m, 2H), 7.28 (d, J = 2.4 Hz, 1H), 7.17-7.10 (m, 1H), 6.57 (s, 1H), 5.11 (dd, J = 12.9, 5.4 Hz, 1H), 4.29-4.21 (m, 4H), 3.82-3.75 (m, 4H), 3.58 (dd, J = 13.9, 3.9 Hz, 8H), 2.92 (s, 1H), 2.58 (d, J = 17.5 Hz, 2H), 2.18 (t, J = 7.5 Hz, 1H),1.35 (t, J = 6.9 Hz, 3H)

### Synthesis example 45

### Synthesis of Compound 56

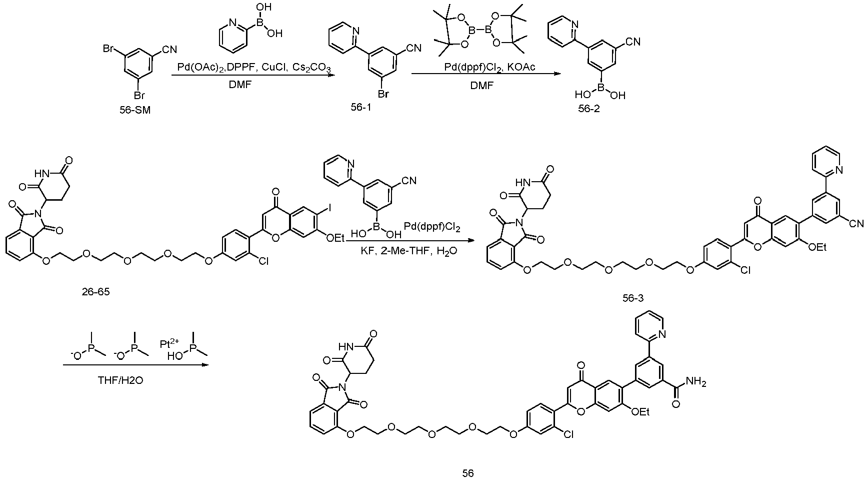

### (1) Synthesis of Compound 56-1

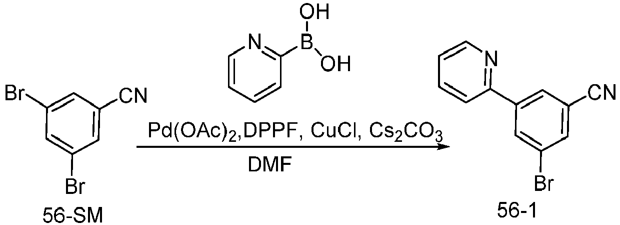

2 g of 56-SM was added into 100 mL of dioxane and 20 mL of H₂O, followed by 1.55 g of pyridine boronic acid, 860 mg of Pd(OAc)₂ and 429 mg of DPPF, 1g of CuCI and 5 g of Cs₂CO₃ , stirred after the addition, then stirred at 100°C for 16h under N₂ protection. The disappearance of the raw materials and the generation of product were confirmed by liquid quality monitor, the system was concentrated and evaporated, water, ethyl acetate was added for extraction, the organic phase was dried and concentrated, the residue was applied to a silica gel column, eluted with 40% EtOAc in petroleum ether, concentrated to obtain 1.2 g of target product (yield: 60%, purity: 97.90%). MS: [M+H] 259.46

### (2) Synthesis of Compound 56-2

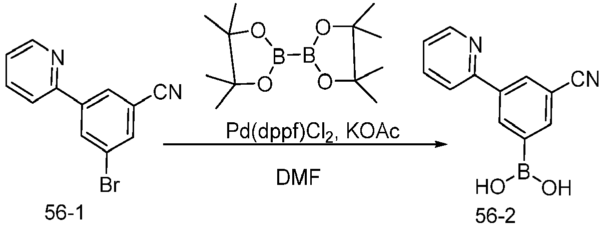

1.2 g of 56-1 was added into 50 ml of DMF, followed by 1.2 g of bisphenopinacol borate, followed by 336 mg of Pd(dppf)Cl₂ and 900 mg of KOAc, stirred after the addition, then stirred at 90°C for 16h under N₂ protection. The disappearance of the raw materials and the generation of product were confirmed by liquid quality monitor, the system was concentrated and evaporated, a proper amount of DMF was used for dissolving the residue, a C18 reversed-phase column was used for loading, reversed-phase separation was carried out, Eluted with 10% ACN to obtain 600 mg of the target product (yield: 60%, purity: 97.90%). MS: [M+H] 225.15.

### (3) Synthesis of Compound 56-3

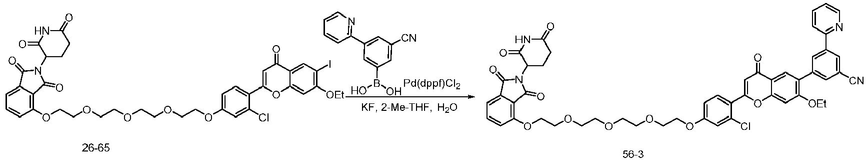

100 mg of 56-2 and 100 mg of 26-65 were added into 10 ml of 2-Me-THF and 2 mL of H₂O, followed by 17 mg of Pd(dppf)Cl₂ and 40 mg of KF, stirred after the addition, then stirred at 90°C for 16h under N₂ protection. The disappearance of the raw materials and the generation of product were confirmed by liquid quality monitor, the system was concentrated and evaporated, a proper amount of DMF was used for dissolving the residue, a C18 reversed-phase column was used for loading, reversed-phase separation was carried out, Eluted with 70% ACN to obtain 60 mg of the target product (yield: 56.7%, purity: 95.16%), MS: [M+H] 926.11.

¹H NMR (400 MHz, DMSO-*d*₆) δ 8.74 (d, *J* = 4.3 Hz, 1H), 8.64 (s, 1H), 8.53 (s, 1H), 8.46 (s, 1H), 8.18 (d, *J* = 8.0 Hz, 1H), 8.12 (s, 1H), 8.06 (s, 1H), 8.01 - 7.92 (m, 1H), 7.79 (dd, *J* = 19.2, 8.5 Hz, 2H), 7.49 - 7.42 (m, 2H), 7.41 - 7.32 (m, 2H), 7.27 (d, *J* = 2.4 Hz, 1H), 7.13 (dd, *J* = 8.7, 2.4 Hz, 1H), 6.58 (s, 1H), 5.11 (dd, *J* = 12.9, 5.4 Hz, 1H), 4.34 - 4.20 (m, 6H), 3.78 (q, *J* = 6.3 Hz, 4H), 3.63 - 3.54 (m, 8H), 2.95 - 2.81 (m, 1H), 2.62 - 2.52 (m, 2H), 2.06 - 1.99 (m, 1H), 1.34 (t, *J* = 6.9 Hz, 3H).

### (4) Synthesis of Compound 56

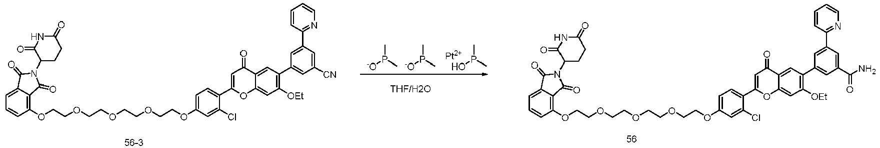

40 mg of 56-3 was added into 10 ml of THF and 2 mL of H₂O, followed by 20 mg of hydrogenated (dimethylphosphinic acid-kP) [hydrobis (dimethylphosphinic acid-kP)] platinum (II), stirred after the addition, then stirred at 90°C for 16h under N₂ protection. The disappearance of the raw materials and the generation of product were confirmed by liquid quality monitor, the system was concentrated and evaporated, a proper amount of DCM was used for dissolving the residue and loaded on Prep-TLC, developed with DCM/MeOH(15:1), and freeze-dried to obtain 12 mg of the target product (yield: 30%, purity: 98.80%). MS: [M+H] 945.38.

¹H NMR (400 MHz, CDCL3) δ 9.51 (s, 1H), 8.77 (m, 1H), 8.33 - 8.23 (m, 2H), 8.05 (m, 2H), 7.90 (d, m 1H), 7.71 (dd, 1H), 7.71 - 7.61 (m, 2H), 7.59 (d, 1H), 7.46 (s, 2H), 7.20 (m, 1H), 7.07 - 6.98 (m, 2H), 6.92 (d,, 1H), 6.80 (m, 1H), 6.75 (s, 1H), 5.24 (m, 1H), 4.18 - 4.06 (m, 6H), 3.78 (m, 4H), 3.74 - 3.61 (m, 8H), 2.93 - 2.80 (m, 1H), 2.65 - 2.48 (m, 2H), 2.16 - 2.02 (m, 1H), 1.37 (m, 3H).

### Synthesis example 46

### Synthesis of Compound 84

Intermediate 26-65 was replaced with 11-16 in the same manner as compound 56.

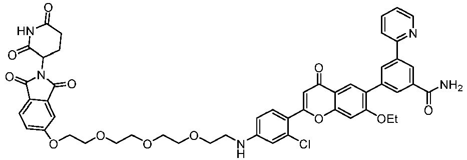

MS: [M+H] 943.28 HNMR: ¹H NMR (400 MHz, DMSO-*d*₆) δ 11.10 (s, 1H), 8.58 (s, 1H), 8.47 (s, 1H), 8.23 (s, 1H), 8.17 - 8.04 (m, 3H), 7.95 (t, *J* = 7.7 Hz, 1H), 7.80 (dd, *J* = 16.0, 8.5 Hz, 2H), 7.51 - 7.32 (m, 5H), 7.28 (d, *J* = 2.4 Hz, 1H), 7.13 (dd, *J* = 8.8, 2.4 Hz, 1H), 6.58 (s, 1H), 5.11 (dd, *J* = 12.9, 5.4 Hz, 1H), 4.33 - 4.21 (m, 6H), 3.83 - 3.75 (m, 4H), 3.65 - 3.54 (m, 8H), 2.07 - 1.95 (m, 2H), 1.35 (s, 3H).

### Synthesis example 47

### Synthesis of Compound 57

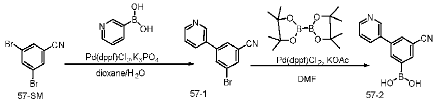

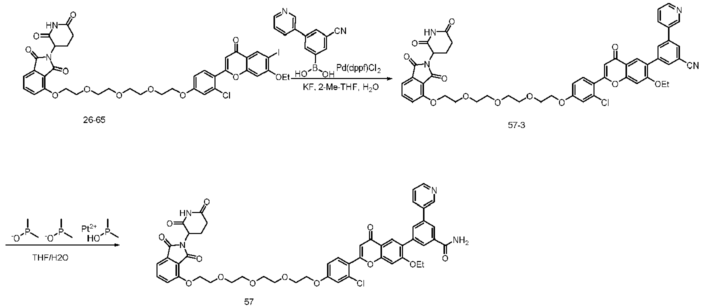

### (1) Synthesis of Compound 57-1

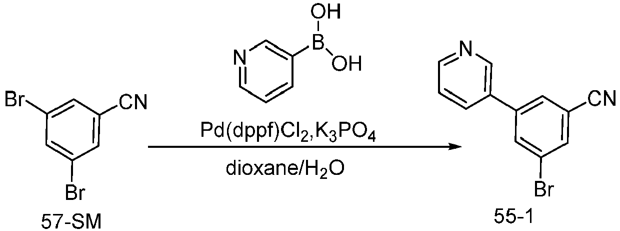

500 mg of 57-SM was added into 50 ml of dioxane and 10 ml of Hz O, followed by 518 mg of pyridine boronic acid, 282 mg of Pd(dppf)Cl₂ and 1.62 g of K₃PO₄, stirred after the addition, then stirred at 90°C for 16h under N₂ protection. The disappearance of the raw materials and the generation of product were confirmed by liquid quality monitor, the system was concentrated and evaporated, a proper amount of DMF was used for dissolving the residue, a C18 reversed-phase column was used for loading, reversed-phase separation was carried out, Eluted with 60% ACN to obtain 200 mg of the target product (yield: 40%, purity: 97.90%). MS: [M+H]259.14

### (2) Synthesis of Compound 57-2

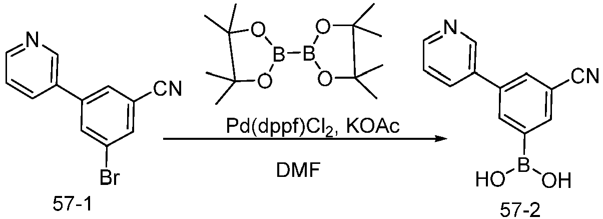

200 mg of 57-1 was added into 50 ml of DMF, followed by 253 mg of bisphenopinacol borate, 56.14 mg of Pd(dppf)Cl₂ and 150 mg of KOAc, stirred after the addition, then stirred at 90°C for 16h under N₂ protection. The disappearance of the raw materials and the generation of product were confirmed by liquid quality monitor, the system was concentrated and evaporated, a proper amount of DMF was used for dissolving the residue, a C18 reversed-phase column was used for loading, reversed-phase separation was carried out, Eluted with 10% ACN to obtain 150 mg of the target product (yield: 86%, purity: 97.90%). MS: [M+H] 225.33.

### (3) Synthesis of Compound 57-3

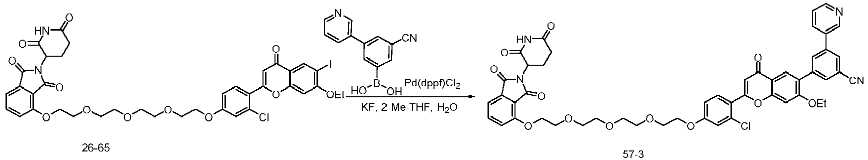

100 mg of 57-2 and 100 mg of 26-65 were added into 10 ml of 2-Me-THF and 2 mL of H₂O, followed by 17 mg of Pd(dppf)Cl₂ and 40 mg of KF, stirred after the addition, then stirred at 90°C for 16h under N₂ protection. The disappearance of the raw materials and the generation of product were confirmed by liquid quality monitor, the system was concentrated and evaporated, a proper amount of DMF was used for dissolving the residue, a C18 reversed-phase column was used for loading, reversed-phase separation was carried out, Eluted with 10% ACN to obtain 40 mg of the target product (yield: 16%, purity: 95.16%), MS: [M+H] 927.21.

¹H NMR (400 MHz, DMSO-d6) δ 11.10 (s, 1H), 9.04 (s, 1H), 8.65 (d, J = 5.0 Hz, 1H), 8.25 (d, J = 17.4 Hz, 3H), 8.10 (d, J = 4.9 Hz, 2H), 7.79 (dd, J = 19.8, 8.5 Hz, 2H), 7.60-7.53 (m, 1H), 7.44 (s, 1H), 7.40-7.32 (m, 2H), 7.27 (d, J = 2.2 Hz, 1H), 6.57 (s, 1H), 5.11 (dd, J = 12.9, 5.2 Hz, 1H), 4.31 (s, 2H), 4.29-4.21 (m, 4H), 3.59 (d, J = 9.9 Hz, 8H), 2.89 (s, 1H), 2.52 (s, 2H), 2.02 (d, J = 7.4 Hz, 1H), 1.36-1.32 (m, 3H).

### (4) Synthesis of Compound 57

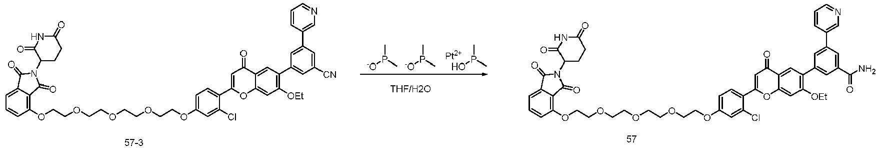

40 mg of 57-3 was added into 10 mL of THF and 2 mL of H₂O, followed by 20 mg of hydrogenated (dimethylphosphinic acid-kP) [hydrobis (dimethylphosphinic acid-kP)] platinum (II), stirred after the addition, then stirred at 90°C for 16h under N₂ protection. The disappearance of the raw materials and the generation of product were confirmed by liquid quality monitor, anhydrous sodium sulfate was added, the organic phase system was concentrated and evaporated, a proper amount of DCM was used for dissolving the residue and loaded on Prep-TLC, developed with DCM/MeOH(15:1), and freeze-dried to obtain 17.8 mg of the target product (yield: 44.5%, purity: 98.80%). MS: [M+H] 945.38. ,

¹H NMR (400 MHz, CDCL3) δ 9.51 (s, 1H), 8.25 (s, 1H), 7.99 (t, *J* = 2.1 Hz, 1H), 7.96 - 7.87 (m, 2H), 7.66 (d, *J* = 8.5 Hz, 1H), 7.62 - 7.32 (m, 9H), 7.06 - 6.99 (m, 2H), 6.92 (d, *J* = 2.1 Hz, 1H), 6.84 - 6.73 (m, 2H), 5.24 (t, *J* = 3.5 Hz, 1H), 4.17 - 4.07 (m, 6H), 3.78 (t, *J* = 5.0 Hz, 4H), 3.74 - 3.62 (m, 7H), 2.92 - 2.81 (m, 1H), 2.64 - 2.49 (m, 2H), 2.15 - 2.03 (m, 1H), 1.37 (d, *J* = 12.5 Hz, 1H).

### Synthesis example 48

### Synthesis of Compound 58

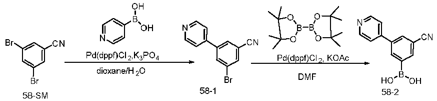

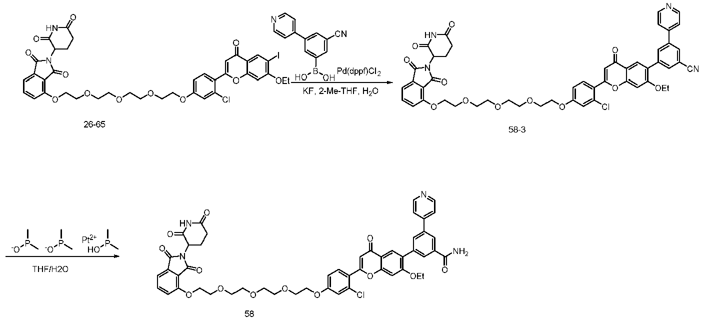

### (1) Synthesis of Compound 58-1

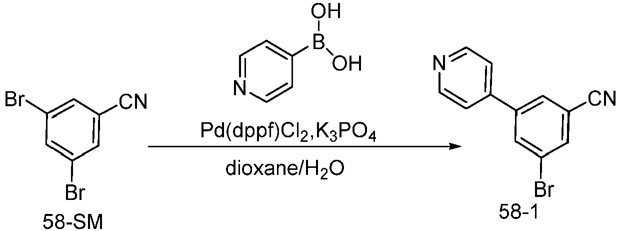

500 mg of 58-SM was added into 50 mL of dioxane and 10 mL of Hz O, followed by 518 mg of pyridine boronic acid, 282 mg of Pd(dppf)Cl₂ and 1.62 g of K₃PO₄ , stirred after the addition, then stirred at 90°C for 16h under N₂ protection. The disappearance of the raw materials and the generation of product were confirmed by liquid quality monitor, the system was concentrated and evaporated, a proper amount of DMF was used for dissolving the residue, a C18 reversed-phase column was used for loading, reversed-phase separation was carried out, Eluted with 60% ACN to obtain200 mg of the target product (yield: 40%, purity: 97.90%). MS: [M+H] 259.32.

### (2) Synthesis of Compound 58-2

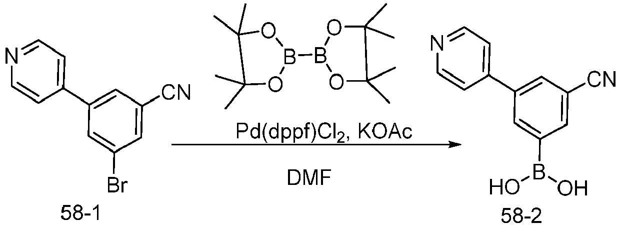

200 mg of 58-1 was added into 50 ml of DMF, followed by 253 mg of bisphenopinacol borate, 56.14 mg of Pd(dppf)Cl₂ and 150 mg of KOAc, stirred after the addition, then stirred at 90°C for 16h under N₂ protection. The disappearance of the raw materials and the generation of product were confirmed by liquid quality monitor, the system was concentrated and evaporated, a proper amount of DMF was used for dissolving the residue, a C18 reversed-phase column was used for loading, reversed-phase separation was carried out, Eluted with 10% ACN to obtain 150 mg of the target product (yield: 86%, purity: 97.90%). MS: [M+H] 225.15.

### (3) Synthesis of Compound 58-3

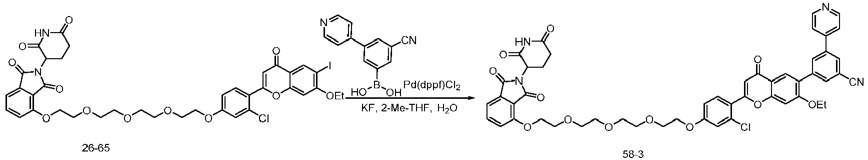

90 mg of 58-2 and 100 mg of 26-65 were added into 10 mL of 2-Me-THF and 2 mL of H₂O, followed by 17 mg of Pd(dppf)Cl₂ and 40 mg of KF, stirred after the addition, then stirred at 90°C for 16h under N₂ protection. The disappearance of the raw materials and the generation of product were confirmed by liquid quality monitor, the system was concentrated and evaporated, a proper amount of DMF was used for dissolving the residue, a C18 reversed-phase column was used for loading, reversed-phase separation was carried out, Eluted with 10% ACN to obtain 40 mg of the target product (yield: 41%, purity: 95.16%), MS: [M+H] 927.23.

¹H NMR (400 MHz, DMSO-d6) δ 11.10 (s, 1H), 9.04 (s, 1H), 8.65 (d, J = 5.0 Hz, 1H), 8.25 (d, J = 17.4 Hz, 3H), 8.10 (d, J = 4.9 Hz, 2H), 7.79 (dd, J = 19.8, 8.5 Hz, 2H), 7.60-7.53 (m, 1H), 7.44 (s, 1H), 7.40-7.32 (m, 2H), 7.27 (d, J = 2.2 Hz, 1H), 6.57 (s, 1H), 5.11 (dd, J = 12.9, 5.2 Hz, 1H), 4.31 (s, 2H), 4.29-4.21 (m, 4H), 3.59 (d, J = 9.9 Hz, 8H), 2.89 (s, 1H), 2.52 (s, 2H), 2.02 (d, J = 7.4 Hz, 1H), 1.36-1.32 (m, 3H).

### (4) Synthesis of Compound 58

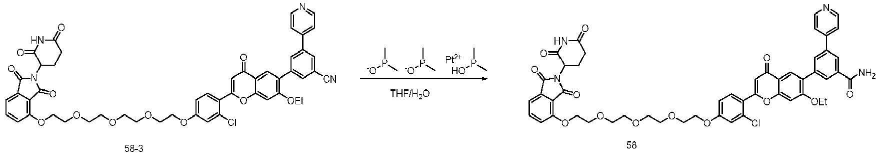

40 mg of 58-3 was added into 10 mL of THF and 2 mL of H₂O, followed by 20 mg of hydrogenated (dimethylphosphinic acid-kP) [hydrobis (dimethylphosphinic acid-kP)] platinum (II), stirred after the addition, then stirred at 90°C for 16h under N₂ protection. The disappearance of the raw materials and the generation of product were confirmed by liquid quality monitor, anhydrous sodium sulfate was added, the organic phase system was concentrated and evaporated, a proper amount of DCM was used for dissolving the residue and loaded on Prep-TLC, developed with DCM/MeOH(15:1), and freeze-dried to obtain 17.8 mg of the target product (yield: 44.5%, purity: 98.80%), MS: [M+H] 945.38.

¹H NMR (400 MHz, DMSO-d6) δ 11.11 (s, 1H), 8.75 (d, J = 5.6 Hz, 2H), 8.35 (d, J = 7.1 Hz, 2H), 8.17 (s, 1H), 8.09 (s, 1H), 7.94 (d, J = 5.8 Hz, 2H), 7.81 (d, J = 8.3 Hz, 1H), 7.76 (d, J = 8.7 Hz, 1H), 7.44 (d, J = 2.1 Hz, 1H), 7.41 - 7.32 (m, 2H), 7.27 (d, J = 2.4 Hz, 1H), 7.13 (dd, J = 8.7, 2.4 Hz, 1H), 6.58 (s, 1H), 5.11 (dd, J = 12.9, 5.2 Hz, 1H), 4.34-4.22 (m, 6H), 3.78 (q, J = 6.2 Hz, 4H), 3.63-3.55 (m, 8H), 2.93-2.81 (m, 1H), 2.56 (s, 2H), 2.06- 1.99 (m, 1H), 1.34 (t, J = 6.9 Hz, 3H).

### Synthesis example 49

### Synthesis of Compound 59

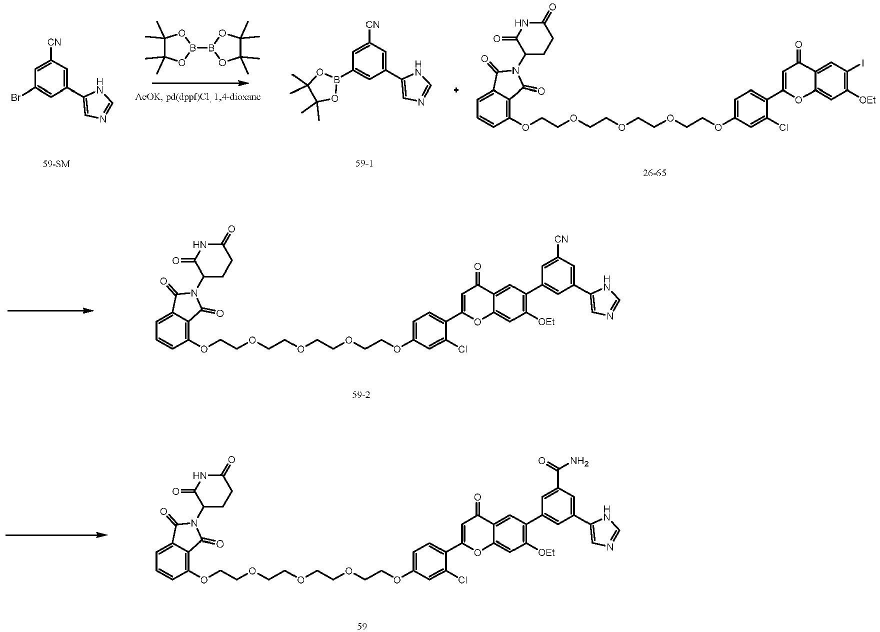

### (1) Synthesis of Compound 59-1

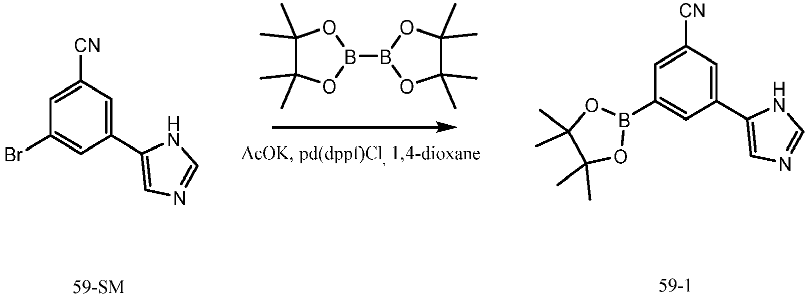

203 mg of 59-SM was dissolved in 20 ml of 1, 4-dioxane, 311 mg of bisphenopinacol borate, 240 mg of potassium acetate, 179 mg of Pd(dppf)Cl₂ were added.

Protected by N₂, replaced 3 times, heated to 80°C and stirred overnight.

Sample was taken and sent to LC-MS, producing boric acid and boric acid ester, and concentrated the reaction solution.

Purification: 40 g C18 silica gel column pre-filled and the concentrate was loaded with 4 ml MeOH.

Eluted with 0-60% ACN\H₂O.

Concentrated at 40°C to obtain 177 mg off-white solid, borate was hydrolyzed to boric acid during purification.

### (2) Synthesis of Compound 59-2

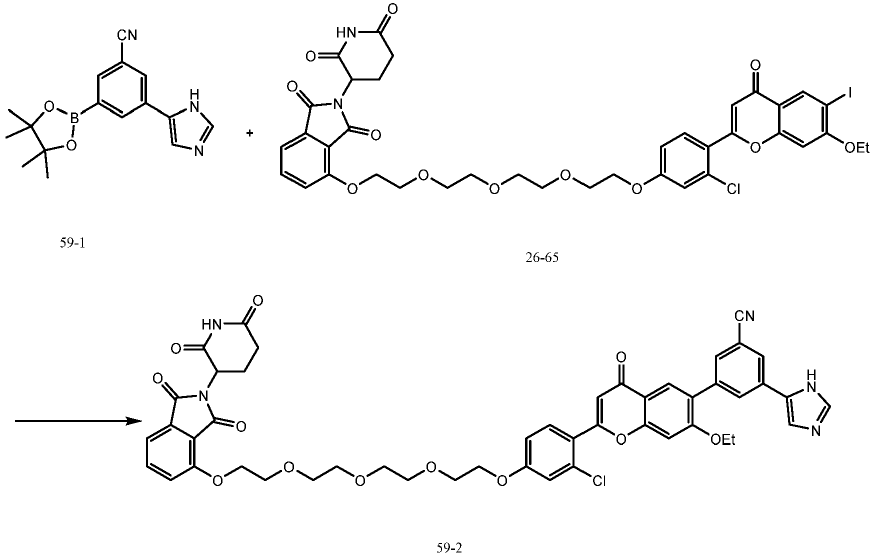

The raw material was dissolved in 2-MTHF, KF and Pd(dppf)Cl₂ were added, followed by 1 ml of water, protected with N₂, 70°C, stirred overnight, anhydrous sodium sulfate was added into the reaction solution, filtered, washed 2 times with DCM, concentrated, purified: silica gel column pre-filled, dissolved and loaded the sample with DCM, eluted with 0-5% DCM\MeOH, collected chromatographic solution, and concentrated to obtain 69mg white solid.

### (3) Synthesis of Compound 59

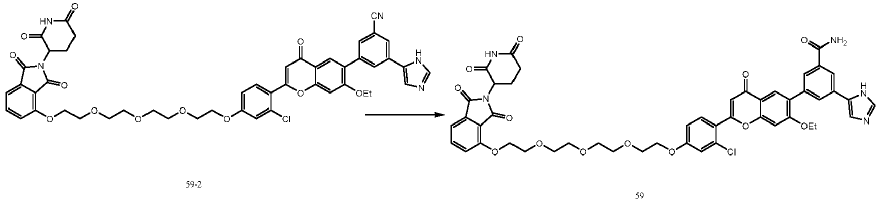

The raw material was dissolved in ultra-dry acetonitrile, a catalytic amount of hydrogenation (dimethyl phosphinic acid-kP) [hydrogen bis (dimethyl phosphinic acid-kP)] platinum (II) was added, stirred overnight, filtered, concentrated and purified: silica gel column pre-filled, dissolved and loaded the sample with DCM, eluted with 0-5% DCM\MeOH, collected chromatographic solution, and concentrated to obtain 29mg white solid. MS: [M+H] 934.35, purity: 97.7%.

¹H NMR (500 MHz, Chloroform-d) δ 9.12 (s, 1H), 8.29 - 8.22 (m, 2H), 8.07 (dt, 2H), 7.71 - 7.61 (m, 3H), 7.56 (d,1H), 7.41 (dd, 1H), 7.13 (d, 1H), 7.05 (s, 1H), 7.00 (s, 1H), 6.87 (dd, 1H), 6.32 - 6.28 (m, 2H), 4.41 (ddt, 2H), 4.28 - 4.17 (m, 3H), 3.94 (ddd, 1H), 3.79 (ddd, 1H), 3.45 - 3.38 (m, 2H), 3.36 - 3.18 (m, 9H), 2.96 - 2.84 (m, 1H), 2.65 - 2.53 (m, 2H), 2.13 - 2.02 (m, 1H), 1.45 (t, 3H).

### Synthesis example 50

### Synthesis of Compound 60

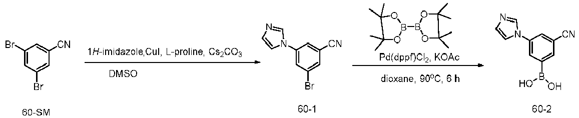

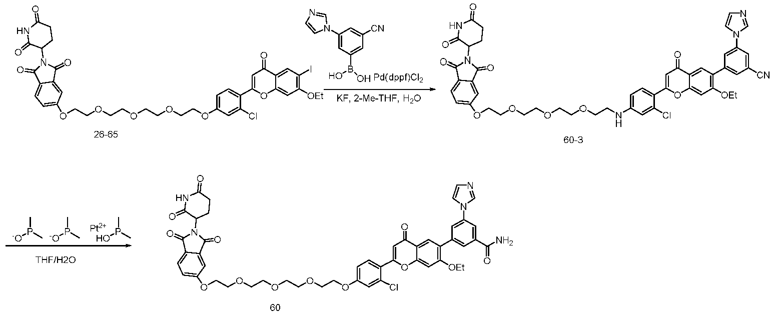

### (1) Synthesis of Compound 60-1

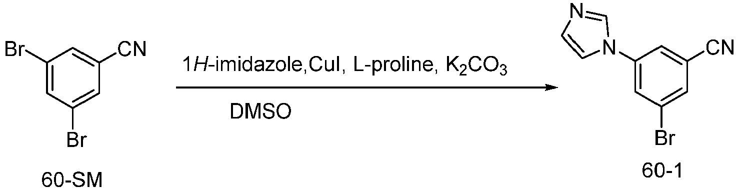

2 g of 60-SM was added into 200 ml of DMSO, followed by 0.79 g of imidazole, 295 mg of Cul and 471 mg of L-proline, 3.2g of K₂CO₃, stirred for 16h at 110°C under protection of N₂. The disappearance of the raw materials and the generation of product were confirmed by liquid quality monitor, the system was concentrated and evaporated, a proper amount of DMF was used for dissolving the residue, a C18 reversed-phase column was used for loading, reversed-phase separation was carried out, Eluted with 60% ACN to obtain 1.1 g of the target product (yield: 57%, purity: 97.90%). MS: [M+H] 248.38.

### (2) Synthesis of Compound 60-2

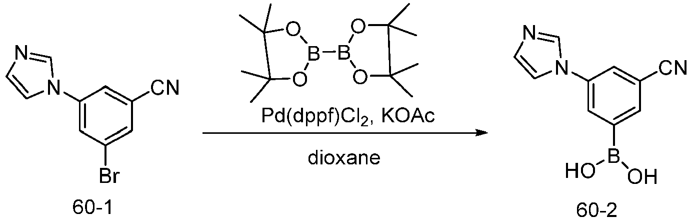

50 ml of DMF was added into 500 mg of 60-1, followed by 768 mg of bisphenopinacol borate, 146 mg of Pd(dppf)Cl₂ and 392 mg of KOAc, stirred after the addition, then stirred at 90°C for 16h under N₂ protection. The disappearance of the raw materials and the generation of product were confirmed by liquid quality monitor, the system was concentrated and evaporated, a proper amount of DMF was used for dissolving the residue, a C18 reversed-phase column was used for loading, reversed-phase separation was carried out, Eluted with 10% ACN to obtain 300 mg of the target product (yield: 69%, purity: 97.90 %). MS: [M+H] 214.

### (3) Synthesis of Compound 60-3

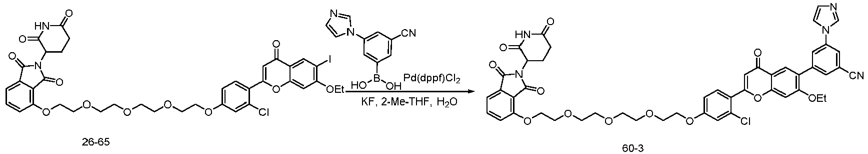

90 mg of 60-2 and 100 mg of 26-65 were added into 10 mL of 2-Me-THF and 2 mL of H₂O, followed by 8.3 mg of Pd(dppf)CI ₂ and 40 mg of KF, stirred after the addition, then stirred at 90°C for 16h under N₂ protection. The disappearance of the raw materials and the generation of product were confirmed by liquid quality monitor, the system was concentrated and evaporated, a proper amount of DMF was used for dissolving the residue, a C18 reversed-phase column was used for loading, reversed-phase separation was carried out, Eluted with 10% ACN to obtain 40 mg of the target product (yield: 41%, purity: 95.16%), MS: MS: [M+H] 916.22.

¹H NMR (400 MHz, DMSO-*d*₆) δ 11.10 (s, 1H), 8.36 (s, 1H), 8.20 (s, 1H), 8.09 (d, *J* = 4.5 Hz, 2H), 8.04 (d, *J* = 1.7 Hz, 2H), 7.88 - 7.74 (m, 3H), 7.73 - 7.66 (m, 1H), 7.60 (s, 1H), 7.44 (d, *J* = 2.1 Hz, 1H), 7.38 - 7.34 (m, 2H), 7.27 (d, *J* = 2.5 Hz, 1H), 7.18 - 7.10 (m, 2H), 5.11 (dd, *J* = 13.0, 5.2 Hz, 1H), 4.31 (s, 2H), 4.27 - 4.21 (m, 4H), 3.79 (s, 4H), 3.63 - 3.55 (m, 8H), 2.12 (s, 1H), 1.48 (d, *J* = 10.3 Hz, 3H).

### (4) Synthesis of Compound 60

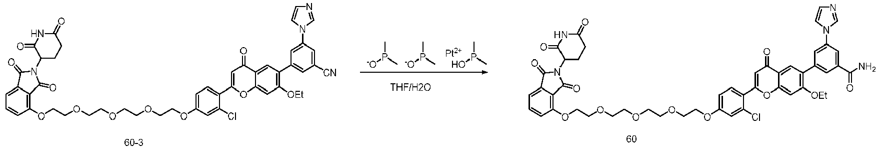

40 mg of 60-3 was added into 10 mL of THF and 2 mL of H₂O, followed by 20 mg of hydrogenated (dimethylphosphinic acid-kP) [hydrobis (dimethylphosphinic acid-kP)] platinum (II), stirred after the addition, then stirred at 90°C for 16h under N₂ protection. The disappearance of the raw materials and the generation of product were confirmed by liquid quality monitor, anhydrous sodium sulfate was added, the organic phase system was concentrated and evaporated, a proper amount of DCM was used for dissolving the residue, the residue was loaded on Prep-TLC, developed with DCM/MeOH(15:1), and freeze-dried to obtain 17.8 mg of the target product (yield: 44.5%, purity: 98.80%), MS: [M+H] 934.35.

¹H NMR (400 MHz, DMSO-*d*₆) δ 11.10 (s, 1H), 8.36 (s, 1H), 8.20 (s, 1H), 8.09 (d, *J* = 4.5 Hz, 2H), 8.04 (d, *J* = 1.7 Hz, 2H), 7.88 - 7.74 (m, 3H), 7.73 - 7.66 (m, 1H), 7.60 (s, 1H), 7.44 (d, *J* = 2.1 Hz, 1H), 7.38 - 7.34 (m, 2H), 7.27 (d, *J* = 2.5 Hz, 1H), 7.18 - 7.10 (m, 2H), 5.11 (dd, *J* = 13.0, 5.2 Hz, 1H), 4.31 (s, 2H), 4.27 - 4.21 (m, 4H), 3.79 (s, 4H), 3.63 - 3.55 (m, 8H), 2.12 (s, 1H), 1.48 (d, J = 10.3 Hz, 3H).

### Synthesis example 51

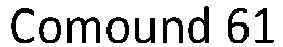

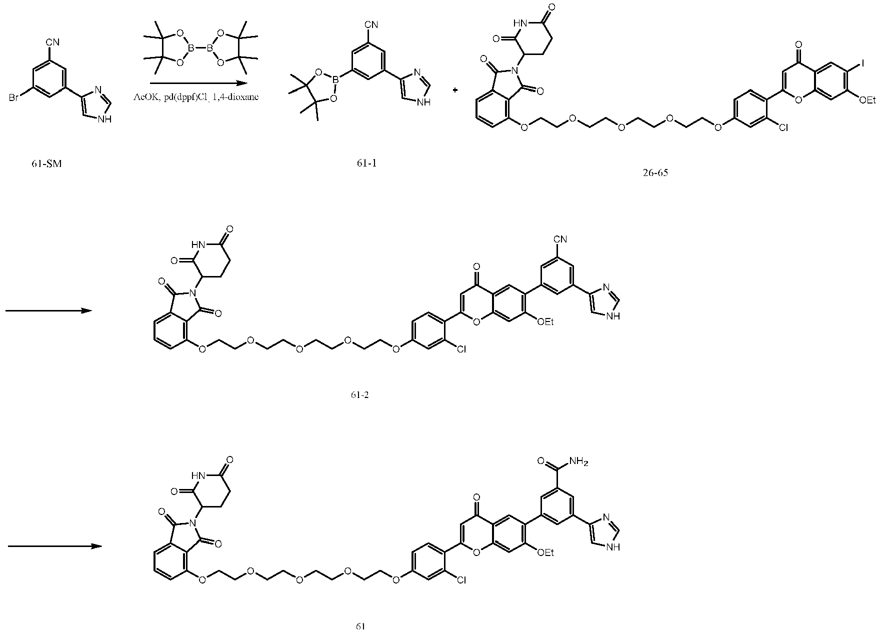

### (1) Synthesis of Compound 61-1

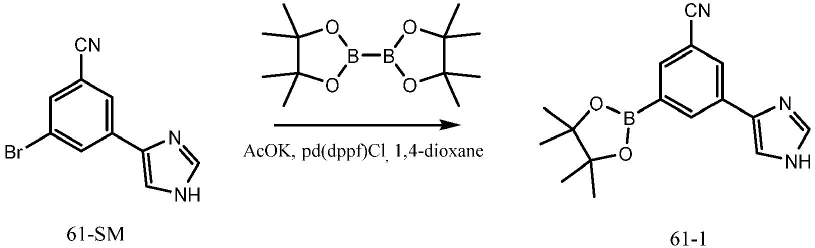

203 mg of 61-SM was dissolved in 20 ml of 1, 4-dioxane, followed by 311 mg of bisphenopinacol borate, 240 mg of potassium acetate, 179 mg of Pd(dppf)Cl₂. Protected by N₂, replaced 3 times, heated to 80°C and stirred overnight. Sent to LC-MS to obtain the products of boric acid and borate, concentrated the reaction solution. C18 silica gel column pre-filled and the concentrate was loaded with 4 ml MeOH. Concentrated at 40°C to obtain 186 mg off-white solid, borate was hydrolyzed to boric acid during purification.

### (2) Synthesis of Compound 61-2

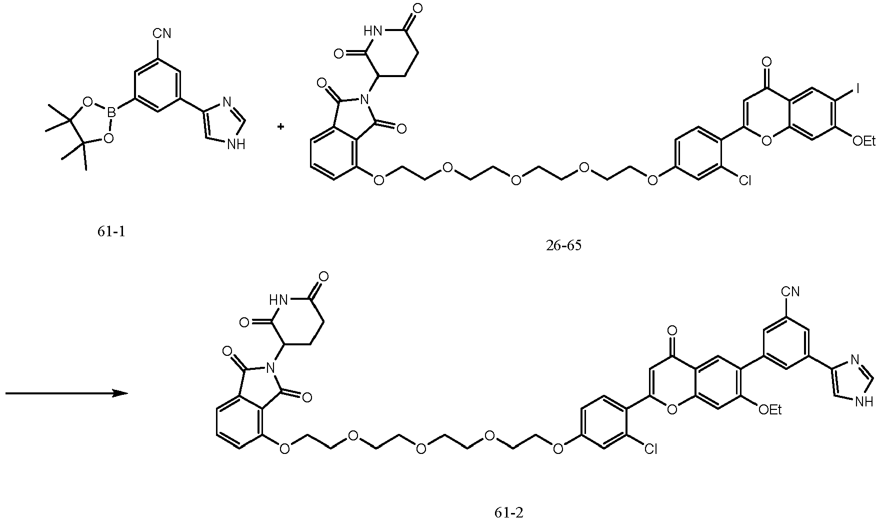

The raw material was dissolved in 2-MTHF, added KF and Pd(dppf)Cl₂, then addedd 1 ml of water, protected by N₂, stirred overnight at 70°C, added anhydrous sodium sulfate into the reaction solution, filtered, washed for 2 times by using DCM, concentrated, silica gel column pre-filled, dissolved and loaded the sample by DCM, eluted by 0-5% DCM\MeOH, collected the chromatographic solution, and concentrated to obtain 74 mg white solid.

### (3) Synthesis of Compound 61

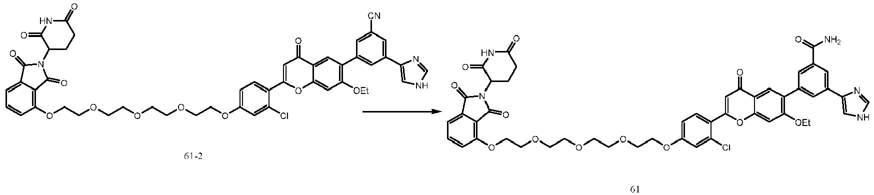

The raw material was dissolved in ultra-dry acetonitrile, added hydrogenated (dimethyl phosphinic acid-kP) [hydrogen bis (dimethyl phosphinic acid-kP)] platinum (II), stirred overnight, filtered, concentrated, silica gel column pre-filled, dissolved and loaded the sample with DCM, eluted with 0-5% DCM\MeOH, collected the chromatographic solution, and concentrated to obtain 23 mg white solid.

MS: [M+H] 934.35, purity: 96.1%.

¹H NMR (500 MHz, Chloroform-d) δ 9.12 (s, 1H), 8.32 (t, 1H), 8.25 (s, 1H), 8.15 (t, 1H), 8.10 (t, 1H), 7.88 (d, 1H), 7.70 - 7.61 (m, 2H), 7.56 (d, 1H), 7.41 (dd, 1H), 7.35 (d, 1H), 7.05 (s, 1H), 7.00 (s, 1H), 6.87 (dd, 1H), 6.34 (s, 2H), 5.12 (t, 1H), 4.40 (p, 2H), 4.28 - 4.17 (m, 3H), 3.94 (ddd, 1H), 3.80 (ddd, 1H), 3.45 - 3.38 (m, 2H), 3.36 - 3.18 (m, 9H), 2.96 - 2.84 (m, 1H), 2.65 - 2.53 (m, 2H), 2.13 - 2.02 (m, 1H), 1.45 (t, 3H).

### Synthesis example 52

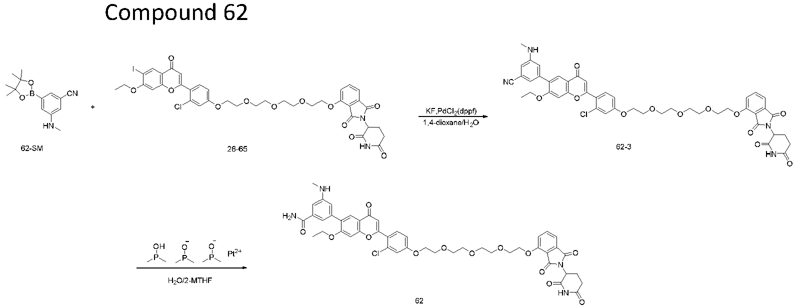

### (1) Synthesis of Compound 62-3

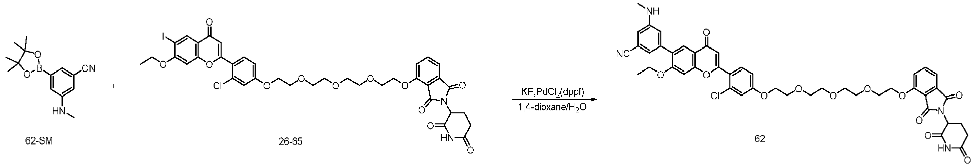

100 mg of 26-65 was added into 3 ml of 2-MTHF, followed by 147 mg of 62-SM, 40 mg of KF, 25 mg of PdCl ₂ (dppf) and 1 ml of H₂O, replaced by N₂ after the addition, raised to 80°C under the protection of N₂ and stirred. Monitored by LC-MS until no TDP102117-66 remained. A proper amount of DCM was added, dried over Na₂SO₄ and suction filtered, the filtrate was concentrated to dryness at 40°C under reduced pressure, the residue was dissolved with a proper amount of DCM and separated by Pre-TLC (DCM:MeOH=20:1) to obtain 93 mg (yield: 92.78%, purity: 95.15%), MS: [M+H] 879.15.

### (2) Synthesis of Compound 62

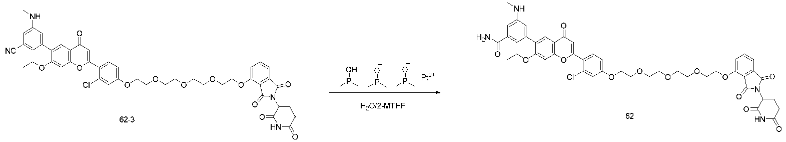

93 mg of 62-3 was added with 1 ml of 2-MTHF, 1 ml of H₂O, followed by 3 mg of PC, heated to 60°C with stirring, monitored by LC-MS until no TDP102163-3 remained. A proper amount of DCM was added, dried by Na₂SO₄ and suction filtered, the filtrate was concentrated to dryness at 40°C under reduced pressure, the residue was dissolved with a proper amount of DCM and separated by Pre-TLC (DCM:MeOH=15:1) to obtain 66 mg (yield: 69.47%, purity: 97.66%), MS: [M+H] 897.33.

¹H NMR (400 MHz, DMSO-*d*₆) δ 11.11 (s, 1H), 7.93 (s, 1H), 7.90 (s, 1H), 7.78 (dd, *J* = 24.5, 8.5 Hz, 2H), 7.44 (d, *J* = 2.0 Hz, 1H), 7.36 (dd, *J* = 8.3, 2.2 Hz, 1H), 7.29 (s, 1H), 7.27 (d, *J* = 2.4 Hz, 1H), 7.22 (s, 2H), 7.12 (dd, *J* = 8.7, 2.4 Hz, 1H), 7.03 (s, 1H), 6.90 (s, 1H), 6.54 (s, 1H), 5.88 (d, *J* = 5.0 Hz, 1H), 5.11 (dd, *J* = 13.0, 5.3 Hz, 1H), 4.30 (d, *J* = 4.5 Hz, 2H), 4.21 (q, *J* = 6.9 Hz, 4H), 3.78 (s, 4H), 3.62 - 3.54 (m, 8H), 2.86 (d, J = 11.9 Hz, 1H), 2.74 (d, *J* = 4.9 Hz, 3H), 2.64 - 2.52 (m, 2H), 2.10 - 1.96 (m, 1H), 1.33 (t, *J* = 6.9 Hz, 3H).

### Synthesis example 53

### Synthesis of Compound 63

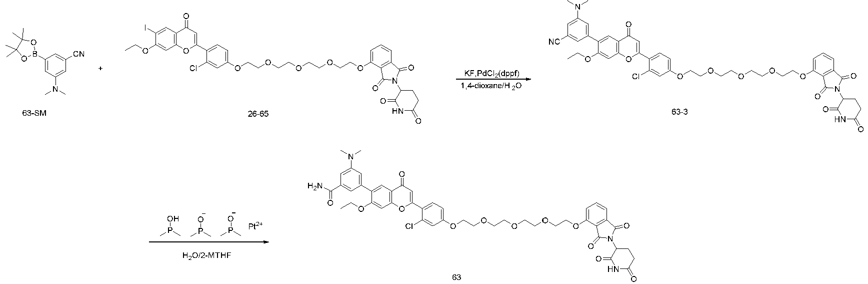

### (1) Synthesis of Compound 63-3

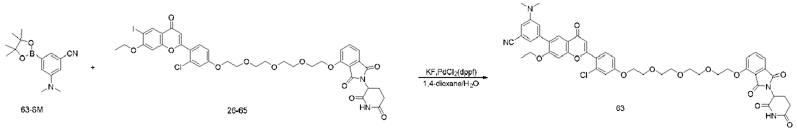

100 mg of 26-65 was added into 3 ml of 2-MTHF, followed by 155 mg of 63-SM, 40 mg of KF, 25 mg of PdCl ₂ (dppf) and 1 ml of H₂O, replaced with N₂ after the addition, the temperature was raised to 80°C under the protection of N₂ and stirred. Monitored by LC-MS until no TDP102117-66 remained. A proper amount of DCM was added, dried over Na₂SO₄ and suction filtered, the filtrate was concentrated to dryness at 40°C under reduced pressure, the residue was dissolved with a proper amount of DCM and separated by Pre-TLC (DCM:MeOH=20: 1) to obtain 94 mg (yield: 92.30%, purity: 91.24%), MS: [M+H] 893.35.

### (2) Synthesis of Compound 63

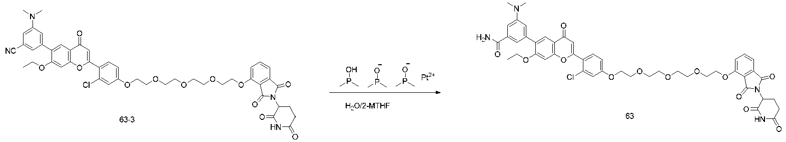

94 mg of 63-3 was added with 1 ml of 2-MTHF, 1 ml of H₂O, followed by 3 mg of PC, heated to 60°C and stirred, monitored by LC-MS until no TDP102164-3 remained. A proper amount of DCM was added, dried over Na₂SO₄ and suction filtered, the filtrate was concentrated to dryness at 40°C under reduced pressure, and the residue was dissolved with a proper amount of DCM and separated by Pre-TLC (DCM:MeOH=15:1) to obtain 52 mg (yield: 54.41%, purity: 95.98%), MS: [M+H] 911.36.

¹H NMR (400 MHz, DMSO-*d*₆) δ 11.11 (s, 1H), 8.00 (s, 0H), 7.96 (s, 1H), 7.81 (d, *J* = 8.3 Hz, 1H), 7.76 (d, *J* = 8.7 Hz, 1H), 7.44 (d, *J* = 1.9 Hz, 1H), 7.36 (dd, *J* = 8.3, 2.2 Hz, 1H), 7.32 (s, 1H), 7.30 (s, 1H), 7.29 (s, 1H), 7.27 (d, *J* = 2.4 Hz, 1H), 7.22 (s, 1H), 7.13 (dd, *J* = 8.7, 2.4 Hz, 1H), 7.07 (s, 1H), 6.55 (s, 1H), 5.11 (dd, *J* = 12.9, 5.4 Hz, 1H), 4.31 (s, 2H), 4.22 (t, *J* = 6.8 Hz, 4H), 3.78 (s, 4H), 3.58 (dd, *J* = 13.7, 3.9 Hz, 8H), 2.98 (s, 6H), 2.93 - 2.83 (m, 1H), 2.63 - 2.53 (m, 2H), 2.05 - 1.98 (m, 1H), 1.33 (t, *J* = 6.9 Hz, 3H).

### Synthesis example 54

### Synthesis of Compound 64

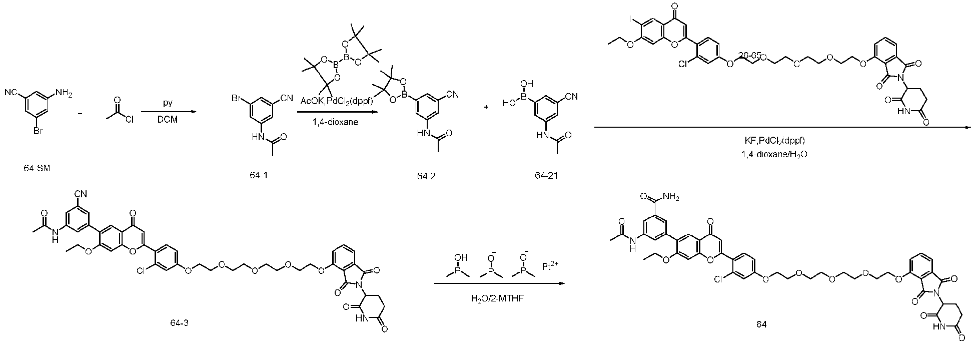

### Synthesis of Compound 64-1

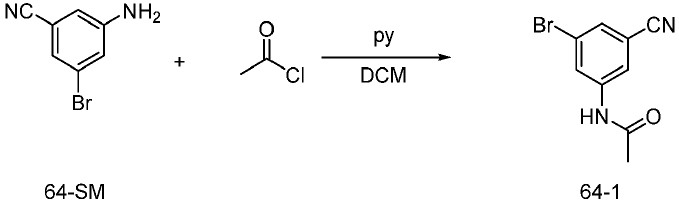

1000 mg of 64-SM was dissolved in 8 ml of DCM, added 602 mg of pyridine, stirred and cooled to 0°C, added 598 mg of acetyl chloride dropwise at 0°C, stirred for 5 min after the addition at 0°C, then stirred at RT, monitored by LC-MS until no TDP102174-SM1 remained. The system was concentrated to dryness at 40°C under reduced pressure, after being dissolved by a proper amount of DMF, the system was separated by reversed-phase Flash, eluted with 40% CAN to obtain 1.2g yellow solid (pruity: 94.44%, yield: 98.90%), MS: [M+H] 239.31.

### (2) Synthesis of Compound 64-2

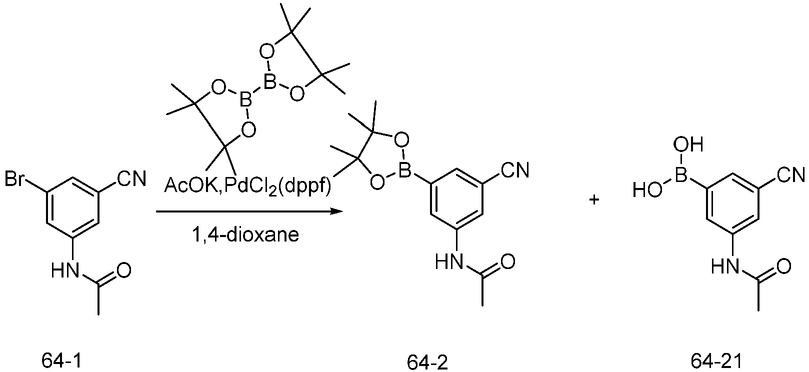

1200 mg of 64-1 was added into 10 ml of 1,4-dioxane, followed by 1912 mg of BPDB, 1478 mg of AcOK, and 1105 mg of PdCl₂(dppf), replaced by N₂ after the addition, the mixture was heated to 85°C under the protection of N₂ and stirred. Monitored by LC-MS until no TDP102169-1 remained. The mixture was concentrated to dryness under reduced pressure at 40°C and the residue was separated by reversed-phase column chromatography, eluted with 46% ACN to obtain 815 mg of the target product (yield: 79.57%, purity: 98%), MS: [M+H] 205.13.

### (2) Synthesis of Compound 64-3

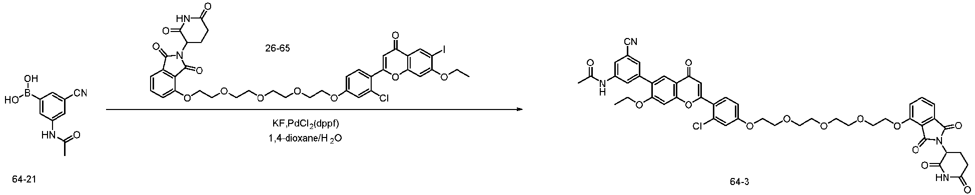

100 mg of 26-65 was added into 3 ml of 2-MTHF, followed by 164 mg of 64-21, 40 mg of KF, 25 mg of PdCl₂(dppf) and 1 ml of H₂O, replaced by N₂ after the addition, the mixture was heated to 85°C under the protection of N₂ and stirred. Monitored by LC-MS until no TDP102117-66 remained. A proper amount of DCM was added, dried over Na₂SO₄ and suction filtered, the filtrate was concentrated to dryness at 40°C under reduced pressure, the residue was dissolved with a proper amount of DCM and separated by Pre-TLC (DCM:MeOH=20: 1) to obtain 30 mg (yield: 29.04%, purity: 88.30%), MS: [M+H] 907.25.

### (3) Synthesis of Compound 64

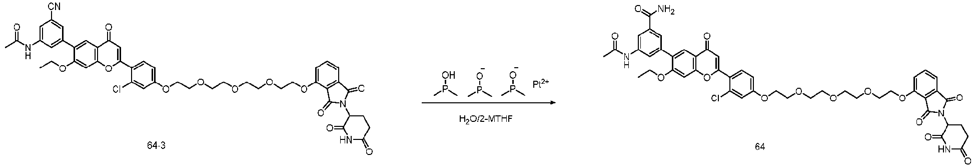

30 mg of 64-3 was added with 1 ml of 2-MTHF, 1 ml of H₂O, followed by 7 mg of PC, heated to 60°C and stirred, monitored by LC-MS until no 64-3 remained. A proper amount of DCM was added, dried over Na₂SO₄ and suction filtered, the filtrate was concentrated to dryness at 40°C under reduced pressure, the residue was dissolved with a proper amount of DCM and separated by Pre-TLC (DCM:MeOH=20:1, developed twice) to obtain 16 mg (yield: 49.18%, purity: 97.86%), MS: [M+H] 925.34.

¹H NMR (400 MHz, DMSO-*d*₆) δ 11.12 (s, 1H), 10.13 (s, 1H), 8.04 (d, *J* = 8.9 Hz, 3H), 7.98 (s, 1H), 7.82 (d, *J* = 8.3 Hz, 1H), 7.79 - 7.72 (m, 2H), 7.45 (d, *J* = 2.2 Hz, 1H), 7.39 - 7.33 (m, 3H), 7.27 (d, *J* = 2.4 Hz, 1H), 7.13 (dd, *J* = 8.7, 2.4 Hz, 1H), 6.56 (s, 1H), 5.11 (dd, *J* = 12.9, 5.3 Hz, 1H), 4.31 (s, 2H), 4.23 (q, *J* = 7.1 Hz, 4H), 3.78 (s, 4H), 3.62 - 3.55 (m, 8H), 2.86 (d, *J* = 13.2 Hz, 1H), 2.57 - 2.52 (m, 2H), 2.07 (s, 3H), 2.01 (s, 1H), 1.34 (t, *J* = 6.9 Hz, 3H).

### Synthesis example 55

### Synthesis of Compound 65

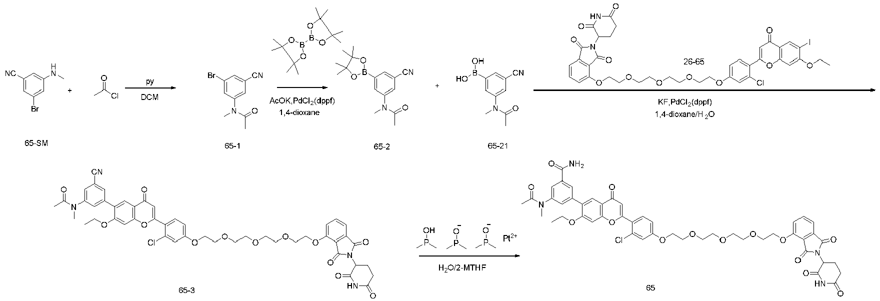

### (1) Synthesis of Compound 65-1

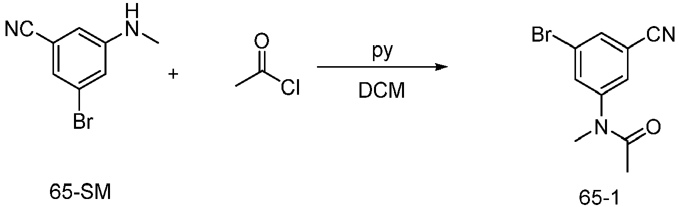

500 mg of 65-SM was added into 3 ml of DCM, followed by 281 mg of pyridine, stirred and cooled to 0°C. 279 mg of acetyl chloride was added dropwise at 0°C and stirred for 5min after the addition at 0°C, then wamed to RT with stirring until no TDP102163-11 remained. The system was concentrated to dryness at 40°C under reduced pressure, the residue was separated by reversed-phase column chromatography, and eluted with 40% can to obtain 531 mg of the target product (purity: 98%, yield: 88.52%), MS: [M+H] 253.15.

### (2) Synthesis of Compound 65-2

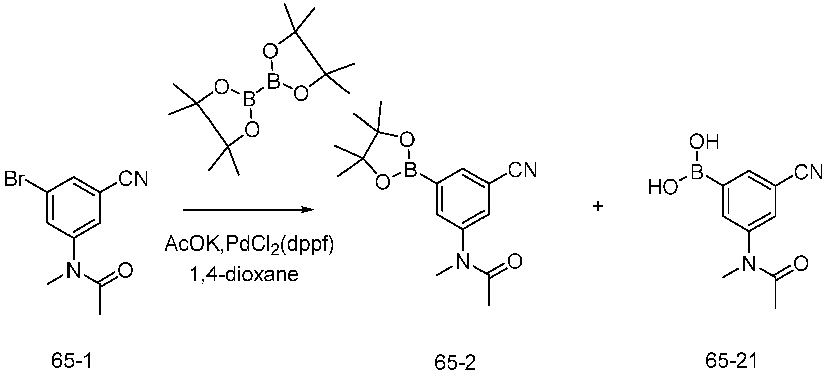

531 mg of 65-1 was added into 10 ml of 1,4-dioxane, followed by 799 mg of BPDB, 618 mg of AcOK, 462 mg of PdCl₂(dppf), replaced by N₂ after the addition, the mixture was heated to 85°C under protection of N₂ and stirred. Monitored by LC-MS until no 65-1 remained. Concentrated to dryness under reduced pressure at 40°C and the residue was separated by reversed-phase column chromatography, eluted with 25% can to obtain 519 mg of the target product (TEA-containing hydrochloride, If calculated according to the 100% yield, the content is about 88%, purity: 91.7%), MS: [M+H] 219.35.

### (3) Synthesis of Compound 65-3

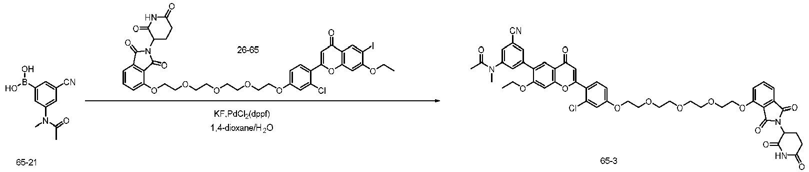

100 mg of 26-65 was added into 3 ml of 2-MTHF, followed by 124 mg of 65-21, 40 mg of KF, 25 mg of PdCl₂(dppf) and 1 ml of H₂O, replaced by N₂ after the additiong, the temperature was raised to 80°C under the protection of N₂ and stirred. Monitored by LC-MS until no TDP102117-66 remained. A proper amount of DCM was added, dried over Na₂SO₄ and suction filtered, the filtrate was concentrated to dryness at 40°C under reduced pressure, the residue was dissolved with a proper amount of DCM and separated by Pre-TLC (DCM:MeOH=20:1, developed twice) to obtain 94 mg (yield: 89.60%, purity: 96.91%), MS: [M+H] 921.12.

### (4) Synthesis of Compound 65

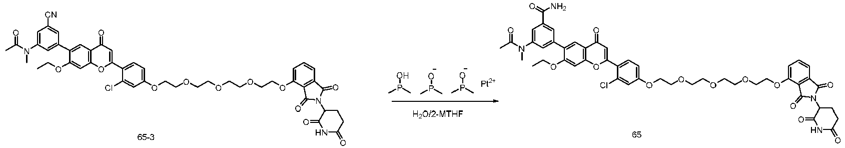

94 mg of 65-3 was added with 1 ml of 2-MTHF, 1 ml of H₂O, followed by 26 mg of PC, heated to 60°C and stirred, monitored by LC-MS until no 65-3 remained. A proper amount of DCM was added, dried over Na₂SO₄ and suction filtered, the filtrate was concentrated to dryness at 40°C under reduced pressure, the residue was dissolved with a proper amount of DCM and separated by Pre-TLC (DCM:MeOH=20:1, developed twice) to obtain 79mg (yield: 59.71%, purity: 98.12%), MS: [M+H] 939.37.

¹H NMR (400 MHz, DMSO-*d*₆) δ 11.12 (s, 1H), 8.17 (s, 1H), 8.03 (s, 2H), 7.85 - 7.71 (m, 4H), 7.54 (s, 1H), 7.45 (d, *J* = 2.1 Hz, 1H), 7.38 - 7.33 (m, 2H), 7.27 (d, *J* = 2.5 Hz, 1H), 7.13 (dd, *J* = 8.7, 2.4 Hz, 1H), 6.57 (s, 1H), 5.11 (dd, *J* = 12.9, 5.4 Hz, 1H), 4.34 - 4.28 (m, 2H), 4.22 (dd, *J* = 8.0, 3.9 Hz, 4H), 3.78 (q, *J* = 6.6 Hz, 4H), 3.62 - 3.55 (m, 8H), 3.22 (s, 3H), 2.94 - 2.82 (m, 1H), 2.62 - 2.51 (m, 2H), 2.05 - 1.98 (m, 1H), 1.32 (t, *J* = 6.9 Hz, 3H).

### Synthesis example 56

### Synthesis of Compound 66

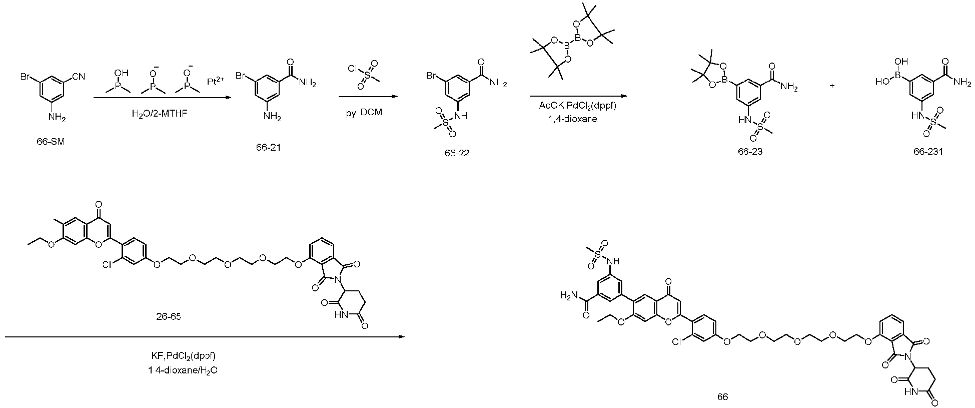

### (1) Synthesis of Compound 66-21

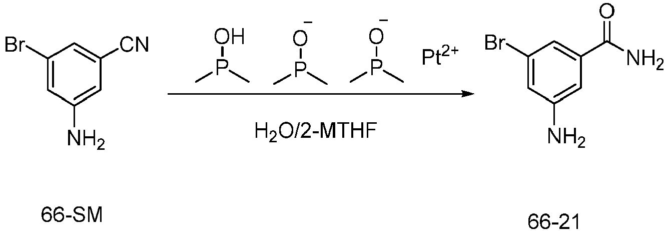

4000 mg of 66-SM was added with 4 ml of 2-MTHF, 4 ml of H₂O, followed by 214 mg of PC, heated to 60°C with stirring, and monitored by LC-MS until no 66-SM remained. A proper amount of DCM:MeOH=10:1 was added, dried over Na₂SO₄ and suction filtered, and the filtrate was filtered through silica gel and concentrated to dryness at 40°C under reduced pressure to obtain 4.2g light yellow solid (yield: 96.21%, purity: 98.71%), MS: [M+H] 214.91, 216.91.

### (2) Synthesis of Compound 66-22

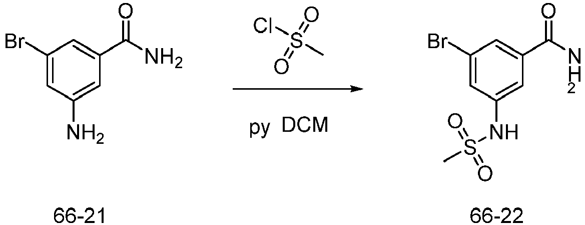

1000 mg of 66-21 was dissolved in 4 ml of DCM, added 4 ml pyridine with stirring and cooled, 800 mg of methanesulfonyl chloride was added dropwise slowly, removed ice bath after the addition and stirred until no 66-21 remained. The system was concentrated to dryness at 40°C under reduced pressure, after being dissolved by a proper amount of DMF, the system was separated by reversed-phase Flash, eluted with 50% ACN to obtain 860 mg of the target product (purity: 98%, yield: 63.35%), MS: [M+H] 292.91, 294.92.

### (3) Synthesis of Compound 66-23

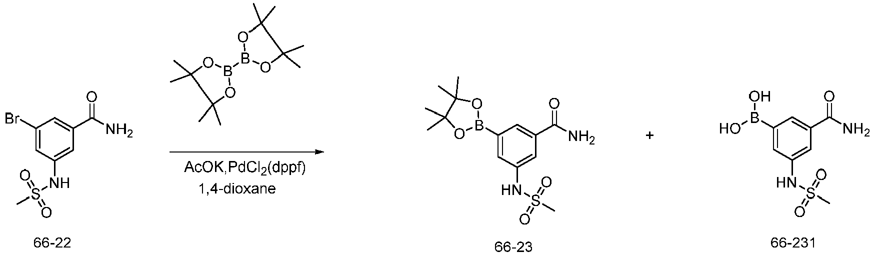

860 mg of 66-22 was added into 10 ml of 1,4-dioxane, followed by 1118 mg of BPDB, 864 mg of AcOK, 646 mg of PdCl₂(dppf), replaced by N₂ after the addition, heated to 85°C under the protection of N₂ and stirred. Monitored by LC-MS until no 66-22 remained. The mixture was concentrated to dryness under reduced pressure at 40°C and the residue was subjected to reversed-phase column chromatography, eluted with 38% ACN to obtain 733 mg of the target product (purity: 174-23/174-231=50/14.6, yield: 73.45%), MS: [M+H] 341.77, 259.56.

### (4) Synthesis of Compound 66

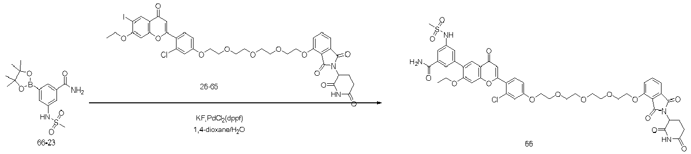

100 mg of 26-65 was added into 3 ml of 2-MTHF, followed by 116 mg of 66-23, 40 mg of KF, 25 mg of PdCl₂(dppf) and 1 ml of H₂O, replaced by N₂ after the addition, raised to 80°C under the protection of N₂ and stirred. Monitored by LC-MS until no 26-65 remained. A proper amount of DCM was added, dried over Na₂SO₄ and suction filtered, the filtrate was concentrated to dryness at 40°C under reduced pressure, and the residue was dissolved with a proper amount of DCM and separated by Pre-TLC (DCM:MeOH=15:1) to obtain 63 mg (yield: 57.55%, purity: 95.88%), MS: [M+H] 961.39.

¹H NMR (400 MHz, DMSO-*d*₆) δ 11.12 (s, 1H), 9.95 (s, 1H), 8.14 (s, 1H), 8.01 (s, 1H), 7.83 - 7.74 (m, 3H), 7.71 (s, 1H), 7.62 (s, 1H), 7.45 (s, 2H), 7.36 (d, *J* = 9.8 Hz, 2H), 7.27 (d, *J* = 2.2 Hz, 1H), 7.13 (dd, *J* = 8.8, 2.1 Hz, 1H), 6.57 (s, 1H), 5.11 (dd, *J* = 12.9, 5.4 Hz, 1H), 4.31 (s, 2H), 4.23 (d, *J* = 6.5 Hz, 4H), 3.78 (s, 4H), 3.62 - 3.54 (m, 8H), 3.05 (s, 3H), 2.93 - 2.82 (m, 1H), 2.62 - 2.52 (m, 2H), 2.07 - 1.98 (m, 1H), 1.35 (t, *J* = 6.8 Hz, 3H).

### Synthesis example 57

### Synthesis of Compound 67

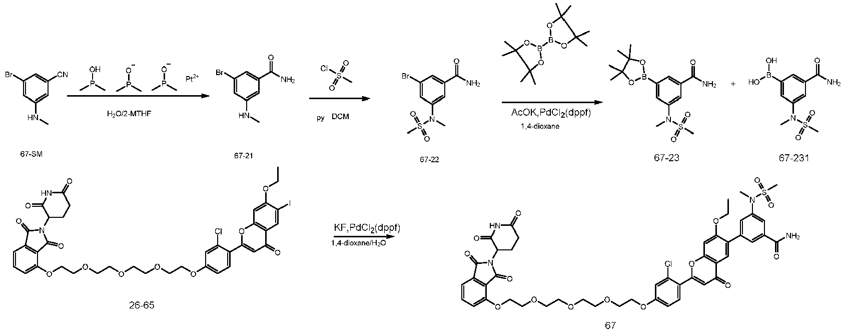

### (1) Synthesis of Compound 67-21

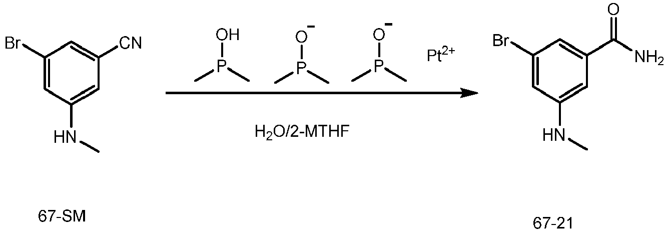

4.0 g of 67-SM was added with 4 ml of 2-MTHF, 4 ml of H₂O, followed by 214 mg of PC, heated to 60°C with stirring, monitored by LC-MS until no 67-SM remained. A proper amount of DCM:MeOH=10:1 was added, dried over Na₂SO₄ and filtered, the filtrate was filtered through silica gel and then concentrated to dryness at 40°C under reduced pressure to obtain 4.3 g light yellow solid (yield: 96.21%, purity: 97.62%), MS: [M+H] 228.99, 230.99.

### (2) Synthesis of Compound 67-22

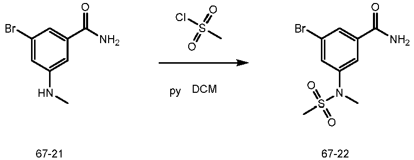

1.1 g of 67-21 was dissolved in 4 ml of DCM, added 4 ml of pyridine with stirring and cooled, 800 mg methanesulfonyl chloride was added dropwise slowly, removed ice bath after the addition and stirred until no 67-21 remained. The system was concentrated to dryness at 40°C under reduced pressure, after being dissolved by a proper amount of DMF, the system was separated by reversed-phase Flash, eluted with 50% ACN to obtian 887 mg of the target product (purity: 97.77%, yield: 63.35%), MS: [M+H] 306.97, 308.97.

### (3) Synthesis of Compound 67-23

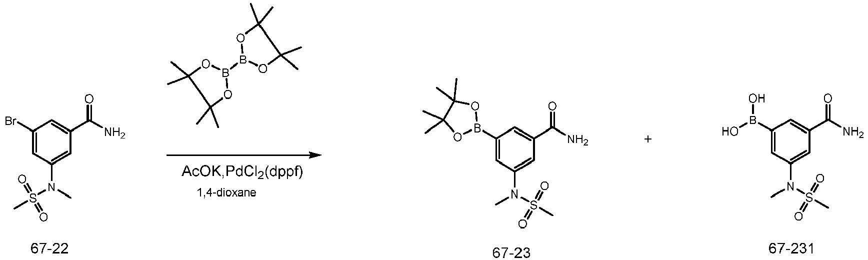

887 mg of 67-22 was added into 10 ml of 1,4-dioxane, followed by 1118 mg of BPDB, 864 mg of AcOK, 646 mg of PdCl₂(dppf), replaced by N₂ after the addition, heated to 85°C under protection of N₂ and stirred. Monitored by LC-MS until no 67-22 remained. The mixture was concentrated to dryness under reduced pressure at 40°C and the residue was separated by reversed-phase column chromatography, eluted with 38% ACN to obtain 762 mg of the target product (yield: 72.11%), MS: [M+H] 354.07, 273.06.

### (4) Synthesis of Compound 67

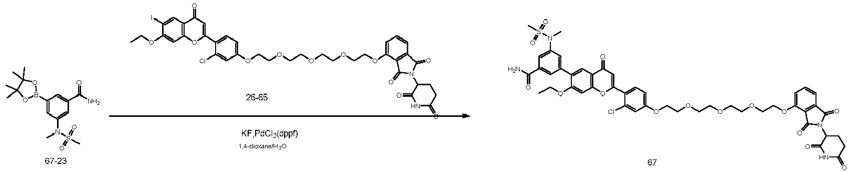

100 mg of 26-65 was added into 3 ml of 2-MTHF, followed by 117 mg of 67-23, 40 mg of KF, 25 mg of PdCl₂(dppf) and 1 ml of H₂O, replaced by N₂ after the addition, raised to 80°C under the protection of N₂ and stirred. Monitored by LC-MS until no 26-65 remained. A proper amount of DCM was added, dried over Na₂SO₄ and filtered, the filtrate was concentrated to dryness at 40°C under reduced pressure, the residue was dissolved with a proper amount of DCM and separated by Pre-TLC (DCM:MeOH=15: 1) to obtain 57 mg (yield: 56.23%, purity: 96.22%), MS: [M+H] 975.42.

¹H NMR (400 MHz, CDCL3) δ 9.12 (s, 1H), 8.23 (s, 1H), 7.93 (d, 1H), 7.88 (t, 1H), 7.59 - 7.52 (m, 2H), 7.20 - 7.11 (m, 2H), 7.05 (s, 1H), 6.98 (s, 1H), 6.90 - 6.81 (m, 2H), 6.74 (t, 1H), 6.27 (s, 2H), 4.36 (ddq,2H), 4.29 - 4.14 (m, 4H), 3.48 - 3.37 (m, 4H), 3.37 - 3.26 (m, 11H), 3.20 (s, 3H), 2.96 - 2.84 (m, 1H), 2.63 - 2.53 (m, 2H), 2.13 - 2.03 (m, 1H), 1.45 (t, 3H).

### Synthesis example 58

### Synthesis of Compound 68

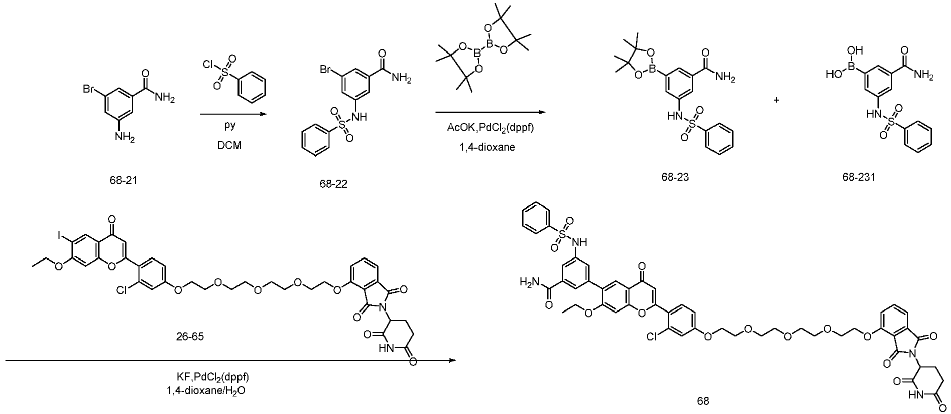

### (1) Synthesis of Compound 68-22

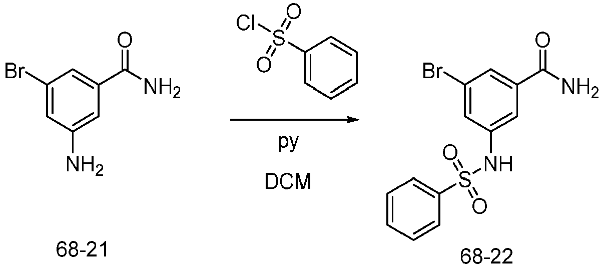

1000 mg of 68-21 was dissolved in 4 ml of DCM, added 4 ml of pyridine with stirring and cooled to 0°C, 1233 mg of benzenesulfonyl chloride was added dropwise slowly, removed ice bath after the addition and stirred until no 68-21 remained. The system was concentrated to dryness at 40°C under reduced pressure, after being dissolved by a proper amount of DMF, the system was separated by reversed-phase Flash, eluted with 55% ACN to obtain 663 mg of the target product (purity: 98%, yield: 40.28%), MS: [M+H] 354.97, 356.97.

### (2) Synthesis of Compound 68-23

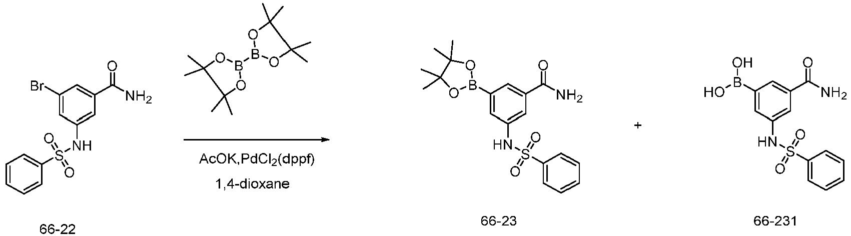

663 mg of 68-22 was added into 10 ml of 1,4-dioxane, followed by 711 mg of BPDB, 550 mg of AcOK, 411 mg of PdCl₂(dppf), replaced by N₂ after the addition, heated to 85°C under protection of N₂ and stirred. Monitored by LC-MS until no 68-22 remained. The mixture was concentrated to dryness under reduced pressure at 40°C and the residue was separated by reversed-phase column chromatography, eluted with 48% ACN to obtain 615 mg of the target product (purity: 18023/180-231=3/80, yield: 81.94%), MS: [M+H] 403.13, 321.45.

### (3) Synthesis of Compound 68

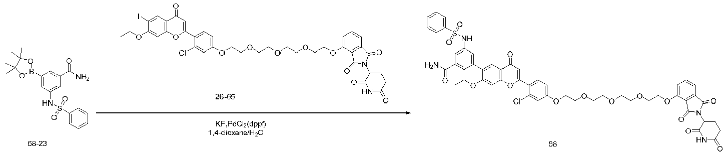

100 mg of 26-65 was added into 3 ml of 2-MTHF, followed by 138 mg of 68-23, 40 mg of KF, 25 mg of PdCl₂(dppf) and 1 ml of H₂O, replaced by N₂ after the addition, raised to 80°C under the protection of N₂ and stirred. Monitored by LC-MS until no 26-65 remained. A proper amount of DCM was added, dried over Na₂SO₄ and suction filtered, the filtrate was concentrated to dryness at 40°C under reduced pressure, the residue was dissolved with a proper amount of DCM and separated by Pre-TLC (DCM:MeOH=15:1) to obtain 63 mg (yield: 54.06%, purity: 95.40%), MS: [M+H] 1023.46.

¹H NMR (400 MHz, DMSO-*d*₆) δ 11.12 (s, 1H), 10.52 (s, 0H), 8.08 (s, 1H), 7.90 (s, 1H), 7.82 - 7.72 (m, 5H), 7.63 (s, 1H), 7.58 (q, *J* = 7.7, 7.1 Hz, 3H), 7.50 (s, 1H), 7.45 (d, *J* = 1.9 Hz, 1H), 7.41 (s, 1H), 7.36 (dd, *J* = 8.3, 2.1 Hz, 1H), 7.32 (s, 1H), 7.27 (d, *J* = 2.2 Hz, 1H), 7.13 (dd, *J* = 8.7, 2.2 Hz, 1H), 6.56 (s, 1H), 5.11 (dd, *J* = 13.0, 5.3 Hz, 1H), 4.31 (s, 2H), 4.21 (dd, *J* = 13.1, 5.8 Hz, 4H), 3.78 (s, 4H), 3.59 (dd, *J* = 15.2, 5.1 Hz, 8H), 2.93 - 2.83 (m, 1H), 2.63 - 2.53 (m, 2H), 2.14 - 1.86 (m, 1H), 1.29 (t, *J* = 6.9 Hz, 3H).

### Synthesis example 59

### Synthesis of Compound 69

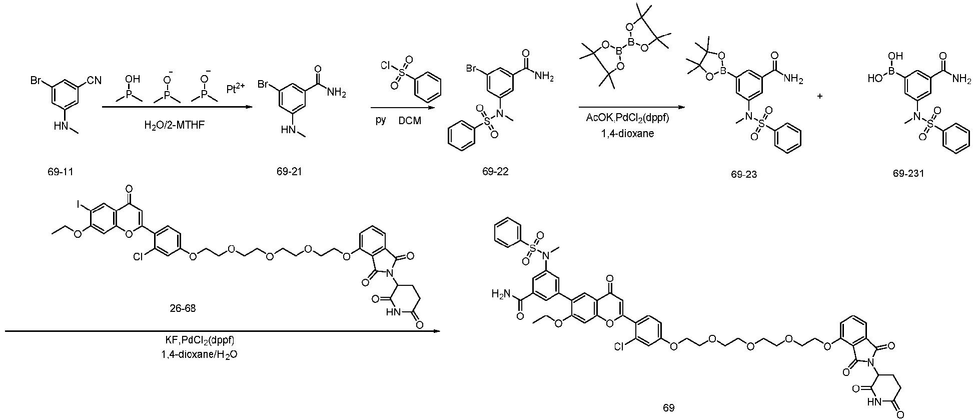

### (1) Synthesis of Compound 69-21

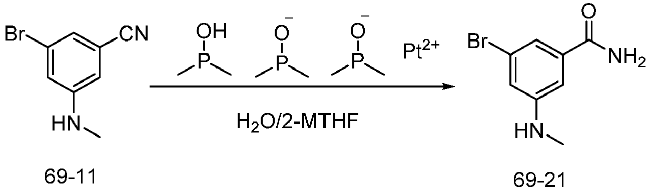

800 mg of 69-11 was added with 4 ml of 2-MTHF, 4 ml of H₂O, followed by 40 mg of PC, heated to 60°C with stirring, monitored by LC-MS until no 69-11 remained. A proper amount of DCM was added, dried over Na₂SO₄ and suction filtered, the filtrate was concentrated to dryness at 40°C under reduced pressure and separated the residue by reversed-phase Flash, eluted with 50% can to obtain 740 mg of the target product (yield: 85.64%, purity: 99.64%), MS: [M+H] 228.99, 230.99.

### (2) Synthesis of Compound 69-22

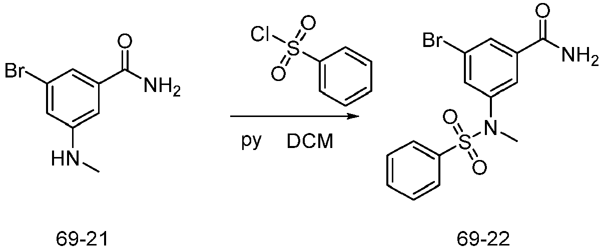

570 mg of 69-21 was dissolved in 3 ml of DCM, added 3 ml of pyridine with stirring and cooled to 0°C, 659mg of benzene sulfonyl chloride was added dropwise slowly, removed ice bath after the addition and stirred until no 69-21 remained. The system was concentrated to dryness at 40°C under reduced pressure, after being dissolved by a proper amount of DMF, the system was separated by reversed-phase Flash, eluted with 65% ACN to obtain 530 mg of the target product (purity: 99.73%, yield: 57.68%), MS: [M+H] 369.12, 371.24.

### (3) Synthesis of Compound 69-231

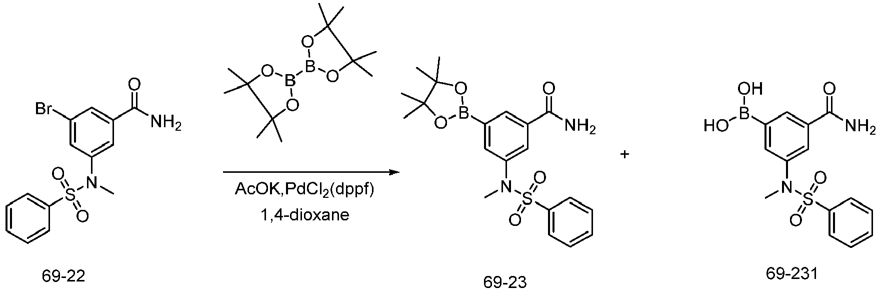

530 mg of 69-22 was added into 10 ml of 1,4-dioxane, followed by 547 mg of BPDB, 422 mg of AcOK, 317 mg of PdCl₂(dppf), replaced by N₂ after the addition, raised to 85°C under the protection of N₂ and stirred. Monitored by LC-MS until no 69-22 remained. Concentrated to dryness under reduced pressure at 40°C and the residue was subjected to reversed-phase column chromatography, eluted with 55% ACN to obtain 400 mg of the target product (purity: 96.37%, yield: 83.15%), MS: [M+H] 335.17.

### (4) Synthesis of Compound 69

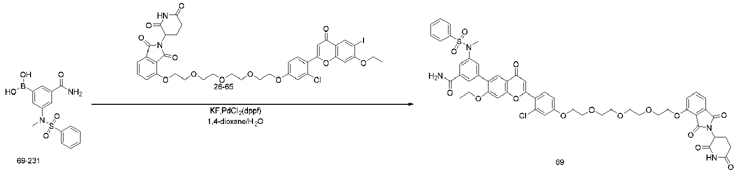

100 mg of 26-65 was added into 3 ml of 2-MTHF, followed by 114 mg of 69-231, 40 mg of KF, 25 mg of PdCl₂(dppf) and 1 ml of H₂O, replaced by N₂ after the addition, raised to 70°C under the protection of N₂ and stirred. Monitored by LC-MS until no 26-65 remained. A proper amount of DCM was added, dried over Na₂SO₄ and suction filtered, the filtrate was concentrated to dryness at 40°C under reduced pressure, and the residue was dissolved with a proper amount of DMF and separated by reversed-phase Flash, eluted with 85% ACN to obtain 60 mg of the product (yield: 50.80%, purity: 98.95%), MS: [M+H] 1037.49.

¹H NMR (400 MHz, DMSO-*d*₆) δ 11.10 (s, 1H), 8.12 (s, 1H), 8.01 (s, 1H), 7.92 (s, 1H), 7.81 (d, *J* = 8.3 Hz, 1H), 7.74 (dd, *J* = 14.8, 7.9 Hz, 2H), 7.60 (dt, *J* = 10.7, 6.1 Hz, 5H), 7.49 - 7.42 (m, 3H), 7.40 - 7.30 (m, 2H), 7.27 (d, *J* = 2.3 Hz, 1H), 7.13 (dd, *J* = 8.7, 2.3 Hz, 1H), 6.56 (s, 1H), 5.11 (dd, *J* = 12.9, 5.3 Hz, 1H), 4.34 - 4.27 (m, 2H), 4.25 - 4.17 (m, 4H), 3.79 (d, *J* = 7.7 Hz, 4H), 3.62 - 3.54 (m, 8H), 3.21 (s, 3H), 2.95 - 2.82 (m, 1H), 2.64 - 2.52 (m, 2H), 2.09 - 1.97 (m, 1H), 1.28 (t, *J* = 6.9 Hz, 3H).

### Synthesis example 60

### Synthesis of Compound 70

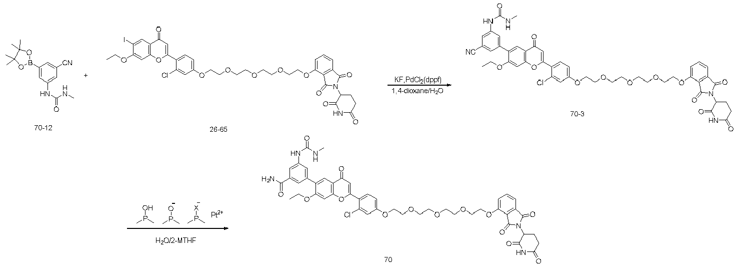

### (1) Synthesis of Compound 70-3

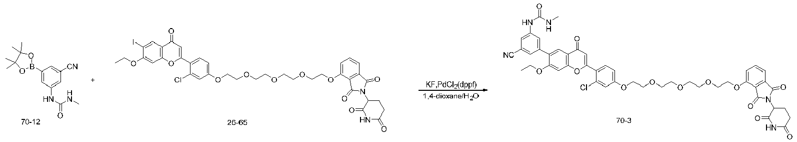

100 mg of 26-65 was added into 3 ml of 2-MTHF, followed by 172 mg of 70-12, 40 mg of KF, 25 mg of PdCl₂(dppf) and 1 ml of H₂O, replaced by N₂ after the addition, raised to 80°C under the protection of N₂ and stirred. Monitored by LC-MS until no 26-65 remained. A proper amount of DCM was added, dried over Na₂SO₄ and suction filtered, the filtrate was concentrated to dryness at 40°C under reduced pressure, the residue was dissolved with a proper amount of DCM and separated by Pre-TLC (DCM:MeOH=20:1) to obtain 70 mg (yield: 66.67%, purity: 95.89%), MS: [M+H] 922.13.

### (2) Synthesis of Compound 70

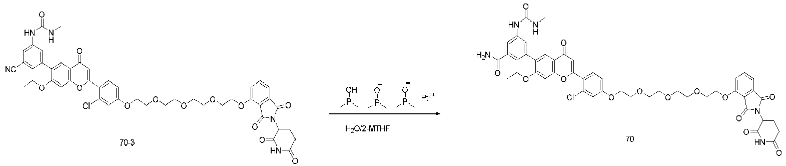

70 mg 70-3 was added with 1 ml 2-MTHF, 1 ml H₂ O, followed by 2 mg PC, heated to 60°C with stirring, monitored by LC-MS until no 70-3 remained. A proper amount of DCM was added, dried over Na₂SO₄ and suction filtered, the filtrate was concentrated to dryness at 40°C under reduced pressure, the residue was dissolved with a proper amount of DCM and separated by Pre-TLC (DCM:MeOH=15:1) to obtain 57 mg (yield: 79.85%, purity: 97.92%), MS: [M+H] 940.36.

¹H NMR (400 MHz, DMSO-*d*₆) δ 11.11 (s, 1H), 8.70 (s, 1H), 7.98 (s, 1H), 7.95 (s, 1H), 7.88 (s, 1H), 7.83 - 7.75 (m, 3H), 7.59 (s, 1H), 7.45 (d, *J* = 2.1 Hz, 1H), 7.36 (dd, *J* = 8.3, 2.2 Hz, 1H), 7.32 (s, 1H), 7.30 (s, 1H), 7.27 (d, *J* = 2.4 Hz, 1H), 7.13 (dd, *J* = 8.8, 2.4 Hz, 1H), 6.55 (s, 1H), 6.06 (d, *J* = 4.7 Hz, 1H), 5.11 (dd, *J* = 12.8, 5.3 Hz, 1H), 4.30 (d, *J* = 4.6 Hz, 2H), 4.23 (d, *J* = 6.6 Hz, 4H), 3.78 (s, 4H), 3.62 - 3.55 (m, 8H), 2.93 - 2.83 (m, 1H), 2.66 (d, *J* = 4.6 Hz, 3H), 2.62 - 2.54 (m, 2H), 2.07 - 1.98 (m, 1H), 1.33 (d, *J* = 6.9 Hz, 3H).

### Synthesis example 61

### Synthesis of Compound 71

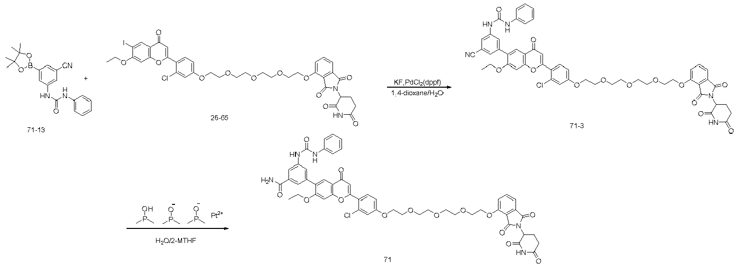

### (1) Synthesis of Compound 71-3

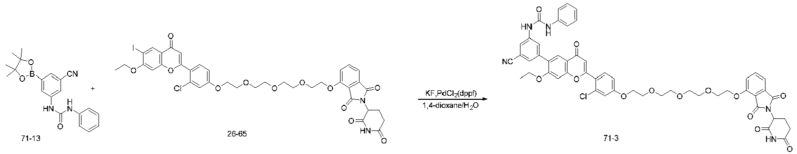

100 mg of 26-65 was added into 3 ml of 2-MTHF, followed by 207 mg of 71-13, 40 mg of KF, 25 mg of PdCl₂(dppf) and 1 ml of H₂O, replaced by N₂ after the addition, raised to 80°C under the protection of N₂ and stirred. Monitored by LC-MS until no 26-65 remained. A proper amount of DCM was added, dried over Na₂SO₄ and suction filtered, the filtrate was concentrated to dryness at 40°C under reduced pressure, and the residue was dissolved with a proper amount of DCM and separated by Pre-TLC (DCM:MeOH=20:1) to obtain 77 mg (yield: 68.61%, purity: 89.45%), MS: [M+H] 984.35.

### (2) Synthesis of Compound 71

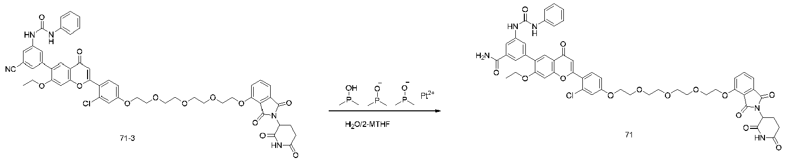

77 mg of 71-3 was added with 1 ml of 2-MTHF, 1 ml of H₂O, followed by 3 mg of PC, heated to 60°C and stirred, monitored by LC-MS until no TDP102184-3 remained. A proper amount of DCM was added, dried over Na₂SO₄ and suction filtered, the filtrate was concentrated to dryness at 40°C under reduced pressure, the residue was dissolved with a proper amount of DCM and separated by Pre-TLC (DCM:MeOH=15:1) to obtain 73 mg (yield: 93.47%, purity: 98.76%), MS: [M+H] 1002.43.

¹H NMR (400 MHz, DMSO-*d*₆) δ 11.11 (s, 1H), 8.87 (s, 1H), 8.72 (s, 1H), 8.05 (s, 1H), 7.99 (s, 1H), 7.92 (s, 1H), 7.87 (s, 1H), 7.82 (d, *J* = 8.3 Hz, 1H), 7.77 (d, *J* = 8.7 Hz, 1H), 7.68 (s, 1H), 7.48 (d, *J* = 7.7 Hz, 2H), 7.45 (d, *J* = 2.2 Hz, 1H), 7.38 - 7.33 (m, 3H), 7.31 - 7.26 (m, 3H), 7.13 (dd, *J* = 8.8, 2.4 Hz, 1H), 6.98 (t, *J* = 7.3 Hz, 1H), 6.56 (s, 1H), 5.11 (dd, *J* = 12.9, 5.3 Hz, 1H), 4.33 - 4.29 (m, 2H), 4.24 (q, *J* = 6.5, 6.0 Hz, 4H), 3.78 (t, *J* = 8.1 Hz, 4H), 3.62 - 3.54 (m, 8H), 2.93 - 2.83 (m, 1H), 2.55 (d, *J* = 9.3 Hz, 2H), 2.02 (dd, *J* = 14.6, 6.6 Hz, 1H), 1.36 (t, *J* = 6.9 Hz, 3H).

### Synthesis example 62

### Synthesis of Compound 72

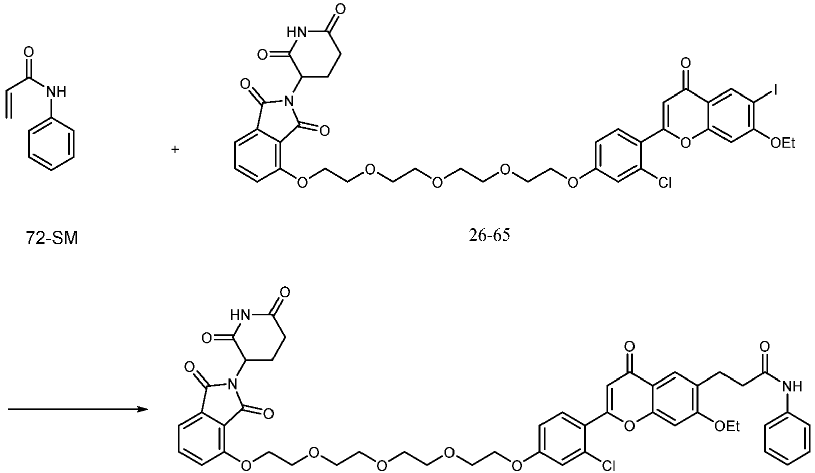

84 mg of 72-SM was dissolved in 5 ml of DMF, added 100 mg of 26-65, 52 mg of tris-(o-tolyl) phosphine, and added 47 mg of potassium carbonate with stirring.

Stirred overnight.

Sample was taken and sent to LC-MS, filtered the reaction solution, concentrated, added 50 ml of water for dilution, extracted by EA for 3 times, combined, washed by brine once, and concentrated.

Purification: 40 g C18 silica gel column pre-filled and the concentrate was loaded with 4 ml MeOH.

Eluted with 0-60% ACN\H₂O.

Concentrated, lyophilized to obtain 56 mg off-white solid.

MS: [M+H] 896.34, purity: 94.9%.

¹H NMR (500 MHz, Chloroform-d) δ 9.12 (s, 1H), 8.09 (s, 1H), 7.93 (d,1H), 7.71 - 7.60 (m, 2H), 7.60 - 7.52 (m, 3H), 7.41 (dd,1H), 7.30 (t, 2H), 7.13 (d, 1H), 7.11 - 7.03 (m, 1H), 7.05 (s, 1H), 6.90 - 6.83 (m, 2H), 5.16 - 5.07 (m, 1H), 4.45 (ddt, 2H), 4.29 - 4.16 (m, 3H), 3.94 (ddd, 1H), 3.79 (ddd, 1H), 3.45 - 3.37 (m, 2H), 3.36 - 3.19 (m, 9H), 3.24 - 3.14 (m, 1H), 2.96 - 2.85 (m, 2H), 2.83 (tdd, 1H), 2.70 - 2.60 (m, 1H), 2.64 - 2.52 (m, 2H), 2.14 - 2.01 (m, 1H), 1.43 (t, 3H).

### Synthesis example 63

### Synthesis of Compound 73

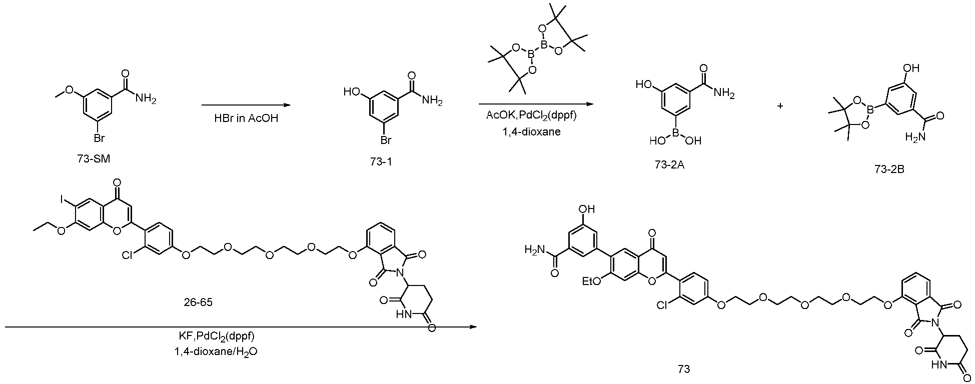

### (1) Synthesis of Compound 73-1

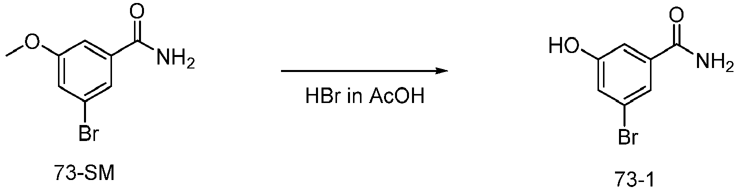

1000 mg of 73-SM was added into 15 ml of HBr in acetic acid, heated to 50°C with stirring, monitored by LC-MS and supplemented with HBr in acetic acid until 73-SM remained unchanged. The system was concentrated to dryness under reduced pressure at 40°C and the residue was separated by reversed-phase Flash, eluted with 47% ACN to obtain 700 mg of the target product (yield: 74.86%, purity: 99.39%), MS: [M+H] 215.96, 217.96.

### (2) Synthesis of Compound 73-2

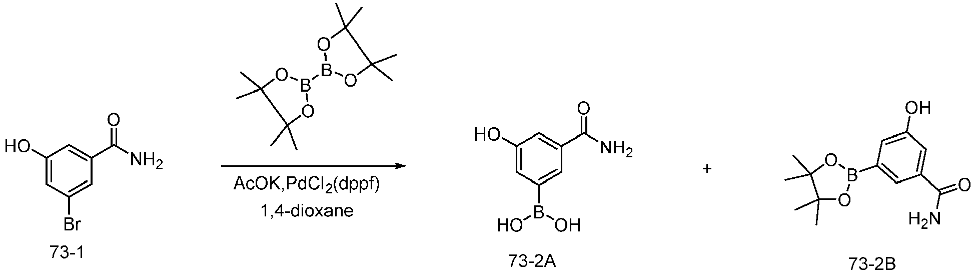

700 mg of 73-1 was added into 10 ml of 1,4-dioxane, followed by 1234 mg of BPDB, 954 mg of AcOK and 713 mg of PdCl₂(dppf), replaced by N₂ after the addition, raised to 85°C under the protection of N₂ and stirred. Monitored by LC-MS until no 736-1 remained. The mixture was concentrated to dryness under reduced pressure at 40°C, and the residue was separated by reversed-phase column chromatography, eluted with 45% ACN to obtain 98 mg of the target product (purity: 80.23%, yield: 16.71%), MS: [M+H] 182.32.

### (3) Synthesis of Compound 73

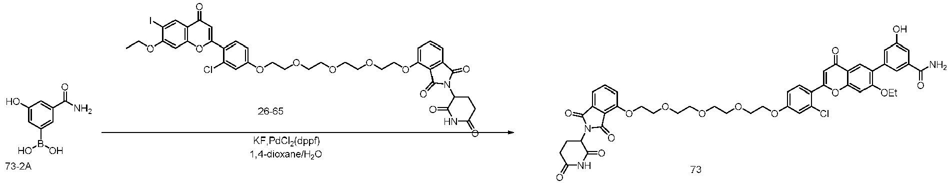

40 mg of 26-65 was added into 3 ml of 2-MTHF, followed by 25 mg of 73-2A, 16 mg of KF, 10 mg of PdCl₂(dppf) and 1 ml of H₂O, replaced by N₂ after the addition, raised to 70°C under the protection of N₂ and stirred. Monitored by LC-MS until no 26-65 remained. A proper amount of DCM was added, dried over Na₂SO₄ and suction filtered, the filtrate was concentrated to dryness at 40°C under reduced pressure, and the residue was separated by Pre-TLC (DCM:MeOH=15:1) to obtain 21 mg (yield: 51.70%, purity: 96.47%), MS: [M+H] 884.29.

¹H NMR (400 MHz, DMSO-*d*₆) δ 11.11 (s, 1H), 9.71 (s, 1H), 7.98 (s, 1H), 7.94 (s, 1H), 7.82 (d, *J* = 8.3 Hz, 1H), 7.75 (d, *J* = 8.7 Hz, 1H), 7.49 (s, 1H), 7.45 (d, *J* = 1.9 Hz, 1H), 7.36 (dd, *J* = 8.3, 2.1 Hz, 1H), 7.32 (s, 2H), 7.27 (s, 2H), 7.13 (d, *J* = 8.7 Hz, 2H), 6.55 (s, 1H), 5.11 (dd, *J* = 12.9, 5.4 Hz, 1H), 4.31 (s, 2H), 4.22 (d, *J* = 6.6 Hz, 4H), 3.78 (s, 4H), 3.58 (dd, *J* = 13.7, 3.7 Hz, 8H), 2.92 - 2.83 (m, 1H), 2.64 - 2.52 (m, 2H), 2.06 - 1.99 (m, 1H), 1.33 (d, *J* = 6.9 Hz, 3H).

### Synthesis example 64

### Synthesis of Compound 74

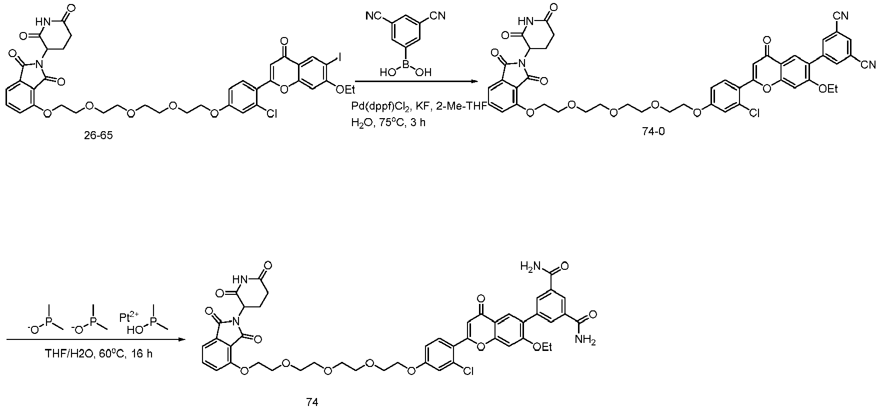

### (1) Synthesis of Compound 74-0

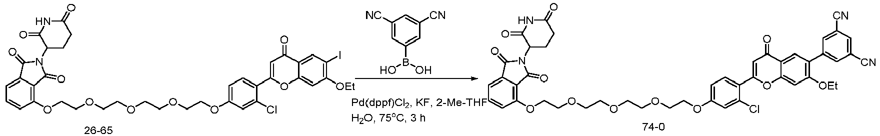

26-65 (90 mg, 1 eq.), (3, 5-dicyclopropenyl) boronic acid (95 mg, 3 eq.) and KF (70 mg, 6.0 eq.) were added into 2-methyltetrahydrofuran (3 ml) and water (1 ml) with stirring, Pd(dppf)Cl₂ (11 mg, 0.1 eq.) was added under N₂ atmosphere, heated to 75°C and reacted for 8 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 45 mg off-white solid. MS: [M+H]=875.21.

¹H NMR (400 MHz, DMSO-*d*₆) δ 8.38 (dd, *J* = 6.7, 1.5 Hz, 3H), 8.04 (s, 1H), 7.79 (t, *J* = 7.9 Hz, 1H), 7.72 (d, *J* = 8.7 Hz, 1H), 7.49 (d, *J* = 8.5 Hz, 1H), 7.42 (d, *J* = 7.3 Hz, 1H), 7.31 (s, 1H), 7.22 (d, *J* = 2.5 Hz, 1H), 7.10 (dd, *J* = 8.7, 2.6 Hz, 1H), 6.57 (s, 1H), 4.74 (q, *J* = 3.6, 2.4 Hz, 2H), 4.32 (t, *J* = 4.3 Hz, 2H), 4.25 - 4.18 (m, 4H), 3.65 (dd, *J* = 5.9, 3.3 Hz, 2H), 3.58 (dq, *J* = 9.2, 4.8, 4.2 Hz, 8H), 3.24 (t, *J* = 5.6 Hz, 1H), 2.86 (s, 1H), 2.61 (d, *J* = 18.6 Hz, 1H), 2.04 (d, *J* = 6.4 Hz, 1H), 1.35 (d, *J* = 6.9 Hz, 3H).

### (2) Synthesis of Compound 74

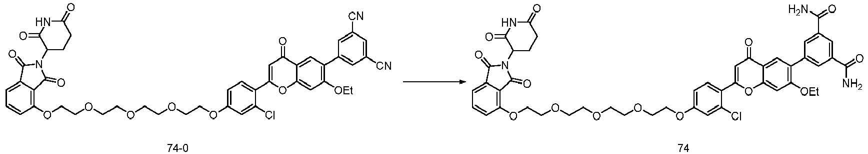

74-0 (30 mg, 1 eq.), Pt (0.1 eq.) were added into tetrahydrofuran (3 ml) and water (3 ml) with stirring, heated to 60°C and continued the reaction for 16 h under N₂ atmosphere, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 15 mg off-white solid. MS: [M+H]=911.31.

¹H NMR (400 MHz, DMSO-*d*₆) δ 8.34 (d, *J* = 14.0 Hz, 1H), 8.23 (s, 1H), 8.06 (s, 1H), 8.00 (m, 1H), 7.82 - 7.74 (m, 2H), 7.51 (s, 1H), 7.43 (d, *J* = 7.3 Hz, 1H), 7.33 (s, 1H), 7.23 (d, *J* = 2.4 Hz, 1H), 7.12 (d, *J* = 10.0 Hz, 1H), 6.59 (s, 1H), 5.06 (dd, *J* = 12.8, 5.5 Hz, 1H), 4.32 (d, *J* = 4.9 Hz, 2H), 4.24 (dd, *J* = 14.5, 7.6 Hz, 4H), 3.82 (s, 2H), 3.78 (s, 2H), 3.68 - 3.64 (m, 2H), 3.57 (dd, *J* = 7.3, 4.8 Hz, 7H), 3.53 (d, *J* = 4.3 Hz, 1H), 2.89 - 2.81 (m, 1H), 2.63 (d, *J* = 17.7 Hz, 2H), 2.21 (t, *J* = 7.4 Hz, 1H), 1.33 (s, 3H).

### Synthesis example 65

### Synthesis of Compound 75

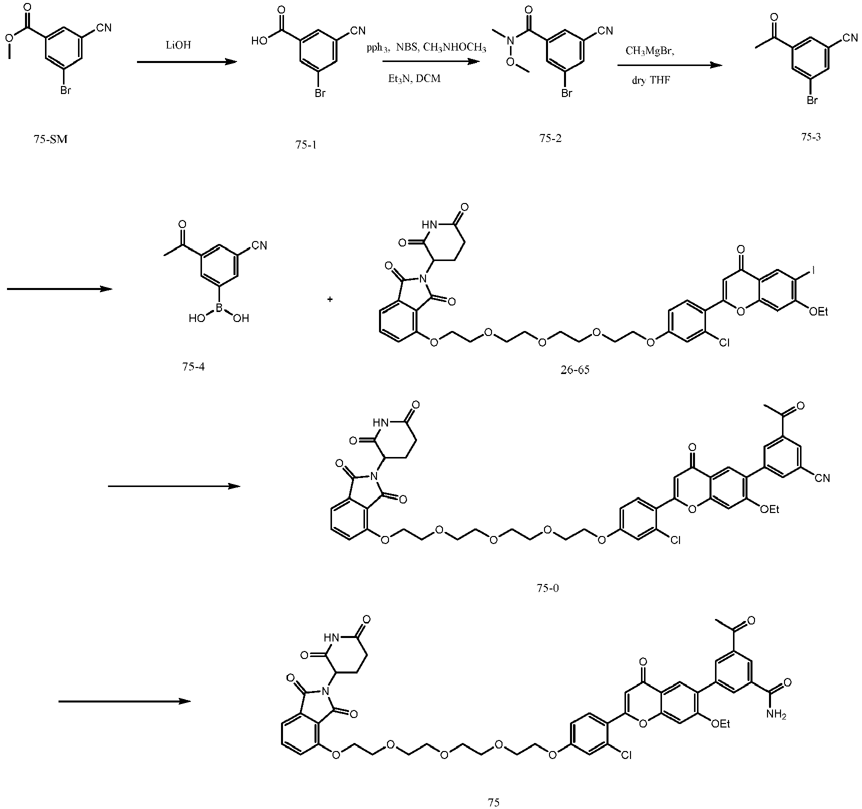

### (1) Synthesis of Compound 75-1

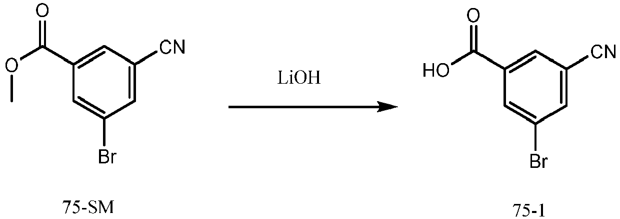

2.00 g of 75-SM was dissolved in 20 ml THF, added 4 ml MeOH, 4 ml H₂O, and 1.75 g LiOH.H₂ O was added with stirring.

After 3h, the complete reaction of raw materials was monitored by TLC (PE: EA=3:1).

The reaction solution was concentrated, 80 ml of water was added into the concentrate, 1N HCl was added with stirring until pH was 3, a white solid precipitated, extracted with EA 50 ml for 3 times, the organic phases were combined, washed once with 80 ml of saturated brine, dried over anhydrous sodium sulfate, filtered, and concentrated to obtain 1.9 g white solid and was used directly in the next reaction.

### (2) Synthesis of Compound 75-2

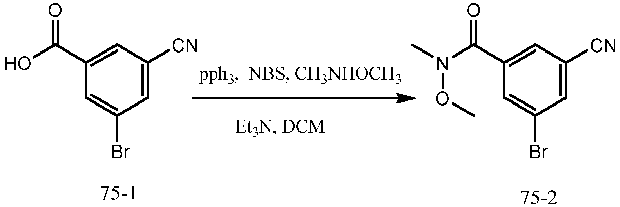

1.00 g of 75-1 was dissolved in DCM, at 0°C, added 1.74 g of triphenylphosphine, white suspension, 1.18 g of NBS, orange clear solution, raised to room temperature after 15min, 0.65 g of dimethylhydroxylamine hydrochloride and 0.49 g of triethylamine were sequentially added, and the color gradually became dark.

After 1h, sample was taken and sent to LC-MS, showing predominantly target product 268.

The reaction solution was poured into 100 ml of saturated sodium bicarbonate solution, with white smoke, extracted 3 times with 75 ml of DCM, the organic phases were combined, washed once with 100 ml of saturated brine, dried over anhydrous sodium sulfate and concentrated.

Purification: dissolved in DCM, stirred with 3g of silica gel, eluted with 0-50% EA\PE, collected and concentrated to obtain 0.99g white solid.

### (3) Synthesis of Compound 75-3

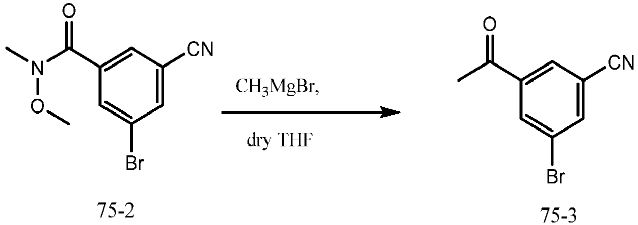

77 mg of 75-2 was put into a single-mouth bottle, protected by nitrogen, added with 2 ml of ultra-dry tetrahydrofuran for dissolution, and added with 0.3 ml of 1M tetrahydrofuran solution of methyl magnesium bromide at 0°C to obtain a light yellow clear solution.

Stirred at 0°C for 1 h.

5 ml of 5% HCl\EtOH solution was added, after stirring for 5min, 20 ml of saturated brine was added, then extracted with 20 ml of DCM for 3 times, the organic phases were combined, washed once with saturated brine, dried over anhydrous sodium sulfate, filtered and concentrated to obtain 56 mg white solid.

### (4) Synthesis of Compound 75-4

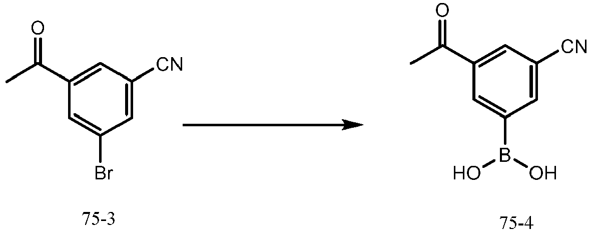

100 mg of 75-3 was dissolved into 5 ml of 1, 4-dioxane, added with 170 mg bisphenopinacol borate, 131 mg potassium acetate, 98 mg Pd(dppf)Cl₂ , replaced by N₂ protection for 3 times, and stirred at 80°C overnight. A sample was taken and sent to LC-MS, mainly ionizing to 190, and concentrated the reaction solution. Purification: 80 g C18 silica gel column pre-filled and the concentrate was loaded with 2ml MeOH. Eluted with 0-60% ACN\H₂O, concentrated at 40°C to obtain 66 mg off-white solid, borate was hydrolyzed to boric acid during purification.

### (5) Synthesis of Compound 75-0

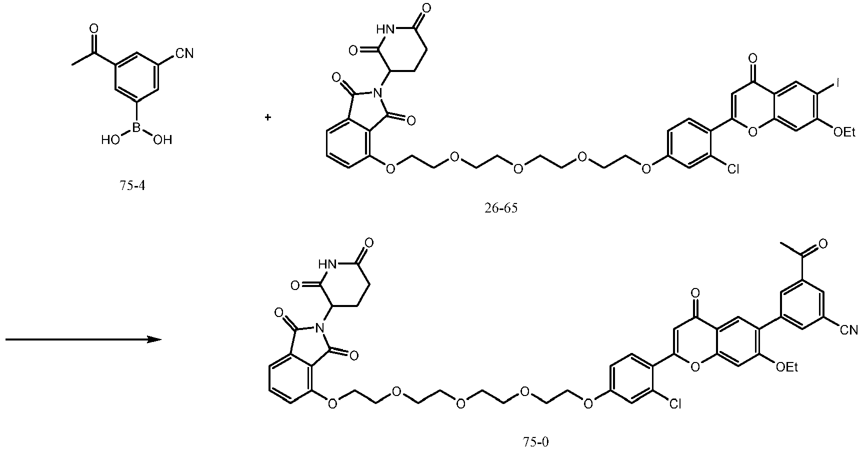

105 mg of 75-4 and 100 mg of 26-65 raw materials were dissolved into 3 ml of 2-MTHF, added with 40 mg of KF and 25 mg of Pd(dppf)Cl₂, followed by 1 ml of water, protected by N₂, 70°C, stirred overnight, anhydrous sodium sulfate was added into the reaction solution, filtered, washed 2 times with DCM, and concentrated for purification: 30 g of silica gel column pre-filled, dissolved and loaded the sample with DCM, eluted with 0-5% DCM\MeOH, collected the chromatographic solution, and concentrated to obtain 78 mg white solid.

### (6) Synthesis procedure for Compound 75

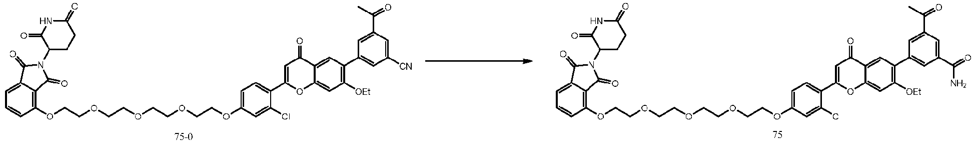

78 mg of 75-0 was dissolved into 3 ml of ultra-dry acetonitrile, and a catalytic amount of hydrogenated (dimethylphosphinic acid-kP) [hydrobis (dimethylphosphinic acid-kP)] platinum (II) was added, stirred overnight, filtered, concentrated, purified: 30 g of silica gel column pre-filled, dissolved and loaded the sample with DCM, eluting with 0-5% DCM\MeOH, collected the chromatographic solution, and concentrated to obtain 56 mg white solid. MS: [M+H] 910.33, purity: 91.66%

¹H NMR (400 MHz, DMSO-*d*₆) δ 8.28 (t, *J* = 2.1 Hz, 1H), 8.11 - 8.03 (m, 3H), 7.81 - 7.75 (m, 1H), 7.77 (s, 2H), 7.63 (d, *J* = 8.6 Hz, 1H), 7.54 (dd, *J* = 7.9, 7.2 Hz, 1H), 7.16 (s, 1H), 7.08 (dd, *J* = 7.9, 1.1 Hz, 1H), 7.02 (d, *J* = 2.2 Hz, 1H), 6.80 (dd, *J* = 8.6, 2.3 Hz, 1H), 6.74 (s, 1H), 5.40 (t, *J* = 3.5 Hz, 1H), 4.16 (t, *J* = 4.9 Hz, 2H), 4.16 - 4.06 (m, 4H), 3.78 (td, *J* = 5.0, 3.0 Hz, 4H), 3.73 - 3.61 (m, 8H), 2.92 - 2.81 (m, 1H), 2.64 - 2.49 (m, 2H), 2.14 - 2.03 (m, 1H), 1.32 (t, *J* = 6.3 Hz, 3H).

### Synthesis example 66

### Synthesis of Compound 76

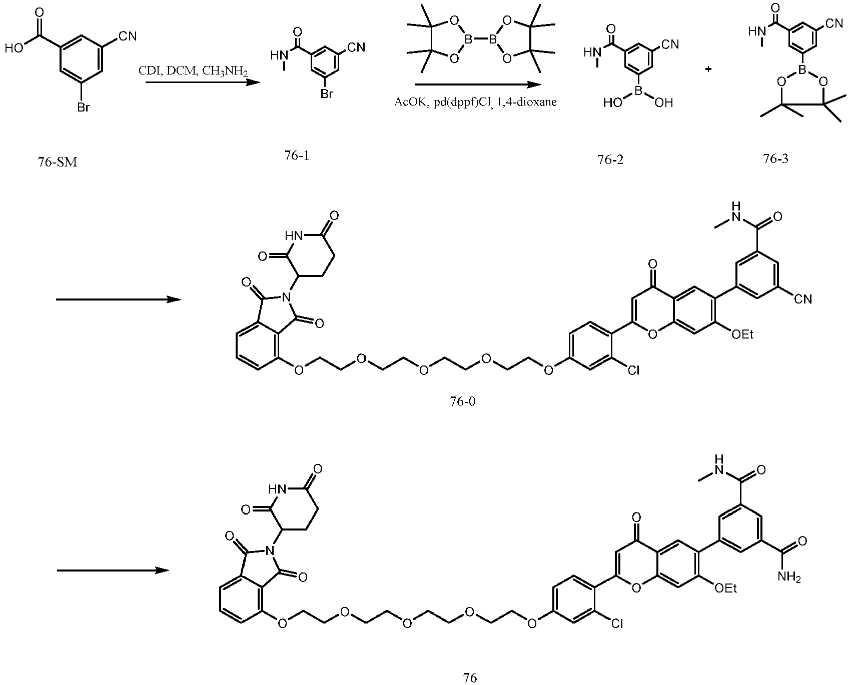

### (1) Synthesis of Compound 76-1

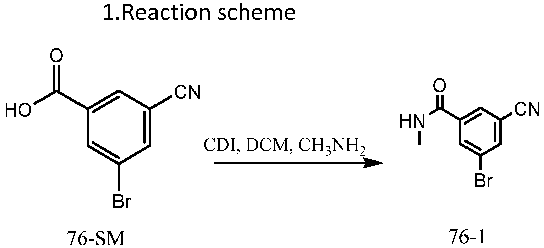

210 mg of 76-SM was dissolved into 10 ml of DCM, added with 151 mg of CDI, the solution became clear, after stirring for 20 min, methylamine alcohol solution was dropped, after 2 h, sample was taken and sent to LC-MS, showing predominantly target product, purification: directly loaded the reaction solution, eluted with 0-10% MeOH/DCM, collected the chromatographic solution, combined and concentrated to obtain 195 mg white solid.
76-2

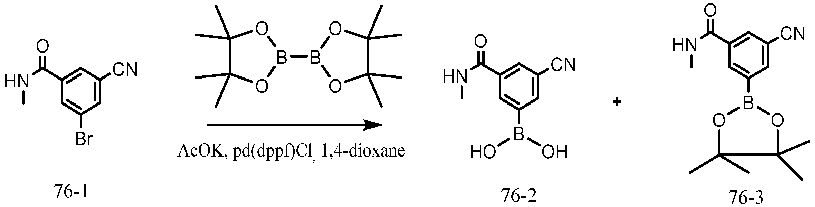

### Experimental procedures

195 mg 76-1 was dissolved into 20 ml 1, 4-dioxane, added with 311 mg bisphenopinacol borate, 240 mg potassium acetate, 179 mg Pd(dppf)Clz , replaced by N₂ protection for 3 times, and stirred at 80°C overnight. Sample was taken and sent to LC-MS, mainly ionizing to 205 and 287 as boric acid and borate respectively, and concentrated the reaction solution. 40 g C18 silica gel column pre-filled and the concentrate was loaded with 4 ml MeOH. Concentrated at 40°C to obtain 185 mg off-white solid, borate was hydrolyzed to boric acid during purification.

### (2) Synthesis of Compound 76-0

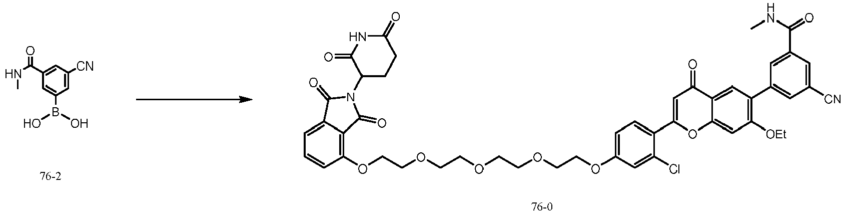

76-2 and 850 mg of 26-52 in the above reaction were dissolved in 2-MTHF, KF and Pd(dppf)Cl₂ were added, followed by 1 ml of water, protected by N₂, stirred overnight at 70°C, anhydrous sodium sulfate was added to the reaction solution, filtered and washing with DCM for 2 times, concentrated and passed through 30 g of pre-filled silica gel column, dissolved and loaded the sample with DCM, eluted with 0-5% DCM\MeOH and collected the chromatographic solution, concentrated to obtain 65 mg white solid.

### (3) Synthesis of Compound 76

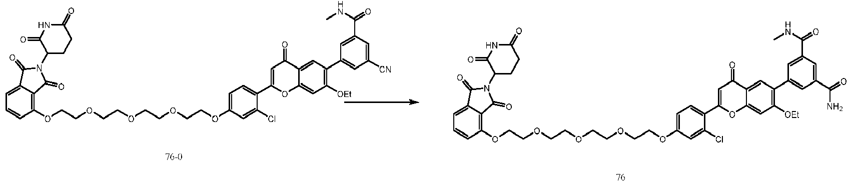

65 mg of 76-0 was dissolved in ultra-dry acetonitrile, a catalytic amount of hydrogenated (dimethylphosphinic acid-kP) [hydrogen bis (dimethylphosphinic acid-kP)] platinum (II) was added, stirred overnight and filtered, concentrated and passed through 30 g of pre-filled silica gel column, dissolved and loaded the sample with DCM, eluted with 0-5% DCM\MeOH and collected the chromatographic solution, concentrated to obtain 25 mg white solid.

MS: [M+H] 925.34, purity: 93.04%.

¹H NMR (400 MHz, DMSO-*d*₆) δ 11.11 (s, 1H), 8.34 (s, 1H), 8.22 (s, 1H), 8.17 (s, 2H), 8.06 (s, 1H), 7.79 (dd, *J* = 17.7, 8.5 Hz, 2H), 7.49 (s, 2H), 7.36 (d, *J* = 6.4 Hz, 2H), 6.58 (s, 1H), 4.23 (s, 4H), 3.78 (s, 4H), 3.59 (d, *J* = 10.0 Hz, 8H), 2.82 (d, *J* = 4.4 Hz, 3H), 1.34 - 1.29 (m, 6H).

### Synthesis example 67

### Synthesis of Compound 77

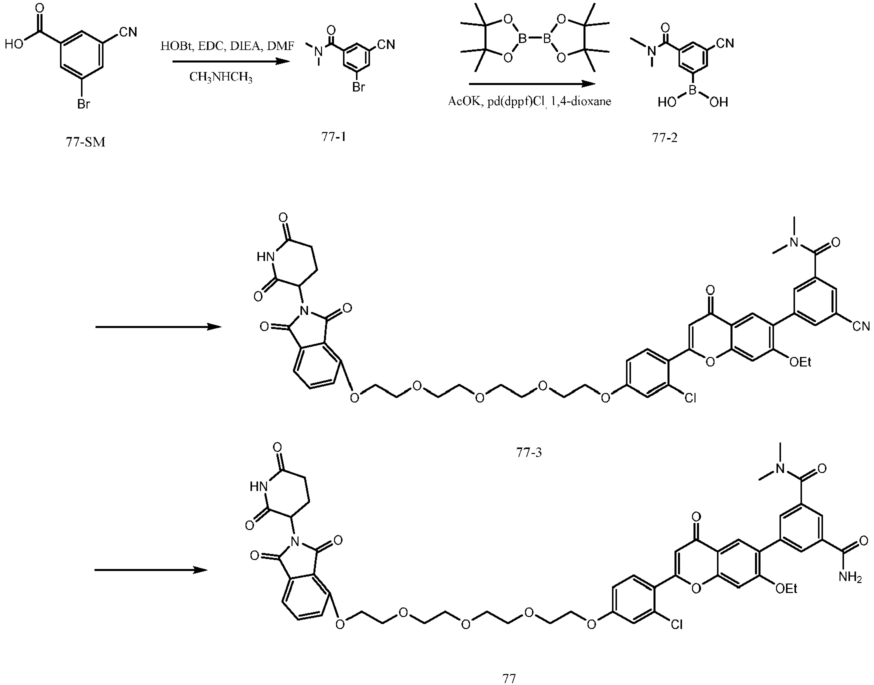

### (1) Synthesis of Compound 77-1

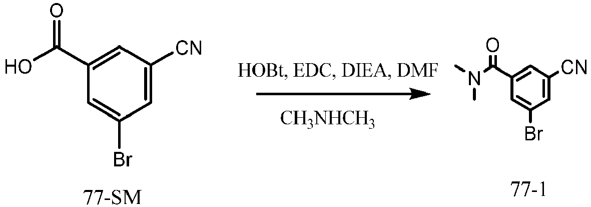

502 mg of 77-SM was dissolved in 15 ml of DMF, 570 mg of HOBt, 809 mg of EDCI and 947 mg of DIPEA were sequentially added, the solution was turned brown from colorless, 344 mg of dimethylamine hydrochloride was added, and stirred at room temperature overnight.

A sample was taken and sent to LC-MS showing mainly as target product.

The reaction solution was diluted with 60 ml of water, extracted three times with 40 ml of EA, the organic phases were combined and washed once with 60 ml of water and once with brine, dried over anhydrous sodium sulfate, filtered and concentrated to obtain 0.71g orange oil.

Purification: diluted the crude product with DCM, added 1.50 g of silica gel for loading, eluted with 0-30% EA\PE.

470 mg of orange syrup.

### (2) Synthesis of Compound 77-2

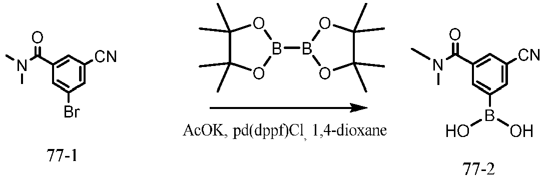

470 mg of 77-1 was dissolved into 45ml of 1,4-dioxane, added with 707 mg bisphenopinacol borate, 547 mg potassium acetate, 409 mg Pd(dppf)Cl₂.

Replaced by N₂ protection for 3 times, heated to 80°C and stirred overnight.

A sample was taken and sent to LC-MS, mainly ionizing to 218, and concentrated the reaction solution.

Purification: 80 g C18 silica gel column pre-filled and the concentrate was loaded with 4 ml MeOH.

Eluted with 0-60% ACN\H₂O.

Concentrated at 40°C to obtain 185 mg off-white solid, borate was hydrolyzed to boric acid during purification.

### (3) Synthesis of Compound 77-3

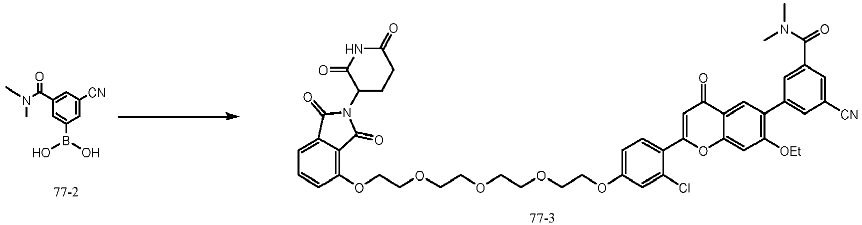

124 mg of 77-2 and 100 mg of 26-65 were dissolved in 3 ml of 2-MTHF, added with 40 mg of KF and 25 mg of Pd(dppf)Cl₂, followed by 1 ml of water, protected by N₂, stirred overnight at 70°C, anhydrous sodium sulfate was added to the reaction solution, filtered, washed 2 times with DCM, and concentrated.

Purification: 30 g of silica gel column pre-filled, dissolved and loaded with DCM, eluted with 0-5% DCM/MeOH.

The chromatographic solution was collected and concentrated to obtain 68 mg white solid.

¹H NMR (400 MHz, DMSO-*d*₆) δ 11.10 (s, 1H), 8.12 (s, 1H), 7.99 (s, 1H), 7.90 (s, 2H), 7.78 (dd, *J* = 21.9, 8.5 Hz, 2H), 7.44 (d, *J* = 2.1 Hz, 1H), 7.39 - 7.32 (m, 2H), 7.27 (d, *J =* 2.4 Hz, 1H), 7.13 (dd, *J* = 8.7, 2.4 Hz, 1H), 6.56 (s, 1H), 5.11 (dd, *J* = 13.1, 5.3 Hz, 1H), 4.27 (dd, *J* = 26.1, 5.8 Hz, 6H), 3.82 - 3.76 (m, 4H), 3.58 (dd, *J* = 14.4, 3.1 Hz, 9H), 3.02 (s, 3H), 2.96 (s, 3H), 2.05 - 1.97 (m, 2H), 1.32 (t, *J* = 6.9 Hz, 3H), 1.07 (s, 2H).

### (4) Synthesis of Compound 77

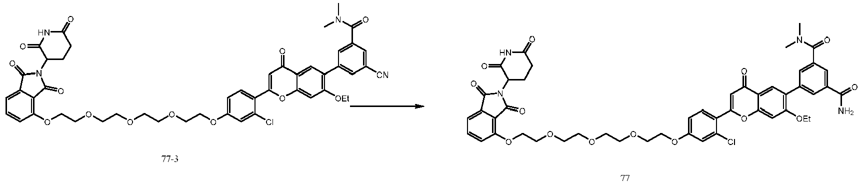

68 mg of 77-3 was dissolved into 3 ml of ultra-dry acetonitrile, 10 mg of catalyst Ghaffar-Parkins was added, and stirred overnight.

Filtered and concentrated.

Purification: 30 g silica gel column pre-filled, dissolved and loaded with DCM, eluted with 0-5% DCM/MeOH.

The chromatographic solution was collected and concentrated to obtain 33 mg white solid.
MS: [M+H] 939.37, purity: 95.24%
¹H NMR (400 MHz, DMSO-*d*₆) δ 11.10 (s, 1H), 8.18 (s, 1H), 8.12 (s, 1H), 8.00 (s, 1H), 7.90 (s, 1H), 7.79 (dd, *J* = 19.7, 8.4 Hz, 2H), 7.73 (s, 1H), 7.50 (s, 1H), 7.44 (s, 1H), 7.36 (s, 2H), 7.27 (s, 1H), 7.14 (s, 1H), 6.56 (s, 1H), 5.32 (s, 1H), 4.31 (s, 2H), 4.23 (d, *J* = 7.1 Hz, 4H), 3.78 (s, 4H), 3.58 (d, *J* = 9.5 Hz, 9H), 3.00 (d, *J* = 15.1 Hz, 7H), 2.67 (s, 1H), 2.33 (s, 1H), 2.05 - 1.95 (m, 6H).

### Synthesis example 68

### Synthesis of Compound 78

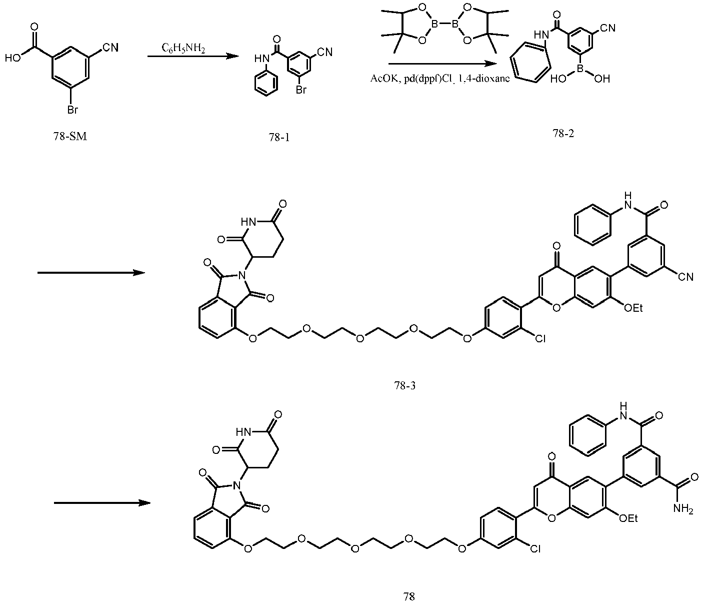

### (1) Synthesis of Compound 78-1

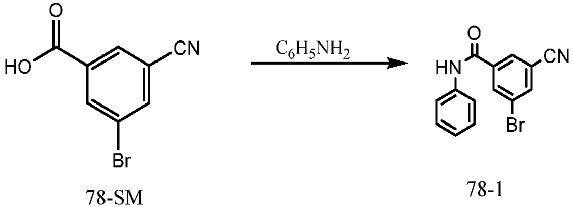

1.04 g of 78-SM was dissolved in 15 ml of DMF, 0.43 g of aniline, 0.56 g of DMAP, 1.15 g of EDCI were added.

Stirred at room temperature to obtain yellow clear liquid.

After 48h, a sample was taken and sent to LC-MS showing mainly as target product.

The reaction solution was diluted with 60 ml of water, extracted three times with 40 ml of EA, the organic phases were combined and washed once with 60 ml of water and once with brine, dried over anhydrous sodium sulfate, filtered and concentrated to obtain 0.71g orange oil.

Purification: the crude product was diluted with DCM, added 1.50 g of silica gel for loading, eluted with 0-30% EA\PE to obtain 0.98 g light yellow solid.

### (2) Synthesis of Compound 78-2

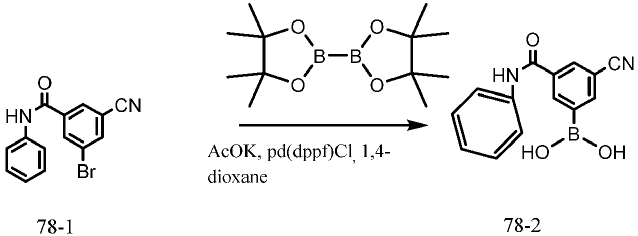

559 mg of 78-1 was dissolved into 45ml of 1,4-dioxane, added with 707 mg bisphenopinacol borate, 547 mg potassium acetate, 409 mg Pd(dppf)Cl₂.

Replaced by N₂ protection for 3 times, heated to 80°C and stirred overnight.

A sample was taken and sent to LC-MS, mainly ionizing to 218, and concentrated the reaction solution.

Purification: 80 g C18 silica gel column pre-filled and the concentrate was loaded with 4 ml MeOH.

Eluted with 0-60% ACN\H₂O.

Concentrated at 40°C to obtain 185 mg off-white solid, borate was hydrolyzed to boric acid during purification.
480 mg

### (3) Synthesis of Compound 78-3

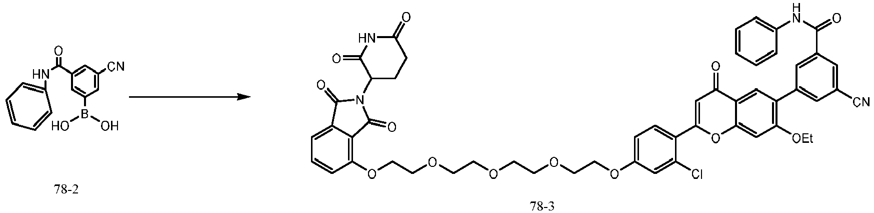

151 mg of 78-2 and 100 mg of 16-65 were dissolved in 3 ml of 2-MTHF, added with 40 mg of KF and 25 mg of Pd(dppf)Cl₂, followed by 1 ml of water, protected by N₂, stirred overnight at 70°C, added anhydrous sodium sulfate into the reaction solution, filtered, washed 2 times with DCM, and concentrated.

Purification: 30 g of silica gel column pre-filled, dissolved and loaded the sample with DCM, eluted with 0-5% DCM\MeOH, collected the chromatographic solution, and concentrated to obtain 77 mg white solid.

### (4) Synthesis of Compound 78

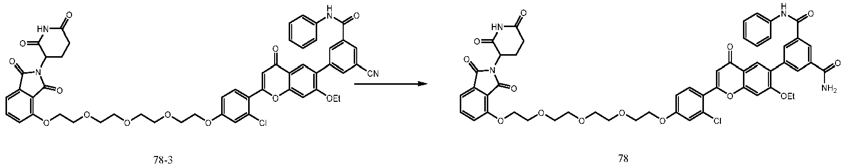

77 mg of 78-3 was dissolved into 1 ml of ultra-dry acetonitrile, a catalytic amount of hydrogenation (dimethyl phosphinic acid-kP) [hydrogen bis (dimethyl phosphinic acid-kP)] platinum (II) was added, stirred overnight, filtered and concentrated, 30 g of silica gel column pre-filled, dissolved and loaded with DCM, eluted with 0-5% of DCM\MeOH and collected the chromatographic solution, concentrated to obtain 45 mg white solid. MS: [M+H] 987.41, purity: 97.03%.

¹H NMR (400 MHz, DMSO-*d*₆) δ 11.11 (s, 1H), 10.44 (s, 1H), 8.46 (s, 1H), 8.29 (s, 2H), 8.22 (s, 1H), 8.11 (s, 1H), 7.80 (q, *J* = 8.4 Hz, 4H), 7.56 (s, 1H), 7.45 (d, *J* = 2.0 Hz, 1H), 7.37 (dt, *J* = 10.2, 3.1 Hz, 4H), 7.28 (d, *J* = 2.4 Hz, 1H), 7.12 (t, *J* = 7.3 Hz, 2H), 6.59 (s, 1H), 5.11 (dd, *J* = 12.9, 5.4 Hz, 1H), 4.34 - 4.20 (m, 6H), 3.79 (d, *J* = 8.6 Hz, 4H), 3.63 - 3.54 (m, 8H), 2.63 - 2.52 (m, 2H), 2.01 (s, 1H), 1.33 (d, *J* = 6.9 Hz, 3H).

### Synthesis example 69

### Synthesis of Compound 79

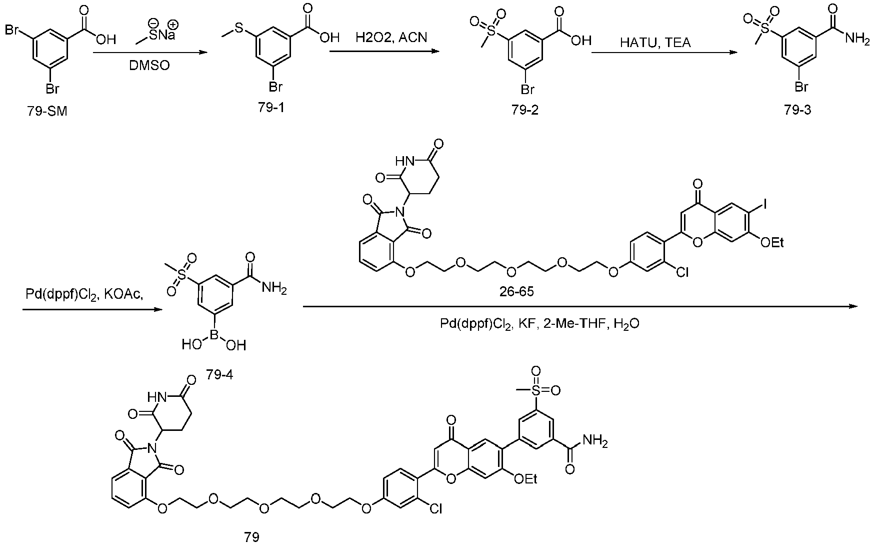

### (1) Synthesis of Compound 79-1

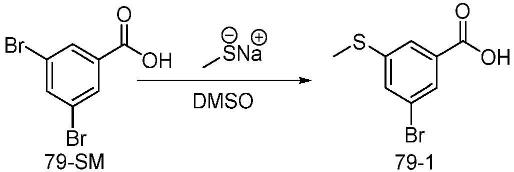

5 g of 79-SM was added into 200 ml of DMSO, followed by 1.38 g of sodium sulfide, stirred after the addition, then stirred at room temperature for 16h under the protection of N₂. The disappearance of the raw materials and the generation of product were confirmed by liquid quality monitor, and added diluted hydrochloric acid into the product. Extracted with ethyl acetate and dried the organic phase. The target product was spin-dried to obtain 2 g of crude product and used directly in the next step without purification. MS: [ M-H ] 245.15.

### (2) Synthesis of Compound 79-2

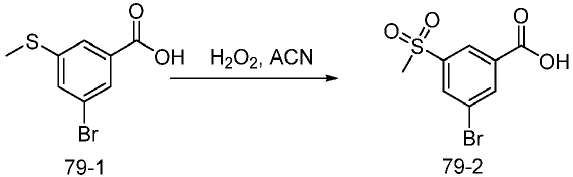

1 g of 79-1 was added into 50 ml of ACN, then added hydrogen peroxide (10 mL) dropwise slowly, stirred after the addition, and then stirred for 16h at 90°C under the protection of N₂. The disappearance of the raw materials and the generation of product were confirmed by liquid quality monitor, the system was concentrated and evaporated, a proper amount of DMF was used for dissolving the residue, a C18 reversed-phase column was used for loading, reversed-phase separation was carried out, eluted with 10% ACN to obtain 300 mg of the target product (yield: 30%, Purity: 97.90%). MS: [ M-H ] 277.27.

### (3) Synthesis of Compound 79-3

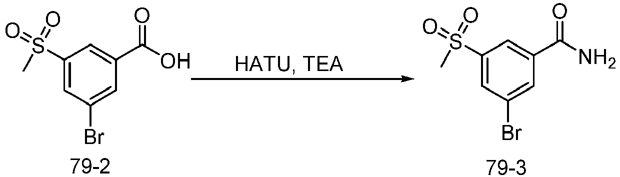

300 mg of 79-2 was added into 10 ml of DMF, followed by 200 mg of HATU and 100 mg of TEA, stirred after the addition, and then stirred at room temperature for 0.5h under the protection of N₂. Then, ethanol solution of ammonia (10 mL) was added, the disappearance of the raw materials and the generation of product were confirmed by liquid quality monitor, the system was concentrated and evaporated, a proper amount of DMF was used for dissolving the residue and loaded on a C18 reversed-phase column, reversed-phase was carried out, eluted with 10% can to obtain 60 mg of the target product (yield: 20%, Purity: 95.16%), MS: [M+H] 278.34.

### (4) Synthesis of Compound 79-4

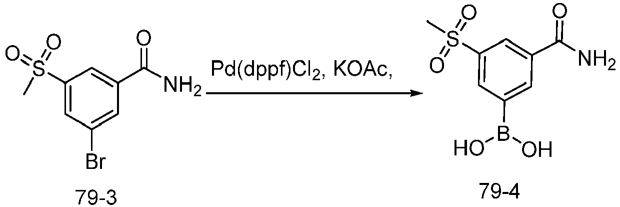

60 mg of 79-3 was added into 10 ml of DMF, followed by 60 mg of bisphenopinacol borate, 17.14 mg of Pd(dppf)Cl₂ and 40 mg of KOAc, stirred after the addition, and stirred for 16h at 90°C under the protection of N₂. The disappearance of the raw materials and the generation of product were confirmed by liquid quality monitor, the system was concentrated and evaporated, a proper amount of DMF was used for dissolving the residue and loaded on a C18 reversed-phase column, after reversed-phase separation, eluted with 10% ACN to obtain the target product. The crude product was spin-dried to obtain 50 mg, and the reaction was used directly for the next synthesis. MS: [M+H] 244.42.

### (5) Synthesis of Compound 79

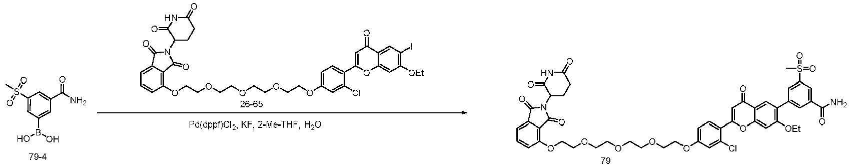

50 mg of 26-65 and 50 mg of 79-4 were added into 10 ml of 2-Me-THF and 2 mL of H₂O, followed by 17 mg of Pd(dppf)Cl₂ and 40 mg of KF, stirred after the addition, then stirred at 90°C for 16 h under protection of N₂. The disappearance of the raw materials and the generation of product were confirmed by liquid quality monitor, the system was concentrated and evaporated, a proper amount of DMF was used for dissolving the residue and loaded on a C18 reversed-phase column, after reversed-phase separation, eluted with 60% ACN to obtain 9.1 mg of the target product (yield: 16%, purity: 95.16%), MS: [M+H] 946.37.

¹H NMR (400 MHz, DMSO-*d*₆) δ 11.11 (s, 1H), 8.44-8.35 (m, 3H), 8.31 (s, 1H), 8.14 (s, 1H), 7.79 (dd, *J* = 16.0, 8.5 Hz, 2H), 7.69 (s, 1H), 7.44 (s, 1H), 7.41-7.33 (m, 2H), 7.28 (d, *J* = 2.1 Hz, 1H), 7.14 (d, *J* = 8.8 Hz, 1H), 6.59 (s, 1H), 5.11 (dd, *J* = 12.7, 5.3 Hz, 1H), 4.31 (s, 2H), 4.25 (d, *J* = 7.0 Hz, 4H), 3.79 (s, 4H), 3.64-3.54 (m, 8H), 2.87 (t, *J* = 9.0 Hz, 1H), 2.56 (s, 2H), 2.02 (d, J = 7.4 Hz, 1H), 1.33 (t, *J* = 6.8 Hz, 3H).

### Synthesis example 70

### Synthesis of Compound 80

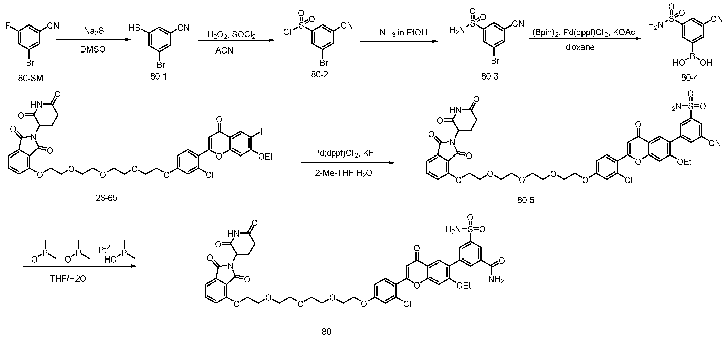

### (1) Synthesis of Compound 80-1

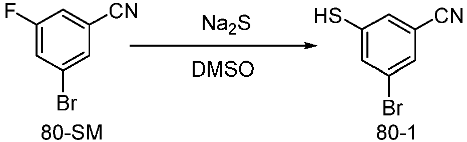

5 g of 80-SM was added into 200 ml of DMSO, followed by 1.38 g of sodium sulfide, stirred after the addition, then stirred at room temperature for 16 h under the protection of N₂. The disappearance of the raw materials and the generation of product were confirmed by liquid quality monitor, and added diluted hydrochloric acid into the product. Extracted with ethyl acetate and dried the organic phase. The target product was spin-dried to obtain 2 g of crude product and was used directly in the next step without purification. MS: [ M-H ] 212.17.

### (2) Synthesis of Compound 80-2

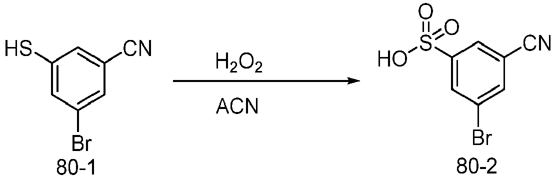

1.2 g of 80-1 was added into 50 ml of ACN, then added hydrogen peroxide (10 mL) dropwise slowly, stirred after the addition, then stirred for 16h at 90°C under the protection of N₂. The disappearance of the raw materials and the generation of product were confirmed by liquid quality monitor, the system was concentrated and evaporated to obtain 1.3 g of crude product, the reaction was used directly for the next synthesis. MS: [M-H] 260.15.

### (3) Synthesis of Compound 80-3

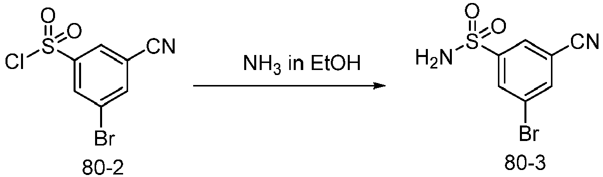

1.2 g of 80-2 was added into 58 mL of SOCl₂ , stirred for 16h at 90°C under the protection of N₂, spin-dried the reaction solution, then ethanol solution of ammonia (10 mL) was added at 0°C, the disappearance of the raw materials and the generation of product were confirmed by liquid quality monitor, the system was concentrated and evaporated, a proper amount of DMF was used for dissolving the residue and loaded on a C18 reversed-phase column, reversed-phase was carried out, eluted with 40% ACN to obtain 106 mg of the target product (yield: 8%, purity: 96.26%), MS: [M+H] 261.33.

### (4) Synthesis of Compound 80-4

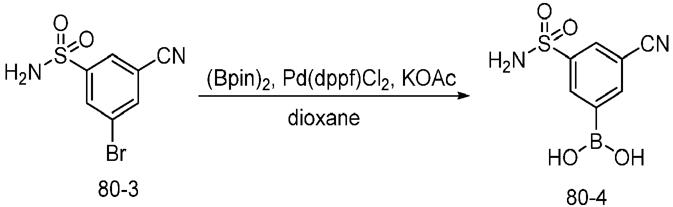

106 mg of 80-3 was added into 10 ml of dioxane, followed by 126 mg of bisphenopinacol borate, 17 mg of Pd(dppf)Cl₂ and 38 mg of KOAc, stirred after the addition, and stirred for 16 h at 90°C under the protection of N₂. The disappearance of the raw materials and the generation of product were confirmed by liquid quality monitor, the system was concentrated and evaporated, a proper amount of DMF was used for dissolving the residue, a C18 reversed-phase column was used for loading, reversed-phase separation was carried out, eluted with 10% ACN to obtain the target product. The crude product was spin-dried to obtain 109 mg, and the reaction was used directly for the next synthesis. MS: [M+H] 227.

### (5) Synthesis of Compound 80-5

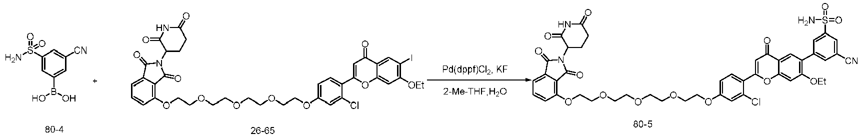

80 mg of 26-65 and 100 mg of 80-4 were added into 20 mL of 2-Me-THF and 5 mL of H₂O, followed by 17 mg of Pd(dppf)Cl₂ and 40 mg of KF, stirred after the addition, then stirred for 16 h at 90°C under protection of N₂. The disappearance of the raw materials and the generation of product were confirmed by liquid quality monitor, the system was concentrated and evaporated, a proper amount of DMF was used for dissolving the residue, a C18 reversed-phase column was used for loading, reversed-phase separation was carried out, eluted with 60% ACN to obtain 37mg of the target product (yield: 46%, Purity: 97.24%), MS: [M+H] 929.18.

### (6) Synthesis of Compound 80

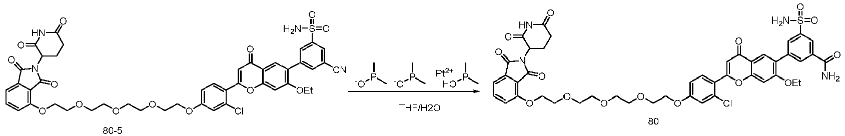

37 mg of 80-5 was added into 10 ml of THF and 2 mL of H₂O, followed by 15 mg of hydrogenated (dimethylphosphinic acid-kP) [hydrobis (dimethylphosphinic acid-kP)] platinum (II), stirred after the addition, then stirred for 16 h at 90°C under the protection of N₂. The disappearance of the raw materials and the generation of product were confirmed by liquid quality monitor, anhydrous sodium sulfate was added, and the organic phase system was concentrated and evaporated, a proper amount of DCM was used for dissolving the residue and loaded on Prep-TLC, developed with DCM/MeOH(15:1), and freeze-dried to obtain 12 mg of the target product (yield: 70.1%, purity: 97.40%). MS: [M+H] 947.36.

¹H NMR (400 MHz, Chloroform-d) δ 8.67 (t, 1H), 8.56 (t, 1H), 8.49 (t, 1H), 8.24 (s, 1H), 7.89 (d, 1H), 7.78 (s, 2H), 7.53 (d,1H), 7.46 (d, 3H), 7.25 (dd, 1H), 7.08 (s, 1H), 6.97 (s, 1H), 6.91 (dd, 1H), 5.16 (t, , 1H), 4.53 (dq, 1H), 4.38 - 4.27 (m, 2H), 4.23 - 4.08 (m, 3H), 3.77 (ddd, 1H), 3.65 (ddtd, 3H), 3.51 - 3.06 (m, 8H), 2.96 - 2.83 (m, 1H), 2.63 - 2.53 (m, 2H), 2.13 - 2.02 (m, 1H), 1.45 (t, 3H).

### Synthesis example 71

### Synthesis of Compound 81

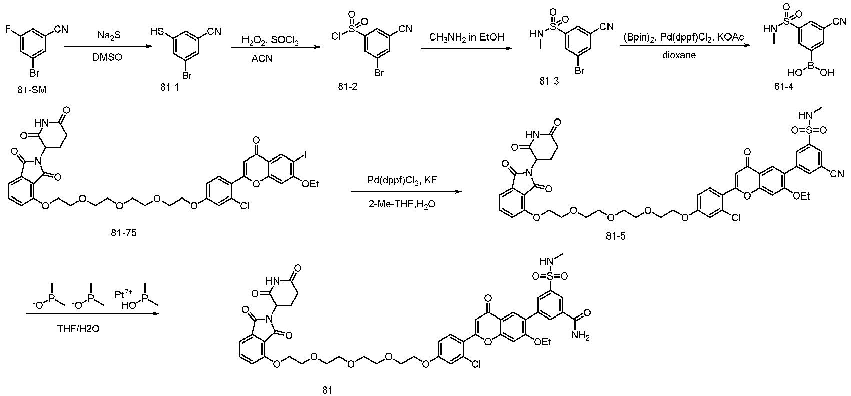

### (1) Synthesis of Compound 81-1

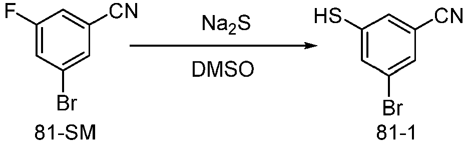

5 g of TDP102178-SM was added into 200 ml of DMSO, followed by 1.38 g of sodium sulfide, stirred after the addition, then stirred at room temperature for 16 h under the protection of N₂. The disappearance of the raw materials and the generation of product were confirmed by liquid quality monitor, and added diluted hydrochloric acid into the product. Extracted with ethyl acetate and dried the organic phase. The target product was spin-dried to obtain 2 g of crude product and was used directly in the next step without purification. MS: [M-H] 212.25.

### (2) Synthesis of Compound 81-2

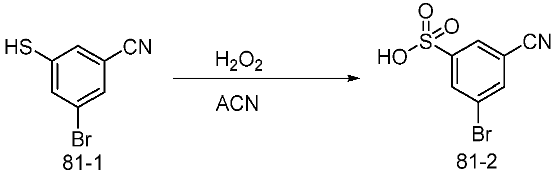

1.2 g of 811 was added into 50 ml of ACN, then added hydrogen peroxide (10 mL) dropwise slowly, stirred after the addition, then stirred for 16 h at 90°C under the protection of N₂. The disappearance of the raw materials and the generation of product were confirmed by liquid quality monitor, the system was concentrated and evaporated to obtain 1.3 g of crude product, the reaction was used directly for the next synthesis. MS: [M-H] 260.35.

### (3) Synthesis of Compound 81-3

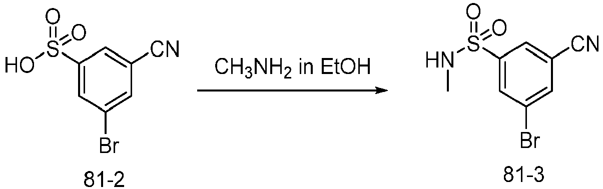

1.3 g of 81-2 was added into 60 mL of SOCl₂ , then stirred for 16h at 90°C under the protection of N₂, spin-dried the reaction solution, then ethanol solution of ammonia (10 mL) was added at 0°C, the disappearance of the raw materials and the generation of product were confirmed by liquid quality monitor, the system was concentrated and evaporated, a proper amount of DMF was used for dissolving the residue and loaded on a C18 reversed-phase column, reversed-phase was carried out, eluted with 40% ACN to obtain 110 mg of the target product (yield: 8%, purity: 95.16%), MS: [M+H]275.51.

### (4) Synthesis of Compound 81-4

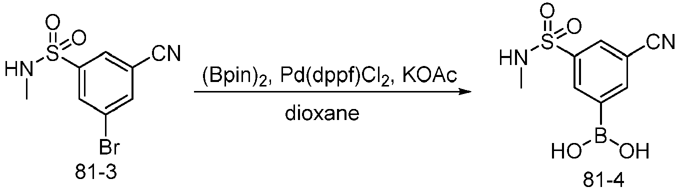

110 mg of 81-3 was added into 10 ml of dioxane, followed by 130 mg of bisphenopinacol borate, 17.14 mg of Pd(dppf)Cl₂ and 40 mg of KOAc, stirred after the addition, then stirred for 16 h at 90°C under the protection of N₂. The disappearance of the raw materials and the generation of product were confirmed by liquid quality monitor, the system was concentrated and evaporated, a proper amount of DMF was used for dissolving the residue and loaded on a C18 reversed-phase column, reversed-phase was carried out, eluted with 10% ACN to obtain the target product. Spin-dried the crude product to obtain 100 mg, the reaction was used directly for the next synthesis. MS: [M+H] 240.35.

### (5) Synthesis of Compound 81-5

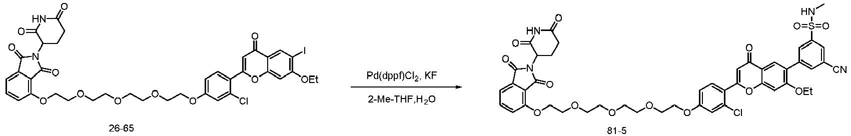

80 mg of 26-65 and 100 mg of TDP102175-4 were added into 20 mL of 2-Me-THF and 5 mL of H₂O, followed by 17 mg of Pd(dppf)Cl₂ and 40 mg of KF, stirred after the addition, then stirred for 16 h at 90°C under the protection of N₂. The disappearance of the raw materials and the generation of product were confirmed by liquid quality monitor, the system was concentrated and evaporated, a proper amount of DMF was used for dissolving the residue, a C18 reversed-phase column was used for loading, reversed-phase separation was carried out, eluted with 60% ACN to obtain 40 mg of the target product (yield: 46%, Purity: 95.16%), MS: [M+H] 943.12.

### (6) Synthesis of Compound 81

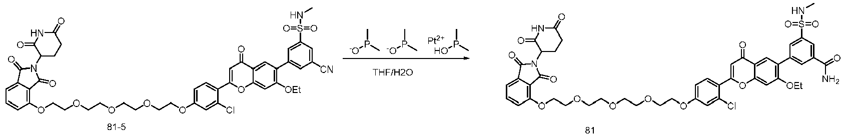

40 mg of TDP102178-5 was added into 10 ml of THF and 2 mL of H₂O, followed by 17 mg of hydrogenated (dimethylphosphinic acid-kP) [hydrobis (dimethylphosphinic acid-kP)] platinum (II), stirred after the addition, then stirred for 16 h at 90°C under the protection of N₂. The disappearance of the raw materials and the generation of product were confirmed by liquid quality monitor, anhydrous sodium sulfate was added, and the organic phase system was concentrated and evaporated, a proper amount of DCM was used for dissolving the residue and loaded on Prep-TLC, developed with DCM/MeOH(15:1), and freeze-dried to obtain 14.1 mg of the target product (yield: 70.1%, purity: 98.80%). MS: [M+H] 961.39.

¹H NMR (400 MHz, DMSO-*d*₆) δ 11.11 (s, 1H), 8.37 (s, 1H), 8.34 (s, 1H), 8.28 (s, 1H), 8.15 (s, 1H), 8.11 (s, 1H), 7.79 (dd, *J* = 17.0, 8.5 Hz, 2H), 7.67- 7.60 (m, 2H), 7.45 (d, *J =* 1.9 Hz, 1H), 7.39 (s, 1H), 7.36 (dd, *J* = 8.4, 2.1 Hz, 1H), 7.28 (d, *J* = 2.3 Hz, 1H), 7.13 (dd, *J* = 8.7, 2.2 Hz, 1H), 6.59 (s, 1H), 5.11 (dd, *J* = 12.8, 5.2 Hz, 1H), 4.33-4.20 (m, 6H), 3.79 (s, 4H), 3.58 (dd, *J* = 13.7, 3.8 Hz, 8H), 2.58 (d, *J* = 17.6 Hz, 2H), 2.47 (d, *J* = 5.0 Hz, 3H), 2.11-1.93 (m, 2H), 1.33 (t, *J* = 6.9 Hz, 3H).

### Synthesis example 72

### Synthesis of Compound 85

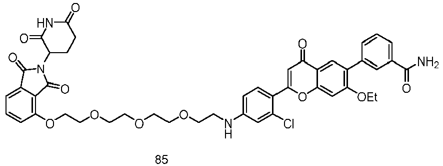

The preparation method is the same as the preparation method of compound 53, wherein the intermediate 26-65 was converted to intermediate 11-16.
MS: [M+H]=867.30
¹H NMR (400 MHz, CDCL3) δ 9.51 (s, 1H), 8.21 (s, 1H), 8.01 (t, 1H), 7.83 (m, 1H), 7.74 (m, 1H), 7.66 (m, 1H), 7.64 - 7.54 (m, 2H), 7.48 (m, 1H), 7.05 (m, 1H), 6.91 (s, 1H), 6.84 (d, , 1H), 6.75 (s, 1H), 6.57 (m, 1H), 5.41 (m, 1H), 4.59 (t, 1H), 4.18 (m, 2H), 4.11 (m, 2H), 3.79 (t, *J* = 4.9 Hz, 2H), 3.74 - 3.60 (m, 10H), 3.42 (m, , 2H), 2.92 - 2.81 (m, 1H), 2.64 - 2.49 (m, 2H), 2.15 - 2.03 (m, 1H), 1.37 (m, 1H).

### Synthesis example 73

### Synthesis of Compound 86

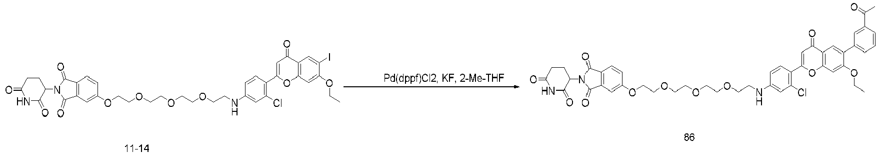

Steps: 1.0 g of 11-14 (1.0 eq), 570 mg (3.0 eq) of 3-acetylphenylboronic acid, 260 mg (0.3 eq) of Pd(dppf)Cl₂ and 390 mg (6.0 eq) of potassium fluoride were added into a 20 ml bottle, followed by 15 ml of 2-methyltetrahydrofuran and 15 ml of water, raised to 70°C to react for 18 h under the protection of N₂. the completion of the reaction was monitored by LC/MS and cooled to room temperature, 100 ml of ethyl acetate and 100 ml of saturated sodium chloride aqueous solution were added for washing, dried with anhydrous sodium sulfate, filtered and concentrated, and the residue was purified by column chromatography (CH₂Cl₂/MeOH, 0-5%, 0-40 min, gradient elution) to obtain 786.25 mg yellow solid with yield of 80.0%. MS [M+H] 866.31.

¹H NMR (400 MHz, DMSO-*d*₆) δ 11.10 (s, 1H), 8.14 (s, 1H), 8.00-7.90 (m, 2H), 7.82 (t, *J* = 8.8 Hz, 2H), 7.65-7.54 (m, 2H), 7.43 (d, *J* = 2.2 Hz, 1H), 7.38-7.29 (m, 2H), 6.80 (d, *J* = 2.2 Hz, 1H), 6.75-6.63 (m, 2H), 6.52 (s, 1H), 5.10 (dd, *J* = 12.9, 5.4 Hz, 1H), 4.33-4.27 (m, 2H), 4.23 (q, *J* = 7.0 Hz, 2H), 3.82-3.75 (m, 2H), 3.62-3.58 (m, 3H), 3.56 (s, 6H), 3.31-3.26 (m, 2H), 2.95-2.81 (m, 1H), 2.64 (s, 3H), 2.62-2.51 (m, 2H), 2.06-1.99 (m, 1H), 1.33 (t, *J* = 6.9 Hz, 3H).

### Synthesis example 74

### Synthesis of Compound 87

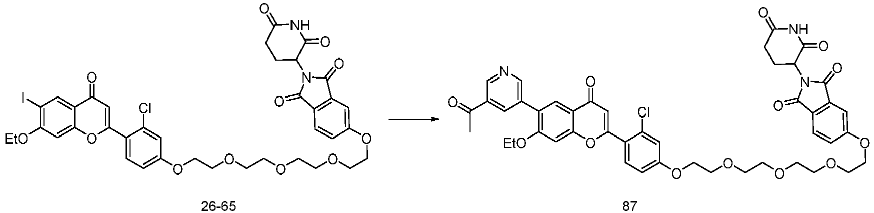

26-65 (1.2 g, 1 eq.), (5-acetylpyridine-3-yl) boronic acid (680 mg,3 eq.) and KF (494 mg, 6 eq.) were added into 2-Me-THF (30 ml) and water (10 ml) with stirring, Pd(dppf)Cl₂ (100 mg, 0.1 eq.) was added under N₂ atmosphere, raised to 75°C to continue the reaction for 16 h, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 520 mg of compound 87 as an off-white solid. MS: [M+H]=868.29.

¹H NMR (400 MHz, DMSO-*d*₆) δ 11.09 (s, 1H), 9.11 (d, *J* = 2.0 Hz, 1H), 8.99 (d, J = 2.1 Hz, 1H), 8.46 (t, *J* = 2.1 Hz, 1H), 8.04 (s, 1H), 7.81 (d, J = 8.3 Hz, 1H), 7.76 (d, *J* = 8.7 Hz, 1H), 7.44 (d, *J* = 2.1 Hz, 1H), 7.39 (s, 1H), 7.35 (dd, *J* = 8.3, 2.2 Hz, 1H), 7.27 (d, *J* = 2.4 Hz, 1H), 7.13 (dd, *J* = 8.7, 2.4 Hz, 1H), 6.58 (s, 1H), 5.10 (dd, *J* = 12.9, 5.3 Hz, 1H), 4.34-4.28 (m, 2H), 4.27-4.21 (m, 4H), 3.78 (q, *J* = 6.4 Hz, 4H), 3.60 (d, *J* = 2.6 Hz, 4H), 3.58-3.54 (m, 4H), 2.86 (d, *J* = 12.4 Hz, 1H), 2.69 (s, 3H), 2.63-2.52 (m, 2H), 2.04-1.98 (m, 1H), 1.33 (d, *J* = 6.9 Hz, 3H).

### Synthesis example 75

### Synthesis of Compound 88

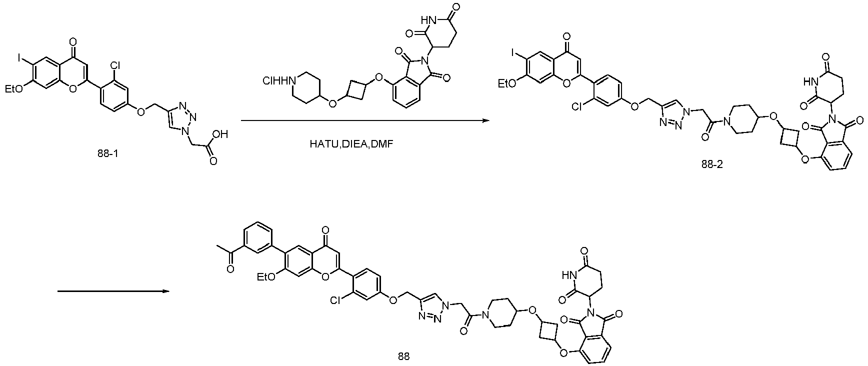

### (1) Synthesis of Compound 88-2

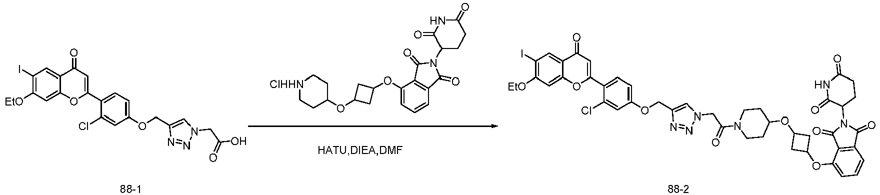

88-1 (60 mg, 1.1 eq.), DIEA (31 mg, 2 eq.) and HATU (68.6 mg, 1.5 eq.) were added into DMF (2mL) with stirring, continued the reaction for 4 h at room temperature, the completion of the reaction was monitored by LCMS. Saturated brine, ethyl acetate were added directly to the reaction flask, liquid separation, filtration, spin-drying were carried out, and passed through Prep-TLC (DCM:MeOH=20:1) to obtain 60 mg off-white solid. MS: [M+H]=991.11.

¹H NMR (400 MHz, DMSO-*d*₆) δ 8.24 (s, 1H), 8.15 (s, 1H), 7.78 (m, 1H), 7.67-7.55 (m, 2H), 7.15 (m,1H), 7.09 (d, 1H), 6.86-6.72 (m, 3H), 5.40 (m, 1H), 5.23 (s, 2H), 4.98 (s, 2H), 4.61 (m, H), 4.11 (m, H), 4.05 (m, H), 3.86 (m, 1H), 3.54-3.34 (m, 4H), 2.92-2.81 (m, 1H), 2.64-2.49 (m, 2H), 2.17-2.02 (m, 5H), 1.93-1.75 (m, 4H), 1.39 (t, *J* = 6.2 Hz, 3H).

### (2) Synthesis of Compound 88

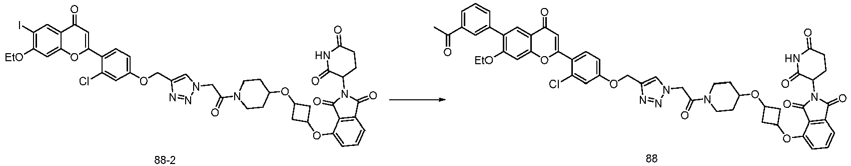

88-2 (60 mg, 1 eq.), (3-acetylphenyl) boronic acid (30.1 mg, 3.0 eq.), KF (21.8 mg, 6 eq.) and Pd(dppf)Cl₂ (4.5 mg, 0.1 eq.) were added into 2-MeTHF (3 ml) and water 1 (mL) with stirring, continued to react for 4 h at 70°C, the completion of the reaction was monitored by LCMS. The reaction flask was directly charged with anhydrous sodium sulfate, filtered, spin-dried, and passed through pre-TLC (DCM:MeOH=20:1) to obtain 30 mg off-white solid. MS: [M+H]=983.42.

¹H-NMR (400 MHz, DMSO-d₆) δ 8.24 (s, 1H), 8.10-8.02 (m, 2H), 7.78 (m, 1H), 7.63 (m, 1H), 7.64-7.55 (m, 3H), 7.50-7.41 (m, 1H), 7.15 (m, H), 7.12-7.07 (m, 2H), 6.82 (m,1H), 6.74 (s, 1H), 5.40 (m, H), 5.23 (s, 2H), 4.98 (s, 2H), 4.61 (m, H), 4.11 (m, H), 4.05 (m, 1H), 3.86 (m, H), 3.54-3.34 (m, 4H), 2.92-2.81 (m, 1H), 2.64-2.49 (m, 2H), 2.17- 2.02 (m, 5H), 1.93-1.75 (m, 4H), 1.32 (m, H).

### Synthesis example 76

### Synthesis of Compound 89

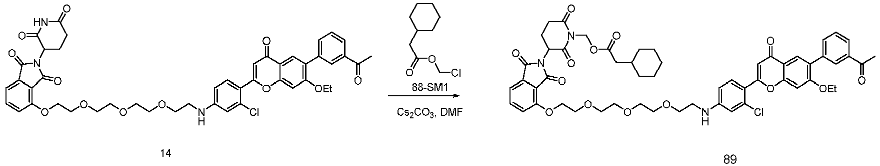

100 mg of compound 14 and 30 mg of SM1 were added into 10 ml of DMF, followed by 75 mg of Cs₂CO₃ , stirred after the addition, then stirred for 16h at 60°C under the protection of N₂. The disappearance of the raw materials and the generation of product were confirmed by liquid quality monitor, the system was concentrated and evaporated, a proper amount of DMF was used for dissolving the residue and loaded on a C18 reversed-phase column, reversed-phase was carried out, eluted with 10% ACN to obtain 43 mg of the target product (yield: 36.4%, Purity: 96.1%), MS: [M+H] 1020.51.

¹H NMR (400 MHz, DMSO-*d*₆) δ 8.10-8.02 (m, 2H), 7.78 (m, 1H), 7.67-7.57 (m, 2H), 7.60-7.50 (m, 2H), 7.50-7.41 (m, 1H), 7.13-7.04 (m, 2H), 6.84 (m,1H), 6.74 (s, 1H), 6.45 (m, 1H), 5.93 (s, 2H), 5.87 (m, H), 5.82 (m, 1H), 4.20-4.08 (m, 4H), 3.78 (m, 2H), 3.73-3.66 (m, 2H), 3.70-3.58 (m, 9H), 3.42 (m, 2H), 2.72-2.56 (m, 2H), 2.26 (m, 2H), 2.23-2.09 (m, 2H), 2.05-1.90 (m, 1H), 1.43-1.34 (m, 2H), 1.37 (s, 4H), 1.38-1.27 (m, 5H), 1.31-1.26 (m, 1H), 1.30-1.19 (m, 2H).

### Synthesis example 77

### Synthesis of Compound 90

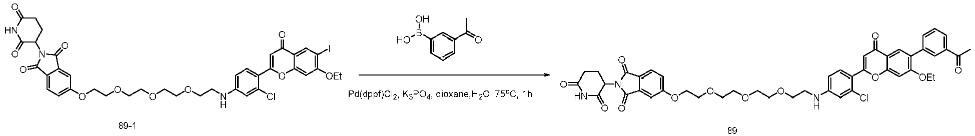

11-16(100 mg, 1 eq.), 3-acetylphenylboronic acid (56.3 mg, 3 eq.) and K₃PO₄ (72.8 mg, 3.0 eq.) were added into 1,4-dioxane (3 ml) and water (1 ml) with stirring, Pd(dppf)Cl₂ (8.3 mg, 0.1 eq.) was added under N₂ atmosphere, continued the reaction for 1 h at 75°C, the completion of the reaction was monitored by LCMS and cooled to room temperature. The reaction flask was directly charged with anhydrous sodium sulfate to remove water, filtered, spin-dried, and passed through pre-TLC (DCM:MeOH=20:1) to obtain 31.76 mg off-white solid (32% yield, 92% purity). MS: [M+H]=866.31.

¹H NMR (400 MHz, DMSO-*d*₆) δ 8.10 - 8.02 (m, 2H), 7.91 (m, 1H), 7.67-7.58 (m, 2H), 7.62-7.53 (m, 2H), 7.50 - 7.41 (m, 1H), 7.10 (s, 1H), 7.05 (m, 1H), 6.84 (m, H), 6.74 (s, 1H), 6.45 (m, H), 5.87 (m, 1H), 5.23 (m, 1H), 4.16-4.06 (m, 4H), 3.78 (m, 1H), 3.73 - 3.58 (m, 10H), 3.42 (m, 2H), 2.92 - 2.81 (m, 1H), 2.64 - 2.49 (m, 2H), 2.14 - 2.03 (m, 1H), 1.32 (m, 3H).

### Synthesis example 78

### Synthesis of Compound 91

### (1) Synthesis of Compound 91-2

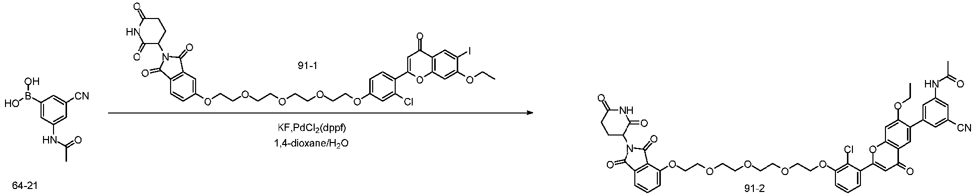

### (2) Synthesis of Compound 91

100 mg of 91-1 was added into 3 ml of 2-MTHF, followed by 164 mg of 64-21, 40 mg of KF, 25 mg of PdCl₂(dppf) and 1 ml of H₂O, replaced by N₂, raised to 80°C under the protection of N₂ and stirred. Monitored by LC-MS until no 91-1 remained. A proper amount of DCM was added, dried with Na₂SO₄ and filtered, the filtrate was concentrated to dryness at 40°C under reduced pressure, the residue was dissolved with a proper amount of DCM and separated by Pre-TLC (DCM:MeOH=20:1) to obtain 29.3 mg (yield: 28.25%, purity: 88.30%), MS: [M+H] 907.08.

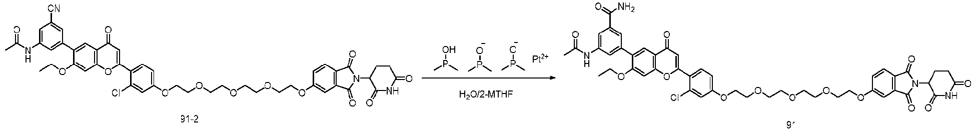

30 mg of 64-3 was added into 1 ml of 2-MTHF, 1 ml of H₂O, followed by 7 mg of PC, heated to 60°C and stirred, monitored by LC-MS until no 64-3 remained. A proper amount of DCM was added, dried with Na₂SO₄ and suction filtered, the filtrate was concentrated to dryness at 40°C under reduced pressure, the residue was dissolved with a proper amount of DCM and separated by Pre-TLC (DCM:MeOH=20:1, developed twice) to obtain 16 mg (yield: 49.18%, purity: 97.86%), MS: [M+H] 925.33.

¹H NMR (400 MHz, DMSO-*d*₆) δ 11.12 (s, 1H), 10.13 (s, 1H), 8.04 (d, *J* = 8.9 Hz, 3H), 7.98 (s, 1H), 7.82 (d, *J* = 8.3 Hz, 1H), 7.79 - 7.72 (m, 2H), 7.45 (d, *J* = 2.2 Hz, 1H), 7.39 - 7.33 (m, 3H), 7.27 (d, *J* = 2.4 Hz, 1H), 7.13 (dd, *J* = 8.7, 2.4 Hz, 1H), 6.56 (s, 1H), 5.11 (dd, *J* = 12.9, 5.3 Hz, 1H), 4.31 (s, 2H), 4.23 (q, *J* = 7.1 Hz, 4H), 3.78 (s, 4H), 3.62 - 3.55 (m, 8H), 2.86 (d, *J* = 13.2 Hz, 1H), 2.57 - 2.52 (m, 2H), 2.07 (s, 3H), 2.01 (s, 1H), 1.34 (t, *J* = 6.9 Hz, 3H).

### Pharmacological activity test experiments

The biological activity of the compounds of the invention is determined by the following method.

Reagent: complete DMEM medium, Gibco products. Fetal bovine serum, manufactured by ThermoFisher. Trypsin, manufactured by ThermoFisher. The SHP-2 antibody is produced by CST.

Cell line, MV 411, the blast cells of patients with bi-phenotypic B-myelomonocytic leukemia, was established by Rovera.

Measuring method: took the cells growing in the logarithmic phase, collected and suspended the cells in DMEM containing 10% fetal calf serum, gently blowed and beated the cells into single cell suspension by using a pipette, and counted the living cells under a microscope. 9 ml of the cultured cell suspension is inoculated into a 10 cm culture dish, the final concentration of the cells is 2×10⁵/ml, after pre-culturing for 24 hours under the culture condition of 37°C and 5% CO₂, DMSO solutions (the final concentration of the DMSO does not exceed 0.5%) of the compounds with different concentrations were added, and the negative control group was added with the same volume of DMSO. Then cultured for 24 hours. All cells were collected and washed with PBS, lysed with RIPA lysate and collected into a 1.7 mltube. 95% metal bath for 5 minutes to fully denature the protein. 100Vconstant voltage electrophoresis after Western condensation and loading, stopped when bromophenol blue band reached the end point. The cellulose acetate membrane is transferred by a wet method, 45 minutes ata constant flow of 300-500 mA, 5% BSA is used for blocking for 1-2 hours at room temperature. Primary antibody of 1:500-1:8000 were used for incubation at 25°C for 40 minutes and then washed three times. Secondary antibody was used for incubation for 15-40 min at room temperature. The proteins were detected using ultrasensitive ECL luminophores such as Enlight, using an X-Ray film automatic developing machine. The concentration of the median degradation was calculated by exposure gray scale. As shown in Table 2, the compounds of the present invention have SHP2 inhibitory activity.

**Table. 2**

| No. | **DC50(nM)** |
|---|---|
| Compound 11 | 50 |
| Compound 12 | 920 |
| Compound 13 | 50 |
| Compound 14 | 25 |
| Compound 15 | 250 |
| Compound 16 | 250 |
| Compound 17 | 250 |
| Compound 18 | >1000 |
| Compound 26 | 3500 |
| Compound 27 | 2 |
| Compound 28 | 110 |
| Compound 29 | 15 |
| Compound 30 | 2000 |
| Compound 31 | 300 |
| Compound 32 | 850 |
| Compound 33 | 3500 |
| Compound 34 | 42 |
| Compound 35 | 25 |
| Compound 36 | 20 |
| Compound 40 | 750 |
| Compound 41 | 1000 |
| Compound 42 | 750 |
| Compound 43 | >250 |
| Compound 44 | 750 |
| Compound 45 | >250 |
| Compound 46 | 6 |
| Compound 50 | >750 |
| Compound 51 | >250 |
| Compound 52 | >250 |
| Compound 53 | 5 |
| Compound 54 | >250 |
| Compound 55 | >1000 |
| Compound 56 | >1000 |
| Compound 57 | >1000 |
| Compound 58 | 500 |
| Compound 59 | >750 |
| Compound 60 | >250 |
| Compound 61 | >250 |
| Compound 62 | >250 |
| Compound 63 | >250 |
| Compound 64 | >250 |
| Compound 65 | >250 |
| Compound 66 | >250 |
| Compound 67 | >250 |
| Compound 68 | >250 |
| Compound 69 | >250 |
| Compound 70 | >250 |
| Compound 71 | >250 |
| Compound 72 | >250 |
| Compound 73 | >250 |
| Compound 74 | 2 |
| Compound 75 | 24.57 |
| Compound 76 | >250 |
| Compound 77 | >250 |
| Compound 78 | >250 |
| Compound 79 | 500 |
| Compound 80 | >500 |
| Compound 81 | >500 |
| Compound 86 | 50 |
| Compound 87 | 45 |
| Compound 88 | 55 |
| Compound 89 | 75 |

The present invention is illustrated in detail by the examples given above, but the present invention is not limited to the details given above, which does not mean that the present invention must rely on the above detailed methods to be implemented. It should be understood by those skilled in the art that any modifications of the present invention, equivalent substitutions of the raw materials of the product of the present invention, and the addition of auxiliary components, selection of specific modes, etc., are within the scope and disclosure of the present invention.

## Claims

1. A compound of formula 1 or a pharmaceutically acceptable salt, ester, optical isomer, stereoisomer, polymorph, solvate, N-oxide, isotopically labeled compound, metabolite, chelate, complex, clathrate, or prodrug thereof, 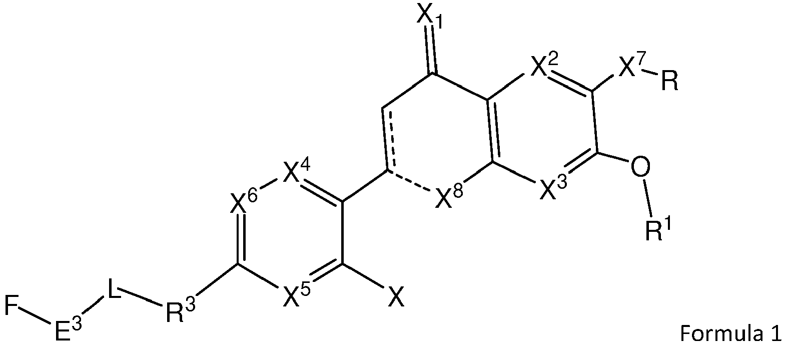 In formula 1,
"- - -" indicates the chemical bond is a double or single bond; "- - -" indicates that the chemical bond is a single bond or absent,
X is halogen, C1-6 alkyl, C2-6 alkenyl, C2-6 alkynyl, saturated or partially unsaturated C3-10 cyclic hydrocarbyl, preferably halogen, methyl, ethyl, isopropyl, cyclopropyl, more preferably halogen,
X¹ is NR⁴, O or S, preferably O or S, more preferably O,
X⁷ is a chemical bond or a divalent group formed by combining one or more selected from the group consisting of C1-6 alkylene, C2-6 alkenylene, C2-6 alkynylene, saturated or partially unsaturated C3-10 cyclic hydrocarbylene, -O-, -CO-, -C(= O)O-, -CONH-, -NHCO-, - NHCONH-, -NH-, -S-, -sulfinyl, and sulfonyl, preferably a chemical bond or a divalent group formed by combining one or more selected from the group consisting of C1-6 alkylene, -O-, - CO-, -CONH-, -NHCO-, -NHCONH-, and NHCONH-,
X², X³, X⁴, X⁵and X⁶ are each independently CR⁴ or N, preferably CR⁶,
When the chemical bond represented by "---" is a single bond, X⁸ is NR², O or S, preferably O or S, more preferably O,
When the chemical bond represented by "---" is absent, X⁸ is H, N(R²)₂, OR² or SR², preferably OR² or SR², more preferably OR²,
R¹ is H, C1-6 alkyl, C2-6 alkenyl, C2-6 alkynyl, saturated or partially unsaturated C3-10 cyclic hydrocarbyl, preferably C1-6 alkyl, more preferably one selected from methyl, ethyl, isopropyl and cyclopropyl,
R² is H, C1-6 alkyl, C2-6 alkenyl, C2-6 alkynyl, saturated or partially unsaturated C3-10 cyclic hydrocarbyl, preferably H, C1-6 alkyl, more preferably one selected from H, methyl and ethyl, particularly preferably H,
R³ is CR⁴R⁵ , NR⁴, 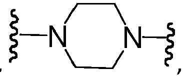 O or S, preferably NR⁴, 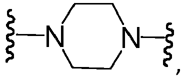 O or S, more preferably NR⁴, 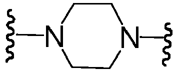 or O,
R ⁴ and R⁵ are each independently H, C1-6 alkyl, C2-6 alkenyl, C2-6 alkynyl, saturated or partially unsaturated C3-10 cyclic hydrocarbyl, saturated or partially unsaturated 3-10 membered heterocyclyl, C6-10 aryl, 5-14 membered heteroaryl, or C6-12 aralkyl,
R⁶ is H, halogen, C1-6 alkyl, C2-6 alkenyl, C2-6 alkynyl, saturated or partially unsaturated C3-10 cyclic hydrocarbyl, saturated or partially unsaturated 3-10 membered heterocyclyl, C6-10 aryl, 5-14 membered heteroaryl, or C6-12 aralkyl,
R is H, halogen, NH₂, OH, SH, amino, substituted or unsubstituted C2-10 aliphatic hydrocarbon group, substituted or unsubstituted saturated or partially unsaturated 3-10 membered heterocyclyl, substituted or unsubstituted C6-10 aryl, or substituted or unsubstituted 5-14 membered heteroaryl; R is preferably halogen, OH, SH, haloalkyl, amino, saturated C3-10 cyclic hydrocarbyl, saturated or partially unsaturated 3-10 membered heterocyclyl, C1-6 alkyl substituted amino, 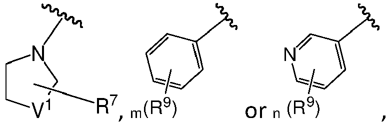
Wherein V¹ is one selected from C(R⁸)₂, C(R⁸)₂ -C(R⁸)₂, CR⁸=CR⁸, C=O, C(=O)C(R⁸)₂, C(R⁸)₂C(=O), C(=O)O, OC(=O), C(= O)NR, N=CR⁸, CR⁸=N, NR⁸-C(R⁸)₂ or C(R⁸)₂-NR⁸;
R⁷ is one selected from H, halogen, cyano, nitro, hydroxymethyl, hydroxyl, amide, C1-C6 alkyl, C1-C6 alkoxy, C1-C6 alkoxy-C1-C6 alkylene, or 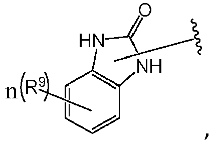
R⁹ are each independently selected from the group consisting of halogen, hydroxyl, oxo, amino, C1-6 alkyl-substituted amino, cyano, nitro, -Si(R⁸)₃, C1-6 alkyl, C1-6 alkoxy, saturated or partially unsaturated C3-6 cyclic hydrocarbyl, saturated or partially unsaturated 3-10 membered heterocyclyl, C6-10 aryl, 5-14 membered heteroaryl, C6-12 aralkyl, -C(=O)R⁸, -OC(=O)R⁸, -C(=O)OR⁸, -OR⁸, -SR⁸, -S(=O)R⁸, -S(=O)₂R⁸, -S(=O)₂N(R⁸)₂, -N(R⁸)₂, -C(=O)N(R⁸),-NR⁸-C(=O)R⁸, -NR⁸-C(=O)OR⁸, -NR⁸-S(=O)₂-R⁸, -NR⁸-C(=O)-N(R⁸)₂, -C1-6 alkylene-N(R⁸)₂, -C1-6 alkylene-OR⁸, -C1-6 alkenylene-OR⁸ and-O-C1-6 alkylene-N(R⁸)₂; m is an integer from 0 to 5, n is an integer from 0 to 4, when m is not 0, a plurality of R⁹ can be connected with each other to form a ring structure, and when n is not 0, a plurality of R⁹ can be connected with each other to form a ring structure;
R⁸ is H, C1-C6 alkyl, saturated or partially unsaturated C3-6 cyclic hydrocarbyl, saturated or partially unsaturated 3-10 membered heterocyclyl or C6-10 aryl, the expression of the ring structure with "-" represents the connecting site at any position on the ring structure that can form a bond;
L is a linking group which represents a linear or branched C3-C29 alkylene chain, wherein the linear or branched C3-C29 alkylene chain is optionally interrupted one or more times by one or more divalent radicals selected from -O-, -CO-, -C(= O)O-, -CONH-, -NHCO-, - NHCONH-, -NH-, -NR⁸-, -C(R⁸)₂-, -S-, sulfinyl, sulfonyl, sulfinyloxy, sulfonyloxy, - aminosulfonylamino-, alkynylene, alkenylene, cycloalkylene, 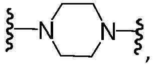 or any combination thereof,
E³ is the following group, 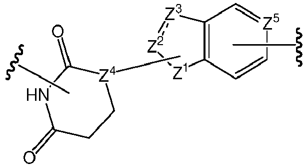
------ represents a single or double bond, preferably a single bond;
Z¹ is O, S, NH, CH₂ or C=O, preferably CH₂ or C=O;
When - is a double bond, Z² is N or CH, Z³ is N or CH;
When ------ is a single bond, Z² is O, S, NH, CH₂ or C=O, preferably NH; Z³ is O, S, NH, CH₂ or C=O, preferably CH₂ or C=O;
Z⁴ is N or CH, Z⁴ is connected with any connectable position of Z¹, Z² and Z³ ;
Z⁵ is N or CH, preferably CH;
E³ is further preferably 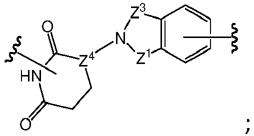
F is H or 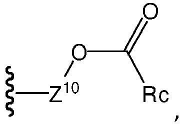
Z¹⁰ is selected from a chemical bond, C1-6 alkylene, C2-6 alkenylene, C2-6 alkynylene, saturated or partially unsaturated C3-10 cyclic hydrocarbylene, preferably a chemical bond, C1-6 alkylene;
Rc is halogen, cyano, nitro, hydroxyl, acylamino, C1-C6 alkyl, C1-C6 alkoxy, substituted C1-C6 alkyl, saturated or partially unsaturated C3-10 cyclic hydrocarbyl, saturated or partially unsaturated 3-10 membered heterocyclyl, C6-10 aryl, 5-14 membered heteroaryl or C6-12 aralkyl, preferably C1-C6 alkyl, saturated or partially unsaturated C3-10 cyclic hydrocarbyl, C6-10 aryl, C6-12 aralkyl,
In the above expression, 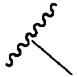 denotes the position of the linkage, and the expression of the ring straucture with "-" represents the connecting site at any position on the ring structure that can form a bond.

2. The compound of claim 1, or a pharmaceutically acceptable salt, ester, optical isomer, stereoisomer, polymorph, solvate, N-oxide, isotopically labeled compound, metabolite, chelate, complex, clathrate, or prodrug thereof, having the structure of formula 2, 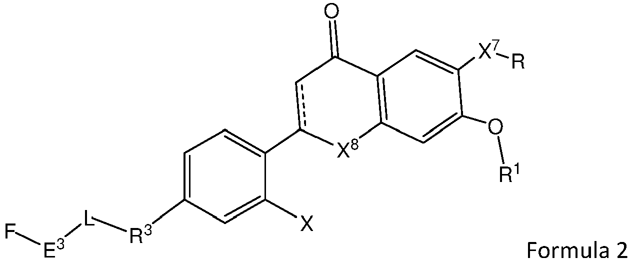 Wherein "- - -" represents a double bond or a single bond,
In formula 2, X is halogen, C1-6 alkyl, saturated or partially unsaturated C3-10 cyclic hydrocarbyl, preferably halogen,
In formula 2, R¹ is H, C1-6 alkyl, C2-6 alkenyl, C2-6 alkynyl, saturated or partially unsaturated C3-10 cyclic hydrocarbyl, preferably C1-6 alkyl, further preferably ethyl,
In formula 2, X⁸ is O or S, preferably O,
In formula 2, R³ is NR⁴, 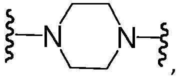 O or S, preferably NR⁴, 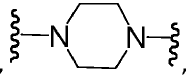 O or S, further preferably NR⁴, 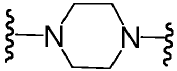 or O, in formula 2, R⁴ are each independently H, C1-6 alkyl, R⁴ is preferably H,
In formula 2, R, L, E³ and F have the same meanings as in claim 1, and in the above expression, 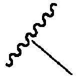 represents a position of linkage.

3. The compound of claim 1, or a pharmaceutically acceptable salt, ester, optical isomer, stereoisomer, polymorph, solvate, N-oxide, isotopically labeled compound, metabolite, chelate, complex, clathrate, or prodrug thereof, having the structure of formula 3, 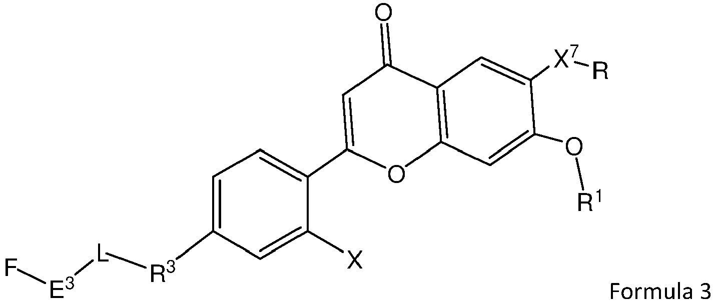
In formula 3, X is halogen, C1-6 alkyl, saturated or partially unsaturated C3-10 cyclic hydrocarbyl, preferably halogen, R¹ is H, C1-6 alkyl, C2-6 alkenyl, C2-6 alkynyl, saturated or partially unsaturated C3-10 cyclic hydrocarbyl, preferably C1-6 alkyl, further preferably ethyl,
In formula 3, R³ is CR⁴R⁵, NH, 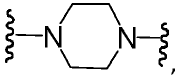 O or S, preferably NR⁴, O or S, R⁴ and R⁵ are each independently H, C1-6 alkyl, C2-6 alkenyl, C2-6 alkynyl, saturated or partially unsaturated C3-10 cyclic hydrocarbyl, saturated or partially unsaturated 3-10 membered heterocyclyl, C6-10 aryl, 5-14 membered heteroaryl or C6-12 aralkyl, R³ is further preferably NH or O,
In the formula 3, R, L, E³ and F have the same meanings as in claim 1, and in the above expression, 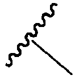 represents a position of linkage.

4. A compound of any one of claims 1 to 3, or a pharmaceutically acceptable salt, ester, optical isomer, stereoisomer, polymorph, solvate, N-oxide, isotopically-labeled compound, metabolite, chelate, complex, clathrate, or prodrug thereof, wherein,
E³ is one of the following groups, 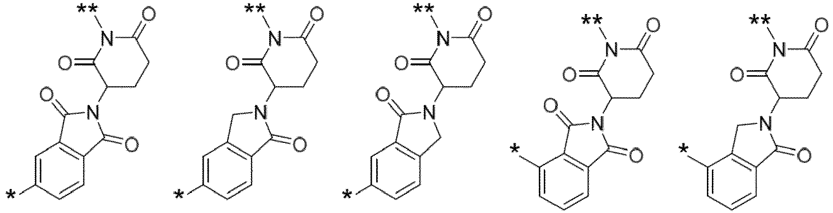 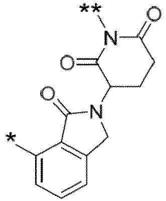
The above groups are linked to L via one of the two positions labeled with *-or**- and the other position is linked to F.
F is H or one of the following groups,

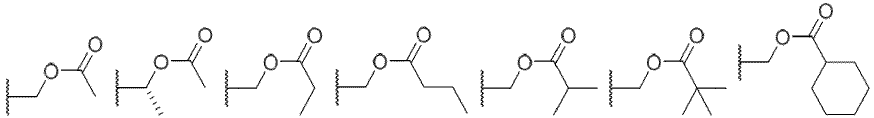

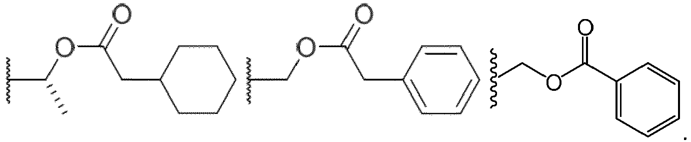

5. The compound of any one of claims 1-4, or a pharmaceutically acceptable salt, ester, optical isomer, stereoisomer, polymorph, solvate, N-oxide, isotopically-labeled compound, metabolite, chelate, complex, clathrate, or prodrug thereof, wherein,
X⁷ is a chemical bond or a divalent group formed by combining one or more selected from the group consisting of methylene, ethylene, propylene, -O-, -CONH, -and NHCO-, preferably a chemical bond, methylene, ethylene, methyleneoxy, ethylene-CONH-, methylene-CONH-, 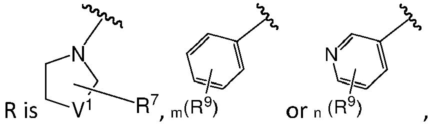 wherein V¹ is one selected from CH₂, CH₂-CH₂, CH=CH, NH-CH₂ or CH₂-NH; R⁷ is one selected from H, C1-C6 alkyl, C1-C6 alkoxy substituted C1-C6 alkylene or 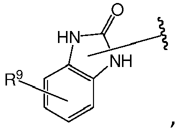 and R⁹ is as defined in claim 1; 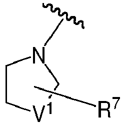 is preferably one selected from 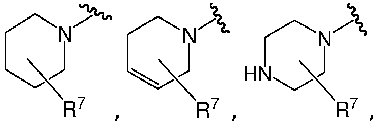 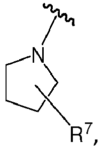 wherein R⁷ is further preferably one selected from H, methyl, ethyl, CONH₂, 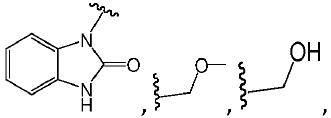
R⁹ are each independently selected from halogen, amino, C1-6 alkyl-substituted amino, cyano, C1-6 alkyl, C1-6 alkoxy, saturated or partially unsaturated C3-6 cyclic hydrocarbyl, saturated or partially unsaturated 3-10 membered heterocyclyl, C6-10 aryl, C6-12 aralkyl, -C(=O)R⁸, -OC(=O)R⁸, -C(=O)OR⁸, -OR⁸, -SR⁸, -S(=O)R⁸, -S(=O)₂R⁸, -S(= O)₂N(R⁸)₂, -N(R⁸)₂, -C(=O)N(R⁸)₂, -NR⁸-C(=O)R⁸, -NR⁸-C(=O)OR⁸, -NR⁸-S(=O)₂-R⁸, -NR⁸-C(=O)-N(R⁸)₂; m is an integer from 0 to 5, n is an integer from 0 to 4, when m is not 0, a plurality of R⁹ can be connected with each other to form a ring structure, and when n is not 0, a plurality of R⁹ can be connected with each other to form a ring structure;
R⁸ is H, C1-C6 alkyl, saturated or partially unsaturated C3-6 cyclic hydrocarbyl, saturated or partially unsaturated 3-10 membered heterocyclyl, or C6-10 aryl, and the expression of the ring straucture with "-" represents the connecting site at any position on the ring structure that can form a bond, 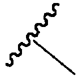 denotes the position of the linkage.

6. The compound of claim 1, or a pharmaceutically acceptable salt, ester, optical isomer, stereoisomer, polymorph, solvate, N-oxide, isotopically labeled compound, metabolite, chelate, complex, clathrate, or prodrug thereof, wherein R is one of the following groups, 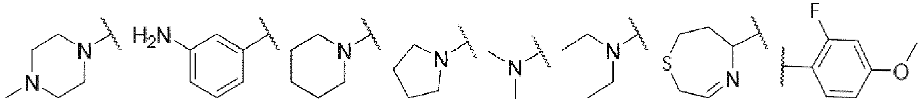 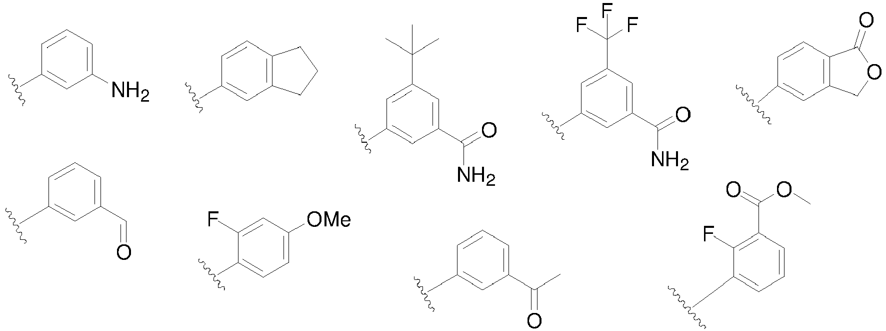 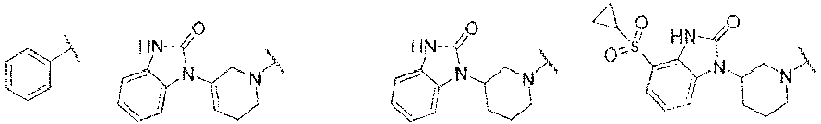 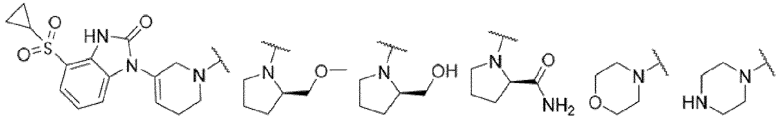 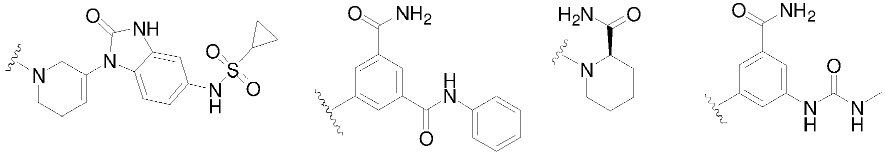 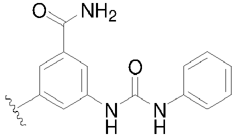 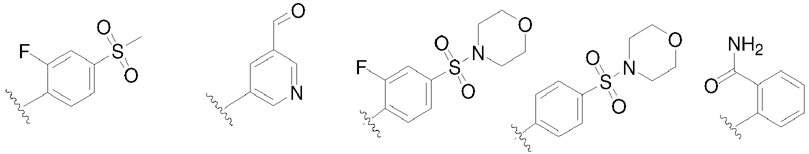 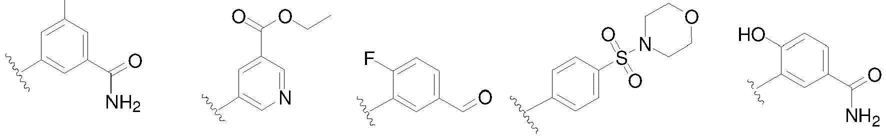 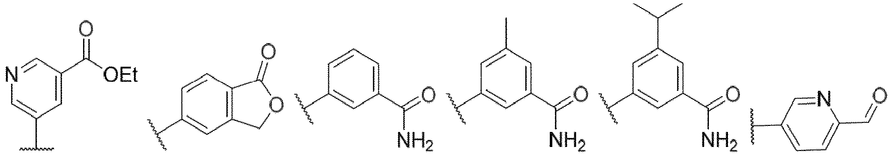 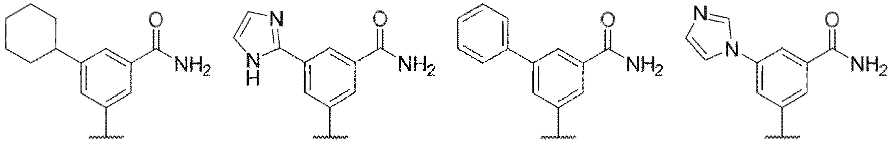 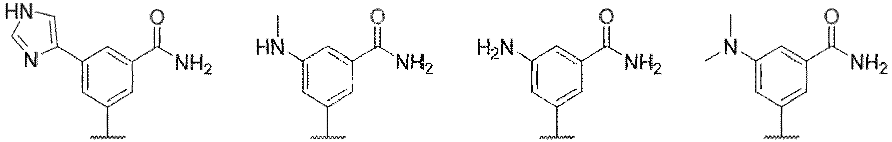 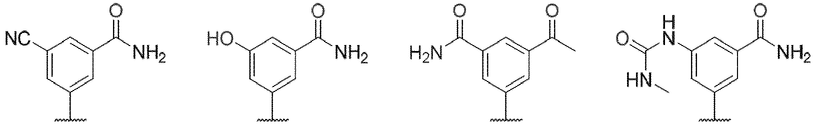 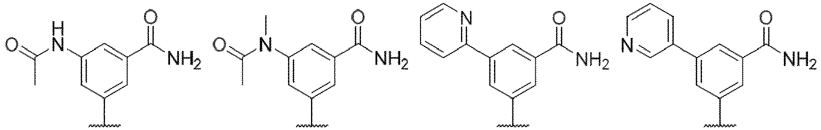 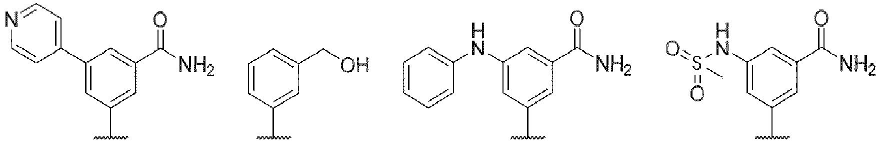 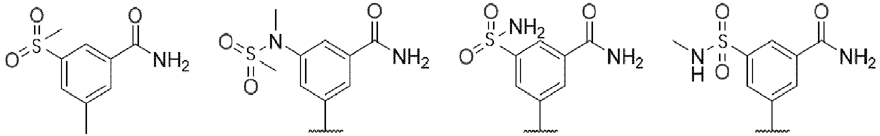 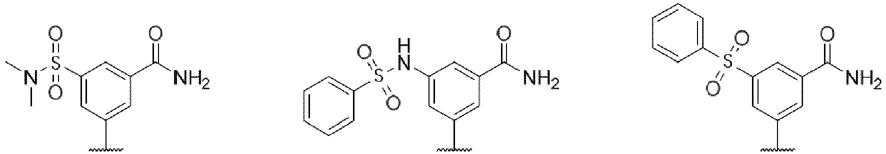 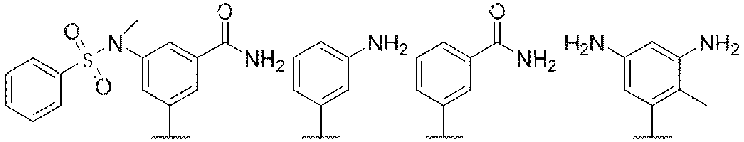 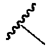 denotes the position of the linkage.

7. The compound of any one of claims 1-6, or a pharmaceutically acceptable salt, ester, optical isomer, stereoisomer, polymorph, solvate, N-oxide, isotopically-labeled compound, metabolite, chelate, complex, clathrate, or prodrug thereof, wherein L is a divalent group represented by formula 5, 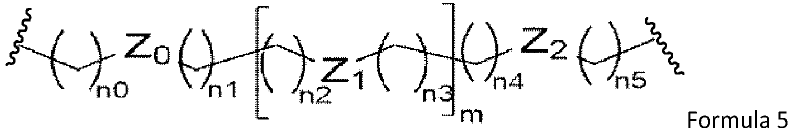
n₀ is 0 or 1,
m is an integer from 1 to 5, preferably 2 or 3; n₁ is an integer from 0 to 3, preferably 1; n₂ is an integer from 0 to 3, preferably 1; n₃ is an integer from 0 to 3, preferably 1; n₄ is an integer from 0 to 3, preferably 1; n₅ is an integer from 0 to 3, preferably 1;
Z₀ is -CH₂-, -NH-, -O-, -S-, 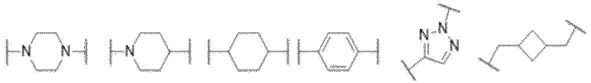 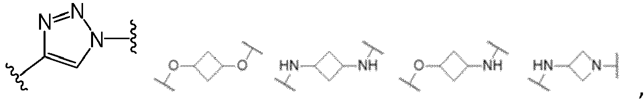 -CO- or -C(=O)O-;
Z₁ is -CH₂-, -NH-, -O-, -S-, 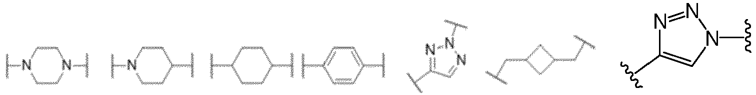 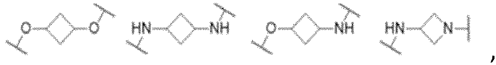 -CO- or -C(=O)O-;
Z₂ is -CH₂-, -NH-, -O-, -S-, 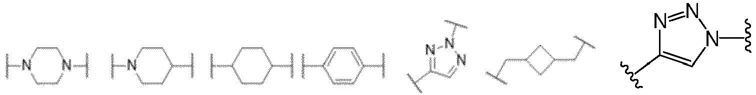 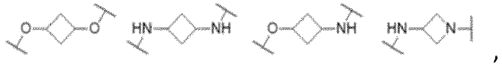 -CO- or -C(= O)O-; 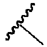 denotes the position of the linkage, and the connecting directions of the two ends are arbitrarily changed.

8. The compound of claim 7, or a pharmaceutically acceptable salt, ester, optical isomer, stereoisomer, polymorph, solvate, N-oxide, isotopically labeled compound, metabolite, chelate, complex, clathrate, or prodrug thereof, wherein L is one of the following divalent groups, 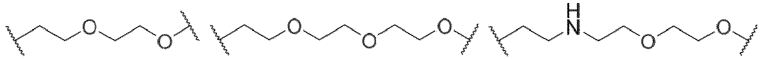 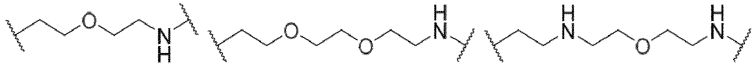 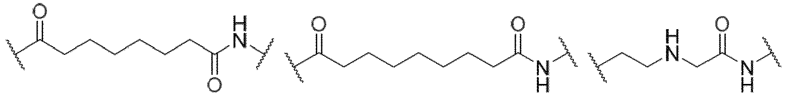 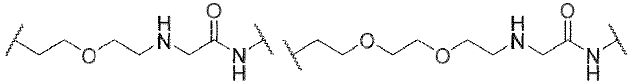 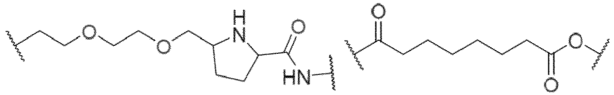 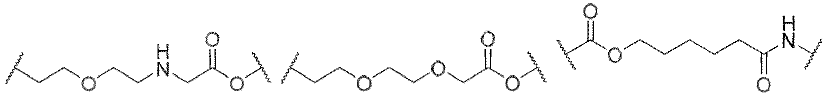 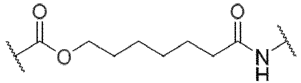 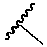 denotes the position of the linkage, and the connecting directions of the two ends are arbitrarily changed.

9. The compound of claim 1, or a pharmaceutically acceptable salt, ester, optical isomer, stereoisomer, polymorph, solvate, N-oxide, isotopically labeled compound, metabolite, chelate, complex, clathrate, or prodrug thereof, wherein the compound has the following structures.

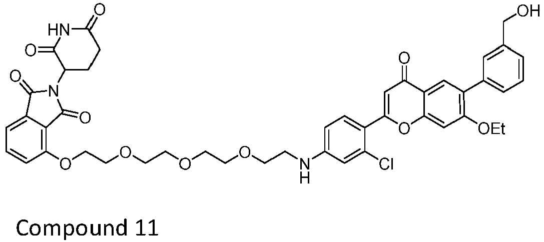

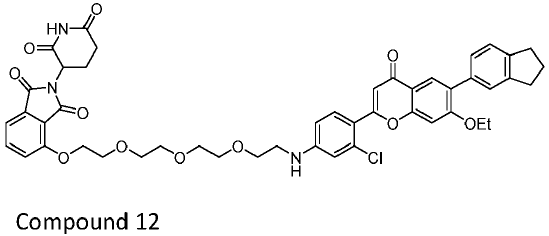

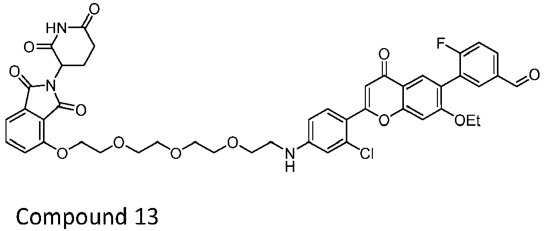

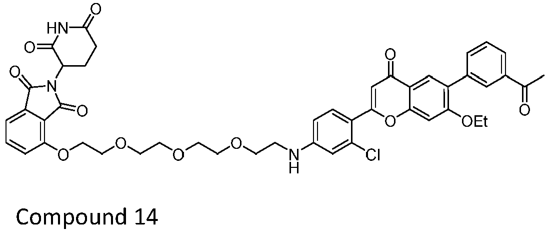

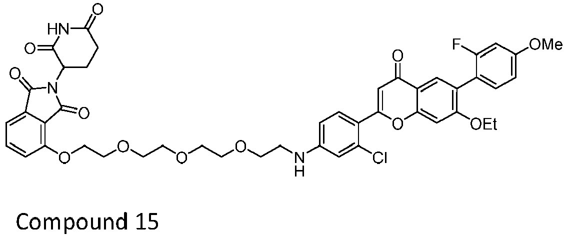

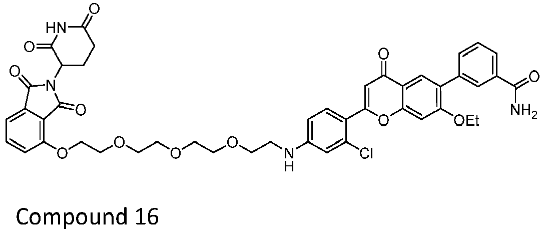

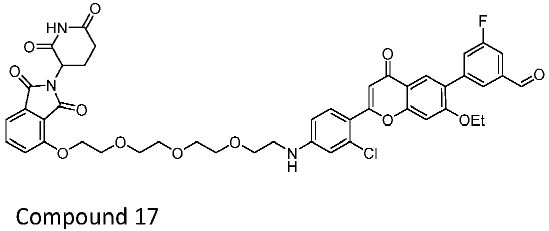

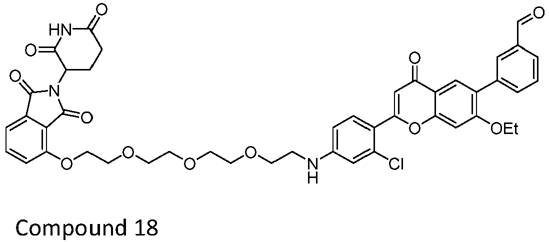

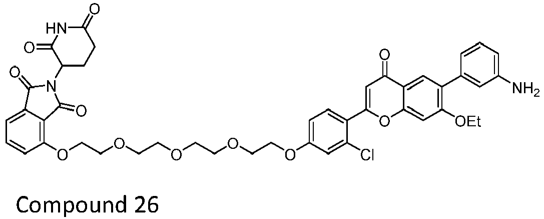

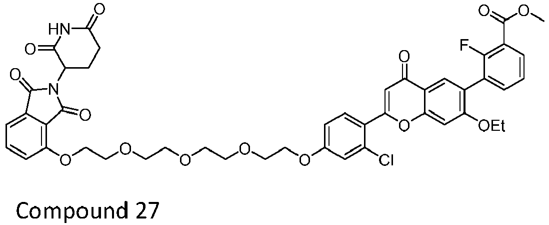

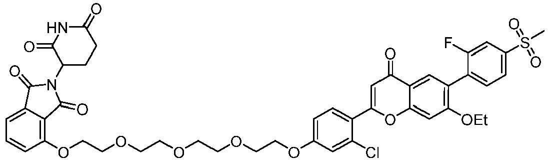

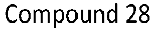

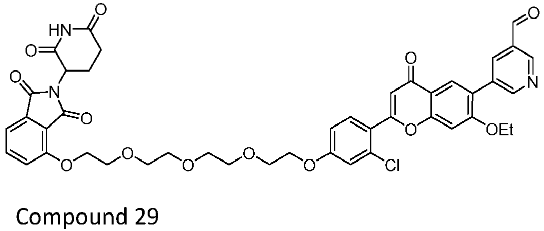

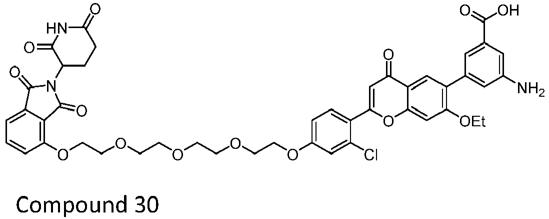

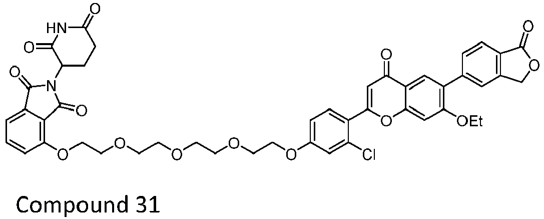

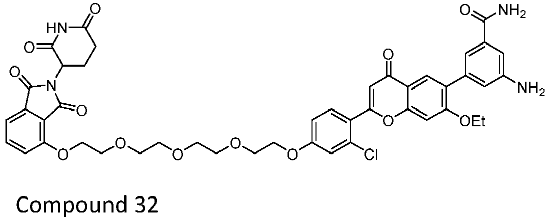

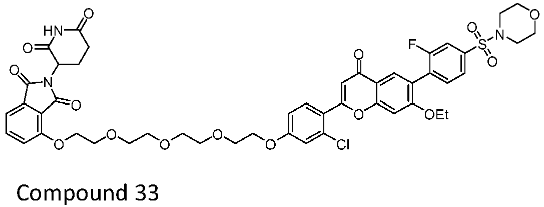

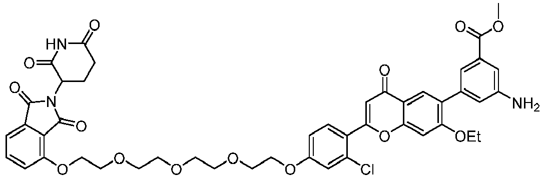

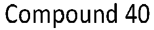

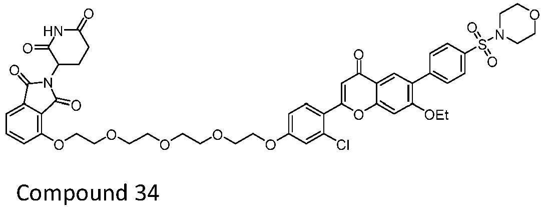

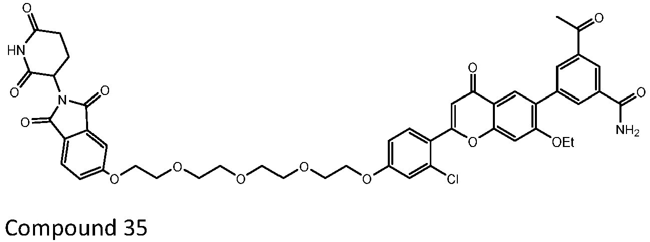

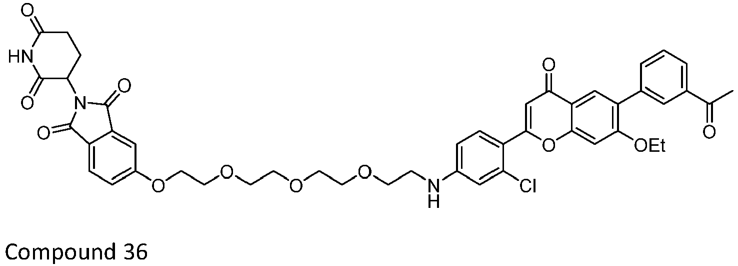

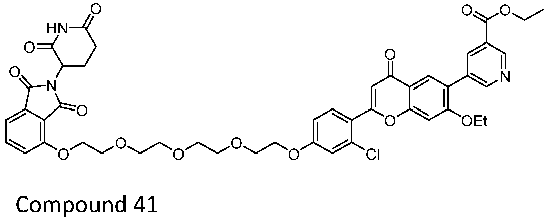

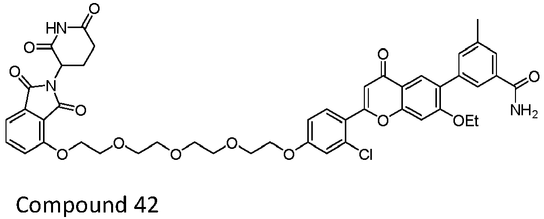

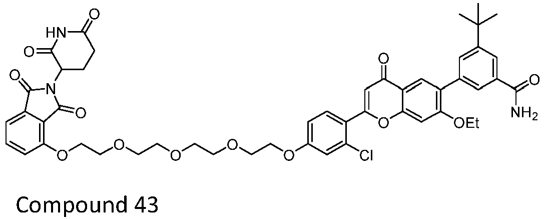

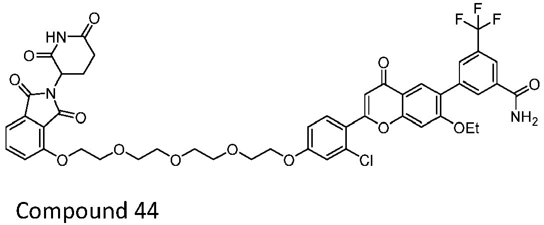

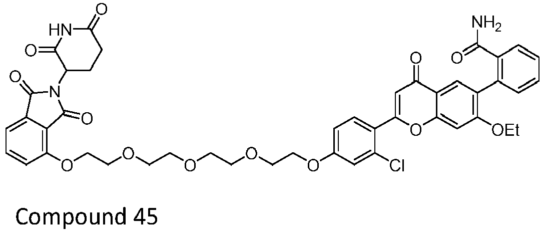

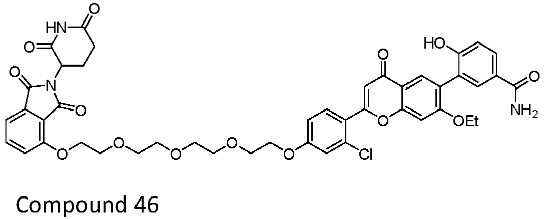

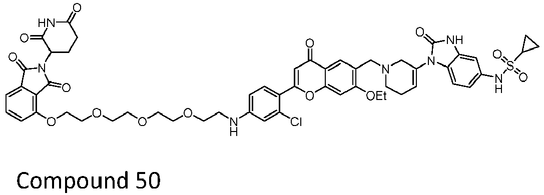

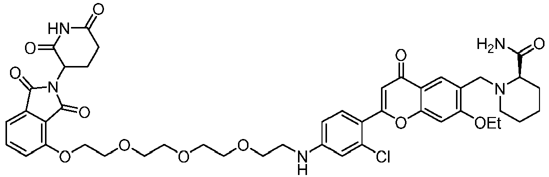

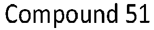

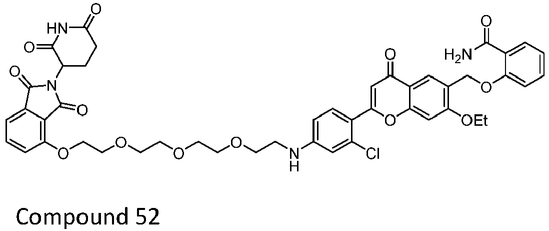

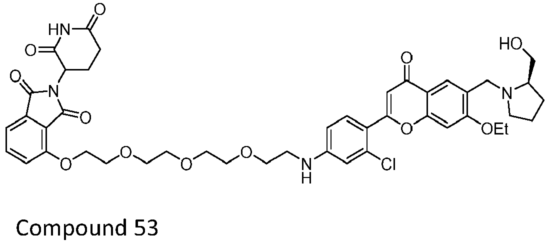

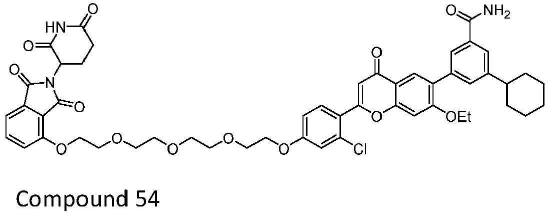

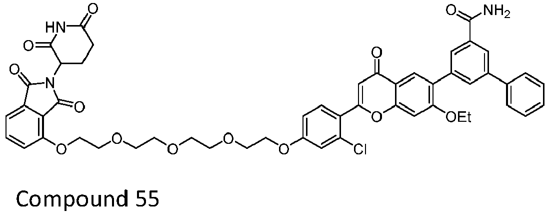

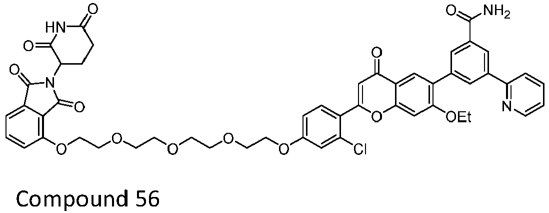

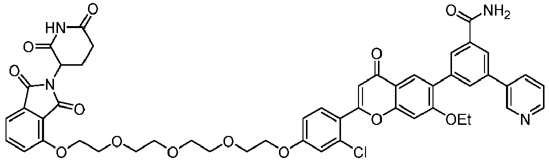

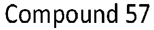

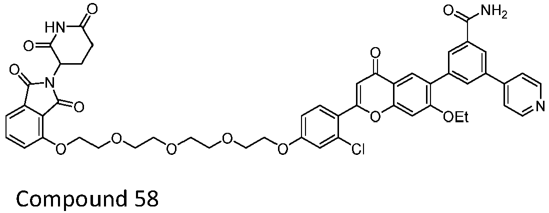

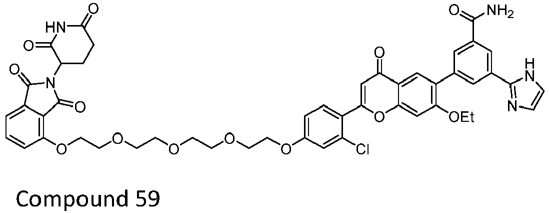

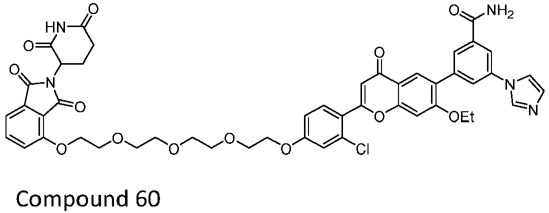

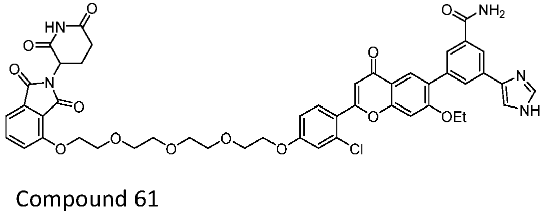

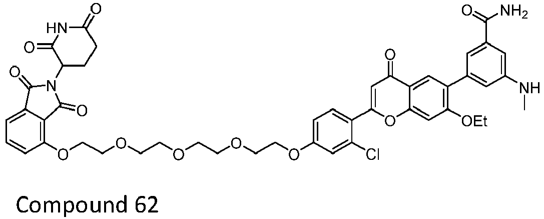

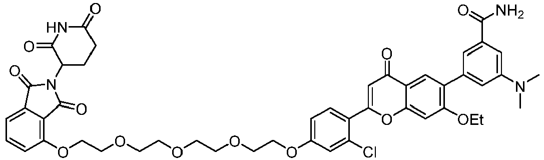

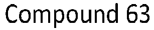

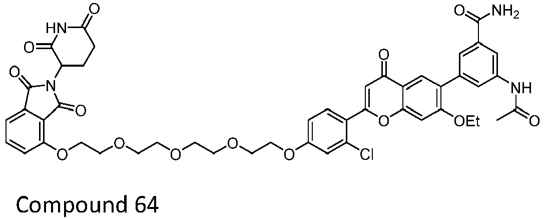

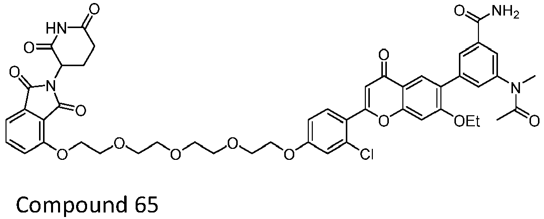

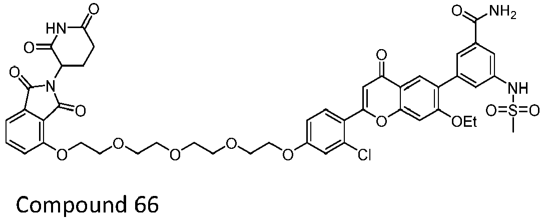

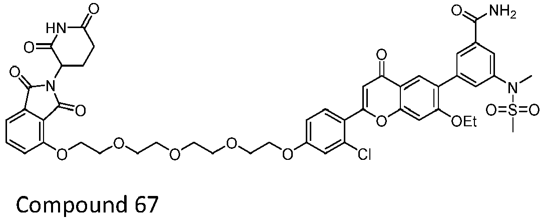

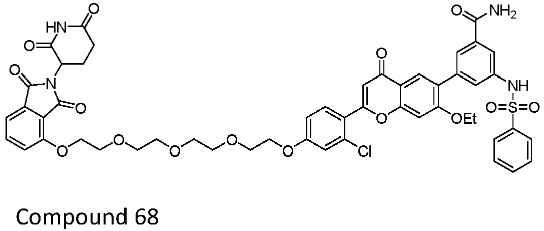

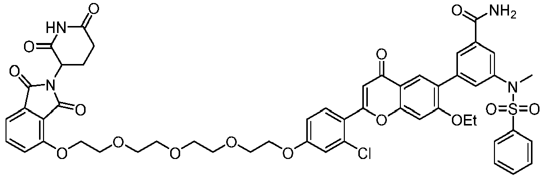

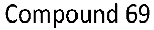

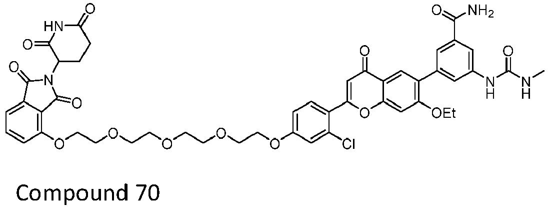

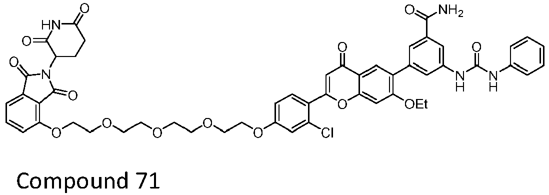

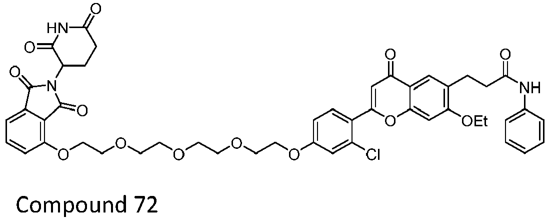

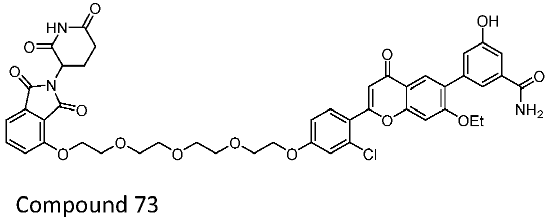

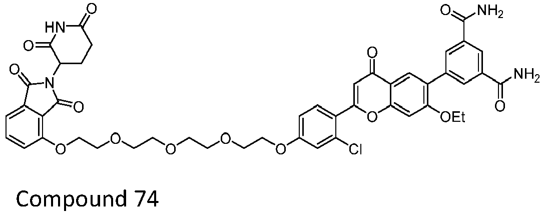

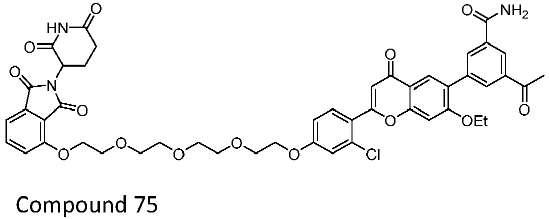

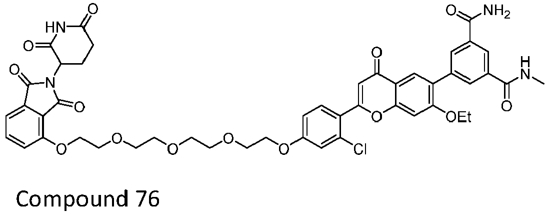

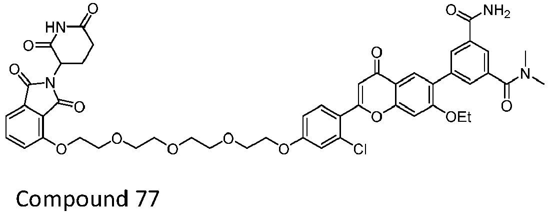

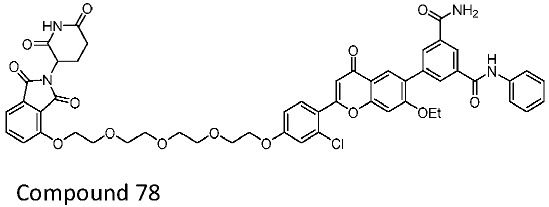

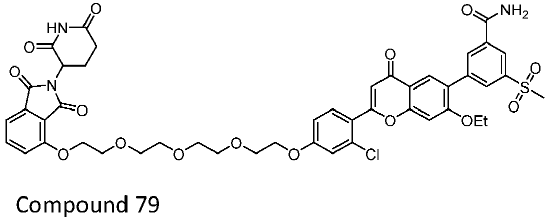

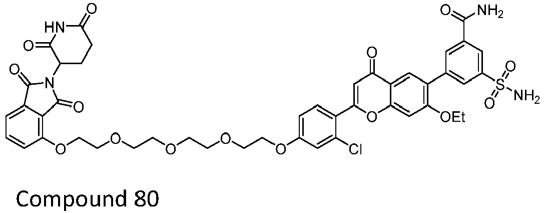

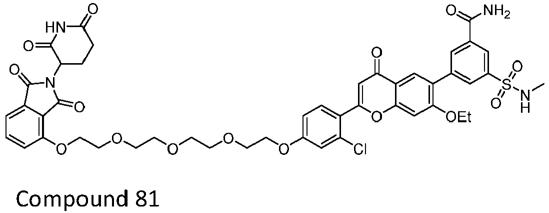

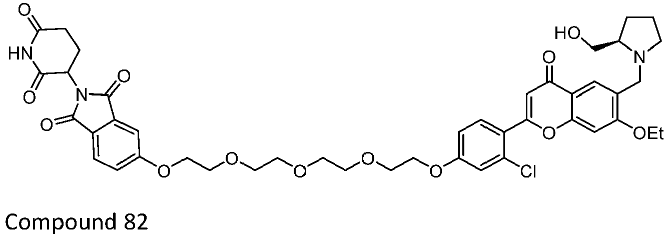

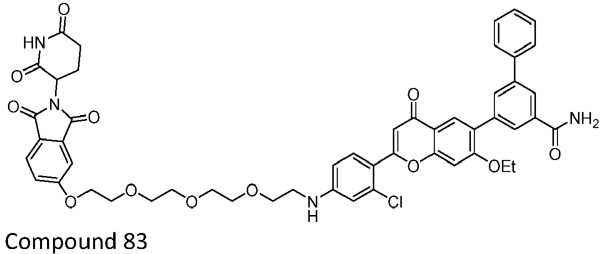

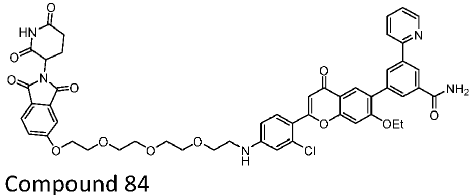

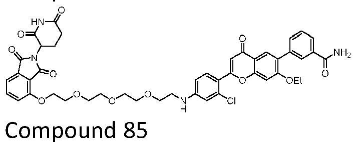

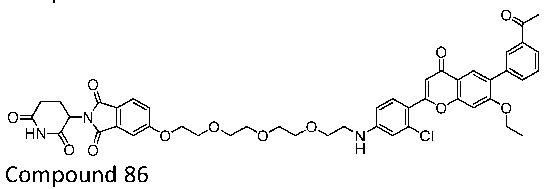

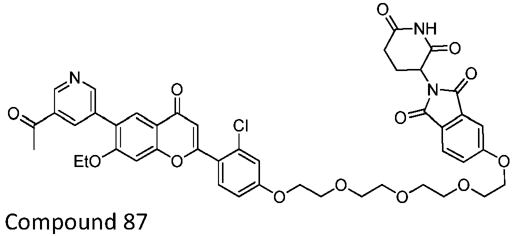

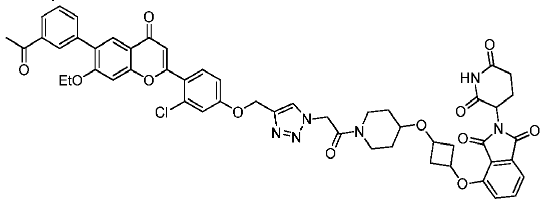

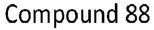

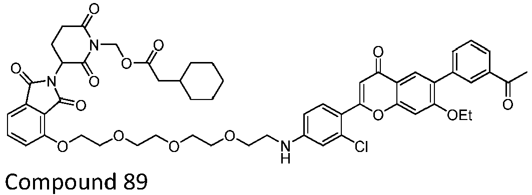

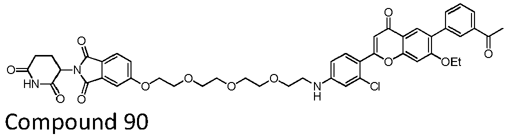

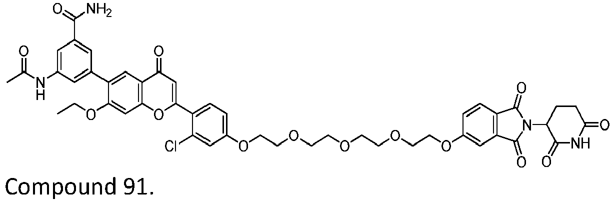

10. A pharmaceutical composition comprising a prophylactically or therapeutically effective amount of the compound according to any one of claims 1 to 9, or a pharmaceutically acceptable salt, ester, optical isomer, stereoisomer, polymorph, solvate, N-oxide, isotopically labeled compound, metabolite, chelate, complex, clathrate, or prodrug thereof, and a pharmaceutically acceptable carrier, preferably in a solid, semi-solid, liquid, or gaseous formulation.

11. Use of the compound of any one of claims 1-9, or a pharmaceutically acceptable salt, ester, optical isomer, stereoisomer, polymorph, solvate, N-oxide, isotopically-labeled compound, metabolite, chelate, complex, clathrate, or prodrug thereof, or the pharmaceutical composition of claim 10 in the manufacture of a medicament for the treatment of a SHP2 phosphatase modulated disease.

12. The use according to claim 11, wherein the disease is a tumor, such as a solid tumor, a hematological tumor, a malignancy, a refractory tumor, a primary or metastatic recurrent tumor, and the like.

13. The use of according to claim 11, wherein the disease is selected from primary or metastatic recurrent NSCLC, squamous carcinoma of the lung, adenocarcinoma of the lung, squamous carcinoma of the head and neck, gastric cancer, colorectal cancer, pancreatic cancer, and the like; also included are breast cancer, esophageal cancer, lung cancer, colon cancer, brain cancer, neuroblastoma, neuroblastoma, melanoma, squamous carcinoma of the head and neck, anaplastic large cell lymphoma and glioblastoma, hematological malignancies of cachexia including juvenile myelomonocytic leukemia, acute myelogenous leukemia, noonan syndrome, leopard syndrome, type II diabetes, and obesity.

14. A method of treatment of a SHP2 phosphatase modulated disease, comprising administering to a human in need of such treatment an effective amount of the compound of any one of claims 1 to 9 or a pharmaceutically acceptable salt, ester, optical isomer, stereoisomer, polymorph, solvate, N-oxide, isotopically labeled compound, metabolite, chelate, complex, clathrate, or prodrug thereof, or a pharmaceutical composition of claim 10.

15. The method of treatment according to claim 14, wherein the disease is a tumor, such as a solid tumor, a hematological tumor, a malignancy, a refractory tumor, a primary or metastatic recurrent tumor, and the like.

16. The method of treatment of claim 14, wherein the disease is selected from primary or metastatic relapsed NSCLC, squamous carcinoma of the lung, adenocarcinoma of the lung, squamous carcinoma of the head and neck, gastric cancer, colorectal cancer, pancreatic cancer, and the like; also included are breast cancer, esophageal cancer, lung cancer, colon cancer, brain cancer, neuroblastoma, neuroblastoma, melanoma, squamous carcinoma of the head and neck, anaplastic large cell lymphoma and glioblastoma, hematological malignancies of cachexia including juvenile myelomonocytic leukemiass, acute myelogenous leukemia, noonan syndrome, leopard syndrome, type II diabetes, and obesity.
